# EUROPEAN PATENT SPECIFICATION

(11) **EP 1 012 248 B1**
(45) Date of publication and mention of the grant of the patent: **02.07.2008**
(21) Application number: 98944849.3
(22) Date of filing: 11.09.1998
(51) Int. Cl.: C12N 9/12, C12N 15/54, C12Q 1/68, G01N 33/53

(54) **NOVEL THERMOPHILIC POLYMERASE III HOLOENZYME**
NEUES THERMOPHILES POLYMERASE III HOLOENZYM
NOUVEL HOLOENZYME POLYMERASE III THERMOPHILE

(30) Priority: 12.09.1997 US 928213; 11.09.1998 US 151888
(43) Date of publication of application: 28.06.2000
(73) Proprietor: Enzyco, Inc., Denver, CO 80206 (US)
(72) Inventor: MCHENRY, Charles, S., Denver, CO 80206 (US); SEVILLE, Mark, Denver, CO 80206 (US); CULL, Millard, G., Denver, CO 80207 (US); CHEN, Joe, Yu, Westminster, CO 80021 (US); KERY, Vladimir, Denver, CO 80220 (US)
(74) Representative: Glawe, Delfs, Moll
(86) International application number: PCT/US1998/018946
(87) International publication number: WO 1999/013060

(56) References cited:
- WO-A-96/10640
- WO-A-98/45452
- MCHENRY C S ET AL: "A DNA polymerase III holoenzyme-like subassembly from an extreme thermophilic eubacterium" JOURNAL OF MOLECULAR BIOLOGY, LONDON, GB, vol. 272, no. 2, 19 September 1997 (1997-09-19), pages 178-189, XP002075007 ISSN: 0022-2836
- DALLMANN H GARRY ET AL: "DnaX complex of Escherichia coli DNA polymerase III holoenzyme: Central role of tau in initiation complex assembly and in determining the functional asymmetry of holoenzyme" JOURNAL OF BIOLOGICAL CHEMISTRY, vol. 270, no. 49, 8 December 1995 (1995-12-08), pages 29555-29562, XP002292630 ISSN: 0021-9258
- FLOWER et al., "The Adjacent dnaZ and dnaX Genes of Escherichia Coli are Contained within One Continuous Open Reading Frame", NUCLEIC ACIDS RESEARCH, 1986, Vol. 14, No. 20, pages 8091-8101, XP002913041

## Description

### FIELD OF THE INVENTION

The present invention relates to gene and amino acid sequences encoding DNA polymerase III holoenzyme subunits and structural genes from thermophilic organisms. In particular, the present invention provides DNA polymerase III holoenzyme subunits of *T. thermophilus.* The present invention also provides antibodies and other reagents useful to identify DNA polymerase III molecules.

### BACKGROUND

Bacterial cells contain three types of DNA polymerases termed polymerase I, II and III. DNA polymerase III (pol III) is responsible for the replication of the majority of the chromosome. Pol III is referred to as a replicative polymerase; replicative polymerases are rapid and highly processive enzymes. Pol I and II are referred to as non-replicative polymerases although both enzymes appear to have roles in replication. DNA polymerase I is the most abundant polymerase and is responsible for some types of DNA repair, including a repair-like reaction that permits the joining of Okazaki fragments during DNA replication. Pol I is essential for the repair of DNA damage induced by UV irradiation and radiomimetic drugs. Pol II is thought to play a role in repairing DNA damage which induces the SOS response and in mutants which lack both pol I and III, pol II repairs UV-induced lesions. Pol I and II are monomeric polymerases while pol III comprises a multisubunit complex.

In *E. coli,* pol III comprises the catalytic core of the *E. coli* replicase. In *E. coli,* there are approximately 400 copies of DNA polymerase I per cell, but only 10-20 copies of pol III (Kornberg and Baker, DNA Replication, 2d ed., W.H. Freeman & Company, [1992], pp. 167; and Wu et al. J. Biol. Chem., 259:12117-12122 [1984]). The low abundance of pol III and its relatively feeble activity on gapped DNA templates typically used as a general replication assays delayed its discovery until the availability of mutants defective in DNA polymerase I (Kornberg and Gefter, J. Biol. Chem., 47:5369-5375 [1972]).

The catalytic subunit of pol III is distinguished as a component of *E. coli* major replicative complex, apparently not by its intrinsic catalytic activity, but by its ability to interact with other replication proteins at the fork. These interactions confer upon the enzyme enormous processivity. Once the DNA polymerase III holoenzyme associates with primed DNA, it does not dissociate for over 40 minutes-the time required for the synthesis of the entire 4 Mb *E. coli* chromosome (McHenry, Ann. Rev. Biochem., 57:519-550 [1988]). Studies in coupled rolling circle models of the replication fork suggest the enzyme can synthesize DNA 150 kb or longer without dissociation *in vitro* (Mok and Marians, J. Biol. Chem., 262:16644-16654 [1987]; Wu et al., J. Biol. Chem., 267:4030-4044 [1992]). The essential interaction required for this high processivity is an interaction between the α catalytic subunit and a dimer of β, a sliding clamp processivity factor that encircles the DNA template like a bracelet, permitting it to rapidly slide along with the associated polymerase, but preventing it from falling off (LaDuca et al., J. Biol. Chem., 261:7550-7557 [1986]; Kong et al., Cell 69:425-437 [1992]). The β-α association apparently retains the polymerase on the template during transient thermal fluctuations when it might otherwise dissociate.

The β₂ bracelet cannot spontaneously associate with high molecular weight DNA, it requires a multiprotein DnaX-complex to open and close it around DNA using the energy of ATP hydrolysis (Wickner, Proc. Natl. Acad. Sci. USA 73:35411-3515 [1976]; Naktinis et al., J. Biol. Chem., 270:13358-13365 [1985]; and Dallmann et al., J. Biol. Chem., 270:29555-29562 [1995]). In E. *coli,* the *dna*X gene encodes two proteins, τ and γ. γ is generated by a programmed ribosomal frameshifting mechanism five-sevenths of the way through *dna*X mRNA, placing the ribosome in a -1 reading frame where it immediately encounters a stop codon (Flower and McHenry Proc. Natl. Acad. Sci. USA 87:3713-3717 [1990]; Blinkowa and Walker, Nucl. Acids Res., 18:1725-1729 [1990]; and Tsuchihashi and Kornberg, Proc. Natl. Acad. Sci. USA 87:2516-2520 [1990]). In E. *coli,* the DnaX-complex has the stoichiometry γ₂τ₂δ₁δ'₁χ₁τ₁ (Dallmann and McHenry, J. Biol. Chem., 270:29563-29569 [1995]). The τ protein contains an additional carboxyl-terminal domain that interacts tightly with the polymerase, holding two polymerases together in one complex that can coordinately replicate the leading and lagging strand of the replication fork simultaneously (McHenry, J. Biol. Chem., 257:2657-2663 [1982]; Studwell and O'Donnell, Biol. Chem., 266:19833-19841 [1991]; McHenry, Ann. Rev. Biochem. 57:519-550 [1988]).

Pol IIIs are apparently conserved throughout mesophilic eubacteria. In addition to *E. coli* and related proteobacteria, the enzyme has been purified from the firmicute *Bacillus subtilis* (Low et al., J. Biol. Chem., 251:1311-1325 [1976]; Hammond and Brown [1992]). With the proliferation of bacterial genomes sequenced, by inference from DNA sequence, pol III exits in organisms as widely divergent as *Caulobacter, Mycobacteria, Mycoplasma, B. subtilis* and *Synechocystis.* The existence of *dna*X and *dna*N (structural gene for β) is also apparent in these organisms. These general replication mechanisms are conserved even more broadly in biology. Although eukaryotes do not contain polymerases homologous to pol III, eukaryotes contain special polymerases devoted to chromosomal replication and β-like processivity factors (PCNA) and DnaX-like ATPases (RFC, Activator I) that assemble these processivity factors on DNA (Yoder and Burgers, J. Biol. Chem., 266:22689-22697 [1991]; Brush and Stillman, Meth. Enzymol., 262:522-548 [1995]; Uhlmann et al., Proc. Natl. Acad. Sci. USA 93:6521-6526 [1996]).

In spite of the apparent ubiquity of pol IIIs and their associated factors required to function as a replicase, the identification of such enzymes remains to be accomplished for many other organisms.

### SUMMARY OF THE INVENTION

The present invention relates to gene and amino acid sequences encoding DNA polymerase III holoenzyme subunits and structural genes from thermophilic organisms. In particular, the present invention provides DNA polymerase III holoenzyme subunits of *T. thermophilus.* The present invention also provides antibodies and other reagents useful to identify DNA polymerase III molecules.

In particular, the present invention A DNA polymerase III holoenzyme isolated from a thermophilic organism. In particularly preferred embodiments, the thermophilic organism is a thermophilic eubacteria. In other preferred embodiments, the thermophilic organism is selected from a member of the genera *Thermus, Thermotoga,* and *Aquiflex.*

The present invention provides nucleotide sequences, including the nucleotide sequence set forth in SEQ ID NO:7, as well as sequences comprising fragments of SEQ ID NO:7, and sequences that are complementary to SEQ ID NO:7. In alternative embodiments, the present invention provides the nucleotide sequence of SEQ ID NO:7, wherein the nucleotide sequence further comprises 5' and 3' flanking sequences, and/or intervening regions.

The present invention also provides recombinant DNA vectors, such as vectors comprising SEQ ID NO:7. In an alternative embodiment, the present invention provides host cells containing these recombinant vectors.

The present invention also provides a purified dnaX protein encoded by an oligonucleotide comprising a nucleotide sequence substantially homologous to the coding strand of the nucleotide sequence of SEQ ID NO:7. The present invention provides full-length, as well as fragments of any size comprising the protein (*i.e.*, the entire amino acid sequence of the protein, as well as short peptides). In particularly preferred embodiments, the dnaX protein is from *Thermus thermophilus.* In other preferred embodiments, the dnaX proteins comprises at least a portion of the amino acid sequence set forth in SEQ ID NO:9.

The present invention also provides a fusion protein(s) comprising a portion of the dnaX protein and a non-dnaX protein sequence. In some preferred embodiments, the dnaX protein comprises SEQ ID NO:9.

The present invention also provides isolated amino acid sequences as set forth in SEQ ID NO:2. In yet other embodiments, the present invention provides an isolated nucleotide sequence encoding the amino acid sequence set forth in SEQ ID NO:2. In additional embodiments, the present invention provides a purified tau protein encoded by a polynucleotide sequence substantially homologous to the coding strand of the nucleotide sequences encoding tau protein. In alternative embodiments, the present invention provides nucleotide sequences encoding at least a portion of tau protein, wherein the nucleotide sequence further comprises 5' and 3' flanking regions and/or intervening regions. Thus, the present invention also encompasses fragments of any size, comprising tau protein amino acid or nucleic acid sequences. In preferred embodiments, the tau protein of the present invention is from *Thermus thermophilus.*

In an alternative embodiment, the present invention provides recombinant vectors comprising at least a portion of the nucleotide sequence encoding tau protein. In yet other embodiments, the present invention provides host cells containing at least one recombinant DNA vector comprising at least a portion of tau protein. In further embodiments, the present invention provides fusion protein(s) at least a portion of tau protein and a non-tau protein sequence.

The present invention also provides the amino acid sequence set forth in SEQ ID NO:1. In one embodiment, the present invention provides an isolated nucleotide sequence encoding the amino acid sequence set forth in SEQ ID NO:1. In other embodiments, the present invention provides at least a portion of purified gamma protein(s) encoded by a polynucleotide sequence substantially homologous to the coding strand of the nucleotide sequence that encodes gamma protein. In yet other embodiments, the present invention provides nucleotide sequences that further comprise 5' and 3' flanking regions and/or intervening regions. In preferred embodiments, the present invention provides gamma protein that is from *Thermus thermophilus.*

The present invention also provides recombinant vectors comprising at least a portion of a nucleotide sequence that encodes gamma protein. In yet other embodiments, the present invention also provides host cells containing the recombinant DNA vectors comprising at least a portion of nucleotide sequence encoding gamma protein. In alternative embodiments, the present invention provides fusion protein(s) comprising a portion of the gamma protein, and non-gamma protein sequence(s).

The present invention further provides the isolated nucleotide sequence set forth in SEQ ID NO:196. In some embodiments, the nucleotide sequence further comprises 5' and 3' flanking regions and/or intervening regions. In yet other embodiments, the present invention provides at least a portion of purified DnaE protein encoded by an oligonucleotide comprising at least a portion of nucleotide sequence substantially homologous to the coding strand of the nucleotide sequence SEQ ID NO:196. In particularly preferred embodiments, the DnaE protein is from *Thermus thermophilus.* In still further embodiments, the DnaE protein comprises the amino acid sequence set forth in SEQ ID NO:197.

The present invention also provides recombinant vectors comprising at least a portion of the nucleotide sequence that encodes the DnaE protein. In alternative embodiments, the recombinant vectors are present within a host cell. In still other embodiments, the present invention provides fusion protein comprising at least a portion of the DnaE protein and a non-DnaE protein sequence. In preferred embodiments of the fusion proteins, the DnaE protein comprises SEQ ID NO:197.

The present invention also provides an isolated nucleotide sequence as set forth in SEQ ID NO:214. In some embodiments, the nucleotide sequence further comprises 5' and 3' flanking and/or intervening regions. In yet other embodiments, the present invention provides at least a portion of purified DnaQ protein encoded by an oligonucleotide comprising at least a portion of nucleotide sequence substantially homologous to the coding strand of the nucleotide sequence SEQ ID NO:214. In particularly preferred embodiments, the DnaQ protein is from *Thermus thermophilus.* In still further embodiments, the DnaQ protein comprises the amino acid sequence set forth in SEQ ID NO:215 and/or SEQ ID NO:216.

The present invention also provides recombinant vectors comprising at least a portion of the nucleotide sequence that encodes the DnaQ protein. In alternative embodiments, the recombinant vectors are present within a host cell. In still other embodiments, the present invention provides fusion protein comprising at least a portion of the DnaQ protein and a non-DnaQ protein sequence. In preferred embodiments of the fusion proteins, the DnaQ protein comprises SEQ ID NO:215 and/or SEQ ID NO:216.

The present invention also provides the isolated nucleotide sequence set forth in SEQ ID NO:221. In some embodiments, the nucleotide sequence further comprises 5' and 3' flanking and/or intervening regions. In yet other embodiments, the present invention provides at least a portion of purified DnaA protein encoded by an oligonucleotide comprising at least a portion of nucleotide sequence substantially homologous to the coding strand of the nucleotide sequence SEQ ID NO:221. In particularly preferred embodiments, the DnaA protein is from *Thermus thermophilus.* In still further embodiments, the DnaA protein comprises the amino acid sequence set forth in SEQ ID NO:222.

The present invention also provides recombinant vectors comprising at least a portion of the nucleotide sequence that encodes the DnaA protein. In alternative embodiments, the recombinant vectors are present within a host cell. In still other embodiments, the present invention provides fusion protein comprising at least a portion of the DnaQ protein and a non-DnaQ protein sequence. In preferred embodiments of the fusion proteins, the DnaQ protein comprises SEQ ID NO:222.

The present invention also provides the isolated nucleotide sequence set forth in SEQ ID NO:230. In some embodiments, the nucleotide sequence further comprises 5' and 3' flanking and/or intervening regions. In yet other embodiments, the present invention provides at least a portion of purified DnaN protein encoded by an oligonucleotide comprising at least a portion of nucleotide sequence substantially homologous to the coding strand of the nucleotide sequence SEQ ID NO:230. In particularly preferred embodiments, the DnaN protein is from *Thermus thermophilus.* In still further embodiments, the DnaN protein comprises the amino acid sequence set forth in SEQ ID NO:231.

The present invention also provides recombinant vectors comprising at least a portion of the nucleotide sequence that encodes the DnaN protein. In alternative embodiments, the recombinant vectors are present within a host cell. In still other embodiments, the present invention provides fusion protein comprising at least a portion of the DnaN protein and a non-DnaN protein sequence. In preferred embodiments of the fusion proteins, the DnaN protein comprises SEQ ID NO:231.

The present invention also provides methods for detecting DNA polymerase III comprising: providing in any order, a sample suspected of containing DNA polymerase III, an antibody capable of specifically binding to a at least a portion of the DNA polymerase III; mixing the sample and the antibody under conditions wherein the antibody can bind to the DNA polymerase III; and detecting the binding. In preferred embodiments of the methods, the sample comprises a thermophilic organism. In alternative preferred embodiments, the thermophilic organism is member of the genus *Thermus.* The methods of the present invention encompass any method for detection.

The present invention also provides methods for detection of polynucleotides encoding at least a portion of DNA polymerase III holoenzyme (or DNA polymerase III holoenzyme subunit) in a biological sample comprising the steps of: a) hybridizing at least a portion of the polynucleotide sequence comprising at least fifteen nucleotides, which hybridizes under stringent conditions to at least a portion of the polynucleotide sequence selected from the group consisting of the DNA sequences set forth in SEQ ID NOS:7, 20, 21, 22, 40, 41, 42, 58, 63, 64, 65, 66, 67, 77, 78, 79, 88, 91, 92, 97, 110, 111, 112, 113, 114, 115, 116, 134, 135, 136, 137, 156, 157, 173, 174, 174, 176, 177, 178, 179, 180, 190, 196, 214, 221, and 230, to nucleic acid material of a biological sample, thereby forming a hybridization complex; and b) detecting the hybridization complex, wherein the presence of the complex correlates with the presence of a polynucleotide encoding at least a portion of DNA polymerase III holoenzyme (or DNA polymerase III holoenzyme subunit) in the biological sample. In one alternative embodiment of the methods, the nucleic acid material of the biological sample is amplified by the polymerase chain reaction.

The present invention also provides an antibody, wherein the antibody is capable of specifically binding to at least one antigenic determinant on the protein encoded by an amino acid sequence selected from the group comprising SEQ ID NOS:1, 2, 8, 9, 26, 31, 34, 37, 59, 70, 75, 82, 85, 197, 198, 215, 216, 222, 223, 225, 226, and 231. The present invention encompasses polyclonal, as well as monoclonal antibodies.

The present invention also provides methods for producing anti-DNA polymerase III holoenzyme and anti-DNA polymerase III holoenzyme subunit antibodies comprising, exposing an animal having immunocompetent cells to an immunogen comprising at least an antigenic portion of DNA polymerase III holoenzyme (or holoenzyme subunit) protein, under conditions such that immunocompetent cells produce antibodies directed against the portion of DNA polymerase III protein holoenzyme or holoenzyme subunit. In one embodiment, the method further comprises the step of harvesting the antibodies. In an alternative embodiment, the method comprises the step of fusing the immunocompetent cells with an immortal cell line under conditions such that an hybridoma is produced. In yet another embodiment, the portion of DNA polymerase III protein or subunit protein used as an immunogen to generate the antibodies is selected from the group consisting of SEQ ID NOS:1, 2, 8, 9, 16, 17, 18, 19, 23, 26, 31, 34, 37, 45, 50, 55, 59, 601, 70, 75, 82, 88, 89, 90, 105, 106, 107, 109, 117, 181, 184, 187, 197, 198, 215, 216, 222, and 231. In other embodiments, the immunogen comprises a fusion protein. In yet another embodiment, the fusion protein comprises at least a portion of DNA polymerase III holoenzyme or holoenzyme subunit protein.

The present invention also provides methods for detecting DNA polymerase III holoenzyme or holoenzyme subunit expression comprising the steps of: a) providing a sample suspected of containing DNA polymerase III holoenzyme or holoenzyme III subunit; and a control containing a quantitated DNA polymerase III holoenzyme or holoenzyme III subunit protein, as appropriate; and b) comparing the test DNA polymerase III holoenzyme or holoenzyme subunit, in the sample with the quantitated DNA polymerase III holoenzyme or holoenzyme subunit in the control to determine the relative concentration of the test DNA polymerase III holoenzyme or holoenzyme III subunit in the sample. In addition, the methods may be conducted using any suitable means to determine the relative concentration of DNA polymerase III holoenzyme or holoenzyme subunit in the test and control samples, including but not limited to the means selected from the group consisting of Western blot analysis, Northern blot analysis, Southern blot analysis, denaturing polyacrylamide gel electrophoresis, reverse transcriptase-coupled polymerase chain reaction, enzyme-linked immunosorbent assay, radioimmunoassay, and fluorescent immunoassay. Thus, the methods may be conducted to determine the presence of DNA polymerase III holoenzyme or holoenzyme III subunit in the genome of the source of the test sample, or the expression of DNA polymerase III holoenzyme or holoenzyme subunit (mRNA or protein), as well as detect the presence of abnormal or mutated DNA polymerase holoenzyme or holoenzyme subunit proteins or gene sequences in the test samples.

In one preferred embodiment, the presence of DNA polymerase III holoenzyme or holoenzyme subunit is detected by immunochemical analysis. For example, the immunochemical analysis can comprise detecting binding of an antibody specific for an epitope of DNA polymerase III holoenzyme or holoenzyme subunit (*e.g.*, SEQ ID NO:2 or 3). In an another preferred embodiment of the method, the antibody comprises polyclonal antibodies, while in another preferred embodiment, the antibody is comprises monoclonal antibodies.

The antibodies used in the methods invention may be prepared using various immunogens. In one embodiment, the immunogen is DNA polymerase III holoenzyme or holoenzyme subunit peptide, to generate antibodies that recognize DNA polymerase III holoenzyme or holoenzyme subunit(s). Such antibodies include, but are not limited to polyclonal, monoclonal, chimeric, single chain, Fab fragments, and an Fab expression library.

Various procedures known in the art may be used for the production of polyclonal antibodies to DNA polymerase III holoenzyme or holoenzyme subunit. For the production of antibody, various host animals can be immunized by injection with the peptide corresponding to the DNA polymerase III holoenzyme or holoenzyme subunit epitope including but not limited to rabbits, mice, rats, sheep, goats, etc. In a preferred embodiment, the peptide is conjugated to an immunogenic carrier (*e.g.,* diphtheria toxoid, bovine serum albumin (BSA), or keyhole limpet hemocyanin [KLH]). Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanins, and dinitrophenol.

For preparation of monoclonal antibodies directed toward DNA polymerase III holoenzyme or holoenzyme subunit, any technique that provides for the production of antibody molecules by continuous cell lines in culture may be used (*See, e.g.,* Harlow and Lane, Antibodies: A Laboratory Manual, Cold Spring Harbor Laboratory Press, Cold Spring Harbor, NY). These include but are not limited to the hybridoma technique originally developed by Köhler and Milstein (Köhler and Milstein, Nature 256:495-497 [1975]), as well as other techniques known in the art.

According to the invention, techniques described for the production of single chain antibodies (U.S. Patent 4,946,778) can be adapted to produce DNA polymerase III holoenzyme or holoenzyme subunit-specific single chain antibodies. An additional embodiment of the invention utilizes the techniques described for the construction of Fab expression libraries (Huse et al., Science 246:1275-1281 [1989]) to allow rapid and easy identification of monoclonal Fab fragments with the desired specificity for DNA polymerase III holoenzyme or holoenzyme subunit.

Antibody fragments which contain the idiotype (antigen binding region) of the antibody molecule can be generated by known techniques. For example, such fragments include but are not limited to: the F(ab')2 fragment which can be produced by pepsin digestion of the antibody molecule; the Fab' fragments which can be generated by reducing the disulfide bridges of the F(ab')2 fragment, and the Fab fragments which can be generated by treating the antibody molecule with papain and a reducing agent.

In the production of antibodies, screening for the desired antibody can be accomplished by techniques known in the art (*e.g.*, radioimmunoassay, ELISA [enzyme-linked immunosorbent assay], "sandwich" immunoassays, immunoradiometric assays, gel diffusion precipitin reactions, immunodiffusion assays, *in situ* immunoassays [using colloidal gold, enzyme or radioisotope labels, for example], Western Blots, precipitation reactions, agglutination assays (*e.g.*, gel agglutination assays, hemagglutination assays, etc.), complement fixation assays, immunofluorescence assays, protein A assays, and immunoelectrophoresis assays, etc.

In one embodiment, antibody binding is detected by detecting a label on the primary antibody. In another embodiment, the primary antibody is detected by detecting binding of a secondary antibody or reagent to the primary antibody. In a further embodiment, the secondary antibody is labeled. Many means are known in the art for detecting binding in an immunoassay and are within the scope of the present invention. (As is well known in the art, the immunogenic peptide should be provided free of the carrier molecule used in any immunization protocol. For example, if the peptide was conjugated to KLH, it may be conjugated to BSA, or used directly, in a screening assay.)

The foregoing antibodies can be used in methods known in the art relating to the localization and structure of DNA polymerase III holoenzyme or holoenzyme subunit (*e.g.*, for Western blotting), measuring levels thereof in appropriate biological samples, etc. The biological samples can be tested directly for the presence of DNA polymerase III holoenzyme or holoenzyme subunit using an appropriate strategy (*e.g.,* ELISA or radioimmunoassay) and format (*e.g.,* microwells, dipstick [*e.g.,* as described in International Patent Publication WO 93/03367], etc.). Alternatively, proteins in the sample can be size separated (*e.g.*, by polyacrylamide gel electrophoresis (PAGE), in the presence or not of sodium dodecyl sulfate (SDS), and the presence of DNA polymerase III holoenzyme or holoenzyme subunit detected by immunoblotting (Western blotting). Immunoblotting techniques are generally more effective with antibodies generated against a peptide corresponding to an epitope of a protein, and hence, are particularly suited to the present invention.

The present invention also provides methods for identification of polymerase subunits comprising the steps of: a) providing a test sample suspected of containing amplifiable nucleic acid encoding at least a portion of a DNA polymerase III subunit protein; b) isolating the amplifiable nucleic acid from the test sample; c) combining the amplifiable nucleic acid with amplification reagents, and at least two primers selected from the group consisting of primers having the nucleic acid sequence set forth in SEQ ID NOS:21, 22, 86, 87, 91, 97, 110, 111, 112, 113, 114, 115, 116, 134, 135, 136, 137, 156, 157, 173, 174, 176, 177, 208, 209, 210, 211, 212, and 213, to form a reaction mixture; and d) combining the reaction mixture with an amplification enzyme under conditions wherein the amplifiable nucleic acid is amplified to form amplification product.

In some embodiments, the methods further comprise the step of detecting the amplification product. In some preferred embodiments, the detecting is accomplished by hybridization of the amplification product with a probe. In yet other embodiments, the primers are capable of hybridizing to nucleic acid encoding at least one polymerase subunit selected from the group consisting of DnaA, DnaN, DnaE, DnaX, DnaQ, and DnaB. In still other embodiments, the test sample comprises nucleic acid obtained from a thermophilic organism. In particularly preferred embodiments, the thermophilic organism is a member of the genus *Thermus.*

The present invention also provides amplification methods comprising the steps of: a) providing a test sample suspected of containing amplifiable nucleic acid, amplification reagents, DNA polymerase, an adjunct component comprising at least one subunit of DnaX, DnaE, DnaQ, DnaN, and DnaA, and at least two primers; isolating the amplifiable nucleic acid from the test sample; c) combining the amplifiable nucleic acid with the amplification reagents, the adjunct component, the DNA polymerase, and the primers under conditions such that amplifiable nucleic acid is amplified to form amplification product. In some preferred embodiments, the method further comprises the step of detecting the amplification product. In yet other preferred embodiments, the detecting is accomplished by hybridization of the amplification product with a probe. In still other embodiments, the primers are capable of hybridizing to nucleic acid encoding at least one polymerase subunit selected from the group consisting of DnaA, DnaN, DnaE, DnaX, DnaQ, and DnaB. In yet other embodiments, the DNA polymerase is selected from the group consisting of *Taq* polymerase, *E. coli* DNA polymerase I, Klenow, *Pfu* polymerase, *Tth* polymerase, *Tru* polymerase, *Tfl* polymerase, *Thermococcus* DNA polymerase, and *Thermotoga* DNA polymerase. In yet other embodiments, the amplification reagent comprises components selected from the group consisting of single-stranded binding proteins, helicases, and accessory factors.

The foregoing explanations of particular assay systems are presented herein for purposes of illustration only, in fulfillment of the duty to present an enabling disclosure of the invention. It is to be understood that the present invention contemplates a variety of immunochemical, amplification, and detection assay protocols within its spirit and scope.

### DESCRIPTION OF THE FIGURES

In all of the following Figures that show alignments (DNA or amino acids), the "+" indicates similar, but not identical residues. In the DNA sequences with underlined regions, unless otherwise indicated, the underlining indicates bases generated by the degenerate primers used to generate the DNA of interest. Also unless otherwise indicated, the sequences between the sequences generated by the primers were used in the searches to generate deduced amino acid sequences (*i.e.*, the primer-generated sequences were excluded from the searches).
Figure 1 shows a graph depicting the inactivation of the M13Gori assay mixture at elevated temperatures.
Figure 2 shows the elution profile of Fraction II applied to a cation-exchange BioRex-70 column. Fraction II prepared from (A) 2.4 kg cells, and (B) 300 g of cells was applied at fraction I in both A and B.
Figure 3 shows the elution profile of Fraction III applied to a hydrophobic ToyoPearl-Ether 65-M column to separate the majority of *T. thermophilus* DNA polymerase form pol III.
Figure 4 shows a Western blot of ToyoPearl-Ether fractions 41-52 developed monoclonal antibodies specific for *E. coli* DNA polymerase III α subunit.
Figure 5 shows the elution profile of Fraction IV applied to an anion exchange Q-Sepharose Fast Flow column. Fraction IV was applied at fraction 1.
Figure 6 shows a Coomassie blue-stained SDS-polyacrylamide gel containing Q-Sepharose fractions 28-40.
Figure 7 shows the DNA polymerase gap-filling activity of *T. thermophilus* Fraction V at different temperatures.
Figure 8 shows the amino terminal sequence of the isolated candidate γ (SEQ ID NO:1) and τ (SEQ ID NO:2) *T. thermophilus dnaX* gene products and comparison to the homologous sequences of *E. coli* (SEQ ID NO:3) and *B. subtilis* (SEQ ID NO:4). *T. thermophilus* is abbreviated as *Tth.* The sequences where identical or similar matches occur are also shown (SEQ ID NOS:5 and 6).
Figure 9A shows the DNA sequence (SEQ ID NO:7) of *T. thermophilus dna*X and flanking sequences.
Figure 9C shows a comparison of *T. thermophilus dna*X (SEQ ID NO:9); with homologous eubacterial *dna*X sequences from *E. coli* (SEQ ID NO:10), *B. subtilis* (SEQ ID NO: 11), *Mycoplasma pneumoniae* (SEQ ID NO: 12), *Caulobacter crescentus* (SEQ ID NO: 13), and *Synechocystis sp.* (SEQ ID NO: 14)(a consensus sequence is also shown [SEQ ID NO: 15]).
Figure 9D shows the deduced sequence of the carboxyl-terminus of *T. thermophilus* γ subunit depending on whether the frameshift is -1 (as in *E. coli*) or +1 (SEQ ID NOS:16-19).
Figure 10A shows the nucleotide sequence (SEQ ID NO:20) of *T. thermophilus dna*X product obtained from PCR amplification of *T. thermophilus* chromosomal DNA with primers X1Fa (SEQ ID NO:21) and X139R (SEQ ID NO:22); the sequences generated by the primers are underlined in this Figure (SEQ ID NOS:190 and 85).
Figure 10B shows an alignment of the amino acid sequence (SEQ ID NO:23) deduced from the underlined primers of 10A (*i.e., T. thermophilus* DnaX product), with *B. subtilis* DNA polymerase dnaX sequence (SEQ ID NO:24). As with prior Figures, matching amino acids are indicated (SEQ ID NO:25).
Figure 11A shows an alignment of *T. thermophilus* 130 kDa polypeptide N-terminal amino acid sequence (dnaE)(SEQ ID NO:26) with sequences from *M. tuberculosis* (SEQ ID NO:27) and *H. influenzae* (SEQ ID NO:28) dnaE gene sequences. The sequences that are identical between the corresponding peptides are also shown (SEQ ID NOS:29 and 30).

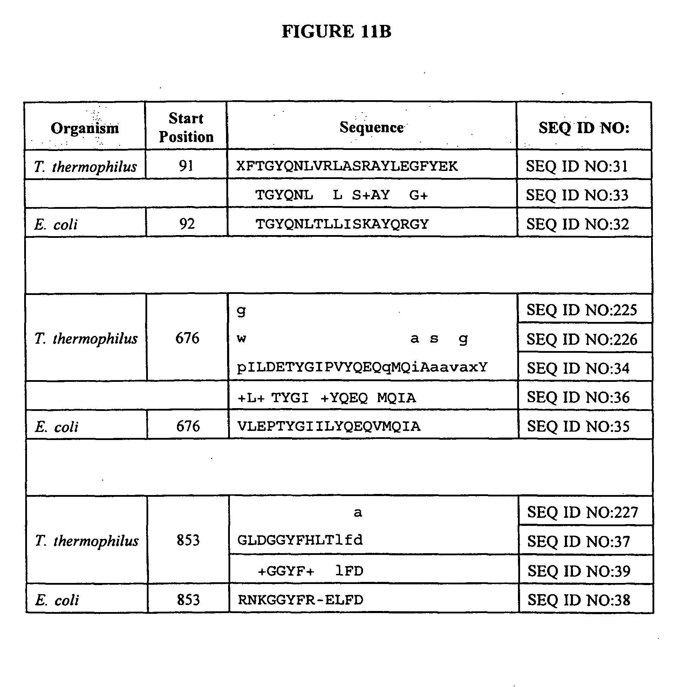

Figure 11B shows comparisons between the internal amino acid sequences of three *T. thermophilus* dnaE regions compared with sequences from *E. coli.* In this Figure, the three *T. thermophilus* peptides were #91 (SEQ ID NO:31), #676 (SEQ ID NO:34), and #853 (SEQ ID NO:37). For *E. coli,* the peptide number refers to the first amino acid residue in the amino acid sequence of the *E. coli* DNA pol III α subunit with which the *T. thermophilus* 130 kDa polypeptide aligned (*i.e.*, 92 [SEQ ID NO:32]; 676 [SEQ ID NO:35]; and 38 [SEQ ID NO:38]). As with other Figures, identical or similar amino acids are indicated between the *T. thermophilus* and *E. coli* sequences (SEQ ID NOS:33, 36, and 39).
Figure 12A shows the nucleotide sequence (SEQ ID NO:40) of *T. thermophilus dna*E product obtained from PCR amplification of *T. thermophilus* chromosomal DNA with primers E1F (SEQ ID NO:87) and E91R (SEQ ID NO:86). The sequences underlined in this Figure are the sequences are the regions recognized by these primers (SEQ ID NOS:41 and 42).
Figure 12B shows alignments of the *T. thermophilus* dnaE amino acid sequence (SEQ ID NOS:45, 50, and 55) deduced from Figure 12A (between the underlined primers), compared with *Synechocystis sp.* sequences (SEQ ID NO:43, 48, and 53), and *M. tuberculosis* sequences (SEQ ID NO:47, 52, and 57). As with other Figures, identical or similar amino acids are also shown (SEQ ID NOS:44, 46, 49, 51, 54, and 56).
Figure 13A shows the nucleotide sequence (SEQ ID NO:58) of the first approximately 1,109 bases of the *T. thermophilus dna*E gene.
Figure 13B shows the preliminary deduced amino acid sequence (SEQ ID NO:59) corresponding to the N-terminal portion of the *dna*E gene shown in Figure 13A.
Figure 13C shows an alignment of the *T. thermophilus dna*E gene (SEQ ID NO:61) with regions of homology in the *M. tuberculosis* DNA polymerase III α subunit sequence (SEQ ID NO:60) and *Synechocystis sp.* DNA polymerase III α subunit sequence (SEQ ID NO:62).
Figure 14A shows the region of asymmetric PCR product corresponding to the region close to the N-terminal end of the *T. thermophilus dna*E gene (SEQ ID NO:63)(*i.e*., the "front end of the clone"). In this Figure, the region corresponding to the sequence generated by the forward primer is underlined and shown in bold (SEQ ID NO:64).
Figure 14B shows the back end of the clone (SEQ ID NO:180). In this Figure, the underlined bases correspond to part of the *Pst*I site.
Figure 14C shows the alignment of the amino acid sequence of *T. thermophilus* DnaE product deduced from the entire sequence shown in Figure 14B (SEQ ID NOS:181, 184, and 187), with *E. coli* DNA polymerase III sequences (SEQ ID NOS:183, 186, and 189). As with prior Figures, identical and similar amino acids are indicated (SEQ ID NOS:182, 185, and 188).
Figure 15A shows the deduced nucleotide sequence (SEQ ID NO:65) of *T. thermophilus* dnaA product obtained from PCR amplification of *T. thermophilus* chromosomal DNA between the primers A177Fb (SEQ ID NO:135) and A251Rb (SEQ ID NO:157). The sequences recognized by the primers are underlined in this Figure (SEQ ID NOS:66 and 67).
Figure 15B shows an alignment of the deduced amino acid sequences (SEQ ID NOS:70, 75) of *T. thermophilus* dnaA product, with *E. coli* dnaA sequence (SEQ ID NOS:68, and 73), and *B. subtilis dna*A sequence (SEQ ID NOS:72 and 76). As with other Figures, identical and similar residues are also shown (SEQ ID NOS:69, 71, and 74).
Figure 16A shows the nucleotide sequence (SEQ ID NO:60) of *T. thermophilus* dnaQ product obtained from PCR amplification of *T. thermophilus* chromosomal DNA with primers Q12Fa (SEQ ID NO:173) and Q98Ra (SEQ ID NO:176). The sequences generated by these primers are underlined in this Figure (SEQ ID NOS:78 and 79).
Figure 16B shows an alignment of the deduced amino acid sequences (SEQ ID NO:82) of *T. thermophilus* dnaQ product, with *E. coli* DNA polymerase III holoenzyme ε subunit sequence (SEQ ID NO:80), and *B. subtilis* DNA polymerase III subunit sequence (SEQ ID NO:84). As with other Figures, identical and similar residues are also shown (SEQ ID NOS:81 and 83).
Figure 17A shows the sequence of full-length *T. thermophilus dnaE* gene (SEQ ID NO:196). In this Figure, the *dnaE* reading frame is shown in bold and underlined.
Figure 17B shows the deduced amino acid sequence of the full-length *T. thermophilus* DnaE protein (SEQ ID NO:197). In this Figure, sequence corresponding to the peptides sequenced from isolated native *T. thermophilus* DnaE are shown in bold.
Figure 17C shows the alignment of regions from the *T. thermophilus* dnaE protein (SEQ ID NO:198), with homologous regions of eubacterial *dnaE* genes from three representative sequences of *E. coli, B. subtilis* type 1, and *Borrelia burgdorferi* DnaE (SEQ ID NOS:199, 200, and 201, respectively).
Figure 17D shows the deduced amino acid sequence (SEQ ID NO:223) of *Tth* DnaE containing Biotin/Hexahis tag on the amino terminus.
Figure 18A shows a major portion of the *Tth dnaQ* gene encoding epsilon (SEQ ID NO:214).
Figure 18B shows the amino acid sequence (SEQ ID NO:215) of the continuous open reading frame encoded by SEQ ID NO:214.
Figure 18C shows the protein (SEQ ID NO:216) that would be expressed if the GTG at codon 36 of SEQ ID NO:214) is used as the initiating codon.
Figure 18D shows the alignment of regions from the *T. thermophlus dnaQ* gene (SEQ ID NO:217), with homologous regions of eubacterial *dnaQ* genes from three representative sequences of *Treponema pallidum, Aquiflex aeolicus,* and *E. coli* DnaE (SEQ ID NOS:218, 219, and 220, respectively).
Figure 19A shows the DNA sequence (SEQ ID NO:221) of the 3' terminal portion intergenic region of *dna*A that may be the *T. thermophilus* origin of replication.
Figure 19B shows the amino acid sequence (SEQ ID NO:222) of the 5' portion of the *T. thermophilus* DnaA open reading frame.
Figure 20A shows a partial DNA sequence (SEQ ID NO:230) of *dnaN .*
Figure 20B shows the deduced amino acid sequence (SEQ ID NO:231) of the amino-terminal portion of DnaN encoded by the DNA sequence (SEQ ID NO:230) shown in Figure 20A.
Figure 20C shows the alignment of regions of *T. thermophilus* DnaN sequence (SEQ ID NO:232) with homologous regions of DnaN from *E. coli* (SEQ ID NO:233) and *Streptococcus pneumoniae* (SEQ ID NO:234).

### DEFINITIONS

As used herein, the term "DNA polymerase III holoenzyme" refers to the entire DNA polymerase III entity (*i.e.,* all of the polymerase subunits, as well as the other associated accessory proteins required for processive replication of a chromosome or genome), while "DNA polymerase III" is just the core [α, ε, θ]). "DNA polymerase III holoenzyme subunit" is used in reference to any of the subunit entities that comprise the DNA polymerase III holoenzyme. Thus, the term "DNA polymerase III" encompasses "DNA polymerase III holoenzyme subunits" and "DNA polymerase III subunits."

The term "5' exonuclease activity" refers to the presence of an activity in a protein which is capable of removing nucleotides from the 5' end of an oligonucleotide. 5' exonuclease activity may be measured using any of the assays provided herein.

The term "3' exonuclease activity" refers to the presence of an activity in a protein which is capable of removing nucleotides from the 3' end of an oligonucleotide. 3' exonuclease activity may be measured using any of the assays provided herein.

The terms "DNA polymerase activity," "synthetic activity" and "polymerase activity" are used interchangeably and refer to the ability of an enzyme to synthesize new DNA strands by the incorporation of deoxynucleoside triphosphates. The examples below provide assays for the measurement of DNA polymerase activity. A protein which is can direct the synthesis of new DNA strands by the incorporation of deoxynucleoside triphosphates in a template-dependent manner is said to be "capable of DNA synthetic activity."

A "DNA synthesis terminating agent which terminates DNA synthesis at a specific nucleotide base" refers to compounds, including but not limited to, dideoxynucleosides having a 2', 3' dideoxy structure (*e.g.*, ddATP, ddCTP, ddGTP and ddTTP). Any compound capable of specifically terminating a DNA sequencing reaction at a specific base may be employed as a DNA synthesis terminating agent.

The term "gene" refers to a nucleic acid (*e.g.*, DNA) sequence that comprises coding sequences necessary for the production of a polypeptide or precursor (*e.g.*, DNA polymerase III holoenzyme or holoenzyme subunit, as appropriate). The polypeptide can be encoded by a full length coding sequence or by any portion of the coding sequence so long as the desired activity or functional properties (*e.g.*, enzymatic activity, ligand binding, signal transduction, etc.) of the full-length or fragment are retained. The term also encompasses the coding region of a structural gene and the including sequences located adjacent to the coding region on both the 5' and 3' ends for a distance of about 1 kb on either end such that the gene corresponds to the length of the full-length mRNA. The term "gene" encompasses both cDNA and genomic forms of a gene. A genomic form or clone of a gene contains the coding region interrupted with non-coding sequences termed "intervening regions" or "intervening sequences." The mRNA functions during translation to specify the sequence or order of amino acids in a nascent polypeptide.

In particular, the terms "DNA polymerase III holoenzyme" and "holoenzyme subunit gene" refer to the full-length DNA polymerase III holoenzyme, and holoenzyme subunit nucleotide sequence(s), respectively. However, it is also intended that the term encompass fragments of the DNA polymerase III holoenzyme and holoenzyme subunit sequences, such as those that encode particular domains of interest, including subunit proteins, as well as other domains within the full-length DNA polymerase III holoenzyme or holoenzyme subunit nucleotide sequence. Furthermore, the terms "DNA polymerase III holoenzyme," "holoenzyme subunit nucleotide sequence," "DNA polymerase III holoenzyme," and "holoenzyme subunit polynucleotide sequence" encompasses DNA, cDNA, and RNA (*e.g*., mRNA) sequences.

Where "amino acid sequence" is recited herein to refer to an amino acid sequence of a naturally occurring protein molecule, "amino acid sequence" and like terms, such as "polypeptide" or "protein" are not meant to limit the amino acid sequence to the complete, native amino acid sequence associated with the recited proteins.

Genomic forms of a gene may also include sequences located on both the 5' and 3' end of the sequences which are present on the RNA transcript. These sequences are referred to as "flanking" sequences or regions (these flanking sequences are located 5' or 3' to the non-translated sequences present on the mRNA transcript). The 5' flanking region may contain regulatory sequences such as promoters and enhancers which control or influence the transcription of the gene. The 3' flanking region may contain sequences which direct the termination of transcription, post-transcriptional cleavage and polyadenylation.

The term "wild-type" refers to a gene or gene product which has the characteristics of that gene or gene product when isolated from a naturally occurring source. A wild-type gene is that which is most frequently observed in a population and is thus arbitrarily designed the "normal" or "wild-type" form of the gene. In contrast, the term "modified" or "mutant" refers to a gene or gene product which displays modifications in sequence and or functional properties (*i.e.*, altered characteristics) when compared to the wild-type gene or gene product. It is noted that naturally-occurring mutants can be isolated; these are identified by the fact that they have altered characteristics when compared to the wild-type gene or gene product.

As used herein, the terms "nucleic acid molecule encoding," "DNA sequence encoding," and "DNA encoding" refer to the order or sequence of deoxyribonucleotides along a strand of deoxyribonucleic acid. The order of these deoxyribonucleotides determines the order of amino acids along the polypeptide (protein) chain. The DNA sequence thus codes for the amino acid sequence.

The term "oligonucleotide" as used herein is defined as a molecule comprised of two or more deoxyribonucleotides or ribonucleotides, preferably more than three, and usually more than ten. The exact size will depend on many factors, which in turn depends on the ultimate function or use of the oligonucleotide. The oligonucleotide may be generated in any manner, including chemical synthesis, DNA replication, reverse transcription, or a combination thereof.

Because mononucleotides are reacted to make oligonucleotides in a manner such that the 5' phosphate of one mononucleotide pentose ring is attached to the 3' oxygen of its neighbor in one direction via a phosphodiester linkage, an end of an oligonucleotide is referred to as the "5' end" if its 5' phosphate is not linked to the 3' oxygen of a mononucleotide pentose ring and as the "3' end" if its 3' oxygen is not linked to a 5' phosphate of a subsequent mononucleotide pentose ring. As used herein, a nucleic acid sequence, even if internal to a larger oligonucleotide, also may be said to have 5' and 3' ends.

When two different, non-overlapping oligonucleotides anneal to different regions of the same linear complementary nucleic acid sequence, and the 3' end of one oligonucleotide points towards the 5' end of the other, the former may be called the "upstream" oligonucleotide and the latter the "downstream" oligonucleotide. In either a linear or circular DNA molecule, discrete elements are referred to as being "upstream" or 5' of the "downstream" or 3' elements. This terminology reflects the fact that transcription proceeds in a 5' to 3' fashion along the DNA strand. The promoter and enhancer elements which direct transcription of a linked gene are generally located 5' or upstream of the coding region. However, enhancer elements can exert their effect even when located 3' of the promoter element and the coding region. Transcription termination and polyadenylation signals are located 3' or downstream of the coding region.

As used herein the term "coding region" when used in reference to structural gene refers to the nucleotide sequences which encode the amino acids found in the nascent polypeptide as a result of translation of a mRNA molecule. The coding region is bounded on the 5' side by the nucleotide triplet "ATG" which encodes the initiator methionine and on the 3' side by one of the three triplets which specify stop codons (*i.e.,* TAA, TAG, TGA).

As used herein, the terms "an oligonucleotide having a nucleotide sequence encoding a gene" and "polynucleotide having a nucleotide sequence encoding a gene," means a nucleic acid sequence comprising the coding region of a gene or in other words the nucleic acid sequence which encodes a gene product. The coding region may be present in either a cDNA, genomic DNA or RNA form. When present in a DNA form, the oligonucleotide or polynucleotide may be single-stranded (*i.e.*, the sense strand) or double-stranded. Suitable control elements such as enhancers/promoters, splice junctions, polyadenylation signals, *etc.* may be placed in close proximity to the coding region of the gene if needed to permit proper initiation of transcription and/or correct processing of the primary RNA transcript. Alternatively, the coding region utilized in the expression vectors of the present invention may contain endogenous enhancers/promoters, splice junctions, intervening sequences, polyadenylation signals, etc., or a combination of both endogenous and exogenous control elements.

As used herein, the term "regulatory element" refers to a genetic element which controls some aspect of the expression of nucleic acid sequences. For example, a promoter is a regulatory element which facilitates the initiation of transcription of an operably linked coding region. Other regulatory elements are splicing signals, polyadenylation signals, termination signals, etc. (defined *infra*).

Transcriptional control signals in eukaryotes comprise "promoter" and "enhancer" elements. Promoters and enhancers consist of short arrays of DNA sequences that interact specifically with cellular proteins involved in transcription (Maniatis et al., Science 236:1237 [1987]). Promoter and enhancer elements have been isolated from a variety of eukaryotic sources including genes in yeast, insect and mammalian cells and viruses (analogous control elements, *i.e.*, promoters, are also found in prokaryote). The selection of a particular promoter and enhancer depends on what cell type is to be used to express the protein of interest. Some eukaryotic promoters and enhancers have a broad host range while others are functional in a limited subset of cell types (for review see, Voss et al., Trends Biochem. Sci., 11:287 [1986]; and Maniatis *et al., supra*)*.* For example, the SV40 early gene enhancer is very active in a wide variety of cell types from many mammalian species and has been widely used for the expression of proteins in mammalian cells (Dijkema et al., EMBO J. 4:761 [1985]). Two other examples of promoter/enhancer elements active in a broad range of mammalian cell types are those from the human elongation factor 1α gene (Uetsuki et al., J. Biol. Chem., 264:5791 [1989]; Kim et al., Gene 91:217 [1990]; and Mizushima and Nagata, Nucl. Acids. Res., 18:5322 [1990]) and the long terminal repeats of the Rous sarcoma virus (Gorman et al., Proc. Natl. Acad. Sci. USA 79:6777 [1982]) and the human cytomegalovirus (Boshart et al., Cell 41:521 [1985]).

As used herein, the term "promoter/enhancer" denotes a segment of DNA which contains sequences capable of providing both promoter and enhancer functions (*i.e.*, the functions provided by a promoter element and an enhancer element, see above for a discussion of these functions). For example, the long terminal repeats of retroviruses contain both promoter and enhancer functions. The enhancer/promoter may be "endogenous" or "exogenous" or "heterologous." An "endogenous" enhancer/promoter is one which is naturally linked with a given gene in the genome. An "exogenous" or "heterologous" enhancer/promoter is one which is placed in juxtaposition to a gene by means of genetic manipulation (*i.e.,* molecular biological techniques) such that transcription of that gene is directed by the linked enhancer/promoter.

Efficient expression of recombinant DNA sequences in eukaryotic cells requires expression of signals directing the efficient termination and polyadenylation of the resulting transcript. Transcription termination signals are generally found downstream of the polyadenylation signal and are a few hundred nucleotides in length. The term "poly A site" or "poly A sequence" as used herein denotes a DNA sequence which directs both the termination and polyadenylation of the nascent RNA transcript. Efficient polyadenylation of the recombinant transcript is desirable as transcripts lacking a poly A tail are unstable and are rapidly degraded. The poly A signal utilized in an expression vector may be "heterologous" or "endogenous." An endogenous poly A signal is one that is found naturally at the 3' end of the coding region of a given gene in the genome. A heterologous poly A signal is one which is one which is isolated from one gene and placed 3' of another gene. A commonly used heterologous poly A signal is the SV40 poly A signal. The SV40 poly A signal is contained on a 237 bp *Bam*HI/*Bcl*I restriction fragment and directs both termination and polyadenylation (Sambrook, *supra,* at 16.6-16.7).

As used herein, the term "vector" is used in reference to nucleic acid molecules that transfer DNA segment(s) from one cell to another. The term "vehicle" is sometimes used interchangeably with "vector."

The term "expression vector" as used herein refers to a recombinant DNA molecule containing a desired coding sequence and appropriate nucleic acid sequences necessary for the expression of the operably linked coding sequence in a particular host organism. Nucleic acid sequences necessary for expression in prokaryotes usually include a promoter, an operator (optional), and a ribosome binding site, often along with other sequences. Eukaryotic cells are known to utilize promoters, enhancers, and termination and polyadenylation signals.

The term "transfection" as used herein refers to the introduction of foreign DNA into eukaryotic cells. Transfection may be accomplished by a variety of means known to the art including calcium phosphate-DNA co-precipitation, DEAE-dextran-mediated transfection, polybrene-mediated transfection, electroporation, microinjection, liposome fusion, lipofection, protoplast fusion, retroviral infection, and biolistics.

As used herein, the term "selectable marker" refers to the use of a gene which encodes an enzymatic activity that confers the ability to grow in medium lacking what would otherwise be an essential nutrient (*e.g.* the *HIS3* gene in yeast cells); in addition, a selectable marker may confer resistance to an antibiotic or drug upon the cell in which the selectable marker is expressed. Selectable markers may be "dominant"; a dominant selectable marker encodes an enzymatic activity which can be detected in any eukaryotic cell line. Examples of dominant selectable markers include the bacterial aminoglycoside 3' phosphotransferase gene (also referred to as the *neo* gene) which confers resistance to the drug G418 in mammalian cells, the bacterial hygromycin G phosphotransferase (*hyg*) gene which confers resistance to the antibiotic hygromycin and the bacterial xanthine-guanine phosphoribosyl transferase gene (also referred to as the *gpt* gene) which confers the ability to grow in the presence of mycophenolic acid. Other selectable markers are not dominant in that there use must be in conjunction with a cell line that lacks the relevant enzyme activity. Examples of non-dominant selectable markers include the thymidine kinase (*tk*) gene which is used in conjunction with *tk* cell lines, the CAD gene which is used in conjunction with CAD-deficient cells and the mammalian hypoxanthine-guanine phosphoribosyl transferase (*hprt*) gene which is used in conjunction with *hprt⁻* cell lines. A review of the use of selectable markers in mammalian cell lines is provided in Sambrook et al., Molecular Cloning: A Laboratory Manual, 2nd ed., Cold Spring Harbor Laboratory Press, New York (1989) pp.16.9-16.15.

Eukaryotic expression vectors may also contain "viral replicons "or "viral origins of replication." Viral replicons are viral DNA sequences which allow for the extrachromosomal replication of a vector in a host cell expressing the appropriate replication factors. Vectors which contain either the SV40 or polyoma virus origin of replication replicate to high copy number (up to 10⁴ copies/cell) in cells that express the appropriate viral T antigen. Vectors which contain the replicons from bovine papillomavirus or Epstein-Barr virus replicate extrachromosomally at low copy number (~100 copies/cell).

The thermophilic DNA polymerase III holoenzyme or holoenzyme subunits may be expressed in either prokaryotic or eukaryotic host cells. Nucleic acid encoding the thermophilic DNA polymerase III holoenzyme or holoenzyme subunit may be introduced into bacterial host cells by a number of means including transformation of bacterial cells made competent for transformation by treatment with calcium chloride or by electroporation. If the thermophilic DNA polymerase III holoenzyme or holoenzyme subunit are to be expressed in eukaryotic host cells, nucleic acid encoding the thermophilic DNA polymerase III holoenzyme or holoenzyme subunit may be introduced into eukaryotic host cells by a number of means including calcium phosphate co-precipitation, spheroplast fusion, electroporation and the like. When the eukaryotic host cell is a yeast cell, transformation may be affected by treatment of the host cells with lithium acetate or by electroporation.

"Hybridization" methods involve the annealing of a complementary sequence to the target nucleic acid (the sequence to be detected). The ability of two polymers of nucleic acid containing complementary sequences to find each other and anneal through base pairing interaction is a well-recognized phenomenon. The initial observations of the "hybridization" process by Marmur and Lane, (*See e.g.,* Marmur and Lane, Proc. Natl. Acad. Sci. USA 46:453 [1960]); and Doty et al., Proc. Natl. Acad. Sci. USA 46:461 [1960]) have been followed by the refinement of this process into an essential tool of modern biology. Nonetheless, a number of problems have prevented the wide scale use of hybridization as a tool in diagnostics. Among the more formidable problems are: 1) the inefficiency of hybridization; 2) the low concentration of specific target sequences in a mixture of genomic DNA; and 3) the hybridization of only partially complementary probes and targets.

With regard to efficiency, it is experimentally observed that only a fraction of the possible number of probe-target complexes are formed in a hybridization reaction. This is particularly true with short oligonucleotide probes (less than 100 bases in length). There are three fundamental causes: a) hybridization cannot occur because of secondary and tertiary structure interactions; b) strands of DNA containing the target sequence have rehybridized (reannealed) to their complementary strand; and c) some target molecules are prevented from hybridization when they are used in hybridization formats that immobilize the target nucleic acids to a solid surface.

Even where the sequence of a probe is completely complementary to the sequence of the target (*i.e.*, the target's primary structure), the target sequence must be made accessible to the probe via rearrangements of higher-order structure. These higher-order structural rearrangements may concern either the secondary structure or tertiary structure of the molecule. Secondary structure is determined by intramolecular bonding. In the case of DNA or RNA targets this consists of hybridization within a single, continuous strand of bases (as opposed to hybridization between two different strands). Depending on the extent and position of intramolecular bonding, the probe can be displaced from the target sequence preventing hybridization.

Solution hybridization of oligonucleotide probes to denatured double-stranded DNA is further complicated by the fact that the longer complementary target strands can renature or reanneal. Again, hybridized probe is displaced by this process. This results in a low yield of hybridization (low "coverage") relative to the starting concentrations of probe and target.

With regard to low target sequence concentration, the DNA fragment containing the target sequence is usually in relatively low abundance in genomic DNA. This presents great technical difficulties; most conventional methods that use oligonucleotide probes lack the sensitivity necessary to detect hybridization at such low levels.

One attempt at a solution to the target sequence concentration problem is the amplification of the detection signal. Most often this entails placing one or more labels on an oligonucleotide probe. In the case of non-radioactive labels, even the highest affinity reagents have been found to be unsuitable for the detection of single copy genes in genomic DNA with oligonucleotide probes. (*See,* Wallace et al., Biochimie 67:755 [1985]). In the case of radioactive oligonucleotide probes, only extremely high specific activities are found to show satisfactory results. (*See,* Studencki and Wallace, DNA 3:1 [1984]; and Studencki et al., Human Genetics 37:42 [1985]).

With regard to complementarity, it is important for some diagnostic applications to determine whether the hybridization represents complete or partial complementarity. For example, where it is desired to detect simply the presence or absence of pathogen DNA (such as from a virus, bacterium, fungi, mycoplasma, protozoan) it is only important that the hybridization method ensures hybridization when the relevant sequence is present; conditions can be selected where both partially complementary probes and completely complementary probes will hybridize. Other diagnostic applications, however, may require that the hybridization method distinguish between partial and complete complementarity. It may be of interest to detect genetic polymorphisms. For example, human hemoglobin is composed, in part, of four polypeptide chains. Two of these chains are identical chains of 141 amino acids (alpha chains) and two of these chains are identical chains of 146 amino acids (beta chains). The gene encoding the beta chain is known to exhibit polymorphism. The normal allele encodes a beta chain having glutamic acid at the sixth position. The mutant allele encodes a beta chain having valine at the sixth position. This difference in amino acids has a profound (most profound when the individual is homozygous for the mutant allele) physiological impact known clinically as sickle cell anemia. It is well known that the genetic basis of the amino acid change involves a single base difference between the normal allele DNA sequence and the mutant allele DNA sequence.

Unless combined with other techniques (such as restriction enzyme analysis), methods that allow for the same level of hybridization in the case of both partial as well as complete complementarity are typically unsuited for such applications; the probe will hybridize to both the normal and variant target sequence. Hybridization, regardless of the method used, requires some degree of complementarity between the sequence being assayed (the target sequence) and the fragment of DNA used to perform the test (the probe). Of course, those of skill in the art knows that one can obtain binding without any complementarity but this binding is nonspecific and to be avoided.

As used herein, the terms "complementary" or "complementarity" are used in reference to polynucleotides (*i.e.*, a sequence of nucleotides) related by the base-pairing rules. For example, for the sequence "A-G-T," is complementary to the sequence "T-C-A." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods which depend upon binding between nucleic acids.

The term "homology" refers to a degree of complementarity. There may be partial homology or complete homology (*i.e.*, identity). A partially complementary sequence is one that at least partially inhibits a completely complementary sequence from hybridizing to a target nucleic acid is referred to using the functional term "substantially homologous." The inhibition of hybridization of the completely complementary sequence to the target sequence may be examined using a hybridization assay (Southern or Northern blot, solution hybridization and the like) under conditions of low stringency. A substantially homologous sequence or probe will compete for and inhibit the binding (*i.e.*, the hybridization) of a completely homologous to a target under conditions of low stringency. This is not to say that conditions of low stringency are such that non-specific binding is permitted; low stringency conditions require that the binding of two sequences to one another be a specific (*i.e.,* selective) interaction. The absence of non-specific binding may be tested by the use of a second target which lacks even a partial degree of complementarity (*e.g.*, less than about 30% identity); in the absence of non-specific binding the probe will not hybridize to the second non-complementary target.

The art knows well that numerous equivalent conditions may be employed to comprise low stringency conditions; factors such as the length and nature (DNA, RNA, base composition) of the probe and nature of the target (DNA, RNA, base composition, present in solution or immobilized, etc.) and the concentration of the salts and other components (*e.g.,* the presence or absence of formamide, dextran sulfate, polyethylene glycol) are considered and the hybridization solution may be varied to generate conditions of low stringency hybridization different from, but equivalent to, the above listed conditions. In addition, the art knows conditions which promote hybridization under conditions of high stringency (*e.g.*, increasing the temperature of the hybridization and/or wash steps, the use of formamide in the hybridization solution, etc.).

When used in reference to a double-stranded nucleic acid sequence such as a cDNA or genomic clone, the term "substantially homologous" refers to any probe which can hybridize to either or both strands of the double-stranded nucleic acid sequence under conditions of low stringency as described above.

A gene may produce multiple RNA species which are generated by differential splicing of the primary RNA transcript. cDNAs that are splice variants of the same gene will contain regions of sequence identity or complete homology (representing the presence of the same exon or portion of the same exon on both cDNAs) and regions of complete non-identity (for example, representing the presence of exon "A" on cDNA 1 wherein cDNA 2 contains exon "B" instead). Because the two cDNAs contain regions of sequence identity they will both hybridize to a probe derived from the entire gene or portions of the gene containing sequences found on both cDNAs; the two splice variants are therefore substantially homologous to such a probe and to each other.

When used in reference to a single-stranded nucleic acid sequence, the term "substantially homologous" refers to any probe which can hybridize (*i.e.*, it is the complement of) the single-stranded nucleic acid sequence under conditions of low stringency as described.

As used herein, the term "hybridization" is used in reference to the pairing of complementary nucleic acids. Hybridization and the strength of hybridization (*i.e.*, the strength of the association between the nucleic acids) is impacted by such factors as the degree of complementary between the nucleic acids, stringency of the conditions involved, the Tₘ of the formed hybrid, and the G:C ratio within the nucleic acids.

As used herein, the term "Tₘ" is used in reference to the "melting temperature." The melting temperature is the temperature at which a population of double-stranded nucleic acid molecules becomes half dissociated into single strands. The equation for calculating the Tₘ of nucleic acids is well known in the art. As indicated by standard references, a simple estimate of the Tₘ value may be calculated by the equation: Tₘ = 81.5 + 0.41 (% G + C), when a nucleic acid is in aqueous solution at 1 M NaCl (*See e.g.*, Anderson and Young, Quantitative Filter Hybridization, in Nucleic Acid Hybridization [1985]). Other references include more sophisticated computations which take structural as well as sequence characteristics into account for the calculation of Tₘ.

As used herein the term "stringency" is used in reference to the conditions of temperature, ionic strength, and the presence of other compounds such as organic solvents, under which nucleic acid hybridizations are conducted. With "high stringency" conditions, nucleic acid base pairing will occur only between nucleic acid fragments that have a high frequency of complementary base sequences. Thus, conditions of "weak" or "low" stringency are often required with nucleic acids that are derived from organisms that are genetically diverse, as the frequency of complementary sequences is usually less.

"Amplification" is a special case of nucleic acid replication involving template specificity. It is to be contrasted with non-specific template replication *(i.e.,* replication that is template-dependent but not dependent on a specific template). Template specificity is here distinguished from fidelity of replication (*i.e.*, synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.

Template specificity is achieved in most amplification techniques by the choice of enzyme. Amplification enzymes are enzymes that, under conditions they are used, will process only specific sequences of nucleic acid in a heterogeneous mixture of nucleic acid. For example, in the case of Qβ replicase, MDV-1 RNA is the specific template for the replicase (Kacian et al., Proc. Natl. Acad. Sci. USA 69:3038 [1972]). Other nucleic acid will not be replicated by this amplification enzyme. Similarly, in the case of T7 RNA polymerase, this amplification enzyme has a stringent specificity for its own promoters (Chamberlin et al., Nature 228:227 [1970]). In the case of T4 DNA ligase, the enzyme will not ligate the two oligonucleotides or polynucleotides, where there is a mismatch between the oligonucleotide or polynucleotide substrate and the template at the ligation junction (Wu and Wallace, Genomics 4:560 [1989]). Finally, *Taq* and *Pfu* polymerases, by virtue of their ability to function at high temperature, are found to display high specificity for the sequences bounded and thus defined by the primers; the high temperature results in thermodynamic conditions that favor primer hybridization with the target sequences and not hybridization with non-target sequences (Erlich (ed.), PCR Technology, Stockton Press [1989]).

As used herein, the term "amplifiable nucleic acid" is used in reference to nucleic acids which may be amplified by any amplification method. It is contemplated that "amplifiable nucleic acid" will usually comprise "sample template."

As used herein, the term "sample template" refers to nucleic acid originating from a sample which is analyzed for the presence of "target" (defined below). In contrast, "background template" is used in reference to nucleic acid other than sample template which may or may not be present in a sample. Background template is most often inadvertent. It may be the result of carryover, or it may be due to the presence of nucleic acid contaminants sought to be purified away from the sample. For example, nucleic acids from organisms other than those to be detected may be present as background in a test sample.

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (*i.e.,* in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and the use of the method.

A primer is selected to be "substantially" complementary to a strand of specific sequence of the template. A primer must be sufficiently complementary to hybridize with a template strand for primer elongation to occur. A primer sequence need not reflect the exact sequence of the template. For example, a non-complementary nucleotide fragment may be attached to the 5' end of the primer, with the remainder of the primer sequence being substantially complementary to the strand. Non-complementary bases or longer sequences can be interspersed into the primer, provided that the primer sequence has sufficient complementarity with the sequence of the template to hybridize and thereby form a template primer complex for synthesis of the extension product of the primer.

As used herein, the term "nested primers" refers to primers that anneal to the target sequence in an area that is inside the annealing boundaries used to start PCR. (*See,* Mullis, et al., Cold Spring Harbor Symposia, Vol. LI, pp. 263-273 [1986]). Because the nested primers anneal to the target inside the annealing boundaries of the starting primers, the predominant PCR-amplified product of the starting primers is necessarily a longer sequence, than that defined by the annealing boundaries of the nested primers. The PCR-amplified product of the nested primers is an amplified segment of the target sequence that cannot, therefore, anneal with the starting primers.

As used herein, the term "probe" refers to an oligonucleotide (*i.e.*, a sequence of nucleotides), whether occurring naturally as in a purified restriction digest or produced synthetically, recombinantly or by PCR amplification, which is capable of hybridizing to another oligonucleotide of interest. A probe may be single-stranded or double-stranded. Probes are useful in the detection, identification and isolation of particular gene sequences. It is contemplated that any probe used in the present invention will be labelled with any "reporter molecule," so that is detectable in any detection system, including, but not limited to enzyme (*e.g*., ELISA, as well as enzyme-based histochemical assays), fluorescent, radioactive, and luminescent systems. It is not intended that the present invention be limited to any particular detection system or label.

The term "label" as used herein refers to any atom or molecule which can be used to provide a detectable (preferably quantifiable) signal, and which can be attached to a nucleic acid or protein. Labels may provide signals detectable by fluorescence, radioactivity, colorimetry, gravimetry, X-ray diffraction or absorption, magnetism, enzymatic activity, and the like.

As used herein, the term "target," when used in reference to the polymerase chain reaction, refers to the region of nucleic acid bounded by the primers used for polymerase chain reaction. Thus, the "target" is sought to be sorted out from other nucleic acid sequences. A "segment" is defined as a region of nucleic acid within the target sequence.

The term "substantially single-stranded" when used in reference to a nucleic acid target means that the target molecule exists primarily as a single strand of nucleic acid in contrast to a double-stranded target which exists as two strands of nucleic acid which are held together by inter-strand base pairing interactions.

Nucleic acids form secondary structures which depend on base-pairing for stability. When single strands of nucleic acids (single-stranded DNA, denatured double-stranded DNA or RNA) with different sequences, even closely related ones, are allowed to fold on themselves, they assume characteristic secondary structures. An alteration in the sequence of the target may cause the destruction of a duplex region(s), or an increase in stability of a thereby altering the accessibility of some regions to hybridization of the probes oligonucleotides. While not being limited to any particular theory, it is thought that individual molecules in the target population may each assume only one or a few of the structures (*i.e*., duplexed regions), but when the sample is analyzed as a whole, a composite pattern from the hybridization of the probes can be created. Many of the structures that can alter the binding of the probes are likely to be only a few base-pairs long and would appear to be unstable. Some of these structures may be displaced by the hybridization of a probe in that region; others may by stabilized by the hybridization of a probe nearby, such that the probe/substrate duplex can stack coaxially with the target intrastrand duplex, thereby increasing the stability of both. The formation or disruption of these structures in response to small sequence changes results in changes in the patterns of probe/target complex formation.

As used herein, the term "polymerase chain reaction" ("PCR") refers to the method of Mullis U.S. Patent Nos. 4,683,195 4,683,202, and 4,965,188, which describe a method for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing and polymerase extension can be repeated many times (*i.e.*, denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified".

With PCR, it is possible to amplify a single copy of a specific target sequence in genomic DNA to a level detectable by several different methodologies (*e.g.*, hybridization with a labeled probe; incorporation of biotinylated primers followed by avidin-enzyme conjugate detection; incorporation of ³²P-labeled deoxynucleotide triphosphates, such as dCTP or dATP, into the amplified segment). In addition to genomic DNA, any oligonucleotide or polynucleotide sequence can be amplified with the appropriate set of primer molecules. In particular, the amplified segments created by the PCR process itself are, themselves, efficient templates for subsequent PCR amplifications.

As used herein, the terms "PCR product," "PCR fragment," and "amplification product" refer to the resultant mixture of compounds after two or more cycles of the PCR steps of denaturation, annealing and extension are complete. These terms encompass the case where there has been amplification of one or more segments of one or more target sequences.

As used herein, the term "amplification reagents" refers to those reagents (deoxyribonucleotide triphosphates, buffer, etc.), needed for amplification except for primers, nucleic acid template and the amplification enzyme. Typically, amplification reagents along with other reaction components are placed and contained in a reaction vessel (test tube, microwell, etc.).

As used in reference to amplification methods such as PCR, the term "polymerase" refers to any polymerase suitable for use in the amplification of nucleic acids of interest. It is intended that the term encompass such DNA polymerases as the polymerase III of the present invention, as well as *Taq* DNA polymerase (*i.e.*, the type I polymerase obtained from *Thermus aquaticus*), although other polymerases, both thermostable and thermolabile are also encompassed by this definition.

As used herein, the term "RT-PCR" refers to the replication and amplification of RNA sequences. In this method, reverse transcription is coupled to PCR, most often using a one enzyme procedure in which a thermostable polymerase is employed, as described in U.S. Patent No. 5,322,770. In RT-PCR, the RNA template is converted to cDNA due to the reverse transcriptase activity of the polymerase, and then amplified using the polymerizing activity of the polymerase (*i.e.,* as in other PCR methods).

As used herein, the terms "restriction endonucleases" and "restriction enzymes" refer to bacterial enzymes, each of which cut double-stranded DNA at or near a specific nucleotide sequence.

As used herein, the term "recombinant DNA molecule" as used herein refers to a DNA molecule which is comprised of segments of DNA joined together by means of molecular biological techniques.

The terms "in operable combination," "in operable order," and "operably linked" as used herein refer to the linkage of nucleic acid sequences in such a manner that a nucleic acid molecule capable of directing the transcription of a given gene and/or the synthesis of a desired protein molecule is produced. The term also refers to the linkage of amino acid sequences in such a manner so that a functional protein is produced.

The term "isolated" when used in relation to a nucleic acid, as in "an isolated oligonucleotide" or "isolated polynucleotide" refers to a nucleic acid sequence that is identified and separated from at least one contaminant nucleic acid with which it is ordinarily associated in its natural source. Isolated nucleic acid is such present in a form or setting that is different from that in which it is found in nature. In contrast, non-isolated nucleic acids as nucleic acids such as DNA and RNA found in the state they exist in nature. The isolated nucleic acid, oligonucleotide, or polynucleotide may be present in single-stranded or double-stranded form. When an isolated nucleic acid, oligonucleotide or polynucleotide is to be utilized to express a protein, the oligonucleotide or polynucleotide will contain at a minimum the sense or coding strand *(i.e.,* the oligonucleotide or polynucleotide may single-stranded), but may contain both the sense and anti-sense strands (*i.e.,* the oligonucleotide or polynucleotide may be double-stranded).

As used herein, the term "purified" or "to purify" refers to the removal of contaminants from a sample. For example, anti-DNA polymerase III holoenzyme and holoenzyme subunit antibodies are purified by removal of contaminating non-immunoglobulin proteins; they are also purified by the removal of immunoglobulin that does not bind DNA polymerase III holoenzyme or holoenzyme subunit. The removal of non-immunoglobulin proteins and/or the removal of immunoglobulins that do not bind DNA polymerase III holoenzyme or holoenzyme subunit results in an increase in the percent of DNA polymerase III holoenzyme or holoenzyme subunit-reactive immunoglobulins in the sample. In another example, recombinant DNA polymerase III holoenzyme or holoenzyme subunit polypeptides are expressed in bacterial host cells and the polypeptides are purified by the removal of host cell proteins; the percent of recombinant DNA polymerase III holoenzyme or holoenzyme subunit polypeptides is thereby increased in the sample.

The term "recombinant DNA molecule" as used herein refers to a DNA molecule which is comprised of segments of DNA joined together by means of molecular biological techniques.

The term "recombinant protein" or "recombinant polypeptide" as used herein refers to a protein molecule which is expressed from a recombinant DNA molecule.

The term "native protein" as used herein to indicate that a protein does not contain amino acid residues encoded by vector sequences; that is the native protein contains only those amino acids found in the protein as it occurs in nature. A native protein may be produced by recombinant means or may be isolated from a naturally occurring source.

As used herein the term "portion" when in reference to a protein (as in "a portion of a given protein") refers to fragments of that protein. The fragments may range in size from four amino acid residues to the entire amino acid sequence minus one amino acid.

As used herein, the term "fusion protein" refers to a chimeric protein containing the protein of interest (*i.e.*, DNA polymerase III holoenzyme or holoenzyme subunit and fragments thereof) joined to an exogenous protein fragment (the fusion partner which consists of a non-DNA polymerase III holoenzyme or holoenzyme subunit protein). The fusion partner may enhance solubility of the DNA polymerase III holoenzyme or holoenzyme subunit protein as expressed in a host cell, may provide an affinity tag to allow purification of the recombinant fusion protein from the host cell or culture supernatant, or both. If desired, the fusion protein may be removed from the protein of interest (*i.e.,* DNA polymerase III holoenzyme, holoenzyme subunit protein, or fragments thereof) by a variety of enzymatic or chemical means known to the art.

A "variant" of DNA polymerase III holoenzyme or holoenzyme subunit, as used herein, refers to an amino acid sequence that is altered by one or more amino acids. The variant may have "conservative" changes, wherein a substituted amino acid has similar structural or chemical properties (*e.g.*, replacement of leucine with isoleucine). More rarely, a variant may have "nonconservative" changes (*e*.*g*., replacement of a glycine with a tryptophan). Similar minor variations may also include amino acid deletions or insertions, or both. Guidance in determining which amino acid residues may be substituted, inserted, or deleted without abolishing biological or immunological activity may be found using computer programs well known in the art, for example, DNASTAR software.

The term "sequence variation" as used herein refers to differences in nucleic acid sequence between two nucleic acid templates. For example, a wild-type structural gene and a mutant form of this wild-type structural gene may vary in sequence by the presence of single base substitutions and/or deletions or insertions of one or more nucleotides. These two forms of the structural gene are said to vary in sequence from one another. A second mutant form of the structural gene may exist. This second mutant form is said to vary in sequence from both the wild-type gene and the first mutant form of the gene. It is noted, however, that the invention does not require that a comparison be made between one or more forms of a gene to detect sequence variations. Because the method of the invention generates a characteristic and reproducible pattern of complex formation for a given nucleic acid target, a characteristic "fingerprint" may be obtained from any nucleic target without reference to a wild-type or other control. The invention contemplates the use of the method for both "fingerprinting" nucleic acids without reference to a control and identification of mutant forms of a target nucleic acid by comparison of the mutant form of the target with a wild-type or known mutant control.

As used herein, the term "target nucleic acid" refers to the region of nucleic acid bounded by the primers used for polymerase chain reaction. Thus, the "target" is sought to be sorted out from other nucleic acid sequences. A "segment" is defined as a region of nucleic acid within the target sequence.

The term "nucleotide analog" as used herein refers to modified or non-naturally occurring nucleotides such as 7-deaza purines (*i.e.*, 7-deaza-dATP and 7-deaza-dGTP). Nucleotide analogs include base analogs and comprise modified forms of deoxyribonucleotides as well as ribonucleotides. As used herein the term "nucleotide analog" when used in reference to targets present in a PCR mixture refers to the use of nucleotides other than dATP, dGTP, dCTP and dTTP; thus, the use of dUTP (a naturally occurring dNTP) in a PCR would comprise the use of a nucleotide analog in the PCR. A PCR product generated using dUTP, 7-deaza-dATP, 7-deaza-dGTP or any other nucleotide analog in the reaction mixture is said to contain nucleotide analogs.

"Oligonucleotide primers matching or complementary to a gene sequence" refers to oligonucleotide primers capable of facilitating the template-dependent synthesis of single or double-stranded nucleic acids. Oligonucleotide primers matching or complementary to a gene sequence may be used in PCRs, RT-PCRs and the like.

A "consensus gene sequence" refers to a gene sequence which is derived by comparison of two or more gene sequences and which describes the nucleotides most often present in a given segment of the genes; the consensus sequence is the canonical sequence. "Consensus protein," "consensus amino acid," consensus peptide," and consensus polypeptide sequences refer to sequences that are shared between multiple organisms or proteins.

The term "biologically active," as used herein, refers to a protein or other biologically active molecules (*e.g.*, catalytic RNA) having structural, regulatory, or biochemical functions of a naturally occurring molecule. Likewise, "immunologically active" refers to the capability of the natural, recombinant, or synthetic DNA polymerase III holoenzyme or holoenzyme subunit, or any oligopeptide or polynucleotide thereof, to induce a specific immune response in appropriate animals or cells and to bind with specific antibodies.

The term "agonist," as used herein, refers to a molecule which, when bound to DNA polymerase III holoenzyme or holoenzyme subunit, causes a change in DNA polymerase III holoenzyme or holoenzyme subunit, which modulates the activity of DNA polymerase III holoenzyme or holoenzyme subunit. Agonists may include proteins, nucleic acids, carbohydrates, or any other molecules which bind or interact with DNA polymerase III holoenzyme or holoenzyme subunit.

The terms "antagonist" or "inhibitor," as used herein, refer to a molecule which, when bound to DNA polymerase III holoenzyme or holoenzyme subunit, blocks or modulates the biological or immunological activity of DNA polymerase III holoenzyme or holoenzyme subunit. Antagonists and inhibitors may include proteins, nucleic acids, carbohydrates, or any other molecules which bind or interact with DNA polymerase III holoenzyme or holoenzyme subunit.

The term "modulate," as used herein, refers to a change or an alteration in the biological activity of DNA polymerase III holoenzyme or holoenzyme subunit. Modulation may be an increase or a decrease in protein activity, a change in binding characteristics, or any other change in the biological, functional, or immunological properties of DNA polymerase III holoenzyme or holoenzyme subunit.

The term "derivative," as used herein, refers to the chemical modification of a nucleic acid encoding DNA polymerase III holoenzyme or holoenzyme subunit, or the encoded DNA polymerase III holoenzyme or holoenzyme subunit. Illustrative of such modifications would be replacement of hydrogen by an alkyl, acyl, or amino group. A nucleic acid derivative would encode a polypeptide which retains essential biological characteristics of the natural molecule.

The term "Southern blot," refers to the analysis of DNA on agarose or acrylamide gels to fractionate the DNA according to size followed by transfer of the DNA from the gel to a solid support, such as nitrocellulose or a nylon membrane. The immobilized DNA is then probed with a labeled probe to detect DNA species complementary to the probe used. The DNA may be cleaved with restriction enzymes prior to electrophoresis. Following electrophoresis, the DNA may be partially depurinated and denatured prior to or during transfer to the solid support. Southern blots are a standard tool of molecular biologists (Sambrook et al., Molecular Cloning: A Laboratory Manual, Cold Spring Harbor Press, NY, pp 9.31-9.58 [1989]).

The term "Northern blot," as used herein refers to the analysis of RNA by electrophoresis of RNA on agarose gels to fractionate the RNA according to size followed by transfer of the RNA from the gel to a solid support, such as nitrocellulose or a nylon membrane. The immobilized RNA is then probed with a labeled probe to detect RNA species complementary to the probe used. Northern blots are a standard tool of molecular biologists (Sambrook *et al.,* supra, pp 7.39-7.52 [1989]).

The term "Western blot" refers to the analysis of protein(s) (or polypeptides) immobilized onto a support such as nitrocellulose or a membrane. The proteins are run on acrylamide gels to separate the proteins, followed by transfer of the protein from the gel to a solid support, such as nitrocellulose or a nylon membrane. The immobilized proteins are then exposed to antibodies with reactivity against an antigen of interest. The binding of the antibodies may be detected by various methods, including the use of radiolabelled antibodies.

The term "antigenic determinant" as used herein refers to that portion of an antigen that makes contact with a particular antibody (*i.e.,* an epitope). When a protein or fragment of a protein is used to immunize a host animal, numerous regions of the protein may induce the production of antibodies which bind specifically to a given region or three-dimensional structure on the protein; these regions or structures are referred to as antigenic determinants. An antigenic determinant may compete with the intact antigen (*i.e.*, the "immunogen" used to elicit the immune response) for binding to an antibody.

The terms "specific binding" or specifically binding" when used in reference to the interaction of an antibody and a protein or peptide means that the interaction is dependent upon the presence of a particular structure (*i.e.*, the antigenic determinant or epitope) on the protein; in other words the antibody is recognizing and binding to a specific protein structure rather than to proteins in general. For example, if an antibody is specific for epitope "A," the presence of a protein containing epitope A (or free, unlabelled A) in a reaction containing labelled "A" and the antibody will reduce the amount of labelled A bound to the antibody.

As used herein, the term "cell culture" refers to any *in vitro* culture of cells. Included within this term are continuous cell lines (*e.g.*, with an immortal phenotype), primary cell cultures, finite cell lines (*e.g.,* non-transformed cells), and any other cell population maintained *in vitro.*

The terms "test DNA polymerase III holoenzyme" and "test holoenzyme subunit" refers to a sample suspected of containing DNA polymerase III holoenzyme or holoenzyme subunit, respectively. The concentration of DNA polymerase III holoenzyme or holoenzyme subunit in the test sample is determined by various means, and may be compared with a "quantitated amount of DNA polymerase III holoenzyme or holoenzyme subunit" (*i.e.,* a positive control sample containing a known amount of DNA polymerase III holoenzyme or holoenzyme subunit), in order to determine whether the concentration of test DNA polymerase III holoenzyme or holoenzyme subunit in the sample is within the range usually found within samples from wild-type organisms.

The term "microorganism" as used herein means an organism too small to be observed with the unaided eye and includes, but is not limited to bacteria, virus, protozoans, fungi, and ciliates.

The term "microbial gene sequences" refers to gene sequences derived from a microorganism.

The term "bacteria" refers to any bacterial species including eubacterial and archaebacterial species.

The term "virus" refers to obligate, ultramicroscopic, intracellular parasites incapable of autonomous replication (*i.e.*, replication requires the use of the host cell's machinery).

### DESCRIPTION OF THE INVENTION

The present invention relates to gene and amino acid sequences encoding DNA polymerase III holoenzyme subunits and structural genes from thermophilic organisms. In particular, the present invention provides DNA polymerase III holoenzyme subunits of *T. thermophilus.* The present invention also provides antibodies and other reagents useful to identify DNA polymerase III molecules.

Prior to the present invention, only one type of DNA polymerase had been discovered in thermophilic eubacteria, even though others have been actively sought (*See e.g.*, Lawyer et al., J. Biol. Chem., 264:6427-6437 [1989]; Chien et al., J. Bacteriol., 127:1550-1557 [1976]; and Kaledin et al., Biochem., 45:494-501 [1981]). The present invention provides a pol III-class polymerase and an associated pol III holoenzyme auxiliary subunit homolog from the thermophile *T. thermophilus.*

This invention was developed in a step-wise fashion, using various techniques, including of general gap filling assays to monitor polymerase activity and the use of cross-reactive monoclonal antibodies against the α catalytic subunit of *E. coli* DNA polymerase III holoenzyme to distinguish the novel polymerase from the well-characterized DNA polymerase I-like *Thermus thermophilus* DNA polymerase.

Indeed, a survey of 12 monoclonal antibodies directed against the 130 kDa α subunit of the *E. coli* pol III holoenzyme revealed a subset that reacted with a protein of approximately the same size in Western blots of *T. thermophilus* extracts. These antibodies were used to distinguish the pol III polymerase of the present invention, from the characterized *T. thermophilus* polymerase during protein fractionation procedures.

Two proteins migrating with the polymerase and present in approximate stoichiometric ratio after three chromatographic steps were isolated and subjected to partial amino acid sequencing. The amino terminus of both were homologous to the two products of the *E. coli dna*X gene, the γ and τ subunits of the DNA polymerase III holoenzyme. Using this information and sequences conserved among *dna*X-like genes, a gene fragment was isolated by PCR, and used as a probe to isolate the full length *Thermus thermophilus dna*X gene. The deduced amino acid sequence was found to be highly homologous to the DnaX proteins of other bacteria. Examination of the sequence permitted identification of a frameshift site similar to the one used in *E. coli* to direct the synthesis of the shorter γ DnaX-gene product.

Conservation of a frameshifting mechanism to generate related ATPases is significant in that, by analogy to *E. coli,* can both assemble a β processivity factor onto primed DNA. Both a 63 kDa τ subunit that has a molecular weight consistent with its being a full length *dna*X translation product, and a 50 kDa γ subunit that likely arises by translational frameshifting was detected in enzyme purified from *T*. *thermophilus* extracts. Examination of the *dna*X DNA sequence provided confirmation of this. In E. *coli,* ribosomes frameshift at the sequence A AAA AAG into a -1 frame where the lysine UUU anticodon tRNA can base pair with 6As before elongating (Flower and McHenry, Proc. Natl. Acad. Sci. USA 87:3713-3717 [1990]; Blinkowa and Walker, Nucl. Acids Res., 18:1725-1729 [1990]; and Tsuchihashi and Kornberg, Proc. Natl. Acad. Sci. USA 87:2516-2520 [1990]). In *T. thermophilus,* the putative frameshift site has the sequence A AAA AAA A, which would enable either a +1 or -1 frameshift. The +1 frameshift product would extend only one residue beyond the lys-lys encoding sequence where the frameshift occurs, similar to the *E. coli* -1 product. A -1 frameshift would encode a protein with a 12-amino acid extension. Such an extension could permit an interaction that may further distinguish γ from τ functionally or could loop back to stabilize its structure in a thermal environment.

The present invention also provides additional polymerase III holoenzyme subunits. For example, the *dnaQ* sequence of *T. thermophilus* (SEQ ID NO:214) and partial amino acid sequence of DnaQ (SEQ ID NOS:215 and 216) are provided, as well as the *dnaE* sequence of *T. thermophilus* (SEQ ID NO:196), and DnaE amino acid sequence (SEQ ID NO:197).

It is contemplated that purified DnaQ will find use in PCR and other applications in which high fidelity DNA synthesis is required or desirable. Although an understanding of the mechanism is not necessary in order to use the present invention, DnaQ binds to the α subunit of DNA polymerase III, and works with it to efficiently remove errors made by the DNA polymerase III.

It is also contemplated that DnaQ will find use in place of an adjunct proofreading polymerase in PCR and other amplification amplifications. For example, when combined in an amplification reaction with a DNA polymerase that lacks a proofreading exonuclease, the DnaQ will facilitate elongation of PCR product as it is capable of removing mismatches within the PCR product. Thus, it is contemplated that the present invention will find use in such applications as long-range PCR (*e.g.*, PCR involving 5-50 kb targets).

In addition to the DnaQ and DnaE components provided by the present invention, a portion of *dnaN* (SEQ ID NO:230) and the corresponding deduced amino acid sequence (SEQ ID NO:231) are also provided. It is contemplated that the DnaN protein will find use in purification of the β subunit (*i.e.*, the critical subunit that permits pol III to catalyze a processive (*i.e*., long-distance without dissociating) amplification reaction. DnaN is useful with pol III alone (*e.g*., α or α plus ε) on linear templates in the absence of additional subunits, or it can be used with the DnaX complex, as well as with additional proteins (*e.g.*, single-stranded binding proteins, helicases, and/or other accessory factors), to permit very long PCR reactions.

As mentioned above, *E. coli* pol III holoenzyme can remain associated with primed DNA for 40 minutes and replicates DNA at 500-1000 nucleotides/second (McHenry, Ann. Rev. Biochem., 57:519-550 [1988]). Existing PCR technology is limited by relatively non-processive repair-like DNA polymerases. The present invention provides a thermophilic replicase capable of rapid replication and highly processive properties at elevated temperatures. It is contemplated that the compositions of the present invention will find use in many molecular biology applications, including megabase PCR by removing the current length restrictions, long range DNA sequencing and sequencing through DNA with high secondary structure, as well as enabling new technological advances in molecular biology.

### EXPERIMENTAL

The following examples serve to illustrate certain preferred embodiments and aspects of the present invention and are not to be construed as limiting the scope thereof.

In the experimental disclosure which follows, the following abbreviations apply: g (gram); L (liter); µg (microgram); ml (milliliter); bp (base pair); °C (degrees Centigrade); kb or Kb (kilobases); kDa or kd (kilodaltons); EDTA (ethylenediaminetetraacetic acid); DTT (dithiothreitol); LB (Luria Broth); -mer (oligomer); DMV (DMV International, Frazier, NY); PAGE (polyacrylamide gel electrophoresis); SDS (sodium dodecyl sulfate); SDS-PAGE (sodium dodecyl sulfate polyacrylamide gel electrophoresis); SSPE (2x SSPE contains 0.36 mM NaCl, 20 mM NaH₂PO₄, pH 7.4, and 20 mM EDTA, pH 7.4; the concentration of SSPE used may vary), SOP media (20 g/l tryptone (Difco), 10 g/l yeast extract (Difco), 5 g/l NaCl, 2.5 g/l potassium phosphate, dibasic (Fisher), 1 g/l MgSO₄ · 7H₂O (Fisher), pH 7.2); TE buffer (10 mM Tris, 1 mM EDTA); 50 x TAE (242 g Tris base, 57.1 ml glacial acetic acid, 100 ml 0.5 M EDTA pH 8.0); Blotto (10% skim milk dissolved in dH₂O and 0.2% sodium azide); Gel Loading Dye (0.25% Bromophenol blue, 0.25% xylene cyanol, 25% Ficoll (Type 400) in dH₂O); Pre-hybridization mix (50% Formamide, 5X SSPE, 1% SDS, 0.5% CARNATION™ non-fat dried milk, 10% skim milk, 0.2% Na Azide); FBS (fetal bovine serum); ABS, Inc (ABS, Inc., Wilmington, DE); GeneCodes (GeneCodes, Ann Arbor, MI); Boehringer Mannheim (Boehringer Mannheim, Indianapolis, IN); Champion Industries (Champion Industries, Clifton, NJ); Organon (Organon Teknika Corp., Durham NC); Difco (Difco, Detroit, MI); Enzyco (Enzyco Inc., Denver, Co); Fisher Scientific (Fisher Scientific, Fair Lawn, NJ); FMC (FMC, Rockland, Maine); Gibco BRL (Gibco BRL Gaithersburg, MD); Hyclone (Hyclone, Logan UT); Intermountain or ISC (ISC BioExpress, Bountiful, Utah); Invitrogen (Invitrogen, Carlsbad, CA); Millipore (Millipore, Marlborough, MA); MJ Research (MJ Research, Watertown, MA); Molecular Probes (Molecular Probes, Eugene, OR); National Diagnostics (National Diagnostics, Manville, NJ); Pharmacia Biotech (Pharmacia Biotech., Piscataway, NJ); Promega (Promega Corp., Madison, WI); Qiagen (Qiagen, Chatsworth, CA); Sigma PE/ABI (Perkin Elmer Applied Biosystems Division, Foster City, CA); (Sigma, St. Louis, MO); Stratagene (Stratagene, LaJolla CA); Tecan (Tecan, Research Triangle Park, NC); Whatman (Whatman, Maidstone, England); Lofstrand Labs (Lofstrand Labs, Ltd., Gaithersburg, Maryland) and LSPI (LSPI Filtration Products, Life Science Products, Denver, CO); Irvine (Irvine Scientific, Irvine CA); and Jackson Labs (Jackson Labs, Bar Harbor, Maine).

In Examples in which a molecular weight based on SDS-PAGE gels is reported for a protein, the molecular weight values reported are approximate values. In addition, all DNA sequences indicated herein are approximate, with the exception of the full-length gene sequences for *dnaE* and *dnaX,* which are believed to be accurate.

### EXAMPLE 1

### Selection of a DNA Polymerase Assay to Monitor the Purification of a Multi-Subunit DNA Polymerase III from Thermus thermophilus

In order to monitor the purification of a multi-subunit DNA polymerase III from the thermophilic *Thermus thermophilus,* the suitability of a DNA polymerase assay which is commonly used for measurement of the activity of DNA polymerase III in mesophilic bacteria (*e.g., Escherichia coli*) was investigated. However, the unsuitability of the prior art assay for use at high temperatures necessitated the development of an alternative assay for DNA polymerase activity for use at high temperatures. This Example involved a) long single-stranded template mesophilic DNA polymerase assay, and b) development of a gap-filling assay for DNA polymerase activity.

### A. Long Single-Stranded Template Mesophilic DNA Polymerase Assay

For mesophilic bacteria, such as *E. coli,* the assay system used to measure DNA replication employs a relatively long single-stranded DNA template, M13Gori, as substrate that is primed with RNA at a unique site by the action of dnaG primase (Cull and McHenry, Meth. Enzymol., 262:2235 [1995]). The ability to use this assay at high temperature for monitoring polymerase III activity from thermophilic organisms was investigated as follows.

The M13Gori template (Enzyco) was first primed with RNA as described below. The following components were mixed together on ice in the following order: 243 µl of primer-template solution (60 mM HEPES (pH 7.5), 14 mM magnesium acetate, 2.8 mM ATP, GTP, CTP and UTP, 14% glycerol, 56 mM NaCl, 42 mM potassium glutamate, 84 µg/ml bovine serum albumin (BSA) and 4 mM DTT), with 7.2 µl M13Gori (2.98 mg/ml), 63 µl E. *coli* single-stranded binding protein (SSB) (Enzyco) (2.2 mg/ml) and 27 µl *Dna*G primase (Enzyco) (0.39 mg/ml). This mixture was then incubated for 15 min at 30°C to form the "primer-template." The above mixture provided enough primed M13Gori for over 100 polymerase assays.

The DNA polymerase reaction mixture was assembled by adding the following components on ice: 300 µl of primer-template was added to 2.1 ml of polymerization reaction solution (180 mM Bicine (pH 8.0), 25% glycerol, 0.017% Nonidet-P40, 170 µg/ml BSA, 83 mM potassium glutamate, 8 mM DTT, 2.3 mM magnesium acetate, 10 µg /ml rifampicin, 57 µM each of dGTP, dATP and dCTP, and 21 µM TTP) and the solutions were mixed to form the "polymerase reaction mixture." This mixture can be used immediately or stored for several months at -80°C by flash-freezing in liquid N₂.

To test the stability of the polymerase reaction mixture at elevated temperatures, 15 µl aliquots of the reaction mixture were incubated at 55°C, 65°C, or 75°C, for various periods of time varying between 1 and 40 min. The reaction tubes were then immediately placed on ice and allowed to cool to 0°C. *E. coli* DNA polymerase III holoenzyme (Enzyco) was then added and the reaction tubes incubated for 5 min at 30°C before measuring the amount of double-stranded (ds) DNA synthesized in the reaction.

The amount of ds DNA synthesized was measured using a fluorometric assay that relies on the properties of the fluorophor PicoGreen™ (Molecular Probes). This dye displays significant fluorescence in the presence only of ds DNA and is almost completely non-fluorescent in the presence of single stranded DNA. Two hundred fifty microliters of a 1:400 dilution of PicoGreen™ fluorophor in Tris-HCl (pH 7.5), 1 mM EDTA was added to each reaction tube. The fluorescence intensity was measured using an SLT fluorescent plate reader (Tecan) using a 385 nm filter for excitation and a 535 nm filter for emission.

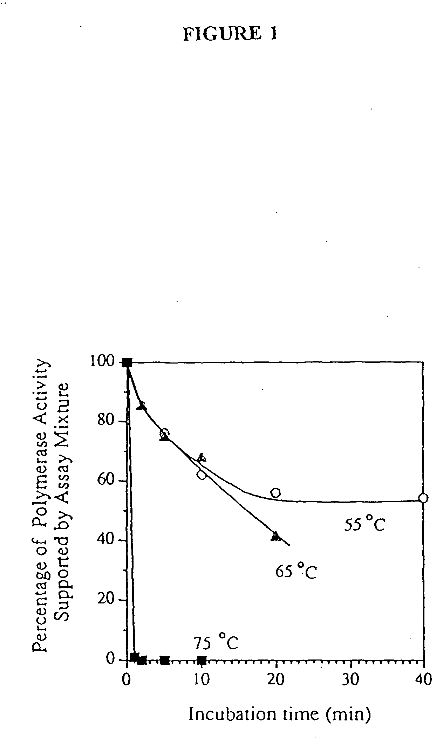

The results of these polymerase assays are summarized in Figure 1. In Figure 1, the percentage of polymerase activity supported by the assay mixture was plotted against the incubation time in minutes. In this Figure, the time points indicated represent the length of time that the reaction mixture was incubated at the elevated temperature, before chilling, adding holoenzyme, and incubating for 5 minutes at 30°C. The solid squares, the solid triangles and the open circles represent assays conducted at 75°C, 65°C and 55°C, respectively.

The data shown in Figure 1 demonstrate that one or more components of the assay mixture were inactivated at the highest temperature tested. For example, at 75°C complete inactivation occurred in less than 1 min. Thus, this system was unsuitable for the measurement of DNA polymerase activity at 75°C.

### B. Development of a Gap-Filling Assay for DNA Polymerase

Because the polymerase reaction mixture in the M13Gori assay was found to be unstable at elevated temperatures, the DNA gap-filling assay was used to monitor polymerase activity during purification, although the DNA gap-filling assay does not discriminate between the different types of polymerases (*e.g.,* polymerase I and III). The majority (>90%) of gap-filling activity in bacterial cells is due to the presence of DNA polymerase I, making it difficult to identify other polymerases, including DNA polymerase III. Nonetheless, as discussed below, this assay was found to be suitable to detect *T. thermophilus* pol III activity during the purification procedures described in the following Examples.

The gap-filling assay was performed as follows. An assay mixture containing 32 mM HEPES (pH 7.5), 13% glycerol, 0.01% Nonidet P40, 0.13 mg/ml BSA, 10 mM MgCl₂, 0.2 mg/ml activated calf-thymus DNA (Enzyco), 57 µM each of dGTP, dATP and dCTP, and 21 µM [³H]TTP (360 cpm/pmol) was assembled.

The reaction was started by the addition of a 0.5 µl of a suitable dilution of DNA polymerase to 15 µl of reaction mix, and incubated at 37°C for 5-50 min. The reaction was then stopped by placing the reaction tube on ice.

The amount of DNA synthesized in the assay was measured by first precipitating the polynucleotide with 2 drops of 0.2 M inorganic pyrophosphate (PPi) and 0.5 ml 10% TCA. Removal of unincorporated nucleotide triphosphates was accomplished by filtering the mixture through GFC filters (Whatman) and washing the filters with 12 ml 0.2 M PPi/1M HCl and then ethanol. The filters were then allowed to dry before scintillation counting; Ecoscint-0 (National Diagnostics) was used as the scintillant. One unit of enzyme activity is defined as one picomole of TTP incorporated per min at 37°C. Positive controls containing *E. coli* DNA pol III were included in the assay since this assay does not distinguish the activity of pol I,pPol II, and pol III.

The gap-filling assay was selected instead of the long stranded template DNA polymerase assay to monitor the purification of DNA polymerase III activity from *T. thermophilus.* In the following Examples, the assay was performed at 37°C, in part for convenience, but also because the decrease in activity for pol III at lower temperatures is less than that of the major *T. thermophilus* DNA polymerase activity, permitting more efficient detection of pol III because it comprised a greater percentage of total polymerase activity when assayed this way. One unit of activity is equivalent to 1 pmol total nucleotide synthesis per minute at 37 °C.

### EXAMPLE 2

### Purification of a DNA Polymerase Multi-Subunit Complex From T. thermophilus

This Example involved a) growth of *T. thermophilus* strain pMF48.kat cells, and b) large-scale purification of a DNA III polymerase multi-subunit complex from *T. thermophilus.*

The MF48.kat strain (Lasa et al., Microbiol., 6:1555-1564 [1992]) was used for the purification of a polymerase multi-subunit complex. Strain pMF48.kat is a *T. thermophilus* strain mutated to be deficient in the protective S-layer protein found on the outer coat. This mutation renders the strain susceptible to lysis by hen-egg white lysozyme. The lysis procedure of Cull and McHenry (Cull and McHenry, Meth. Enzymol., 262:22-35 [1995]) for isolation of E. *coli* DNA polymerase III holoenzyme *(i.e.,* the replicative enzyme of *E. coli*) requires a gentle lysis procedure using lysozyme and the addition of spermidine to precipitate the chromosomal DNA with the cellular debris. The use of the S-layer mutant of *T. thermophilus* was found to allow the use of a modification of the standard *E. coli* gentle lysis procedure (Cull and McHenry *supra*), as described below.

A complex containing at least three proteins (α, τ, and γ) having DNA polymerase activity as determined by the gap-filling assay was purified from *T. thermophilus* MF48.kat Partial amino acid sequencing of these *T. thermophilus* proteins revealed that these proteins share homology with subunits of the *E. coli* DNA polymerase III holoenzyme.

### A. Growth of T. thermophilus Strain pMF48.kat Cells

MF48.kat cells were grown as previously described in (Lasa et al. , Molec. Microbiol., 6:1555-1564 [1992]), harvested, and the pellet stored at -80°C as glycerol stock until ready for use. DNA polymerase was purified from large scale fermentation cultures of MF48.kat cells. These steps are further elaborated in the following sections.

### 1. Preparation of Media for Fermentation of T. thermophilus Strain pMF48.kat

*T. thermophilus* strain pMF48KAT (Lasa *et al.,* 1992) was grown in 180 L batches with aeration in a 250 1 fermentor at 72 °C in a medium containing (per L): 0.27 g ferric chloride hexahydrate, 0.294 g sodium citrate trisodium salt dihydrate, 0.025 g calcium sulfate dihydrate, 0.20 g magnesium chloride hexahydrate, 0.53 g ammonium chloride, 8 g pancreatic digest of casein (DMV International), 4 g yeast extract (Ardamine Z, Champlain Industries), 4.0 g glucose (Cerelose 2001, food grade (Corn Products, International), 0.5 L-glutamic acid monosodium salt, 0.254 g sodium phosphate monobasic (dissolved in 1000/180 ml water), 1.5 g dipotassium phosphate (dissolved in 2000/180 ml water). Following the addition of the final component to the fermentation medium, the pH of the broth was adjusted to pH 7.3 at 72°C with 3N NaOH. Fermentation was conducted so as to avoid precipitation or turbidity, and achieve growth over 1.2 O.D.₆₀₀. During preparation of the fermentation medium, adequate time (approximately 4 minutes) was allowed for medium constituents to go into solution prior to the addition of the next medium component while constantly stirring.

### 2. Growth Conditions

Seed cultures were grown from frozen glycerol stock as follows and stored at -80°C. One ml of frozen glycerol stock was added to 250 ml fermentation medium prepared as described above and additionally containing kanamycin (30 µg/ml) at 72°C in a baffled Fernbach flask with 45 mm screw top closure, and the culture incubated overnight in an environmental shaker (New Brunswick) with low agitation (~100 rpm). At approximately 20 hours, the resulting seed culture was transferred to 180 L of fermentation medium in a fermenter (IF250 New Brunswick Scientific). Fermentation was carried out at 72°C, pH 7.3, 100% O₂ (measured with Ingold O₂ Transmitter 4300 with Ingold probes blanked on non-sparged, non-agitated medium, and at a pressure of 3 pounds per square inch, as determined by the pressure gauge on the fermenter) over atmospheric pressure with air flow at 40 L/min and rpm at 80.

A 5 L sugar mixture consisting of 480 g/L glucose and 60 g/L L-glutamic acid was added during the run. The sugar mixture was prepared by incrementally adding and dissolving the sugar and glutamic acid in hot, sterile deionized water. One liter of the sugar mixture was added at OD₆₀₀ 0.7-0.9, followed by the addition of 1 L of sugar mixture at each doubling thereafter (*i.e.,* at the rate of approximately 50-100 ml sugar solution per minute). At the time of transfer of the seed culture from the flask to the large fermenter, the culture was mixed with aeration (sparge) only in the absence of agitation. Agitation was begun at OD₆₀₀ 0.7- 0.9. As the culture density increased and the available oxygen approached zero (measured with Ingold O₂ Transmitter 4300 with Ingold probes blanked on non-sparged, non-agitated medium), the agitation was increased to keep the detectable O₂ levels just above zero.

pH control was not used during the initial static growth phase until OD₆₀₀ reached approximately 0.7-0.9, at which time pH was controlled with 3N sodium hydroxide to maintain a pH of 7.3 at 72°C. The pH was measured with an Ingold pH controller/transmitter 2500 with Ingold probes. Foaming was controlled mechanically.

Cells were harvested at OD₆₀₀ 2.5-3.0 by transferring the culture via a hose through an Alfa-Laval plate heat exchanger (72°C culture: ~ 10°C water exchange medium) to a Sharples A5 16 continuous flow centrifuge. A cell paste was obtained by centrifugation in a Sharples A5 16 continuous flow centrifuge for approximately 1 hour at 17,000 rpm (13,000xg) at room temperature. The cell paste was resuspended 1:1 (w/w) in Buffer TS (50 mM Tris-HCl, 10% sucrose (pH 7.5) and then frozen in liquid nitrogen as pellets and stored at -20°C.

### B. Partial Purification of T. thermophilus DNA Polymerase III and Associated Proteins

To avoid formation of a precipitate which was observed when protein solutions were kept at 4°C, all of the following operations for large scale purification were performed at room temperature unless otherwise stated. Cells were lysed with lysozyme, and an ammonium sulfate fraction collected. The ammonium sulfate fraction was first resolved by cation exchange chromatography, followed by hydrophobic chromatography. These steps are described below.

### Cell Lysis and Ammonium Sulfate Fractionation

First, 4.7 kg of a 1:1 suspension of cells in Tris-sucrose were added to 6.5 1 tris-sucrose that had been prewarmed to 55 °C. To the stirred mixture, 117 ml of 0.5 M DTT, and 590 ml of 2M NaCl, 0.3M spermidine in Tris-sucrose adjusted to pH 7.5, were added. The pH of the slurry was adjusted to pH 8 by the addition of 2 M Tris base, and 2.35 g lysozyme was added. The slurry was distributed into 250 ml centrifuge bottles and incubated at 30 °C for 1 hour with occasional inversion, and then centrifuged at 23,000 x g for 60 min. at 4°C. The recovered supernatant (8 l) constituted Fraction I (Table 1).

**TABLE 1**

| **Partial Purification of *T. thermophilus* Pol III and Associated Proteins** | | | | |
|---|---|---|---|---|
| **Fraction** | **Method of Purification** | **Units (x 10³)** | **Protein^{c} (mg)** | **Spec. Act. (Units/mg)** |
| I. | Cell lysis | ND^{a} | 73,800 | ND |
| II. | Amm. sulfate precipitation | 2,200 | 6,540 | 360 |
| III. | BioRex-70 chromatography | 6,700 | 686 | 10,000 |
| IV. | ToyoPearl ether chromatography | 1,400 | 54 | 25,000 |
| V. | Q-Sepharose chromatography | 760^{c} | 4.8 | 160,000^{b} |

| | | | | |
|---|---|---|---|---|
| ^{a} Fraction I was not assayed due to a non-linear response, presumably due to nuclease or inhibitory contaminants. ^{b} Assays were conducted at 37°C. The specific activity is 5-fold higher (800,000 units/mg) if assayed at 60°C. ^{c} The reported yield is normalized to a complete preparation using all of the material from Fraction IV. Only 50% of Fraction IV was used in the preparation of Fraction V. | | | | |

To FrI, ammonium sulfate (0.267 g/ initial ml Fraction I) was added over a 15 min interval. The mixture was stirred for an additional 30 min at 4 °C and then centrifuged at 23,000 x g for 60 min at 0 °C. The recovered pellet was resupended (on ice) in 563 ml (0.07 x Fraction I volume) 50 mM Tris-HCl (pH 7.5), 20 % glycerol, 1 mM EDTA, 0.1 M NaCl, 5 mM DTT, 0.18 g (added to each ml of final solution) ammonium sulfate and the resulting suspension centrifuged at 23,000 x g for 60 min at 0°C resulting in Fraction II (Table 1).

The precipitate was thoroughly resuspended on ice in 563 ml (0.07 x vol. of Fraction I) of backwash solution (50 mM Tris-HCl (pH 7.5), 20% glycerol, 1 mM EDTA, 0.1 M NaCl, 5 mM DTT, 0.18 g/ initial ml ammonium sulfate [*i.e.*, 0.18 g ammonium sulfate were added for each 1 ml of initial solution volume, and centrifuged at 12,000 rpm in a GSA rotor for 60 min at 0°C. The pellet (Fraction II) was stored at 4°C.

### C. Identification Of Monoclonal Antibodies Cross-Reactive With The α Subunit Of T. thermophilus DNA Polymerase III Using ELISA

### 1. Generation of Monoclonal Antibodies

Monoclonal antibodies were generated by the University of Colorado Cancer Center Monoclonal Antibody Core. Standard procedures were used as described (*See e.g.,* Oi and Herzenberg, in Mishell and Shiigi (eds.), Selected Methods in Cellular Immunology, W.H. Freeman & Co., San Francisco [1980], p 351-371). Briefly, 100 µg of DNA polymerase III α subunit (Enzyco) in complete Freund's Adjuvant was injected subcutaneously into the back of the neck of a BALB/c Bailey mouse (Jackson Labs). Four weeks later, each mouse was boosted intraperitoneally with 50µg in Incomplete Freund's Adjuvant (IFA). Each animal was boosted again two weeks later with the same injection. Two weeks later, mice were bled from the tail and their polyclonal titer against α determined. The mouse giving the best response was selected for future sacrifice, was and given an additional 20 µg booster (without adjuvant). Three days later, the mouse was sacrificed, the spleen aseptically removed and processed by mincing into several 2-3 mm pieces. The spleen pieces were bathed in RPMI-serum free medium (1 10 L package RPMI Powder (Irvine), 20 g sodium bicarbonate, 43 g HEPES, 100 ml non essential amino acids (NEAA, Irvine), 1.1 g sodium pyruvate, 2.92 g L-Glutamine, 50 ml Pen-Strep (Irvine) adjusted to pH 7.05, in 10 L deionized H₂O, and filtered through a Gelman filter. Cells were transferred to a 50 ml conical tube, pelleted and washed in RPMI-serum free medium. Cells were manipulated to remove red blood cells and centrifuged for 5 min at 1400 rpm. Remaining red blood cells were removed by resuspending the spleen cell pellet in 10 ml of 0.83% NH₄Cl at room temperature for 90 seconds. After 90 sec, 20 ml of RPMI-serum free medium was added, the cells were swirled to mix, and centrifuged at 1400 rpm for 5 min. The cell pellet was washed with RPMI-serum free medium, centrifuged (5 min, 1400 rpm) and resuspended in 20.3 ml RPMI-serum free medium.

Spleen cells and THT myeloma cells (Fox-NY Mouse Myeloma, ATCC 1732-CRL), cultured in RPMI with 10% FBS (Hyclone Lot 2334), and 15 µg/ml 8-azaguanine (pH 7.1-7.2) under 7% CO₂ at 37 °C), were mixed at a 4:1 ratio in a 50 ml conical tube. Mixed cells were centrifuged 10 min at 1400 rpm, and the supernatant was aspirated. PEG (50%) at room temperature was added drop-wide over 1 minute with gentle stirring. Approximately 1 ml was added to 2 x 10⁸ cells. The PEG was then diluted by drop-wise addition of 9 ml RPMI-serum free medium. Cells were centrifuged at 1400 rpm for 10 min.

The supernatant from the centrifuged cells was aspirated and the cells were resuspended in 10 ml of RPMI containing 15% FBS. Then, 10 ml of cells were plated per 100 mm Petri dish, and the cells were incubated overnight at 37 °C in 7 % CO₂. Cells were then harvested, collected by centrifugation and resuspended in 205 ml RPMI containing 15 % FBS + AHAT medium (75 µM adenine, 0.1 mM hypoxanthine, 0.4 µM aminopterin, 16 µM thymine) in a T75 flask. In addition, 2 drops of this suspension were plated into the wells of a 96-well CoStar dish. This procedure was continued to generate twenty 96 well dishes. These were incubated at 37 °C in 7% CO₂. On the third day after plating 2 drops/well of fresh RPMI containing 15% FBS and AHAT, as well as 1x Hybridoma Cloning Supplement (Boehringer-Mannheim) medium were added, with fluid changes every three days thereafter by aspirating ½ of the medium from each well and feeding 2 drops/well of fresh RPMI with 15% FBS and AHAT. Screening was performed when the cells reached 1/3 confluency (*ca.* 10 days post fusion).

### 2. Monoclonal Antibody Screening

Screening was performed by an ELISA procedure. Twenty 96-well dishes (Dynatech Laboratories) were coated with 50 µl/well of a 1 µg/ml solution of the α subunit of the *E. coli* DNA polymerase III holoenzyme diluted in PBS (8 g/l NaCl, 0.2 g/l KCI, 1.15 g/l Na₂HPO₄, 0.2 g/l KE₂PO₄ (pH 7.4)). Plates were washed (3x) in PBS with 0.1%Tween 20, and blocked by the addition of 200 µl/well of 1 % BSA in PBS. After washing, hybridoma supernatant (50 µl/well) was added from the corresponding well of the 96-well culture dish. After washing, Fc specific goat anti-mouse IgG-horse radish peroxidase conjugated (Organon) (100 µl/well) antibody (diluted 1:5000 in 1% BSA in PBS) was added, and incubated for 3 hours at room temperature. Plates were washed and exposed to 100 µl substrate solution and incubated for 15 minutes in the dark. Substrate solution was prepared by adding 150 ml citric acid buffer (10.2 g/l citric acid monohydrate, 26.8 g/l sodium phosphate dibasic, pH adjusted to 4.9 with phosphoric acid) to 60 mg O-phenylenediamine (Sigma). Just prior to use, 60 µl of 30% hydrogen peroxide was added. Reactions were quenched by the addition of 50 µl sulfuric acid/well, and read at 490 nm.

From the preliminary screening, 24 wells appeared positive (O.D. 490 > 1.0 Control wells containing only diluent (*i.e.*, 1% BSA in PBS); growth medium and medium containing AHAT and myeloma cells only, gave an O.D. of 0.2 or less. Cells from 24 wells that produced monoclonal antibodies against the α subunit of DNA polymerase III holoenzyme were expanded in a well of a 24 well plate and tested again by ELISA for continued secretion of antibody against α. Three wells failed to show continued growth or secretion. The remaining candidates were also screened by a Western blot procedure with a mixture of the DNA polymerase III holoenzyme subunits (as described under section describing following *T. thermophilus* pol III by Western on hydrophobic column except 0.5 µg of each of the *E. coli* DNA polymerase III holoenzyme subunits were loaded in a mixture and blotted against undiluted serum).

From the 21 remaining candidates, 12 wells were selected that reacted strongly and specifically with α on Western Blots for cloning by limiting dilution (antibodies # 178, 210, 257, 279, 645, 889, 1018, 1104, 1171, 1283, 1950, 1976). The remaining polyclonal wells were frozen down by the procedure described below. The antibodies produced were isotyped using an ELISA and isotype-specific antibodies. The two antibodies used in this study, 1950 and 1104 were both IgG₁.

For cloning by the limiting dilution technique, cells were removed from the chosen positive wells and placed in a 15 ml conical tube. An aliquot of 600,000 cells was taken and added to a tube containing RPMI-serum free media to bring the final cell density to 60,000 cells/ml. Cells were diluted 1:10 successively with RPMI-serum free media until a dilution of 600 cells/ml was achieved. Cells were then diluted with HCS (Hybridoma Cloning Supplement, Boehringer Mannheim) until concentrations of 50, 10 and 5 cells/ml were achieved. These were then aliquoted into wells of a 96 well plate (2 drops/well), incubated in a 37 °C incubator and visually scored after five days in an inverted microscope, by noting the wells that appear to have colonies that arose from a single cell (one colony/well). These wells were screened by ELISA to identify clones that are producing antibody directed against α.

The 12 candidate positive clones were expanded into 24 well plates and recreened by ELISA. These were expanded further into duplicate wells in a 24 well plate and then grown in 100 mm dishes and supernatant was collected. Cells were frozen away in 2 x 10⁶ cell aliquots in 95% FBS + 5% DMSO) and stored in a liquid nitrogen freezer.

### 3. ELISA Assay of an Ammonium Sulfate Fraction

In general, large asymmetric complexes are relatively insoluble in ammonium sulfate. Ammonium sulfate fractionation provides a 50-fold purification of the *E. coli* pol III holoenzyme. A low ammonium sulfate cut of *T. thermophilus* extracts was used to provide a source of protein enriched sufficiently in pol III that an ELISA assay could be used to screen 12 monoclonal antibodies directed against the *E. coli* pol III a subunit to determine if they cross-reacted with a *T*. *thermophilus* protein. The ammonium sulfate fraction was prepared by addition of 0.246 g ammonium sulfate to each ml of Fraction I using the same approach as described for the preparation of Fraction II in the pol III preparation described under Methods. For the ELISA screening assay, all manipulations were conducted at room temperature. Into each well of a 96-well microtiter plate (Coming Costar High Binding EIA/RIA) was placed 4 µg protein in 150 µl Buffer E7 (10mM Tris-HCl (pH 7.5), 150 mM NaCl, 0.05% Tween-20). After an overnight incubation, each well was blocked by incubation with buffer E7 + 10 µg/ml BSA for 3 h, followed by 3 washes with Buffer E7 containing 10 mg/ml BSA and the addition of 150 µl of hybridoma supernatant, incubated for 3.5 hours. Wells were then washed 3 times with Buffer E7 and once with an equivalent buffer that had been adjusted to pH 8.8 (Buffer E8). Then, 150 µl of a 1:3000 dilution of goat anti-mouse IgG antibody-alkaline phosphatase conjugate (BioRad) was added and incubated for 1 hour. Wells were washed 3 times with Buffer E7, once with Buffer E8 and developed in the presence of p-nitrophenyl phosphate for 10 min. Absorbance was read at 405. Monoclonal antibodies produced from hybridoma lines C1950-F3 and C1104-H2 (Enzyco) gave an absorbance of 0.17 and 0.16 respectively; on average, all other candidate monoclonal supernatants gave an absorbance of 0.06. Equal volumes of both supernatants were combined for future work.

In addition, 40 µg of the 0.246 ammonium sulfate fraction of *T. thermophilus* FrI was subjected to the Western Blotting procedure described below. A band migrating with the same mobility as the α subunit of pol III of *E. coli* was detected.

These data demonstrated that Fraction II of *T. thermophilus* contained an α subunit of DNA polymerase III, and that a mixture of monoclonal antibodies from hybridoma cell lines C1950-F3 and C1104-H2 is capable of detecting the *T. thermophilus* DNA polymerase III α subunit. These data also demonstrated that the ammonium sulfate precipitations were optimized to provide a nearly quantitative precipitation of the candidate *T. thermophilus* α subunit (as judged by Western blots) while removing as much contaminating protein as possible (Fraction II; Table 1).

### D. Cation Exchange Chromatography

Having established that a roughly 130 kDa protein cross-reacted with anti-*E*. *coli* α monoclonals, a lysis procedure and ammonium sulfate fractionation that partially purified the α subunit were optimized.

A BioRex 70 cation exchange chromatography step was developed that resolved polymerase activity from the majority of contaminating protein. Fraction II was resuspended in 100 ml buffer U (50 mM imidazole-HCl (pH 6.8), 20% glycerol, 35 mM ammonium sulfate, 1 mM magnesium acetate, 0.1 mM zinc sulfate, 5 mM β-mercaptoethanol, 0.1 mM ATP) and dialyzed twice successively versus 2 L buffer U. Dialysate was applied to a 300 ml BioRex 70 (BioRad, 100-200 mesh, 5.5 cm diameter) column equilibrated in buffer U and washed with 0.9 1 buffer U. Activity was eluted with a 1.5 1 0→300 mM NaCl gradient in buffer U. All gradient-eluted fractions containing greater than 20,000 units of gap-filling polymerase activity/ml were pooled, constituting Fraction III (Table 1).

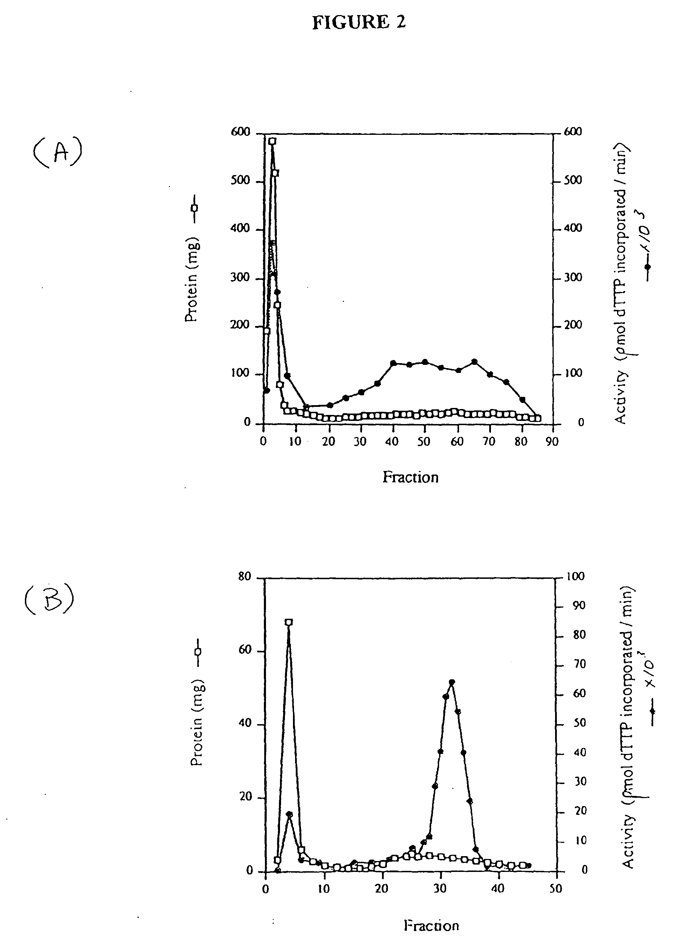

Figure 2 shows the column profile of protein, and DNA polymerase activity. Fractions 20-80 in Figure 2A which contained bound DNA polymerase activity were pooled to generate Fraction III. Ammonium sulfate solution saturated at 4°C was added to 50% (v/v), and centrifuged at 23,000 x g for 60 min at 4°C to obtain a precipitate.

Although the BioRex-70 column separated a large amount of contaminating protein from the polymerase activity and resulted in enrichment of the polymerase fraction, it did not resolve different polymerases, since it did not resolve the pol III antibody reactive fraction from the majority of polymerase activity. Nevertheless, a 25-fold increase in gap-filling polymerase specific activity was achieved (Fraction III, Table 1). Resolution of different DNA polymerase activities was achieved by hydrophobic interaction chromatography as described below. Figure 2A shows the results from one column run, while Figure 2B shows the results from another column run. Although the results are not completely reproducible, these Figures show that the DNA polymerase preparation was enriched by the BioRex-70 column.

### E. Hydrophobic Chromatography

*E. coli* pol III holoenzyme binds tightly to hydrophobic columns (McHenry and Kornberg, J. Biol. Chem., 252:6478-6484 [1977]). Thus, hydrophobic interaction chromatography was attempted to resolve *T. thermophilus* pol III from the smaller and, presumably, more hydrophilic DNA polymerase I-like activity.

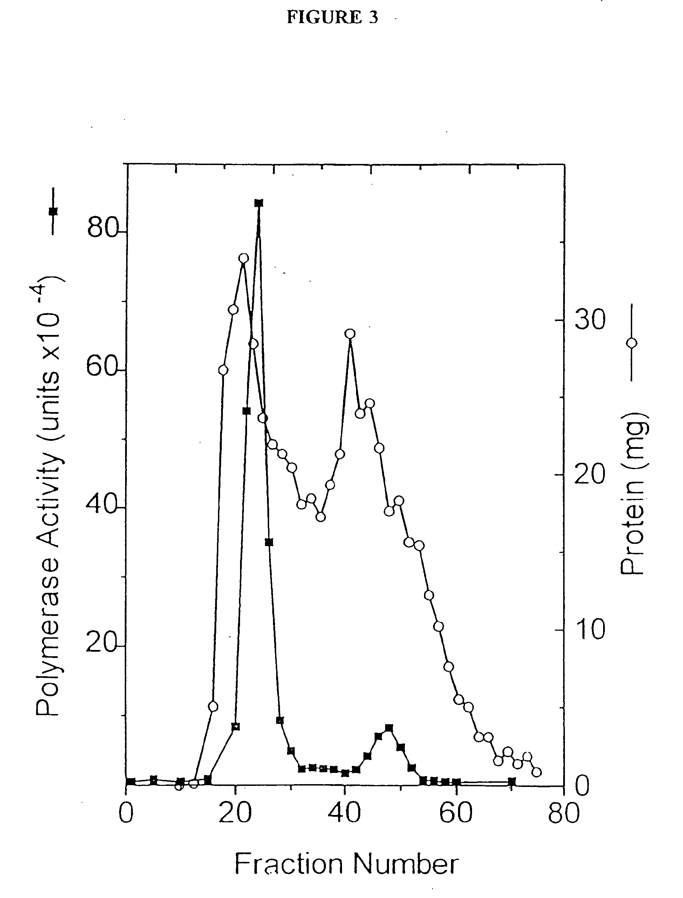

Hydrophobic interaction chromatography was used to resolve a unique polymerase that cross-reacted with antibody directed against *E. coli* pol III α subunit. Fraction III protein was precipitated by the addition of an equal volume of saturated ammonium sulfate and the pellet was collected by centrifugation 23,000 x g, 1 h, 4 °C). The pellet was dissolved in 40 ml buffer U, and 20 ml ToyoPearl-Ether 650M (Toso Haas) equilibrated in buffer U and 1.6 M ammonium sulfate was added. To the stirred suspension was added (dropwise) 33.6 ml 4 M ammonium sulfate and the entire mixture was applied to an 80 ml ToyoPearl-Ether column (3.5 cm diameter) equilibrated in buffer U with 1.6 M ammonium sulfate. The column was washed with 80 ml buffer U + 1.1 M ammonium sulfate and polymerase activity eluted with a 1.5 1 1.1→0.3 M ammonium sulfate gradient in buffer U (Figure 3). Fractions containing polymerase activity were subjected to the Western Blotting procedure. The second peak (fractions 45→50) that contained polymerase activity and reacted with monoclonal antibodies against *E*. *coli* pol III α subunit was pooled, constituting Fraction IV (120 ml, Table 1, Figure 3). Fractions were assayed for gap-filling polymerase activity as described above and for protein content using the Coomassie Protein Assay Reagent (Pierce) with BSA used as a standard.

Figure 3 shows a profile of the hydrophobic column chromatography. Protein (open circles) and gap-filling polymerase activity (filled squares) were plotted against fraction numbers. The gap-filling assay revealed 2 peaks of polymerase activity, with the first peak eluting between fraction numbers 20-30, and the second peak eluting between fraction numbers 45-50. The major *T. thermophilus* DNA polymerase peak eluted early in the gradient, and did not react with anti-α monoclonal antibodies. The second peak, a minor peak comprising approximately 9% of the polymerase activity, bound more tightly to the column (Fraction IV, Table 1).

### F. Western Blotting and Identification of Proteins Cross Reactive with Monoclonal Antibody Against T. thermophilus DNA Polymerase III Subunit α

In order to determine whether the fractions which eluted from the hydrophobic chromatography column contained *T. thermophilus* DNA polymerase III α subunit, fractions containing DNA polymerase activity as determined by the gap filling assay (described in Example 1) were further analyzed by Western blotting using monoclonal antibodies which were specific for *T. thermophilus* DNA polymerase III α subunit (described above). The following steps were carried out at room temperature unless otherwise indicated.

Aliquots of column fractions (50 µl) were subjected to electrophoresis on a 10% SDS-PAGE. Proteins were then transferred from the gel to a PVDF membrane (BioRad). The transfer buffer contained 25 mM Tris, 192 mM glycine, and 20% methanol, and was adjusted to pH 8.5 by the addition of HCl. Transfer was conducted for 2 h at 70V (0.7 A). The membrane was then washed in 10 mM Tris-HCl (pH 7.5), 150 mM NaCl, 0.05% Tween-20 followed by incubation for 1 h in the same buffer with 5% dried milk. The blot was then incubated overnight with C1950-F3, C1104 H2 hybridoma supernatant (Enzyco), washed (3-times) in 100 ml buffer TBS containing 0.5% Tween-20, incubated for 1 hour with a 1:2000 dilution of goat anti-mouse IgG alkaline phosphatase conjugate (BioRad) in buffer TBS containing 0.5% dried milk, washed (3-times) in 100 ml buffer TBS containing 0.5% Tween-20, and washed once in 100 ml buffer P (100 mM Tris-HCl (pH 9.5), 100 mM NaCl, 5 mM MgCl₂). The membrane was developed by incubating in a 1:150 dilution of a GIBCO nitroblue tetrazolium stock solution and a 1:300 dilution of a 5-bromo-4-chloro-3-indoyl phosphate stock solution in buffer B, until the α band reached the desired intensity. The development of color was stopped by washing the membrane in water.

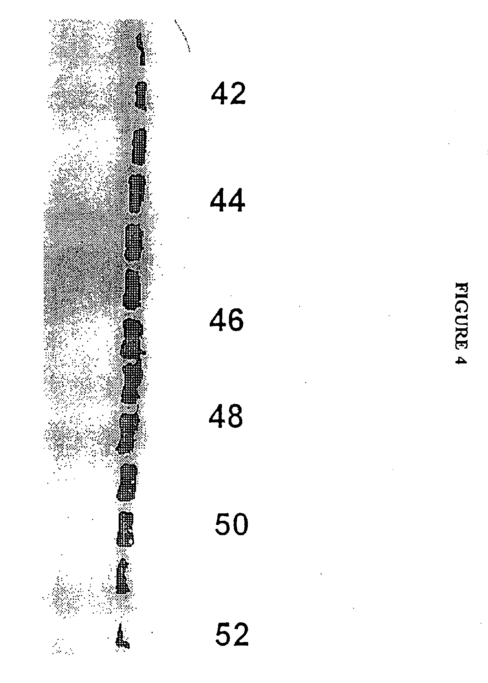

Figure 4 shows the results of the SDS-PAGE / Western blot analysis for fractions 41-52. Lane numbers 41-52 contained ToyoPearl-Ether column fractions 41-52. Western blot analysis showed that the second peak (fractions ~45-50) contained protein molecules with a molecular weight of approximately 130 kDa which cross-reacted with antibodies from cell lines C1950-F3 and C1104-H2 raised against the α subunit of *E. coli* DNA polymerase III holoenzyme (Figure 4).

Figure 4 shows that the cross-reactive 130 kDa band eluted with an intensity parallel to the polymerase activity in the second peak. This demonstrates that the second peak of activity contained a pol III-like polymerase, which is distinct from the characterized *T. thermophilus* DNA polymerase. This is in contrast to fractions 20-30 in the first peak which did not result in detectable binding to *E. coli* DNA polymerase III antibody. Fractions 45-50 were used to generate Fraction IV (stored at 4°C).

### G. Anion Exchange Chromatography

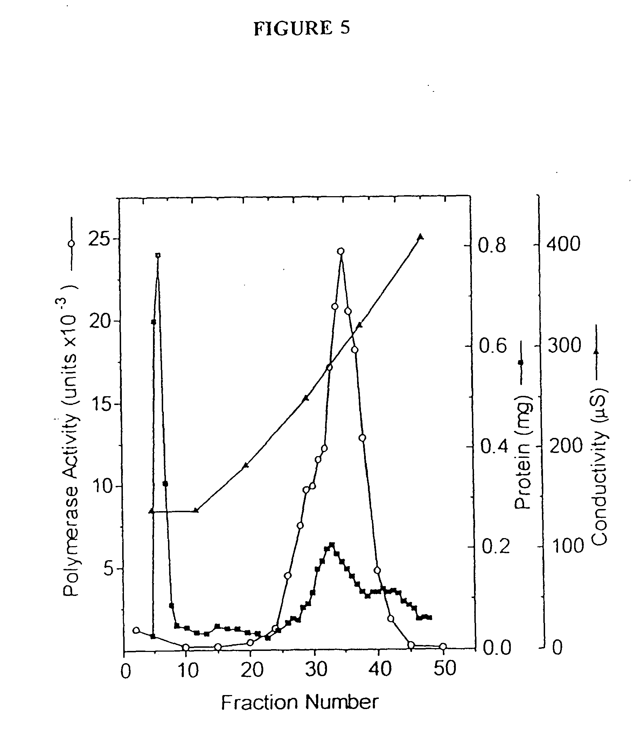

To provide a higher level of purification, and to enable direct examination of the components, an additional chromatographic step was developed. One-half of Fraction IV was combined with an equal volume of saturated ammonium sulfate (4 °C) and the resulting precipitate collected by centrifugation and redissolved in 4 ml buffer U, and dialyzed overnight versus 250 ml buffer U (one buffer change). The dialysate was then applied to a 5 ml Q-Sepharose fast flow column (1.4 cm diameter) and equilibrated in buffer U. The column was washed with 15 ml buffer U, and the polymerase activity was eluted with a 75 ml 0→275 mM NaCl gradient in buffer U. A contaminating protein was found to elute toward the end of the activity peak; thus, fractions were carefully selected for pooling based on purity. All fractions that contained greater than 50 µg protein and a specific activity greater than 104,000, were pooled (Fraction 28-36) to yield Fraction V (25 ml, Table 1, Figure 5). Fractions (1.5 ml each) were collected and assayed for polymerase activity using the gap-filling assay described above, and for conductivity using a Radiometer CDM83 conductivity meter on samples diluted 1:100 in distilled water equilibrated to room temperature. Standards were also run in parallel.

Figure 5 shows polymerase activity (open circles), protein (filled squares) and conductivity (filled triangles) across the column fractions. Chromatography on Q-Sepharose yielded a single peak of polymerase activity between fractions 28-40, and a 6-fold increase in specific activity (Figure 5, Fraction V; Table 1) .

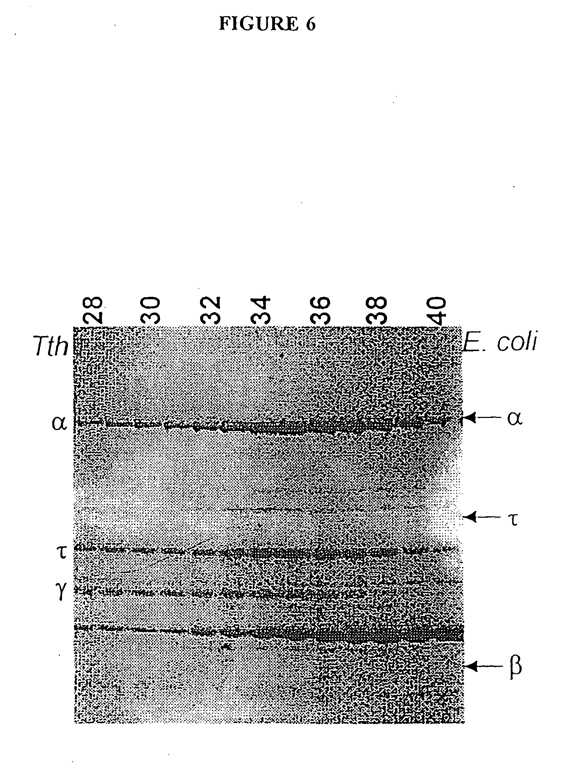

Samples (10 µl) of fractions 28-40 across the polymerase activity peak were analyzed by SDS-PAGE (10%). The Coomassie blue-stained gel is shown in Figure 6. The migration position of the *E. coli* DNA polymerase III holoenzyme (Enzyco) subunits α (130 k), τ (71.1 k) and β (40.5 k) are indicated with arrows on the right hand side of the gel (Figure 6). A 130 kDa protein eluted in parallel to the activity profile (Figure 6). A 43 kDa protein eluted later and did not parallel the eluted activity; this protein is presumably a contaminant. Two additional proteins of 63±6 and 50±5 kDa eluted roughly in parallel with the 130 kDa protein, providing candidates for polymerase III-associated proteins, possible subunits of a *T. thermophilus* pol III holoenzyme. The candidate α and two apparently comigrating proteins of 63 kDa and 50 kDa comprised approximately 50% of the protein resolved on an SDS gel. The 63 kDa protein chromatographed in parallel with the polymerase. The 50 kDa protein was more strongly represented toward the early portion of the peak. These data suggest that the 63 kDa and 50 kDa bands correspond to the *T*. *thermophilus* τ and γ subunits, respectively.

Rigorous quantitation of the extent of purification of *T*. *thermophilus* polymerase III was not possible since the gap-filling assay used was not specific for the polymerase III holoenzyme. However, it is estimated that the *T. thermophilus* polymerase III was purified approximately 50,000-fold, assuming all of the DNA polymerase III activity was recovered in Fraction II and it represented 10% of the Fr II polymerase activity. The specific activity of purified *T. thermophilus* pol III in Fraction V was 800,000 units/mg at 60°C. The specific activity of pure *E. coli* pol III is known to be 2.5 x 10⁶ at 30°C (Kim and McHenry, J. Biol. Chem., 271:20681-20689 [1996]). Thus, after correction for the mass of contaminants and the associated τ and γ proteins, the expected specific activity of *T. thermophilus* pol III core was estimated to be approximately 2 x 10⁶ (*i.e.,* close to the activity of its *E. coli* counterpart when compared at temperatures slightly below their optimal growth temperature).

Fractions containing the highest polymerase specific activity (28-36) as determined from the gap-filling polymerase activity profile of Figure 5 were pooled (Fraction V) and solid ammonium sulfate was added to 75% (w/v).

### H. Enzyme Activity of T. thermophilus Fraction V as a Function of Temperature

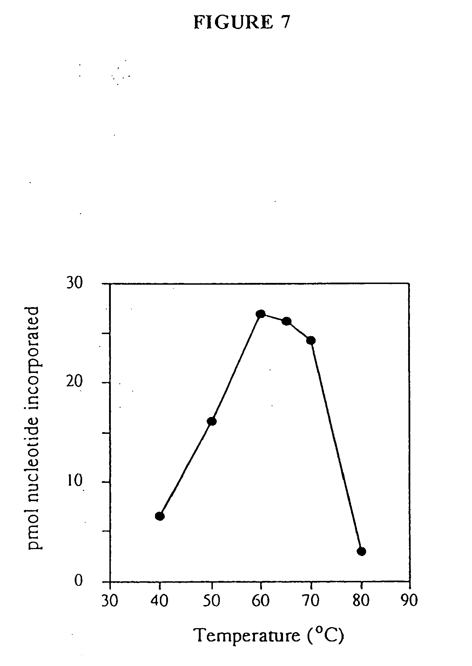

In order to determine the stability of the *T. thermophilus* DNA polymerase III Fraction V, the DNA polymerase activity of the purified *T. thermophilus* DNA polymerase Fraction V was measured as a function of temperature. Polymerase activity was measured using the gap-filling assay described above with the exceptions that the incubation temperature was at selected temperatures between 37°C to 80°C and 0.14 µg of Fraction V protein. Polymerase activity (pmol TTP incorporated in 5 minutes) was plotted against the reaction temperature (Figure 7). The results shown in Figure 7 demonstrate that the gap-filling activity of the purified DNA polymerase complex (Fraction V) has a broad temperature optimum between 60-70°C.

### EXAMPLE 3

### Amino Acid Sequencing of the Purified T. thermophilus DNA Polymerase III τ and γ Proteins

The τ and γ subunits of *E. coli* DNA polymerase III holoenzyme are both encoded by the same gene, *dnaX.* The γ subunit (47.4 kDa) arises as a result of translational frameshifting which yields a truncated version of the τ (71.0 kDa) subunit. Both proteins have identical amino acid sequences at the N-terminus.

The SDS-PAGE analysis of the Q-Sepharose eluant (described in Example 2 and Figure 6) showed that at least 2 other proteins of 63 kDa and 50 kDa, eluted together with the *T. thermophilus* α subunit. As with all other molecular weight values herein reported based on SDS-PAGE, these molecular weight values are approximate values. These molecular weights correspond closely to those of the τ and γ subunits of the *E. coli* DNA polymerase III holoenzyme. To confirm that the 63 kDa and 50 kDa proteins which co-eluted with the *T. thermophilus* α subunit corresponded to the τ and γ subunits of a DNA polymerase III holoenzyme, the 63 and 50 kDa proteins were subjected to N-terminal amino and internal amino acid sequence analysis as follows.

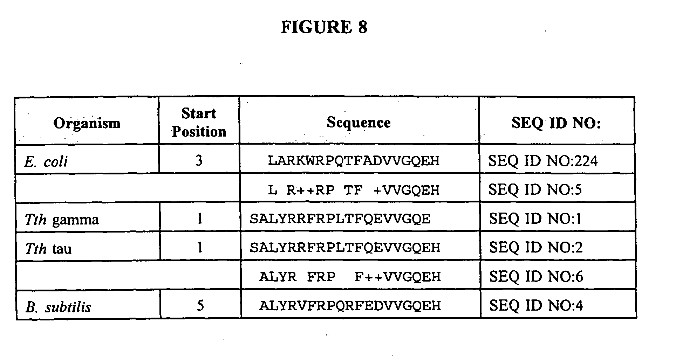

For N-terminal amino acid analysis, 120 µg of Fraction V was fractionated by SDS-PAGE (10%) and transferred to a Hyperbond PVDF membrane as described above. The protein bands at 50 kDa and 63 kDa were excised and subjected to N-terminal amino acid sequencing in an 477 Protein Sequence according to the manufacturer's (ABS, Inc.) instructions. The sequence (SEQ ID NO:1) of the 19 N-terminal amino acids of the 50 kDa *T. thermophilus* protein was identical to the first 19 residues of the 20-amino acid sequence (SEQ ID NO:2) obtained for the 63 kDa protein (*i.e.*, this is consistent with both being the product of the same gene) (*See,* Figure 8). The sequenced regions were 67% identical to the homologous 18-residue *E. coli* DnaX sequence (SEQ ID NO:3), the only bacterial DnaX protein that has been directly characterized on the basis of activity, and 74 % identical to the homologous 19-residue sequence within *B. subtilis* DnaX (SEQ ID NO:4). In Figure 8, homologous regions are also indicated (SEQ ID NOS:5 and 6) between the *T. thermophilus, E. coli,* and *B. subtilis* sequences.

The association of a *T. thermophilus* DnaX homolog with a protein that cross reacts with anti-pol III α monoclonal antibodies and which has the same molecular weight as the *E. coli* pol III α subunit in a 50% pure protein preparation establishes the existence of a multi-subunit pol III holoenzyme in *T. thermophilus.* Furthermore, the results demonstrate that the 63 and 50 kDa proteins are the *T. thermophilus* γ and τ subunits of pol III holoenzyme.

In addition, the 63 kDa band was blotted and treated with Lys-C as described above and an internal peptide was sequenced to yield the sequence ARLLPLAQAHFGVEEVVLVLEGE (SEQ ID NO:88). This peptide did not exhibit detectable homology (*i.e*., no hits were identified in a BLAST search of the entire NR database) with known DnaX sequences), but was important for confirming the correctness of the DNA sequence of the *Thermus thermophilus dna*X gene in subsequent experiments described below.

### EXAMPLE 4

### Cloning and Sequencing The T. Thermophilus dnaX Gene

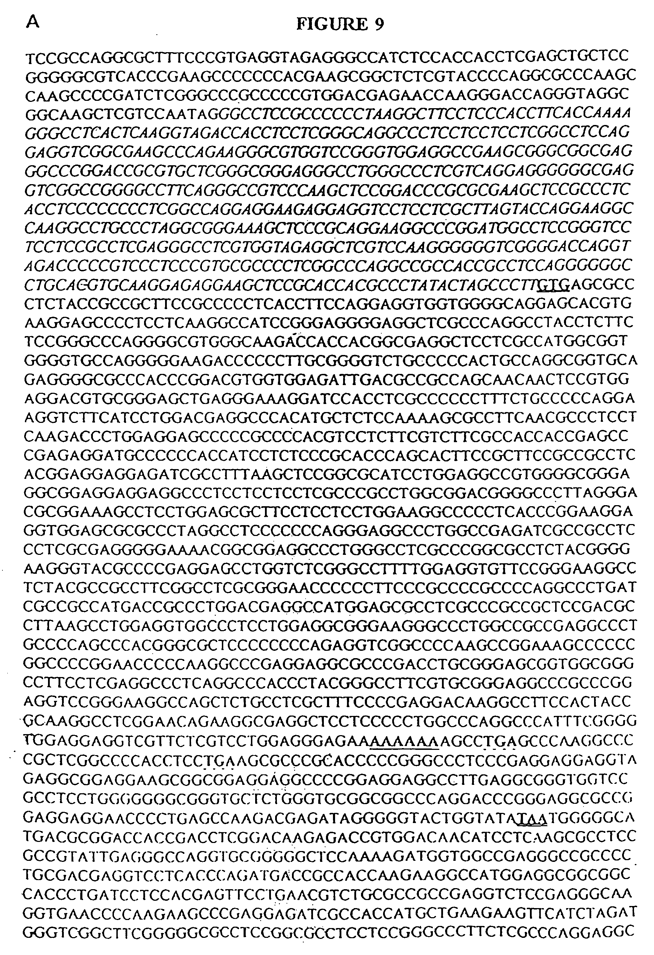

In order to determine whether the 63 kDa and 50 kDa proteins are products of the *T. thermophilus dna*X gene, the structural genes were isolated by a reverse genetics approach using PCR to obtain a fragment of the gene. The gene fragment was used as a probe to obtain the full length of *T. thermophilus dna*X*,* enabling its full sequence to be determined. From the N-terminal sequence, a peptide was selected, FQEVVGQ (SEQ ID NO:89), that would provide a PCR primer with the least degeneracy with all possible codons represented (512-fold). To provide an internal primer, a KTLEEP (SEQ ID NO:90) amino acid sequence common to *E. coli, B. subtilis* (Carter et al., J. Bacteriol., 175:3812-3822 [1993]); and O'Donnell et al., Nuc. Acids Res. 21:1-3 [1993]) and many other eubacteria was exploited. From this consensus sequence a 17-mer primer with 256-fold degeneracy was designed that included all possible combinations of codons. A PCR fragment of the predicted size (388 nucleotides) was obtained. This fragment was close to the spacing of the corresponding regions in the *E. coli dna*X gene (393 nucleotides). The PCR fragment was also highly homologous to *dna*X genes in the fragment between the regions corresponding to the PCR primers, eliminating the bias imposed by primer selection. The PCR-generated probe led to the isolation to a full length gene (Figure 9A) that is highly homologous to other eubacterial *dna*X genes. The deduced amino acid sequence was 42% identical to the corresponding segment of the *B.subtilis dna*X gene. These steps are discussed in detail below.

This example involved (A) Preparation of *T. thermophilus* genomic DNA; (B) Isolation of *T. thermophilus dna*X probe by PCR; (C) Cloning PCR-amplified *dna*X probe; (D) Restriction enzyme digestion of *T. thermophilus* genomic DNA; (E) Southern blotting; (F) Cloning the 7.1 kb Pst1 fragment; and (G) Colony hybridization and sequencing *dna*X*.*

### A. Preparation of T. Thermophilus Genomic DNA

*Thermus thermophilus* strain pMAF.kat (obtained from J. Berenguer; See,Lase *et al,* [1992]) genomic DNA was prepared using previously described methods (Ausubel et al., Current Protocols in Molecular Biology, John Wiley & Sons, New York NY [1995]). Briefly, *T. thermophilus* strain pMAF.kat was grown overnight at 70°C from a single colony in 100 ml of *Thermus* rich media (8 g/l Bacto-tryptone (Difco), 4 g/l yeast extract (Difco), 3 g/l NaCl (Fisher Scientific) (pH 7.5) with vigorous shaking in a New Brunswick Model G25 incubator. The culture was centrifuged in a 250 ml centrifuge bottle in a Sorvall GSA rotor at 6000 rpm (5858 x g) for 6 minutes, followed by resuspension of the pellet from a 100 ml overnight culture in 9.5 ml TE buffer (pH 7.5) in an SS34 centrifuge tube (Sorvall). 0.5 ml 10% SDS and 50 µl of fungal proteinase K (20 mg/ml in dH₂O) (GibcoBRL) was mixed with the suspension, the mixture was incubated at 37°C for 1 h, followed by addition of 2.35 ml of 4M NaCl (Fisher Scientific), and 1.6 ml of cetyltrimethylammonium bromide (CTAB)/NaCl solution (10% CTAB in 0.7 M NaCl). The resulting mixture was incubated at 65°C for 20 minutes followed by extraction with an equal volume of chloroform/isoamyl alcohol (30:1 mixture). The mixture was centrifuged in an SS34 rotor (Sorvall) for 10 minutes at 6000 x g, 0.6 volumes of isopropanol (Fisher) was added to the pellet to form a stringy, white precipitate. The stringy white precipitate was placed into 25 ml 75% ethanol, centrifuged for 5 minutes at 10,000 x g and the supernatant discarded. The pellet was resuspended in 4 ml of TE buffer (pH 7.5), the DNA concentration measured by determining the absorbance at a wavelength of 260 nm in a spectrophotometer, and the DNA concentration adjusted to 100 µg/ml with TE buffer (pH 7.5). 4.3 g of cesium chloride (CsCl) (Sigma cat. # C-4036) and 200 µl of ethidium bromide (10 mg/ml) were added per 4 ml of TE buffer (pH 7.5) used to resuspend the DNA pellet. The mixture was centrifuged in a Sorvall T-1270 rotor in a Sorvall RC70B ultracentrifuge at 55,000 rpm, at 15°C overnight. The chromosomal DNA band (highlighted with UV light) was removed, the ethidium bromide extracted by adding an equal volume of H₂O saturated butanol, mixing thoroughly by inverting the tube, and briefly centrifuging the tube (30 seconds at 14,000 g) to separate the phases. The butanol extraction was repeated until pink color could not be detected, and then the extraction was repeated one more time. The remaining DNA/cesium chloride/TE buffer (pH 7.5) mixture was dialyzed overnight in 500 x volume of TE buffer (pH 7.5) to remove the cesium chloride. DNA was precipitated by the addition of 1/10 volume of 2.5 M sodium acetate (pH adjusted to 5.2 with glacial acetic acid) and 1 volume of isopropanol, and centrifugation at 11,951 x g for 10 minutes. The pellet was washed with 75% ethanol and centrifuged at 11,951 x g for 2 minutes. The supernatant was removed, and the wash repeated. After the final wash, the supernatant was removed and the pellet allowed to dry until it became slightly translucent. The pellet was resuspended in 25 ml of TE buffer (pH 7.5) by gentle rocking overnight at room temperature. DNA was quantitated by spectrophotometry by taking a spectrum of wavelengths from 220 nm to 340 nm. Two preparations were obtained. Preparation A diluted 1/70 in TE buffer gave an OD₂₆₀ of 0.175 consistent with a DNA concentration of 0.612 mg/ml using as a conversion factor A₂₆₀=1=50 µg/ml. Preparation B, with a DNA concentration of 0.906.

### B. Isolation of T. thermophilus dnaX Probe by PCR

An amino-terminal peptide sequence of *T. thermophilus* that showed homology to DnaX from *E. coli, B. subtilis,* and *H. influenza* was used to design oligonucleotide primers which hybridized to the amino-terminal region of the putative *T. thermophilus dna*X*.* Two oligonucleotide primers (X1Fa & XIFb) were designed from the N-terminal peptide sequence to keep codon degeneracy at 512-fold. To design primers for regions located downstream from the N-terminal, conserved regions in DnaX were identified by comparing known sequences from bacteria and bacteriophage. These conserved regions were used to design primers that, with the amino terminal primer, could be used to amplify a portion of the *T. thermophilus dna*X gene by PCR.

A sequence which was homologous to DnaX from *E. coli, B. subtilis,* and *H. influenza-*had the sequence Ser Ala Leu Tyr Arg Arg Phe Arg Pro Leu Thr Phe Gln Glu Val Val Gly Gln Glu (SEQ ID NO:102). The underlined amino acids were chosen for designing an oligonucleotide primer in order to give a primer of sufficient length with minimal degeneracy. The N-terminal primers were 20-mers with 512-fold degeneracy, *i.e.,* forward primer (X1Fa) [5'-TTY CAR GAR GTN GTN GGW CA-3' (SEQ ID NO: 21)] and forward primer (X1FB) [5'-TTY CAR GAR GTN GTN GGS CA-3' (SEQ ID NO:91)].

The conserved DnaX amino acid sequences for design of reverse primer X126R were as follows:

| | | | | | | | | | | | | | | | | |
|---|---|---|---|---|---|---|---|---|---|---|---|---|---|---|---|---|
| *E. coli* | K | V | Y | L | I | **D** | **E** | **V** | **H** | **M** | **L** | **S** | R | H | S | F |
| *B. subtilis* | K | V | Y | I | I | **D** | **E** | **V** | **H** | **M** | **L** | **S** | I | G | A | F |
| *H. influenzae* | K | V | Y | L | I | **D** | **E** | **V** | **H** | **M** | **L** | **S** | R | H | S | F |
| 40 kDa subunit (human RFC) | K | I | I | I | L | **D** | **E** | **A** | **D** | **S** | **M** | T | D | G | A | Q |
| Consensus | | | | | | **D** | **E** | **A/V** | **H** | **M** | **L** | | | | | |

These above sequences correspond to SEQ ID NOS:92-96. The 17-mer, 128-fold degeneracy reverse primer (X126R) had the sequence 5'-ARC ATR TGN RCY TCR TC-3' (SEQ ID NO:97).

The conserved DnaX amino acid sequences for design of reverse primer X139R were as follows:

| | | | | | | | | | | | | | | | | |
|---|---|---|---|---|---|---|---|---|---|---|---|---|---|---|---|---|
| *E. coli* | S | F N | | A | L | L | **K** | **T** | **L** | **E** | **E** | **P** | P | E | H | V |
| *B. subtilis* | A | F | N | A | L | L | **K** | **T** | **L** | **E** | **E** | **P** | P | E | H | C |
| *H. influenzae* | A | F | N | A | L | L | **K** | **T** | **L** | **E** | **E** | **P** | P | E | Y | V |
| Consensus | | | | | | | **K** | **T** | **L** | **E** | **E** | **P** | | | | |

The above sequences correspond to SEQ ID NOS:98-101. The 17-mer, 256-fold degeneracy reverse primer (X139R) had the sequence 5'-GGY TCY TCN ARN GTY TT-3' (SEQ ID NO:22).

For PCR reactions, the Boehringer Mannheim Expand™ long template PCR system was used and following the manufacturer's recommendations except as noted in the following. Reactions were conducted in Boehringer Expand™ buffer 1 supplemented with 0.5 mM extra Mg⁺⁺. The annealing steps were conducted at 48°C, elongation at 68°C and the melting step at 94°C; 26 total cycles were run. Products were separated on 2% FMC Metaphor agarose gels, visualized by Sybr green-1 staining, extracted and cloned into vector pCRII (Invitrogen).

Six µl of gel loading dye was added to each 30 µl PCR reaction described above. Thirty µl of this mixture was loaded into wells of a 2% Metaphor gel in 1x TAE and subjected to gel electrophoresis at 250 volts for 4 hours.

Using the *dna*X primers X1Fa and X139R, and *T. thermophilus* chromosomal DNA preparation A, approximately 10 bands were observed that ranged in size from 350 bp to greater than 1500 bp. Half of these bands were of equal or greater intensity than a band observed at 392 bp as estimated from the gel. Subsequent cloning and sequencing (described below) of the 392 bp gel band showed that this band was 388 bp.

Using *dna*X primers X1Fa and X126R, approximately 18 bands were observed which ranged in size from 100 bp to greater than 1500 bp. Approximately half of the bands were of equal or greater size than the desired 357 bp band. A 329 bp band was selected for cloning as described below.

With the *dna*X primers X1Fb and X126R, approximately 15 bands were observed. These included a band that migrated to the same position as the above described 329 bp product from X1Fa and X126R.

With the X1Fb and X139R primers, approximately 20 bands were observed, with one band at approximately 420 bp appearing near the desired product. However, since the 392 bp product from the X1Fa and X139R primer pair appeared more promising, the 420 bp product was not further pursued.

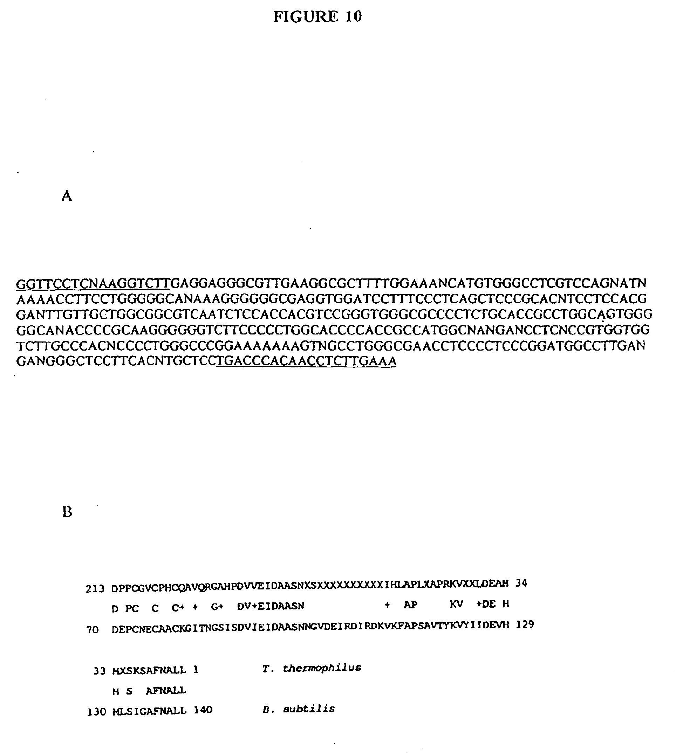

A plasmid isolate (pMGC/1FA-139A; DMSO 1327) containing an *Eco*RI fragment of the predicted size based upon the distance between the homologous region of the *E. coli dna*X gene (approximately 393 bases) was sequenced (Figure 10). In Figure 10A, the sequences corresponding to primers are underlined. The DNA sequence shown in Figure 10A is the complement of the message strand. The sequence shown is of relatively low quality (*i.e.,* approximately 90% estimated accuracy), yet a BLAST search revealed strong homology to bacterial *dna*X genes. *B. subtilis* (shown in the lower line of the alignment of Figure 10B), showed 42% identity over a 71 amino acid stretch. In Figure 10B, the upper peptide sequence is *T. thermophilus.* For, *B. subtilis* the numbers refer to *Dna*X amino acid residues; numbers for *T. thermophilus* refer to the first base of the anticodon for the shown amino acid residue with base #1 being assigned to the C to the left of the corresponding primer sequence near the 3' end of the sequence shown (Figure 10A). In Figure 10, the string of "X"s indicate a defect in the printout from the reported NIH BLAST alignment.

### C. Cloning PCR-Amplified dnaX Probe

PCR-amplified DNA which contained regions encoding the τ and γ subunits of *T. thermophilus* DNA polymerase III was cloned into a modified pCRII vector (Invitrogen) which was used to transform DH5α *E. coli,* and the plasmids containing the PCR-amplified DNA were analyzed by DNA sequencing. These steps are described in more detail in the following sections.

### 1. Cloning of PCR-Amplified DNA Into E. coli

PCR-amplified sequences were extracted from gel slices prepared as described above using freeze-squeeze extraction. Extraction was performed by placing the gel slice in a sterile micro-spin device (0.45 mm pore size cellulose acetate filter (LSPI) that was then inserted into a 2.0 ml Eppendorf tube and the assembly was placed in a -80°C freezer for 10 minutes. The following steps were performed at room temperature. The frozen gel slice was then centrifuged in the micro-spin assembly for 4 minutes at maximum rpm in a microcentrifuge (14,000 x g). The crushed gel slice was then resuspended in 100 µl of 2.5 M Na Acetate, re-frozen for 10 minutes at -80°C, and centrifuged as before. The liquid containing the gel slice DNA was collected in the bottom of the Eppendorf tube (approximately 300 µl) and the DNA was precipitated by the addition of 1 µl glycogen (Boehringer Mannheim) and an equal volume (300 µl) of isopropanol. The precipitate mixture was centrifuged (14,000 x g) in a microcentrifuge for 10 minutes, the supernatant discarded, and the pellet washed with 75% ethanol. The ethanol wash supernatant was discarded, and the pellet was resuspended in 5 µl TE buffer (pH 7.5). The DNA was quantitated by adding 1 µl of DNA to 300 µl of a 1/400 dilution in TE buffer (pH 7.5) of PicoGreen™ (Molecular Probes) in a well of a 96-well microtiter plate (Life Sciences), and compared to known concentrations of *Thermus thermophilus* genomic DNA by excitation on an SLT Fluostar Microplate Fluorometer of the PicoGreen™ at 480 nm and measuring emission at 520 nm.

DNA that PCR-amplified by the Expand High Fidelity enzyme mix had a 1 nucleotide addition (dATP) that allows a compatible cohesive annealing to a vector (pCRII vector, Invitrogen) with a 1 nucleotide T overhang. Fifty or 150 pmoles of PCR amplified DNA was added to 50 pmoles of vector DNA in a 10 µl reaction mixture containing 1 µl of 10x Ligation Buffer (Invitrogen), 1 µl of T4 DNA ligase (1 Weiss unit/µl, GibcoBRL), and sterile dH₂O to bring the volume to 10 µl. Ligation was carried out at 14°C overnight. The DNA in the ligation mixture was precipitated by the addition of 1 µl of glycogen (20 mg/ml, Molecular Biology Grade, Boehringer Mannheim), 1/10 volume (1 µl) 2.5 M sodium acetate (pH 5.2), and 2.5 volumes of ethanol. The precipitate was recovered by centrifugation at 14,000 x g for 10 minutes, the supernatant was removed, and the pellet washed with 0.3 ml of 75% ethanol. The washed pellet was re-centrifuged at 14,000 x g for 2 minutes, the supernatant removed completely with a fine tipped pipet, and the pellet was resuspended in 5 µl of dH₂O. The DNA was used to transform electrocompetent DH5α *E. coli* by electroporation as described below.

Electrocompetent DH5α E. *coli* cells were prepared by picking an isolated colony of DH5α (Gibco-BRL) and growing overnight in 10 ml SOP media. 1 ml of overnight culture was added to 500 mL SOP media, grown to an OD₆₀₀ reading of 0.6-0.8. The culture was placed immediately in an ice water bath to chill rapidly and left on ice for least 15 min. The following steps were carried out in the cold room at 4°C. Cells were centrifuged at 4000 rpm for 15 min, washed with 500 mL ice cold dH₂O and allowed to sit on ice for 30 min. before spinning down in 500 mL centrifuge bottles at 4000 rpm for 15 min. The pellet was washed with 500 ml ice cold dH₂O, spun down in 500 mL centrifuge bottles at 4000 rpm for 15 min. and the pellet resuspended in 20 mL 10% glycerol. The cells were spun down in a 50 mL tube at 5000 rpm for 10 min., resuspended in approximately 0.5 mL 10% glycerol, and 0.2 mL aliquots of competent cell preparation frozen quickly in 1.7 mL centrifuge tubes by placing in liquid nitrogen, then storing at -80°C.

For electroporation, electrocompetent cells were transformed as follows. Cells were removed from the -80°C freezer and thawed on ice. 40 µl of electrocompetent cells were placed into a 1.5 ml Eppendorf tube (pre-chilled for at least 5 minutes on ice) and 2 µl of the dH₂O re-suspended ligated DNA (ligated DNA + 5 µl H₂O) was added and allowed to sit for 5 minutes. The transformation mixture was removed from the 1.5 ml Eppendorf tube and added to pre-chilled electroporation cuvettes (0.2 cm electrode gap, BioRad). The electroporation was performed in an Eppendorf Electroporator 2510 at 2500 V. Then, 1 ml of SOP media was immediately added to the cuvette following electroporation, and the electroporated cells in SOP media were transferred to a capped tube and incubated for 1 h with shaking at 37°C. After 1 hour, aliquots of 2 x 200 µl, 1 x 50 µl, and 1 x 5 µl were plated by spreading onto LB plates containing carbenicillin (50 µg/ml), and incubated overnight at 37°C in a convection heat incubator.

### 2. Restriction Digest Analysis of Plasmids

Plasmids were analyzed for the presence of insert by restriction digestion with *Eco*RI restriction endonuclease. *Eco*RI restriction sites flank insert DNA in the pCRII vector system. For *Eco*RI restriction digestion, 5 µl of Promega Plus Miniprep-isolated plasmid (from 50 µl total) was added to a 10 µl reaction with 1 µl of 10 High Salt reaction buffer (10x H buffer, Boehringer Mannheim), 1 µl of *Eco*RI restriction endonuclease (10 units/µl, GibcoBRL), and 3 µl of dH₂O. The mixture was incubated at 37°C for 2 hours. 2 µl of 6x gel loading dye was added, and all 12 µl of the reaction and dye were electrophoresed in 0.7 % SeaKem GTG agarose (FMC) in 1x TAE for 4 hs at 80 V constant voltage. The presence of a gel band of the expected size confirmed the presence of the insert in the plasmid. Using primers XFIb and X136R, seven colonies were selected for further screening. Six had inserts of approximately the correct size. One (clone A) was selected for sequencing (DMSO 1327). Six colonies were also obtained with the X1Fa-X126R primer pair. The longer X139R-derived sequences were pursued since they were longer.

### 3. Large Scale Plasmid Purification

Large scale plasmid purification was performed in order to obtain enough DNA for sequencing, restriction digestion, etc. Large scale (*i.e*., 50 ml) plasmid purification was performed using Qiagen plasmid preps. An isolated *E. coli* colony was picked from an LB -carbenicillin (70 µg/ml) plate and grown overnight in 50 ml of SOP media. The culture was centrifuged at 12,000 x g in a Sorvall SS34 rotor for 10 minutes in a 50 ml polypropylene tube. The supernatant was discarded. The pellet was resuspended in 4 ml Buffer P1 (50 mM Tris (pH 8.0), 10 mM EDTA, 100 µg/ml Rnase A). 4 ml of Buffer P2 (200 mM NaOH, 1% SDS) was added, mixed gently by inverting the tube 4 to 6 times, and the mixture incubated at room temperature for 5 min. 4 ml of chilled Buffer P3 (3.0 M potassium acetate (pH 5.5)) was added, mixed immediately but gently, the mixture incubated on ice for 15 min., centrifuged at approximately 20,000 g for 30 min at 4°C and the supernatant removed promptly. The supernatant was centrifuged at approximately 20,000 x g for 15 min at 4°C, and the resulting supernatant promptly removed and applied to a Qiagen-tip 100 (Qiagen) which had been previously equilibrated by applying to the resin 4 ml of buffer QBT (750 mM NaCl, 50 mM MOPS (pH 7.0), 15% ethanol, 0.15% Triton X-100). The supernatant was allowed to flow through the resin under gravity. The Qiagen-tip 100 was washed with 2 x 10 ml of Buffer QC (1.0 M NaCl, 50 mM MOPS (pH 7.0), 15% ethanol), and the DNA eluted with 5 ml Buffer QF (1.25 M NaCl, 50 mM Tris-HCl (pH 8.5), 15% ethanol). The eluate was collected in a 12 ml centrifuge tube and the DNA precipitated with 0.7 volumes of isopropanol at room-temperature, and centrifuged immediately at approximately 15,000 x g for 30 min at 4°C. The DNA pellet was washed with 2 ml of 70% ethanol, air-dried for 5 min, and re-dissolved in 500 µl of dH₂O. DNA was quantitated by spectrophotometry over the optical range of 220 nm to 340 nm by blanking against 700 µl dH₂O followed by the addition 10 µl of DNA into the 700 µl dH₂O for the measurement. The ratio of OD at 260 nm to OD at 280 nm was between 1.8 and 2.0. Typically, 5 µg was obtained from a 50 ml culture. The above procedure was used when plasmids were sequenced or used for further cloning steps. The alternative DNA preparation method provided herein was used just for screening methods.

### D. Restriction Enzyme Digestion of T. thermophilus Genomic DNA

Since a large probe that was identical to the gene had been prepared, a directed approach was used to clone the full length *T. thermophilus dna*X rather than screening of a full library. A Southern blot against digested *T. thermophilus* DNA was performed in order to detect a restriction fragment that was large enough to contain the entire *T. thermophilus dna*X gene. This fragment was extracted, cloned, and the resulting colonies were screened by colony hybridization and a candidate clone selected for sequencing. These steps are described as follows.

Restriction endonuclease digests of *Thermus thermophilus* DNA were carried out in order to run the resulting digests on agarose gels and to blot for Southern analysis using PCR-amplified fragments which had been cloned in *E. coli* as described above. Restriction digestion was carried out at 37°C overnight in a 100 µl reaction volume containing 20 µl of genomic DNA (18 µg preparation B chromosomal DNA), 10 µl 10X restriction endonuclease buffer (NEB), 5 µl restriction endonuclease (25 to 50 Units)(NEB), and 65 µl dH₂O. An additional 5 µl of restriction endonuclease was then added and the mixture incubated at 37°C for an additional 2 hours. The digest was precipitated with 1 µl glycogen (20mg/ml, Boehringer Mannheim), 1/10 volume 2.5 M sodium acetate (pH 5.2), 3 volumes of ethanol and centrifuged for 30 minutes at 14,000 x g. The supernatant was removed and the pellet washed with 0.5 ml 75% ethanol prior to resuspension of the pellet in 40 µl TE buffer (pH 7.5).

The prepared genomic DNA was run on agarose gels together with 60,000 cpm of ³²P end-labelled DNA Molecular Weight Marker VII (Boehringer Mannheim) and 1.75 µg (7 µl) of DNA Molecular Weight Marker VII (Boehringer Mannheim). End-labeled molecular weight markers were prepared by incubating an end-labeling reaction mixture [8 µl of DNA Molecular Weight Standard (2 µg, Boehringer Mannheim), 8 µl dH₂O, 3 µl 0.5M Tris (pH 7.5), 0.3 µl 1M MgCl₂, 0.3 µl 1M DTT, 1 µl (2 units/µl), 1.5 µl 1.5 mM each of dCTP, dGTP,and dTTP, 8 µl ³²p-dATP (80 µCi, 3000 Ci/mmole in 5 mM Tris-HCl (pH 7.5), for 1 hour at 37°C. To this mixture was added 2 µl glycogen (20 mg/ml, Boehringer Mannheim), 1/10 volume 2.5 M sodium acetate (pH 5.2) with glacial acetic acid, 3 volumes of ethanol and the resulting mixture was centrifuged in a microcentrifuge at 14,000 x g for 5 minutes, the supernatant discarded and the pellet washed with 400 µl of ethanol. The pellet was resuspend in 50 µl TE buffer (pH 7.5), 1 µl was spotted onto a 24 mm diameter GF/C filter (Whatman), the filter paper dried, and counted with 5 ml scintillation fluid in a scintillation counter, yielding DNA of approximately 10⁸ cpm/µg.

To 10 µl of digested genomic DNA (3 µg) was added 2 µl of gel loading dye and the mixture run on a 0.7% Seakem GTG (FMC) agarose gel in 1x TAE for 14.2 hours, at room temperature, at 35 V constant voltage. The gels were stained for 1 h with gentle rocking with Sybr green in 1X TAE.

### E. Southern Blotting

Southern blotting of *T. thermophilus* genomic DNA with PCR-amplified probes was performed by linking genomic DNA to a positively charged nylon membrane for colony and plaque hybridization (Boehringer Mannheim) using an alkaline transfer buffer as previously described (Ausubel FM *et al.* [1995] *supra*). Briefly, the gel was rinsed with distilled water, and the gel gently shaken with 10 gel volumes of 0.4 M NaOH on a platform shaker for 20 min. The positively charged nylon membrane was placed without prewetting directly onto the gel and alkaline transfer was carried out in 0.4 M NaOH. Since alkaline transfer is quicker than high-salt transfer, the blot could be taken apart any time after 2 h, but the blot was generally performed overnight. The membrane was rinsed in 2X SSPE, and allowed to air dry in order to remove agarose fragments that may adhere to the membrane and to neutralize the membrane. The membrane filters were baked at 80°C in a vacuum oven and were ready to use for hybridization with a labeled probe.

### 1. Preparation of Probe

Probe DNA was prepared from vectors (pCRII, Invitrogen) containing genomic DNA segments which encode portions of subunits of the *Thermus thermophilus* DNA polymerase III holoenzyme subunits, as described above. The gene segments were released from the vector by digestion of 10 µg of the cloned DNA's in a 300 µl reaction with EcoRI (100 Units, Gibco BRL) in 1x Boehringer Mannheim High Salt restriction digest buffer H at 37°C for 2 hours 60 µl of gel loading dye was added to each digest, the entire 360 µl digest was loaded into 260 x 1.5 mm wells, and the digests were electrophoresed in 4% NuSieve GTG agarose (FMC) in 1x TAE buffer, and the gel was run at 100 V (constant voltage) for 4 hours. The gel was stained with 300 ml Sybr green (Molecular Probes, diluted 1/10,000 in 1x TAE) for 1 h with gentle rocking. Bands containing the gene fragments were excised with a clean scalpel, placed into a tared 4 ml Nunc tube (Nunc 4.5 ml Cryotube, NUNC), and the bands were weighed. The slices were melted by putting the tubes in a gravity convection incubator at 72°C for 30 minutes or until the slice was completely melted. Once melted the tubes were cooled to 45°C in a temperature block and 1/10 volume of 10x β-agarase buffer (Gibco BRL; 100 mM Bis-Tris (pH 6.5), 10 mM EDTA) was added, followed by the addition of 4 µl of β-Agarase (GibcoBRL, 1 unit/µl). The mixture was incubated overnight in a gravity convection incubator at 42°C. To precipitate the DNA, 1/10 vol. of 3 M NaOAc (pH 5.2), was added and the mixture chilled on ice. Any remaining undigested agarose was removed by centrifugation for 2 minutes in 2 ml Eppendorf tubes at 14,000 x g in an Eppendorf microcentrifuge. The supernatant was removed and the DNA precipitated by the addition of 1 ml glycogen (Boehringer Mannheim, molecular biology grade, 20 mg/ml) and 2.5 volumes of ethanol, followed by centrifugation at 14,000 x g in an Eppendorf microcentrifuge for 10 minutes. The supernatant was removed and the pellet washed with 0.5 ml of 75% ethanol and centrifuged at 14,000 x g in an Eppendorf microcentrifuge for 2 minutes. The pellets were resuspended in 10 µl of a 1/10 dilution of TE buffer (1 mM Tris (pH 7.5), 0.1 mM EDTA final concentration). The DNA was quantitated by adding 1 µl of DNA to 300 µl of a 1/400 dilution of PicoGreen™ in a well of a 96-well microtiter plate (Life Sciences), and compared to known concentrations of *Thermus thermophilus* genomic DNA by excitation of the PicoGreen™ at 480 nm and measuring emission at 520 nm, on an SLT Fluostar Microplate Fluorometer.

### 2. Labelling of Probe

The probe was labeled by adding 50 ng of DNA to a random hexamer priming reaction mixture (Random Primed DNA Labeling Kit, Boehringer Mannheim) following the manufacture's instructions. Briefly, 50 ng DNA in 10 µl dH₂O was placed into a 0.2 ml thin-walled PCR tube, overlaid with 25 µl mineral oil and the DNA denatured by heating at 95°C for 10 minutes in a thermal cycler (MJ Research). At 9 minutes through the 10 minute denaturation, 2 µl of Boehringer Mannheim hexanucleotide reaction mix (tube 6 in kit) was added and the denaturation continued for an additional minute. Just prior to the end of the 10 minute denaturation, the mixture was rapidly cooled by removing the tube from the thermal cycler and immediately placing it in a beaker of ice-water. One µl each of dATP, dTTP, dGTP (0.5 mM in Tris buffer, final concentration 25 µM) was added to the reaction, followed by 5 µl of ^{α32}P-dCTP (50 µCi, 3000 Ci/mmole, in 5 mM Tris-HCl (pH 7.5)), and 1 µl of Klenow DNA polymerase (Boehringer Mannheim, 2 units/µl in 50% glycerol), and the mixture was incubated at 37°C in a water bath for 1 h.

### 3. Removing Unincorporated Label

The unincorporated label was removed using the PCR Clean-up Kit (Boehringer Mannheim). Briefly, the 20 µl reaction was removed from the mineral oil overlay with a fine-tipped micropipette and the volume adjusted to 100 µl with TE buffer (7.5) in a 0.5 ml Eppendorf tube. 400 1 of the nucleic acid binding buffer was added to the DNA along with 10 µl of the silica suspension mix. The mixture was incubated for 10 minutes at room temperature with frequent vortexing. The mixture was centrifuged for 30 seconds in a microcentrifuge and the supernatant discarded. The matrix containing the DNA was resuspended with 400 µl of nucleic acid binding buffer by vortexing. The mixture was centrifuged and the supernatant discarded as before. The pellet was washed with 400 µl of washing buffer, centrifuged and the supernatant discarded as before. This step was repeated once more. After the final spin the pellet was re-centrifuged and any remnants of supernatant were removed with a fine-tipped micropipette. The pellet was allowed to dry at room temperature for 15 minutes. The DNA was eluted from the silica matrix by the addition of TE buffer (10 mM Tris (pH 8.4), 1 mM EDTA) and incubation at 65°C for 10 minutes with occasional vortexing After centrifugation for 2 minutes at maximum speed in the microcentrifuge, the supernatant was removed to a clean tube and the silica matrix was again re-suspended in 100 µl dH₂O. The elution procedure was repeated and the supernatant combined with the first supernatant to give a final volume of 200 µl. It was then determined that 2 x 10⁷ cpm was incorporated. The estimated specific activity of the DNA is 10⁸⁻⁹ cpm/µg.

### 4. Hybridization

Membranes with bound *Thermus thermophilus* DNA were blocked by incubation with 50 ml blocking fluid (50% formamide, 5x SSPE (20x SSPE comprises 3 M NaCl, 200 mM NaH₂PO₄, 0.02 M Na₂EDTA adjusted to pH 7.4 with NaOH), 1% SDS, and a 1/20 dilution of milk solution (10% skim milk dissolved in dH₂O, 0.2% sodium azide)) at 42°C for at least 2 h in a hybridization bag with spout (Boehringer Mannheim, cat#1666 649). Two µl of the labelled probe was spotted onto 2.4 cm diameter circle GFC filters, placed in 5ml of scintillation fluid in a scintillation tube, and counted in a scintillation counter. The specific activity of the probe should be around 1 x 10⁹ cpm/µg DNA.

The blocking fluid was replaced with 20 ml of fresh blocking buffer fluid. The labeled probe (200 µl as described above in section 3; 1-3 x 10⁷ cpm) was denatured by the addition of 1/5 volume (40 µl) of 0.2 N NaOH and the mixture was incubated at room temperature for 10 minutes. The denatured probe was added to the hybridization bag. Neutralization of this mixture to allow hybridization was accomplished by the addition of 1/5 volume (40 µl) of 2 M Tris-HCl (pH unadjusted). The hybridization mixture was incubated at 42°C with rocking overnight. The filters were washed at least 3x 100 ml each in the bag with 2x SSPE, 0.1% SDS, and placed in a pyrex dish with 500 ml of 0.1x SSPE, 0.1% SDS pre-heated to 42°C for 30 minutes. This wash was repeated 1 more time and the filters were air dried, and exposed to Kodak X-OMAT film or exposed to a phosphorimager plate (Molecular Dynamics) and viewed in a phosphorimager (Molecular Dynamics).

Using as a probe the cloned fragment amplified with dnaX 1Fa and X1Fb primers, Southern blot hybridization showed a 7.1 kb band with genomic DNA digested with *Pst*I. The results of these experiments were 1) The probe hybridized to a 7.1 kb *Pst*I band made by digestion of (22.5 µg of Preparation B chromosomal DNA with 10 µl *Pst*I (100 units), in 25 µl NEB Buffer 3 (NEB) and 190 µl H₂O, overnight at 37°C; and an 8.5 kb band hybridized to the *Hin*dIII digest (22.5µg of Preparation B chromosomal DNA digested with 10 µl *Hin*dIII (100 units) in 25 µl NEB Buffer 2 (NEB) and 190 µl H₂O, overnight at 37°C.

### F. Cloning The 7.1 kb PstI Fragment

PstI-digests of *T. thermophilus* genomic DNA were prepared in order to clone large restriction fragments of a size which was expected to contain the full length *T. thermophilus dna*X gene. The restriction fragments were cloned into a modified pCRII vector (pMGC 707), transformed into *E. coli* and cells containing plasmids that carried *dna*X encoding sequences complementary to probe were identified by a colony hybridization procedure.

### 1. PstI-Restriction Enzyme Digestion of T. Thermophilus Genomic DNA To Prepare the Library

A 250 µl restriction digest reaction mixture was prepared by incubating overnight at 37°C 22.5 µg genomic DNA (Preparation B, prepared as described above), 25 µl of 10 x restriction endonuclease buffer, 10 µl PstI (10 units/µl), and 190 µl dH₂O. Fifty µl of gel loading dye was added to the digest, and the entire 300 µl sample was loaded into a 1.5 mm thickness x 4.3 cm well in a 0.7 % SeaPlaque low melt agarose at 45V constant voltage overnight at 4°C with the DNA Molecular Weight Standards VII (Boehringer Mannheim). The dye front was run until it had reached 2/3 the length of the gel, the gel was stained in Sybr green in 1x TAE for 1 h with gentle shaking. A gel slice that contained at least 2 mm on either side of the region corresponding to a 7.1 kb fragment was excised with a clean scalpel.

The DNA was recovered from the gel by incubating the gel slice at 72°C in a convection heat incubator for 30 minutes or until the gel slice was completely melted. The melted gel slice was placed into a tube which was inserted into a 45°C temperature block and allowed to cool for 5 minutes. Then, 1/10 volume of 10 x β-agarase buffer was added along with 4 µl of β-agarase (1 unit/µl, GibcoBRL) per 200 µl of volume. Digestion was carried out at 42°C overnight in a convection heat incubator and the sample was precipitated by adding 1 µl glycogen (20 mg/ml, Boehringer Mannheim), 1/10 volume of 2.5 M sodium acetate (pH 5.2) with glacial acetic acid, and 1 volume of isopropanol. The precipitate was centrifuged at 14,000 x g for 15 minutes and the supernatant discarded. The pellet was washed once with 1 ml of 75% ethanol, the pellet centrifuged at 14,000 x g for 2 minutes and the supernatant discarded. The pellet was resuspended in 10 µl of millQ de-ionized H₂O.

### 2. Cloning dnaX PstI Fragment into pMGC707

A 7.1 kb fragment that hybridized with the partial *dna*X probe generated by PCR was extracted and cloned into vector pMGC707. pMGC707 was prepared by cutting pCRII (Invitrogen) with *Spe*I and *Not*I and inserting a polylinker resulting from the annealing oligonucleotides and
(5'CTAGTTAATTAACCAGTGCACTGGAAGACGTCATGGAATTCGAACGCGTG CAATTGC) (SEQ ID NO: 104). The polylinker region of the resulting plasmid was cleaved with *Bst*XI to generate *Pst*I compatible termini to receive the extracted 7.1 kb population of fragments. As *Bst*XI does not itself give *Pst*-compatible termini, this vector was designed so that it would give *Pst*I compatible termini.

Vector pMGC707 was cut with *Bst*XI*,* and gel purified to separate linear product from undigested circles. The cut pMGC707 was ligated with a *Pst*I restriction fragment of genomic digest prepared as described above, in a 20 µl Ligation Reaction [2 µl vector DNA (100 ng), 2 µl insert DNA (1000 ng), 2 µl of 10x ligation buffer (Invitrogen), 1 µl ligase (GibcoBRL, 3 units/µl), and 13 µl dH₂O] by incubating for 1 h at room temperature and overnight at 14°C. The reaction mixture was precipitated with 1 µl glycogen (20 mg/ml, Boehringer Mannheim), 1/10 volume of 2.5M sodium acetate (pH 5.2), and 3 volumes ethanol, and washed once with 1 ml 75% ethanol. The resulting plasmids were used to transform DH5α E. *coli* by electroporation as described *supra.* Electroporated cells were incubated for 1 h prior to selection overnight on LB plates containing carbenicillin (50 µg/ml) at 37°C in a convection heat incubator. Transformed cells which grew on carbenicillin-containing plates were ready for colony hybridization to detect the presence of sequences encoding *T. thermophilus* DNA polymerase III holoenzyme subunits. (*i.e.*, "DNA polymerase III holoenzyme" refers to the whole entity, while "DNA polymerase III" is just the core [α, ε, θ]).

### G. Colony Hybridization And Sequencing of T. thermophilus dnaX

Colonies were screened by colony hybridization (Ausubel *et al.* [1995] *supra*) using the *T. thermophilus dna*X PCR-generated probe. Plasmid from positive colonies were purified and those containing 7.1 kb inserts and also a *Bam*HI site as indicated from the sequence of the PCR probe were retained for further characterization. One was submitted for full DNA sequencing (Lark Sequencing Technologies, Houston, TX), resulting in the sequence of the full length gene. These steps are elaborated in detail below.

### 1. Preparation of Replica Filters Containing Transformed Colonies

Replica filters were prepared by filter-to-filter contact with a master filter. A master filter was prepared by placing a sterile dry nylon membrane filter for colony and plaque hybridization (Boehringer Mannheim) onto a one day-old LB agar plate containing carbenicillin (50 µg/ml; Gibco-BRL). The bacteria were applied in a small volume of liquid (<0.8 ml, containing up to 20,000 bacteria for a 137-mm filter; <0.4 ml, containing up to 10,000 bacteria for an 82-mm filter), and spread over the surface of the filter leaving a border 2-3 mm wide at the edge of the filter free of bacteria. The plates were allowed to stand at room temperature until all of the liquid had been absorbed. The plates were inverted and incubated at 37°C until very small colonies (0.1-mm diameter) appeared (about 8-10 hours) on the filter.

Using sterile, blunt-ended forceps (e.g , Millipore forceps), the master filter was gently removed from the first plate and placed on a stack of sterile MC filter paper colony side up. The second, wetted filter was placed on top of the master filter, being careful not to move filters once contact has been made. The filters were pressed together using sterile blotting paper and by applying pressure to a 140 mm petri-dish lid placed on top of the sterile blotting paper. Before filters were peeled apart, 3 to 11 holes were poked through the wedded filters with an 18 gauge hypodermic needle around the perimeter of the filters to aid in orienting the master and replica filters with respect to each other. The filters were gently peeled apart and the filter replica returned to an LB-agar-carbenicillin plate, colony side up, while the master filter was placed back onto an LB-agar-carbenicillin plate.

The plates (containing master filter and replica filter) were incubated at 37°C until colonies 1-2 mm in diameter appeared. Colonies on the master plate reached the desired size more rapidly (6-8 hours). At this stage, while the bacteria were still growing rapidly, the replica filters were transferred to agar plates containing chloramphenicol (170-250 µg/ml) and incubated for a further 8 hours at 37°C. The chloramphenicol plate does not allow growth of the bacterial cell but the plasmid in the cell continues to replicate giving a better signal when the DNA from the colonies is hybridized. The master plates were sealed with parafilm and stored at 4°C in an inverted position until the results of the hybridization reaction were available. Bacteria on the replica filters were lysed and the liberated DNA bound to the replica filters for subsequent hybridization as follows.

### 2. Lysis And Binding Liberated DNA To Filters

Using blunt-ended forceps (*e.g,* Millipore forceps), the replica nylon membrane filter was peeled from the plate and placed for 3 minutes, colony side up, on MC filter paper impregnated with 10% SDS. The filter was transferred to a second sheet of MC paper that had been saturated with denaturing solution (0.5 M NaOH, 1.5 M NaCl) and left for 5 minutes, then to a third sheet of MC paper that had been saturated with neutralizing solution (1.5 M NaCl, 0.5 M Tris • Cl (pH 8.0)) and left for 5 minutes. The filter was allowed to dry, colony side up, at room temperature on a sheet of dry MC paper for 30-60 minutes. The dried filter was sandwiched between two sheets of dry MC paper and baked for 2 hours at 80°C in a vacuum oven prior to hybridization to a ³²P-labeled probe. Colony hybridization with a probe demonstrates that the colony contained plasmids with inserts which contained sequences of *T. thermophilus* DNA that were complementary to the probe.

The hybridization was performed as described for Southern blots. One to two filters were placed in a hybridization bag. If two filters were placed in the bag, the filters were placed back-to-back with the filter surfaces that had not come into contact with the colonies against each other. After completion of the hybridization procedure, the filters were air-dried at least one hour, and the filters were marked with a small dab phosphorescent paint over each of the needle holes. When the filters are exposed to X-ray film, the phosphorescent paint produces a dark spot on the X-ray film that can be used to align the X-ray image with the master filter to identify specific *E. coli* containing plasmids that hybridize to the probe.

The hybridized filters were taped to a piece of Whatman 3 MM paper with Scotch brand Magic tape and the taped filters and Whatman 3MM paper covered with plastic wrap to prevent contamination of the film cassette. In the darkroom, the filters were placed in an X-ray film cassette with an intensifying screen and Kodak XR Omat X-ray film was placed over the top and the cassette sealed. The cassette was placed in a black garbage bag and sealed with tape to act as a secondary barrier to light exposure of the film. The bag was placed overnight at -80°C for exposure of the film.

The film was developed by removing the film in the darkroom, and dipping the film into Kodak developing solution for 3 minutes, followed by a 23 minute soak in cool tap water, and finished by soaking for 3 minutes in fixer (all developing reagents used were Kodak). The film was then rinsed with tap water and allowed to air dry for 15 to 30 minutes.

To align the film with the master filter, the developed X-ray film was placed on a white light box and covered with Plastic wrap. The master filter was identified by the number and orientation of the black spots on the film that aligned with the holes poked in the master filter. The master filter was then carefully removed from the agar plate with sterile forceps and placed on the plastic wrap covered X-ray film to line up the darkened spots on the film with the holes in the master filter. Dark spots on the film were correlated to colonies on the filter, and colonies were picked and struck with a sterile toothpick to LB-agar plates containing carbenicillin at 150 µg/ml.

Isolated colonies from each streak were grown and plasmid isolated as described previously (Promega). For dnaX, 4 strongly hybridizing colonies on one filter from the *Hin*dIII 8.4 to 8.6 Kb fragment clones were chosen for plasmid preparations. Four colonies each from 3 separate filters from the *Pst*I 7.1 Kb fragment clones, (12 total *Pst*I clones) were selected for plasmid preparations. The plasmid DNA from *Hin*dIII and *Pst*I clones was digested with *Bam*HI restriction endonuclease as previously described, and only 1 colony, designated "pAX2-S (DMSO 1386)," showed evidence of an insert. The *Bam*HI digest was repeated on pAX2-S clone, and the restriction pattern gave bands that were roughly estimated against the VII DNA molecular weight standards as 6 Kb,2 Kb, 1.9 Kb, 0.8 Kb. The addition of these fragments gave a plasmid size of around 10.7 Kb, roughly the size of plasmid that would be expected from a 7.1 Kb insert into pMGC707. *Bam*HI restriction endonuclease digests were used because sequence data from the dnaX partial clone obtained by PCR described previously indicated the presence of a *Bam*HI site. This clone was verified to be dnaX by sequencing.

### 3. Restriction Digestion of Clones

In order to confirm the presence of inserts in the plasmids which had been identified as "positives" by colony hybridization, the plasmids were purified and analyzed by restriction enzyme digestion.

### a. Plasmid Purification

Plasmid was prepared and purified from 1-3 ml bacterial culture. Plasmid preparation was performed using a Promega Plus Miniprep technique (Promega). Briefly, 1-3 ml of bacterial cell culture was centrifuged for 1-2 minutes at 10,000 x g in a microcentrifuge, the supernatant poured off and the tube blotted upside-down on a paper towel to remove excess media. The cell pellet was completely resuspended in 200µl of Cell Resuspension Solution (Promega, A711E) (50mM Tris (pH 7.5); 10mM EDTA; 100µg/ml RNase A). 200µl of Cell Lysis Solution (Promega, A712E) (0.2M NaOH and 1% SDS) was added and mixed by inverting the tube 4 times followed by the addition of 200µl of Neutralization Solution (Promega, A713J) (1.32M potassium acetate) and mixing by inverting the tube 4 times. The lysate was centrifuged at 10,000 x g in a microcentrifuge for 5 minutes. If a pellet has not formed by the end of the centrifugation, centrifugation was carried out for an additional 15 minutes.

The plasmid preparation was purified using a vacuum manifold by using ProMega Plus Minipreps (Promega, A721C) which can be easily processed simultaneously with Promega's Vac-Man™ or Vac-Man™ Jr. Laboratory Vacuum Manifold. One ProMega Plus Minipreps column was prepared for each miniprep. The ProMega Plus Minipreps resin (Promega, A767C) was thoroughly mixed before removing an aliquot. If crystals or aggregates were present, they were dissolved by warming the resin to 25-37°C for 10 minutes, then cooling to 30°C before use. One ml of the resuspended resin was pipetted into each barrel of the Minicolumn/Syringe assembly (*i.e.,* the assembly formed by attaching the Syringe Barrels to the Luer-Lok^{®} extension of each Minicolumn), and all of the cleared lysate from each plasmid preparation was transferred to the barrel of the Minicolumn/Syringe assembly containing the resin. A vacuum was applied to pull the resin/lysate mix into the Minicolumn until all of the sample has completely passed though the column. Extended incubation of the resin and lysate was not necessary since at the concentration of plasmid present in most lysates, plasmid binding to the resin was immediate. 320 ml of 95% ethanol was added to the Column Wash solution bottle to yield a Column Wash Solution (Promega, A810E) having a final concentration of 80 mM Potassium acetate, 8.3 mM Tris-HCl (pH 7.5), 40µM EDTA and 55% ethanol. 2 ml of the Column Wash Solution was added to the Syringe Barrel and the vacuum reapplied to draw the solution though the Minicolumn. The resin was dried by continuing to draw a vacuum for a maximum of 30 seconds after the solution has been pulled though the column. The Minicolumn was transferred to a 1.5ml microcentrifuge tube and the Minicolumn centrifuged at 10,000 x g in a microcentrifuge for 2 minutes to remove any residual Column Wash Solution. The Minicolumn was transferred to a new microcentrifuge tube, and eluted with 50 µl water or TE buffer (elution in TE buffer was not used if the DNA was subsequently used in an enzyme reaction, particularly if the DNA was dilute and if large volumes of the DNA solution were required to be added to a reaction, since EDTA may inhibit some enzymes by chelating magnesium required as a co-factor for activity). DNA was eluted by centrifuging the tube at 10,000 x g in a microcentrifuge for 20 seconds. For large plasmids (≥10kb), water or TE buffer preheated to 65-70°C was used since it may increase yields. For plasmids ≥20kb, water or TE buffer preheated to 80°C was used. Plasmids were eluted as soon as possible off the column since, although DNA remains intact on the Minicolumn for up to 30 minutes. The eluted plasmid DNA was stored in the microcentrifuge tube at 4°C or -20°C (50 µl). Typically, the yield was 0.5-2 µg from a 2 ml culture. For plasmids that underwent additional investigation, the process was scaled up, and they were purified using Qiagen's preparation methods.

### b. Sequencing and Analysis

Sequencing was performed by Lark Technologies (Houston TX) as described below. Standard direction for the use of DyeDeoxy termination reaction kit (PE/ABI) Base specific fluorescent dyes were used as labels. Some reaction mixtures were modified to contain DMSO to decrease the formation of secondary structure and allow the determination of sequence in regions of high G-C base content. Sequencing reactions were analyzed on 4.75% PAGE by an ABI 373-S using ABI Sequencing Analysis Software version 2.1.2. Data analysis was performed using Sequencer™ 2.1 software (GeneCodes).

A 2142 bp open reading frame (Figure 9A) (SEQ ID NO:7) was detected. Within the candidate open reading frame, GUG (the 186th codon from the start of the open reading frame) was identified as the actual initiation codon from the sequence of the amino-terminus since it was immediately followed by the previously determined amino-terminal sequence shown in Figure 8, and since GUG is occasionally used as an initiation codon in *T. thermophilus.* Methionine amino-peptidases would be expected to cleave off the terminal methionine (Sherman et al., Bioessays 3:27-31 [1985]), revealing serine as determined experimentally. The initiating GTG could only be conclusively identified by use of the amino-terminal sequence of protein purified from *T. thermophilus.* The preceding sequences in the open reading frame are italicized in Figure 9A. Also in bold and underlined is the AAAAAA sequence that, by analogy to *E. coli,* is probably the frameshifting site that permits synthesis of the shorter γ product. The potential stop codons for γ in the +1 (first) and -1 reading frame are underlined. The stop codon for the full length τ translation product is double-underlined.

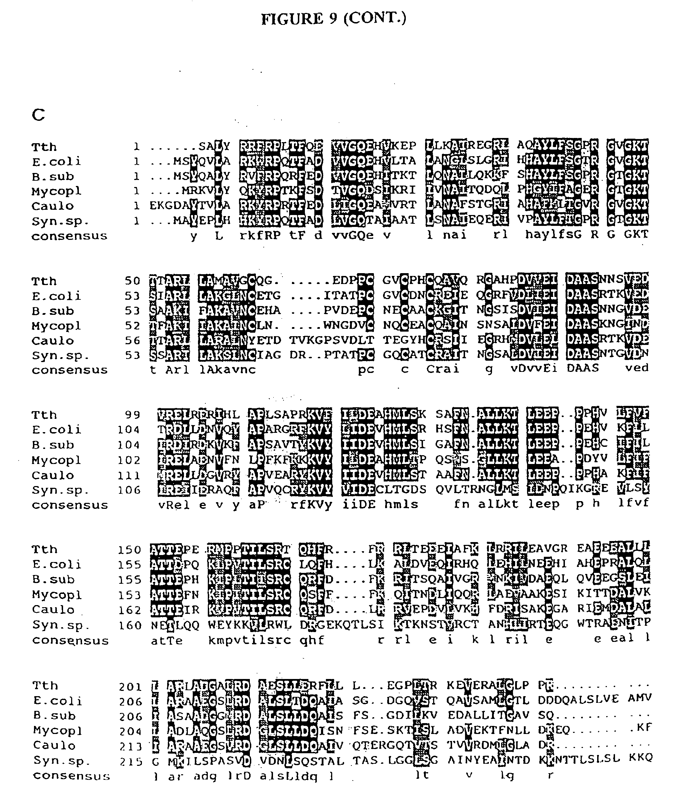

Alignment of the resulting DNA sequence with the identified *E. coli dna*X gene and putative homologous genes from other eubacteria revealed a high level of identity in the amino-terminal region of all bacteria, confirming the identity of the isolated gene to be a *dna*X homolog (Figure 9C). To provide the most useful and concise presentation, only examples from widely divergent organisms are presented in Figure 9C. The amino acid sequences for the organisms listed from the top are: Tth (*T*. *thermophilus Chloroflexaceae*/*Deinococcaceae* group) (SEQ ID NO:9); *E.coli* (proteobacteria group, gamma division) (SEQ ID NO:10), B.sub. (*B. subtilis,* firmicute group, low G+C gram-positive bacteria division) (SEQ ID NO:11), Mycopl (*Mycoplasma pneumoniae,* firmicute group, mycoplasma division) (SEQ ID NO:12), Caulo (*Caulobacter crescentus,* proteobacteria group, alpha division) (SEQ ID NO:13), Syn.sp. (Synechocystis *sp.,* Cyanobacteria group, (blue green algae) (SEQ ID NO:14)). Sequences shaded in black are identical among the indicated bacteria; sequences shaded in gray are similar. The consensus sequence is shown in SEQ ID NO:15.

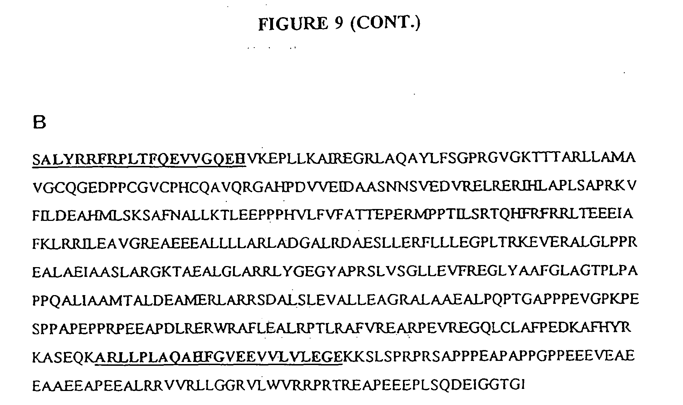

The deduced amino acid sequence with the amino terminal methionine removed as indicated from amino-terminal protein sequencing (SEQ ID NO:8) of the *T. thermophilus* τ subunit is shown in Figure 9B. The peptide sequences directly determined from the isolated *T. thermophilus* DnaX-proteins are underlined. A perfect match was observed with the internal peptide sequence obtained from sequencing of the 63 kDa candidate γ subunit between residues 428 to 450, further confirming the isolation of the structural gene for the protein associated with *T. thermophilus* pol III. The molecular weight of the predicted *T. thermophilus* τ subunit is 58 kDa, in reasonable agreement with the 63±6 kDa determined from SDS PAGE.

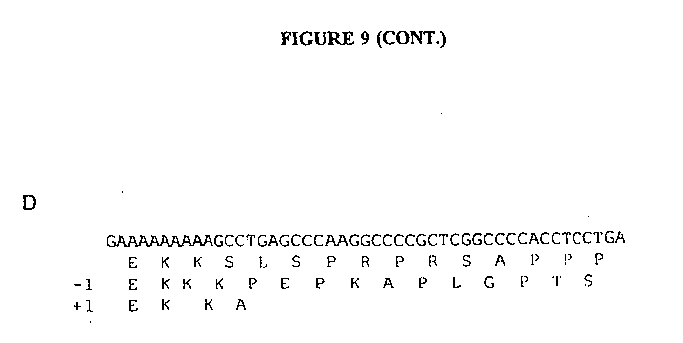

In *E. coli,* the shorter γ product of the *dna*X gene is produced by a translational frameshifting mechanism at the sequence A AAA AAG containing two adjacent lysine codons read by the Lys UUU anticodon tRNA. A potential frameshift site that exploits adjacent AAA lys codons flanked by A residues(A AAA AAA A) was observed at codons 451 and 452 (Figure 9A). Frameshifting into the -1 reading frame at this site would result in a 51 kDa protein, close to the 50 kDa candidate observed in Fraction V (Figure 9D). If, instead, a +1 frameshift occurred, a 50 kDa protein would be produced, indistinguishable within experimental error from the -1 frameshift alternative.

Every region conserved among other eubacterial *dna*X genes is found represented in *T. thermophilus dna*X*.* These sequences include the Walker-type ATP binding site, represented by GVGKTTT (SEQ ID NO:105) in *T. thermophilus dna*X*,* and highly conserved EIDAAS (SEQ ID NO:106), and FNALLKTLEEP sequences (SEQ ID NO:107)(Figure 9C). The conservation beyond the first 220 residues falls off for all *dna*X genes. Thus, the internal peptide sequence obtained that starts at residue 427 (SEQ ID NO:108; the second underlined sequence in Figure 9B) was useful in confirming that the sequence was in the correct reading frame and in providing further confirmation of the isolation of the structural gene for the same proteins isolated in association with *T. thermophilus* pol III.

### EXAMPLE 5

### Cloning and Sequencing the T. Thermophilus dnaE Gene

The *dna*E gene sequence of the *T. thermophilus* DNA polymerase III was obtained using a PCR amplification approach. In this approach, oligonucleotide primers that could be used for amplifying genomic DNA encoding a portion of the α subunit of *Thermus thermophilus* were designed using one of two strategies. The first strategy relied on designing PCR primers which hybridized to DNA polymerase III sequences that were homologous among several species of bacteria. However, insufficient homology was observed between diverse bacterial dnaEs, rendering this approach problematic.

The second strategy relied on designing PCR primers based on the amino acid sequence information which was obtained by sequencing *T. thermophilus* DNA III polymerase α subunit which had been partially purified on protein SDS-PAGE gel (as described in Example 3, *supra*)*.* The designed PCR primers were used to isolate *T*. *thermophilus* genomic sequences which encoded *T. thermophilus* DNA III polymerase subunit α. First, the PCR primers were used to amplify *T. thermophilus* genomic DNA, and the PCR-amplified sequences were used to create clones which contained *dna*E sequences. Second, these clones were used as probes against restriction nuclease digested *T. thermophilus* DNA in a Southern reaction that would allow isolation and cloning of larger segments of genomic DNA of a size expected to contain the entire coding region of *dna*E*.* Third, downstream sequences were isolated by asymmetric PCR. Fourth, downstream sequences were used to probe λ libraries of *T. thermophilus* chromosomal DNA to isolate full length dnaE clones.

This Example involved (A) Partial amino acid sequencing, (B) Isolation of *T. thermophilus dna*E probe by PCR amplification of a segment of *T. thermophilus dna*E, (C) Cloning PCR-amplified *dna*E probe, (D) Southern analysis of *T. thermophilus* DNA using isolated IF-91R *dna*E probe, (E) Cloning and sequencing of the 5' approximately 1100 bp of *T. thermophilus dna*E gene, (F) Isolation of downstream sequences by asymmetric PCR, (G) Southern analysis of *T. thermophilus* using approximately 300 BspHI/EcoRI *dna*E probe isolated from plasmid pB5, (H) Sequencing full-length *T. thermophilus dna*E*.*

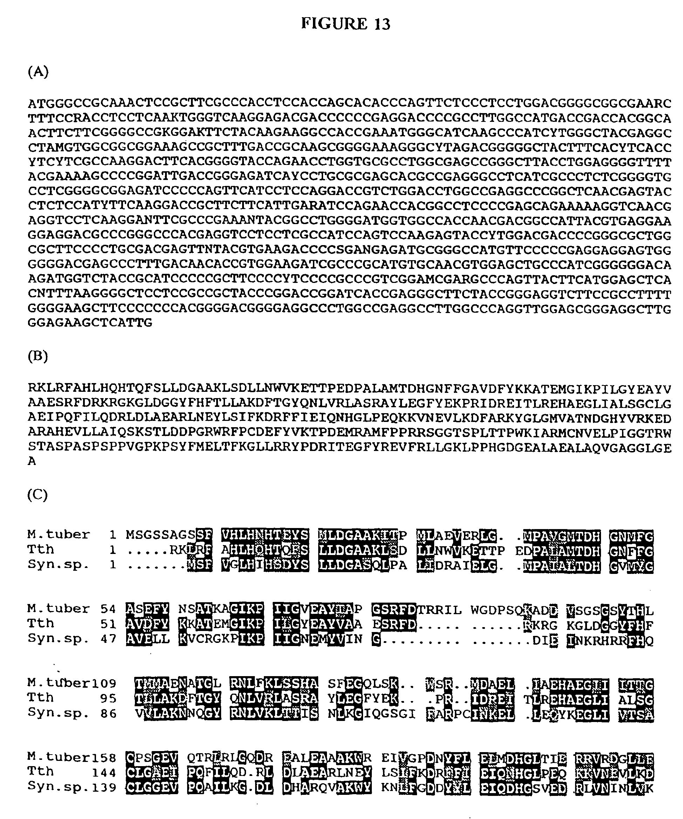

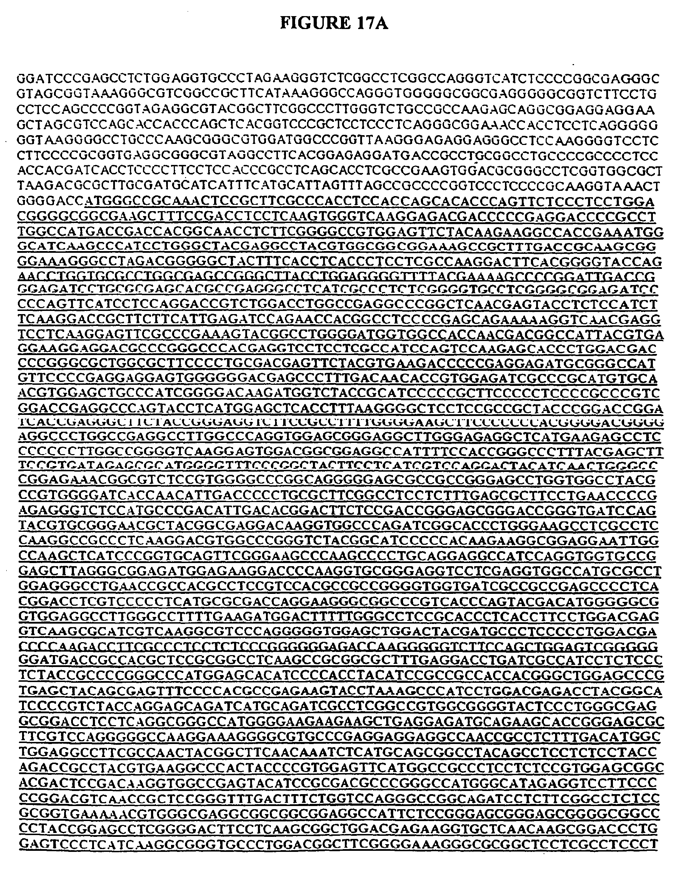

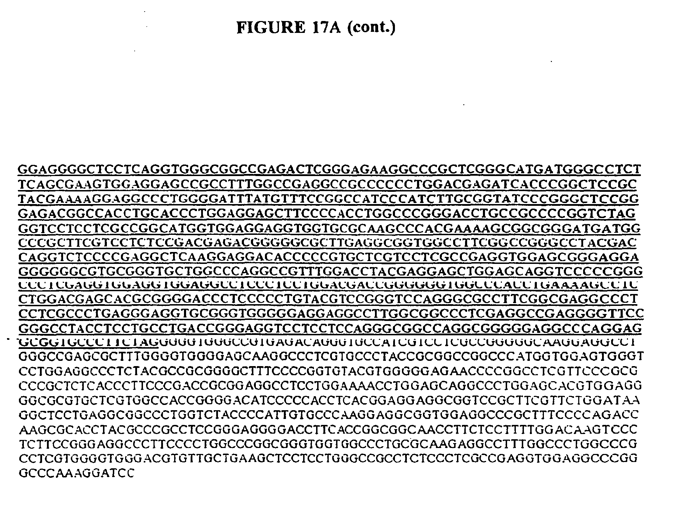

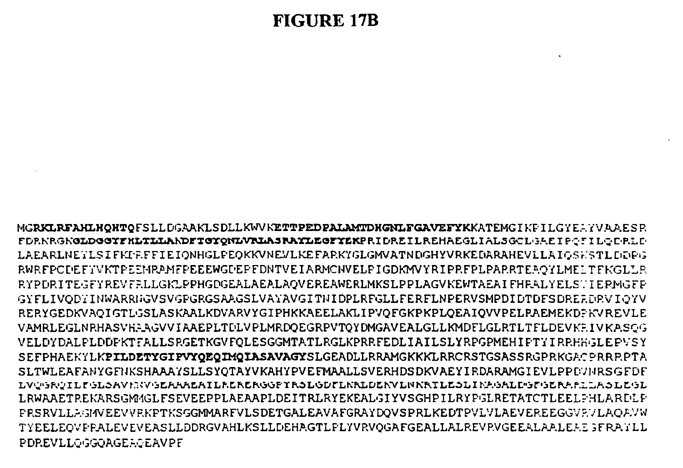

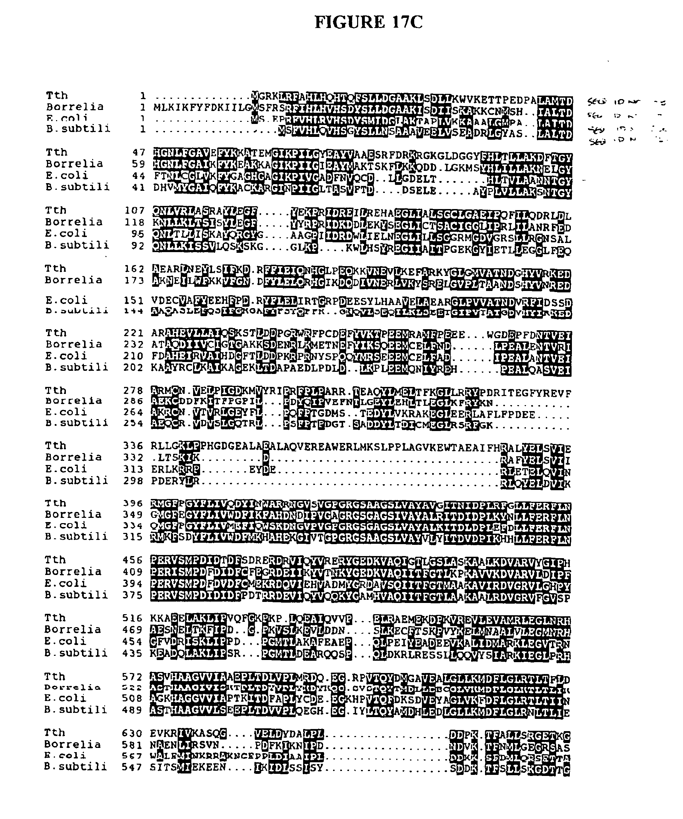

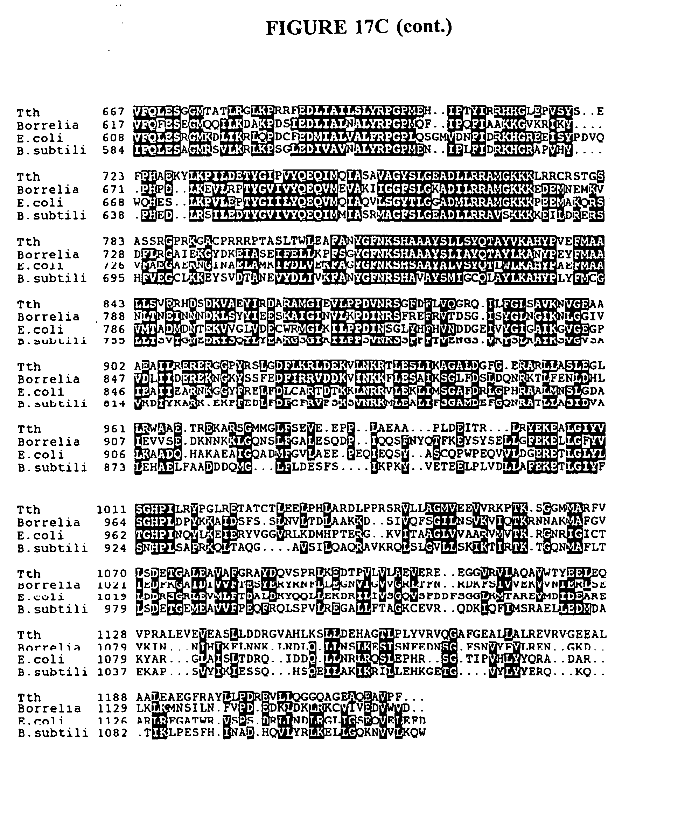

As described below, a segment of the *T. thermophilus* DnaE gene was isolated by PCR and confirmed by DNA sequencing (Figure 13B shows the estimated sequence for the first 366 amino acids *T. thermophilus* DnaE (SEQ ID NO:59). The full-length *dnaE* gene was isolated by using the sequences identified during the development of the present invention to probe lambda *T. thermophilus* chromosomal DNA libraries, subcloning hybridizing sequences, and confirming their identity by DNA sequencing. This full-length sequence is shown in Figure 17A. In this Figure, the *dnaE* reading frame is shown in bold and underlined. The deduced amino acid sequence is shown in Figure 17B. In this Figure, sequence corresponding to the peptides sequenced from isolated native *T. thermophilus* DnaE are shown in bold. Alignment with other eubacterial *dnaE* genes confirmed its identity, as the sequences were strongly homologous in regions conserved between eubacterial *dnaEs.* Figure 17C provides alignments with three representative sequences of *E. coli, B. subtilis* type 1, and *Borrelia burgdorferi* DnaE.

The isolated and sequenced full length *dna*E gene is engineered to overexpress wild type and N- and C-terminal fusions with a peptide containing a hexahistidine sequence, as well as a biotinylation site. The N- and/or C-terminal fusion proteins are purified and used to obtain *T. thermophilus* DnaE-specific monoclonal antibodies and fusion proteins.

The fusion proteins are also used to make affinity columns for the isolation of proteins that bind to DnaE alone, and in a complex with the isolated τ protein, and/or associated factors of the *T. thermophilus* DnaX complex. The structural genes for novel proteins identified by this method are isolated, sequenced, expressed, purified and used to determine whether they make contributions to the functional activity of the *T. thermophilus* DNA polymerase III holoenzyme. Wild type DnaE is purified and used for the reconstitution of DNA polymerase III holoenzyme. In addition, it is developed as an additive to improve the fidelity of thermophilic polymerases that do not contain proofreading exonucleases.

### A. Partial Amino Acid Sequencing

The amino acid sequence at the N-terminus and internal amino acid sequences was determined as follows.

### 1. N-Terminus

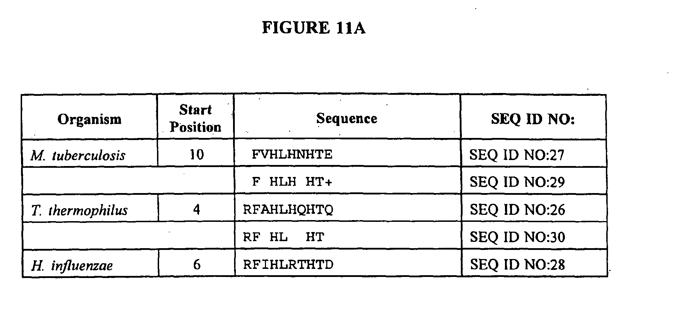

An aliquot of Fraction IV containing 500 µg of protein was precipitated by the addition of an equal volume of saturated ammonium sulfate at 4 °C, centrifuged at 15,000 rpm at 4°C in a SS-34 rotor, resuspended in 50 µl Buffer U and dialyzed overnight versus Buffer U at room temperature. The sample was applied to a 5% SDS polyacrylamide tube gel (0.5 cm diameter) and subjected to electrophoresis in an 230A High Performance Electrophoretic Chromatography module (ABS, Inc.). Fractions (25 µl) were collected, aliquots (5 µl), subjected to 7.5% slab SDS-PAGE, and the proteins visualized by silver staining. Fractions containing the highest concentration of 130 kDa protein were pooled, concentrated approximately 30-fold on a Centricon-10 membrane (Amicon) and analyzed by preparative gel electrophoresis on 7.5% SDS-polyacrylamide gels. The gel-fractionated proteins were transferred to a Hyperbond PVDF membrane (Biorad), and the 130 kd protein band excised and subjected to N-terminal amino acid sequence analysis in an 477A Protein Sequencer according to the manufacturer's (ABS, Inc.) instructions. A sequence of 13 amino-acids was obtained: RKLRFAHLHQHTQ (SEQ ID NO:109). This sequence was aligned with 6/13 residues from the amino terminus *M. tuberculosis* and *H. influenzae* in a BLAST search, and the alignments are shown in Figure 11. In this Figure, "M. tuber" refers to *M. tuberculosis,* while "Tth" refers to *T. thermophilus,* and "H. infl." refers to "*H*. *influenzae*." The numbers in this Figure indicate the amino acid numbers for the respective residues.

### 2. Internal Amino Acid Sequence

Internal peptide sequences were determined at the Harvard Microchemistry facility as described above. An aliquot of Fraction V (same as that used in the DnaX Examples) (230 µg) was subjected to electrophoresis on a 10% polyacrylamide gel, and the fractionated proteins transferred to a PVDF membrane (BioRad) as described above, the Ponceau-S stained 130 kDa band was cut out, subjected to digestion by endo Lys-C, and the resultant peptide separated by HPLC. Three peptides were chosen for sequencing (Figure 11). The peptide sequences aligned with sequences of *E. coli dna*E*.* The peptides were named by the first amino acid in *E. coli* that corresponded to the first residue of the *T. thermophilus* peptide. The peptide sequences obtained were 64% identical (peptide #91)(SEQ ID NOS:31 and 32); 68% identical (peptide #676)(SEQ ID NOS:34 and 35); and 54% identical (peptide #853)(SEQ ID NOS:37 and 38) through the indicated matching segments. Once the complete *dnaE* sequence became available, it was apparent that the peptide 853 alignment was not valid. One additional peptide sequence was obtained (ETTPEDPALAMTDHGNLF; SEQ ID NO:208), that was not used as a PCR probe because of the lack of a reliable alignment with eubacterial dnaE sequences. However, the sequence was useful in verifying the final *dnaE* DNA sequence. Sequences of homology between these peptides are shown in SEQ ID NOS:33, 36, and 39, respectively.

### B. Isolation Of T. thermophilus dnaE Probe By PCR And PCR Amplification of T. thermophilus dnaE

Primers were selected from the amino-terminal protein sequence that would provide the least degeneracy. Because the order of the sequences was known by their alignment with other eubacterial *dna*E sequences, only one primer was made for the extreme amino- and carboxyl sequences, 1F and 853R, respectively. Both forward and reverse primers were made for the internal sequences to enable performing PCR reactions with all possible primer combinations. The peptide sequences selected and the corresponding oligonucleotide probes are summarized in Table 2.

**Table 2. PCR primers used for isolation of T. thermophilus dnaE probes**

| Peptide # | Sequenced used for PCR primer | Primer number¹ | Primer Sequence(s) 5'→3' | Primer Degeneracy (-fold) |
|---|---|---|---|---|
| 2 | HLHQHTQ (SEQ ID NO:191) | 1F | | 512 |
| 91 | EGFYEK (SEQ ID NO:192) | 91F | GARGGNTTYTAYGARAA (SEQ ID NO:111) | 64 |
| | | 91R | TTYTCRTARAANCCYTC (SEQ ID NO:112) | 64 |
| 676 | YQEQQMQ (SEQ ID NO:193) | 676F | | 320 |
| | | 676R | TGCATYTGYTCYTGRTA (SEQ ID NO:114) | 16 |
| 853 | DGGYFH (SEQ ID NO:194) | 853R | TGRAARTANCCNCCRTC (SEQ ID NO:115) | 128 |

| | | | | |
|---|---|---|---|---|
| F, forward primer; R, reverse primer | | | | |

For PCR reactions, the Boehringer Mannheim Expand™ High Fidelity PCR system was used with *T. thermophilus* chromosomal DNA preparation A following the manufacturer's recommendations except as noted. Buffer 1 contained 2 µl of 10 mM dNTP mix (Gibco/BRL; 200 µM final); 4 µl of the forward and reverse primers (3 µM initially; 240 nM final in 100 µl reaction); 1 µl of a 1/30 dilution of *T. thermophilus* genomic DNA (20 ng total), 39 µl distilled H₂O. Buffer 2 contained 10 µl of 10-fold concentrated Boehringer Mannheim Expand HF Buffer with 15 mM MgCl₂, 39 µl H₂O), and 1 µl Expand High Fidelity enzyme mix (mixture of *Taq* and *Pwo* DNA Polymerase -- 3.5 units total). Buffers 1 and 2 were combined in a 0.2 ml thin walled tube (Intermountain) and the PCR reaction was conducted in an MJ Research thermal cycler as follows: annealing steps were conducted at 48°C, elongation at 68°C and the melting step at 94°C, 26 total cycles were run. Reactions were initiated by placing the reaction mixture in a block pre-warmed to 95°C, incubated for 5 min. and the following cycle was initiated: (a) reactions were incubated at 94°C for 30 s (melting step); (b) primer annealing was permitted to occur for 45 s at 48°C; (c) primers were elongated at 68°C for 2 min. After the conclusion of step (c), the reaction block was cycled to 94°C and the cycle was restarted at step (a). After ten cycles, step (c) was increased 20 s for each successive cycle until it reached 7 min.; 26 cycles total were run. The block was then cycled to 4°C and held there until the sample was removed for further use.

DNA was precipitated by the addition of 1 µl glycogen (Boehringer Mannheim) and 100 µl isopropanol, centrifuged in a microfuge for 2 min. at 14,000 x g; the supernatant was discarded and the pellet was washed with 300 µl 75% ethanol (room temperature). The pellet was resuspended in 10 µl TE buffer and 2 µl of gel loading dye (0.25% Bromophenol Blue, 0.25% Xylene cyanol, 25% Ficoll (Type 400) in distilled H₂O) was added. The entire sample was loaded onto a 3% Metaphor agarose gel (FMC) in TAE buffer (0.04 M Tris-acetate, 1 mM EDTA (pH 8.5) along with a 123 bp standard and a 100 bp ladder (Gibco/BRL) in separate lanes. The gel was run at 30 V overnight, stained with 1/10,000 dilution of Sybr Green I (Molecular Probes) in TAE buffer for 1h at room temperature with gentle shaking, and photographed under short uv irradiation. The results of the analysis are summarized below.
A. Primer pair: 1F-91R. Size of band(s) 330 near 314 expected band. Two other faint bands were visible at approximately 250 and 980 bp.
B. Primer pair: 91F-676R. Blank lane on gel -- no band produced.
C. Primer pair: 676F-853R. Size of band 520-550 near 522 expected band. 11 bands ranging from 200 to > 1500 bp. 9 bands were of equal or greater than the intensity of the desired band. (This band did not result in a clone that provided dnaE sequence information.)
D. Primer pair: 1F-676R. Size of band 2100 near 2050 expected band. Only one other band was visible near 1200 bp. (After cloning, this band was sequenced and found to be incorrect -- that is, not *dna*E gene.)
E. Primer pair: 91F-853R. Blank lane -- no bands visible.

### C. Cloning PCR-Amplified dnaE Probe

The products resulting from primer pairs 1F-91R and 1F-676R were extracted using the freeze squeeze technique (described in Example 4, section D) and cloned into vector pCRII (Invitrogen) as described above.

Plasmid isolates were prepared using the Promega Plus Minipreps technique and examined by restriction analysis to determine whether they yielded an *Eco*RI fragment approximately the same size as the desired cloned PCR product (*Eco*RI is flanked by the cloning site). From 8 colonies picked from the 1F-91R cloning, 7 yielded a fragment around the expected 320 bp. From 8 colonies picked from the 1F-676R cloning, 8 yielded a fragment around the expected size. Plasmid DNA was prepared from the 1F-91R and 1F-676R clonings, using the Qiagen Plasmid Midi Kit and sequenced (Colorado State University DNA Sequencing Facility, Ft. Collins, CO).

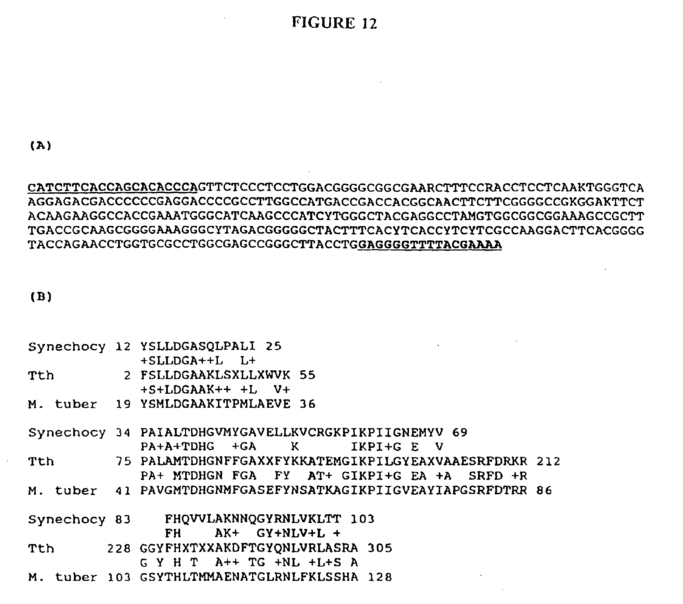

The sequence of the PCR product (SEQ ID NO:40) resulting from amplification of *T. thermophilus* chromosomal DNA with primers 1F and 91R (plasmid pMGC/E1-91.B; DMSO 1322) are shown in Figure 12A. In this Figure, the sequences resulting from the primers underlined and shown in bold (SEQ ID NOS:41 and 42). In Figure 12, a portion of the BLAST search results using only the sequence between the primers is shown. The first segment aligned (base 2-55) in the *T. thermophilus* query sequence) aligned with *M. tuberculosis* and *Synechocystis sp. Dna*E in the +2 reading frame; the remainder of the alignment is in the +3 reading frame indicating a frameshift error in the DNA sequence. The percent identity for three homology stretches was found to be 50%, 63%, and 46% (*M. tuberculosis*) and 57%, 47%, and 47% (*Synechocystis sp*.).

### D. Southern Analysis Of T. thermophilus DNA Using Isolated 1F-91R dnaE Probe

A Southern analysis of *T. thermophilus* chromosomal DNA was conducted as described above (Example 4, section F) to determine the restriction fragments most likely to contain a full-length *T. thermophilus dna*E gene. *T. thermophilus* chromosomal DNA was digested with the indicated restriction enzymes alone and in combination as described above. Approximately 3 µg DNA was subjected to electrophoresis, transfer to membranes and blotting as previously described (Ausubel *et al.* [1995] *supra*). Probe preparation and amount used was as described above.

The size of the bands which hybridized with the probe in restriction enzyme-digested DNA was as follows:

| | |
|---|---|
| *PstI* | 10.2 kb |
| *Pst*I/*Hin*dIII | 0.95 kb |
| *Hin*d III | 0.95 kb |
| *Bsp*HI | 8.5 kb |

### E. Cloning And Sequencing Of The 5' Approximately 1100 bp of T. thermophilus dnaE Gene

A *Bsp*HI digest of *T. thermophilus* chromosomal DNA (preparation B) was subjected to electrophoretic separation on an agarose gel and the region corresponding to the 8.5 kb fragment that hybridized with the *dna*E 1F-91R probe cut out of plasmid pE1-91B.

The isolated 8.5 kb population of *Bsp*HI fragments was cloned into pMGC707 as described above, except that the cleavage site was not within the polylinker. The *Bsp*HI site is at nucleotide 3088 in the parental pCRII vector, approximately 1120 nucleotides away from the polylinker.

Colonies were screened by colony hybridization (Ausubel *et al.* [1995] *supra,* sections 6.1.1 - 6.1.3 and 6.3.1 - 6.3.4; *See* also, production of *T. thermophilus* 1F-91R probe isolated from plasmid pEl-91B. A total of approximately 4,000 colonies from 2 plates was screened. Two positive colonies were selected and restruck on LB plates containing 150 µg/ml carbenicillin to ensure purity of the selected clones. Two separate colonies were selected from each for further examination. Of these, only two (DMSO 708B and 709A) had approximately 8 Kb inserts as judged by *Hin*dIII digestion of plasmids isolated by the Promega Plus Minipreps. Plasmid pBspHIdnaE (DMSO 708B) was sequenced (Colorado State University DNA sequencing facility), resulting in the approximate sequence of the first 1,109 bases of the *T. thermophilus dna*E gene (SEQ ID NO:58) (Figure 13A). In this Figure, the apparent start of this preliminary sequence is underlined; the upstream sequence is not shown in this Figure. The start of this sequence was estimated by alignment with the N-terminal protein sequence. Apparently the initiating Met and the following Gly were removed by proteolysis.

The BLAST search using this sequence indicated that there were at least two frameshift errors in the sequence, since the first part aligned in frame 1, then a long stretch aligned in frame 2, and then a final stretch aligned in frame 1. From these alignments, it was determined that the first frameshift error probably occurred between bases 102 and 119, and the second occurred between bases 787 and 832. The sequences were edited in order to bring all of the homology into one open reading frame, by deleting C₁₀₈, that was a part of a string of 6 Cs. An "N" (*i.e.,* any base) was added after base 787, to produce the sequence shown in Figure 13B (SEQ ID NO:59). The best alignments resulting from a BLAST search with the amino acid sequence shown in Figure 13B are shown in Figure 13C (SEQ ID NOS:60-62. In this Figure, the grey boxes indicate bases that are similar, but not identical between the sequences, while black boxes indicate identical bases.

### F. Isolation Of Downstream Sequences By Asymmetric PCR

Experiments attempting to use primer E853R in combination with primers 91F and 676F failed to isolate downstream *dna*E sequences. Thus, in an attempt to obtain further downstream sequence the following asymmetric PCR procedure was developed so that only relied on an upstream primer and a knowledge of a downstream Pst I restriction site through Southern analysis.

Asymmetric PCR uses one primer to extend a stretch of DNA rather than two primers normally used. The advantage of this technique is useful in that it allows amplification of either a region upstream or downstream of the primer and subsequent cloning without further knowledge of DNA sequence. A single DNA primer is mixed with genomic DNA, and the polymerase used to make a single strand copy of the template DNA as far as it could extend. The single-strand DNA was then made double-stranded by a random hexamer-primer annealing and extension with Klenow polymerase, and the double stranded DNA was cloned directly into the vector after creating the appropriate ends by digestion of the double-stranded DNA with the chosen restriction enzymes. Although the reaction tends to be error-prone, it was thought that it might be useful in providing preliminary sequence information that would aid in the isolation of the full-length gene.

### 1. Primer Design

The primer was selected from known *T. thermophilus dna*E sequence; and additionally was biotinylated at the 5' end and contained a *Pac*I restriction site to allow cloning. The primer sequence was 5'-biotin-CCGCGC**TTAATTAA**CCCAGTTCTCCCTCCTGGACG-3' (SEQ ID NO:116). The *Pac*I restriction site, highlighted in bold, has a rare 8-base recognition sequence (*i.e.,* only contains As and Ts), and would be expected to be extremely rare in *Thermus thermophilus* with its highly (69%) GC rich genome. This is important because it restricts possible clones into the *Pac*I site of the vector to DNA containing the primer. The GCGC region preceding the *Pac*I site clamps the DNA, thus allowing a highly efficient cleavage of the *Pac*I site, and PacI is known to cleave efficiently with as little as 2 base pairs of DNA flanking the cleavage site. Biotin on the 5' end was used to purify the amplified DNA containing the primer, as explained below.

### 2. PCR Amplification

For the single-stranded amplification step, the Boehringer Mannheim Expand™ PCR system was used and following the manufacturer's recommendations except as noted in the following. Buffer A contained 3.5 µl of 10 mM dNTP mix (Gibco/BRL; 350 µM final); 0.5 µl of the forward primer (80 µM stock concentration; 400 nM final in 100 µl reaction), 46 µl distilled H₂O). Buffer B contained 10 µl of 10-fold concentrated Boehringer Mannheim Long Template PCR System Buffer 2 (500 mM Tris-HCl (pH 9.2 at 25°C), 160 mM ammonium sulfate, 22.5 mM MgCl₂), 37.5 µl H₂O), and 1.5 µl Expand Long Template enzyme mix (mixture of Taq and Pwo DNA Polymerase -- 5 units total) and 1 µl of *T. thermophilus* genomic DNA (preparation A, 0.6 µg total). Buffers A and B were combined in a 0.2 ml thin walled tube (Intermountain) and the single primer extension reaction was conducted in an MJ Research thermal cycler as follows.

Annealing steps were conducted at 55°C, elongation at 68°C and the melting step at 95°C; 60 total cycles were run. Reactions were initiated by placing the reaction mixture in a block pre-warmed to 95°C, incubated for 5 min. and the following cycle was initiated: (a) reactions were incubated at 95°C for 30 s (melting step); (b) primer annealing was permitted to occur for 30 s at 55°C; (c) primers were elongated at 68°C for 12 min.. After the conclusion of step (c), the reaction block was cycled to 94°C and the cycle was restarted at step (a). Upon completion of 60 cycles, the block was cycled to 4°C and held there until the sample was removed for workup.

After completion of the PCR reaction, the Boehringer Mannheim PCR cleanup kit was used to remove unextended primer. The PCR cleanup kit efficiently binds DNA that contains greater than 100 bp of double stranded DNA. Most long single-stranded DNA would be expected to contain enough secondary structure to bind to the silica beads, while the primers would not. The PCR cleanup kit was used as described above. Bound DNA was eluted overnight in 50 µl TE buffer, pH 8.4 followed by an additional wash with 50 µl of distilled H₂O.

The extended, biotinylated DNA was removed from the remaining genomic DNA by binding the biotinylated DNA to agarose beads coated with monomeric avidin (Soft-Link, Promega). Soft-Link resin (100 µl settled beads) was incubated 2x w/0.4 mls 10 mM biotin washed 6x with 10% acetic acid by resuspending gel in 1ml and spinning dry in 0.45 µM spin filters (Life Sciences). The resin was then washed once with 0.5 mls of 0.5 M potassium phosphate (pH 7.0), 3x with 50 mM potassium phosphate (pH 7.0), then resuspended in 0.5 mls 50 mM potassium phosphate (pH 7.0). DNA from the single primer elongation reaction was added to 20 µl of the beads (packed volume) (final reaction volume 120 µl), incubated 30 min. with gentle agitation every few minutes, washed with 0.5 ml TE buffer (pH 7.5) followed by centrifugation (14,000 x g, 30 s) in the spin filters to remove unbiotinylated DNA. The wash was repeated 5 times. The biotinylated DNA was eluted by the addition of 40 µl 10 mM biotin to the beads and incubation in a capped spin filter for 5 min. in a 42°C water bath. The DNA was recovered by centrifugation (14,000 x g, 320 s); the elution step was repeated once more. To ensure complete removal of DNA, the beads were washed with 0.1 M NaOH, spun as before and the basic mixture neutralized by the addition of 2 M Tris-HCl. The neutralized solution had a final pH of 7.8 as tested by pH paper. The DNA resulting from the alkaline-eluted and biotin-eluted fractions was ethanol by the addition of 1 µl glycogen, 1/10 volume sodium acetate (adjusted by the addition of acetin acid to pH 5.2), and 3 volumes ethanol followed by centrifugation at 14,000 x g for 10 min. The samples were suspended in 50 µl distilled H₂O and combined.

To convert the single stranded DNA to double-stranded DNA the random hexamer priming was used as previously described, following the protocol of the manufacturer (Boehringer-Mannheim). A 5x reaction mixture was used (100 µl reaction volume) with the following components: approximately 40 µl DNA, 3 µl each dATP, dTTP, dGTP and 5 µl dCTP, 10 µl Boehringer Mannheim random primed DNA labeling kit component 6 "reaction mixture" (containing random hexamer oligonucleotides, 25 µl water, and 5 µl Klenow polymerase (Boehringer, 10 U). The reaction was incubated for 30 min. at 37°C.

DNA from the random-hexamer priming was precipitated using 1 µl glycogen, 1/10 volume sodium acetate, and 3 volumes of ethanol. The pellet was washed 2x with 0.2 mls of 75% ethanol and the pellet resuspended in 200 µl TE buffer, pH 7.5. About 15 µg of total DNA was recovered, quantitated with PicoGreen™ as described above.

A *Pac*I digest on 6.5 µg of the recovered DNA was performed to prepare the primer end of the product for cloning. In reaction containing 90 µl DNA (6.5 µg), was digested (3h, 37°C) in the presence of 25 µl 10 NEB buffer 1(10 mM Bis Tris Propane-HCl, 10 mM MgCl₂, 1 mM DTT (pH 7.0 at 25°C), 10 µl *Pac*I enzyme (NEB, 100 units), 125 µl distilled H₂O). The cleaved DNA was precipitated by the addition of 1 µl glycogen, 25 µl sodium acetate, and 3 volumes of ethanol. The product recovered by centrifugation (5 min., 14,000 x g) was dissolved in 75 µl distilled H₂O.

Analysis of Southern digests showed that there was a *Pst*I site downstream of the *Bsp*HI site that defined the end of the longest *T. thermophilus dna*E clone. Therefore, the asymmetric PCR product was digested with *Pst*I*,* to provide a distal cloning site to enable cloning more downstream sequence.

*Pac*I digested DNA (37 µl containing 3.25 µg DNA) was combined with 12.5 µl 10x NEB buffer #3 50 mM Tris HCl, 10 mM MgCl₂, 100 mM NaCl, 1 mM DTT (pH 7.9 at 25°C), 5 µl PstI (NEB, 25 units), 65.5 µl dH₂O) and digested for 2 h at 37°C. The digested DNA was precipitated by the addition of 1 µl glycogen, 25 µl sodium acetate and 3 volumes of ethanol, recovered by centrifugation, dissolved in 125 µl dH₂O, and quantitated with PicoGreen™ as described. The concentration was 9 ng/µl *(i.e.,* 1125 ng total recovery). DNA was precipitated again as before and dissolved in 2 µl dH₂O.

The vector was digested with *Pac*I and *Bst*XI (gives a *Pst*I compatible end for cloning) as follows: 10 µl DNA (pMGC707, 30 µg) was digested (4h, 55°C) in the presence of 25 µl 10x NEB buffer #3, 10 µl BstXI (NEB, 100 units), 205 µl distilled H₂O). The product was precipitated by the addition of 2 µl glycogen, 1/10 vol. sodium acetate (2.5 M, pH 5.2), 2 volumes ethanol, collected by centrifugation, and redissolved in 50 µl TE buffer (pH 7.5). The redissolved DNA (15 µg) was further digested (37°C overnight) with PacI (5 µl PacI 50 units, NEB) in the presence of 10 µl 10x NEB buffer #1 and 60 µl distilled H₂O.

DNA was run preparatively in 0.6% Seakem agarose in 1x TAE at 85V for 4 hrs. A single band was obtained and recovered by the freeze/squeeze technique described above. DNA was precipitated with ethanol by the standard procedure described above.

### 3. Ligation of PacI/PstI-digested Asymmetric PCR Product Into PacI/BstXI-digested pMGC707 DNA to Make pB5

Digested pMGC707 vector DNA (2 µl (0.1 µg) was mixed with 2 µl of *Pac*I/*Pst*I digested asymmetric PCR product (1 µg) together with 2 µl 10x ligation buffer (Invitrogen), 1 µl T4 DNA ligase (Invitrogen, 4 Weiss units) and 13 µl distilled H₂O and incubated 1 h at 23°C and then overnight at 14°C. DNA was precipitated with ethanol, centrifuged and resuspended in 10 µl distilled H₂O. 4 µl of this mixture was transformed by electroporating as described above. To enrich the population for kanamycin-resistant bacteria, the transformation mixture was grown in liquid culture for 1 hr without selection and 2.5 hrs in the presence of kanamycin (50 µg/ml), then streaked for isolated colonies on LB. Ten colonies were picked and DNA was prepared using Promega Plus minipreps. Two of the ten had inserts of roughly 1.8 kb as judged by comparison of *Pac*I-digested DNA with linearized vector.

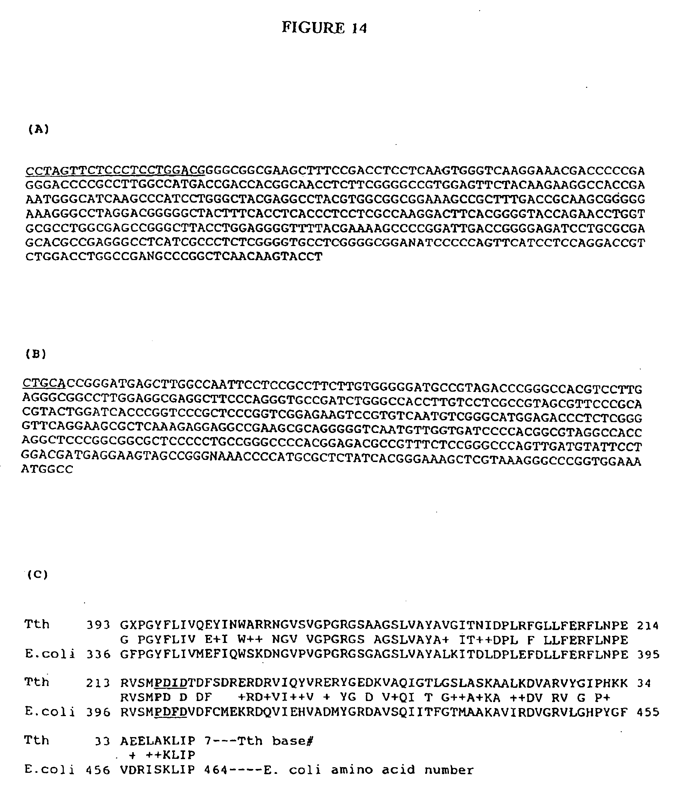

The candidate inserts from isolates pB4 and pB5 also released a 0.95 kb fragment upon *Hin*dIII digestion as predicted from the Southern blot of *T. thermophilus* chromosomal DNA against a *dna*E probe. One (pB5, DMSO 1335) was sequenced and shown to have homology to *E. coli dna*E. The region of asymmetric PCR product corresponding to the more N-terminal end (*i.e.,* the "front end" of the clone) of the *T. thermophilus dna*E gene is shown in Figure 14A (SEQ ID NO:63). In this Figure, the region corresponding to the forward primer is underlined and shown in bold. The distal end of the PCR clone was also sequenced (*i.e.,* the "back end" of the clone), in order to provide an indication of whether extensive new sequence had been gained, or if the full-length gene was obtained by the asymmetric PCR. The sequence of this clone is shown in Figure 14B (SEQ ID NO: 180). The *Pst*I sequence that defined the front end of the *T. thermophilus* DNA is underlined in Figure 14A. BLAST alignment (Figure 14B; SEQ ID NO: 180) indicated that the most distal end corresponded to roughly residue 464 of *E. coli* DnaE, considerably short of the full-length 1160 amino acid protein. Thus, this approach to identify the *T. thermophilus dna*E sequence was abandoned. However, this alignment did reveal the critical "PDXD" motif (SEQ ID NO: 117) that defines two critical aspartate residues making up part of the eubacterial DNA polymerase III active site. This motif is bolded and underlined in Figure 14. *T. thermophilus* DnaE was found to be 62% identical to *E. coli* sequences in this region.

### G. Southern Analysis Of T. thermophilus Using Approximately 300 BspHI/EcoRI dnaE Probe Isolated From Plasmid pB5 AND Sequencing Full-Length T. thermophilus dnaE

Sequence analysis of the *Bsp*HI *T. thermophilus dna*E clone indicated that it encoded only the amino-terminal portion of the sought gene. After obtaining a portion of the *T. thermophilus dna*E gene further downstream sequence by use of the asymmetric PCR cloning method, a probe was designed that was more internal to the gene, enhancing the chances of detecting a restriction fragment that encoded downstream regions of *T. thermophilus dna*E*.* The distal most C-terminal encoding fragment of pB5 bounded by the terminal vector *Eco*RI site and a *Bsp*HI site internal to dnaE was used as a probe for more Southern blots. *T. thermophilus* chromosomal DNA was digested with the indicated restriction enzymes alone and in combination. Approximately 3 µg DNA was subjected to electrophoresis, transferred and blotted as previously described (Ausubel, *supra,* section 2.9.2 to 2.9.11). Following hybridization procedures described and using 120 ng of a probe labeled by the random hexamer priming method (2.5 x 10⁷ cpm) we determined the size of the bands which hybridized with the probe following restriction digestion as follows (all sizes of restriction fragments from this and all Southerns is ± 20%):

| | |
|---|---|
| *Bam*HI | 4.9 kb |
| *Bam*HI/*Kpn*I | 4.2 kb |
| *Kpn*I | 7.4 kb |
| *BgI*II | 13.8 kb |
| *BgI*II/*Nco*I | 2.1 kb |
| *Nco*I | 2.1 kb |
| *Bam*HI/*BgI*II | 4.9 kb |
| *Bam*HI/*Nco*I | 2.1 kb |

| | |
|---|---|
| *Pvu*I, *Spe*I and *Xho*I digests yielded only very high molecular weight DNA | |

A critical part of the restriction map gleaned from the above (with distances here and as determined from all Southerns was ±20%) was:
BamHI---700 bp---KpnI---4200bp---BamHI---3200 bp---KpnI

This method was used to isolate the putative full length *T. thermophilus dna*E clones (Lofstrand Labs) as described briefly below. The bacterial DNA was randomly cut with *Sau*A1 (*i.e.*, a 4 base cutter) by partial digestion and ligated into phage vector lambda GEM 12 at the *Xho* I site (Promega). The library was packaged using Epicenter Technologies packaging extract and plated out for screening. The plaques were lifted in duplicate and probed using ³²P random primed pB5E insert DNA (780 bp fragment isolated from low melt agarose using *Kpn*I and *Bsp*HI). The six primary plates containing greater than 20,000 pfu per plate demonstrated about 100 duplicate positives each. Twelve duplicate positive primary plaque areas were picked and replated to enrich the clones. A third plating was performed using duplicates from the second screen and four duplicate positive isolated plaques were chosen for amplification and DNA purification. The phage were grown in liquid lysate cultures and the virus was purified by two CsCl density gradient centrifugations. The four DNA clones were digested with *Kpn*I and *Bam*HI, individually, and *Kpn*I and *Bam*HI*.* The ethidium bromide stained 0.8% TBE gel indicated that clones BGP2 7.1 and BGP2 8.1 appear to contain full length genes based on the restriction patterns present. A Southern blot was also performed on the four clones cut with *Bam*HI and *Bam*HI*+Kpn*I using the pB5E (probe 2) which showed strong signals in the predicted 4.9 Kb *BamHI* fragment and 7.4 Kb KpnI fragment. The 4.9 Kb *Bam*HI fragment was subcloned into plasmid (pBSIIKS+) for sequencing.

The full-length sequence of *Tth* dnaE (SEQ ID NO:196) was obtained (*See,* Figure 17A). In this Figure, the dnaE reading frame is shown in bold and underlined. The deduced amino acid sequence (SEQ ID NO:197) is shown in Figure 17B). In this Figure, sequence corresponding to the peptides sequenced from isolated native *Tth* dnaE are shown in bold. Alignment with other eubacterial *dnaE* genes confirmed the identity of the full-length *dnaE* sequence. Figure 17C shows alignments of the *Tth dnaE* gene with (SEQ ID NO:198) three representative sequences from *E. coli* (SEQ ID NO:199), *B. subtilis* type 1 (SEQ ID NO:200), and *Borrelia burgdorferi* (SEQ ID NO:201) *dnaE.*

### H. Construction of Vectors Expressing Tth α and Biotin-Tagged α, and Purification of Expressed Proteins

### 1. Construction of Starting Vectors

First, a vector, "pDRK-C" (described by Kim and McHenry, J. Biol. Chem., 271:20690-20698 [1996]), containing a pBR322 origin of replication, a gene expressing the laqI^{Q} repressor protein, and a semisynthetic *E. coli* promoter (pAl), that is repressed by the *lac*I repressor was modified. Plasmid pDRKC DNA was prepared and digested with *Kpn*I*,* the resulting overhanging ends were removed by treatment with T4 DNA polymerase in the presence of Mg⁺⁺ and the four dNTPs (0.1 mM), and resealed with T4 DNA ligase in the presence of 1 mM ATP. Plasmids were transformed into *E. coli,* and plasmid-containing colonies were selected based on ampicillin resistance. Plasmids were prepared from these colonies and screened for loss of the *Kpn*I site. One of the colonies that contained plasmid that was not cleaved by *Kpn*I was selected, grown, and used for preparation of the resulting plasmid, "pDRKC-Kan^{minus}." Approximately 40 µg of DNA as obtained from an 80 ml culture. The resulting plasmid was sequenced in the region of the *Kpn*I site, and was found to contain the expected sequence.

Plasmid pDRKC-Kpn^{minus} DNA was digested with *Xba*I and *Spe*I, to remove a small polylinker that contained sites *Xba*I*--Nco*I*--Not*I*--Dra*III*--Spe*I. The following oligonucleotide (the sequences of each strand are shown below; SEQ ID NOS:202 and 203) was synthesized and inserted into the digested plasmid to replace the polylinker of pDRKC-*Kpn*^{minus}, with *Xba*I*--*AGGAGG*-Pac*I*--Nco*I*--*spacer*--Kpn*I*--*spacer*--Fse*I-*Spe*I (*i.e.,* the following oligonucleotides were synthesized separately, and annealed to form a duplex with sticky ends and inserted into cut plasmid).

Ampicillin-resistant colonies obtained by transformation of the resulting plasmid were screened for production of plasmid that had gained a *Kpn*I site. DNA from one plasmid ("pA1-CB-Nco-1") was prepared, and replacement of the original polylinker with the above oligonucleotide (SEQ ID NOS:202 and 203), was confirmed by DNA sequencing using methods known in the art.

### 2. Construction of a Vector Expressing Wild-Type Tth DNA Polymerase III α Subunit

A 320 bp *Nco*I/*Kpn*I fragment, containing the 5' end of the *Tth dna*E gene was cloned into the corresponding sites of plasmid pA1-CB-Nco-1, to generate plasmid "pAT-TE(5')." The resulting clones were screened for the *Nco*I/*Kpn*I sites. A plasmid DNA preparation was made from one positive clone. Then, a 3454 bp *Kpn*I/*Fse*I fragment (obtained from plasmid pA1-NB-TE, described below), containing the 3'-end of the *Tth dnaE* gene was subcloned into the corresponding sites of plasmid pA1-TE(5'). The resulting clones were screened for the *Kpn*I/*Fse*I sites, and for the correct size *Pac*I/*Fse*I fragments (3.7 kb/5.6 kb), and *Hin*dIII fragments (0.95 kb/9.2 kb), in separate digests. A plasmid DNA preparation from one positive clone yielded approximately 125 µg of DNA from an 80 ml culture. The resulting plasmid, "pA1-TE," was transformed into *E. coli* strain MGC 1030 (mcrA, mcrB, lambda-, lexA3, uvrD::Tc, OmpT::Kn (available from Enzyco). Individual colonies were selected and screened for expression of the *Tth* DNA polymerase III α subunit.

### 3. Verification of Expression of Wild-Type Tth DNA Polymerase III α Subunit by pA1-TE/MGC 1030

Two candidate isolated containing pA1-TE in MGC 1030, were inoculated into a 2 ml of 2x YT culture medium (10 g tryptone, 10 g yeast extract, and 5 g NaCl, per liter), containing 100 µg ampicillin, and grown overnight at 30°C, in a shaking incubator. After 18-24 incubation, 0.5 ml of the now-turbid culture was inoculated into 1.5 ml of fresh 2x YT medium. The cultures were incubated for 1 hour at 37°C, with shaking. Expression was induced by addition of IPTG to a final concentration of 1 mM. After 3 hours, post-induction, cells were harvested by centrifugation. The cell pellets were immediately resuspended in 1/10 culture volume of 2x Laemelli sample buffer (2x solution: 125 mM Tris-HCl (pH 6.8), 20% glycerol, 4% SDS, 5% β-mercaptoethanol, and 0.005% bromphenol blue w/v), and sonicated, to shear the DNA. The samples were heated for 10 minutes at 90-100°C, and centrifuged to remove insoluble debris. A small aliquot of each supernatant (3.5 µl) was loaded onto a 4-20% SDS-PAGE mini-gel (Novex, EC60255; 1 mm thick, with 15 wells/gel) in 25 mM in Tris base, 192 mM glycine, and 0.1 % SDS. A protein, migrating between the β/β' subunits of RNA polymerase, and the high molecular weight standard of the Gibco 10 kDa protein ladder (120 kDa), was observed as a faint band in the induced cultures, but was not observed in the uninduced control. This protein was determined to be consistent with the expected molecular weight of 137,048 kDa. The detected protein represented less than 0.5% of *E. coli* protein.

### 4. Construction of a Vector That Expresses Tth DNA Polymerase III α Subunit with an N-Terminal Biotin/His Tag

Plasmid DRK-N(M), a plasmid designed for expression of proteins with an amino-terminal tag containing a peptide that is biotinylated *in vivo,* a hexahistidine site, and thrombin cleavage site (*See,* Kim and McHenry, J. Biol. Chem., 271:20690-20698 [1996]), and a pBR322 origin of replication, a gene expressing the laqI^{Q} repressor protein, and a semisynthetic *E. coli* promoter (pA1) that is repressed by the *lac*I repressor was modified. The following two oligonucleotides were separately synthesized, annealed to form a duplex with sticky ends, and inserted into cut plasmid. A synthetic linker/adapter consisting of annealed oligonucleotides (SEQ ID NOS:204 and 205, shown below)
ATG #P64-S1 (SEQ ID NO:204):
   CTAGGAAAAAAAAAGGTACCAAAAAAAAAGGCCGGCCACTAGTG
ATG #P64-A1 (SEQ ID NO:205):
   CTTTTTTTTTCCATGGTTTTTTTTTCCGGCCGGTGATCACAGCT
was prepared and cloned into the *Avr*II/*Sal*I sites of plasmid pDRK-N(M), to convert the polylinker following the fusion peptide from *Avr*II*-Dra*III*-Sal*I to *Avr*II--spacer-KpnI--spacer--*Fse*I--*Spe*I--*Sal*I. The resulting colonies were screened for introduction of an *Spe*I site carried by the linker/adapter. One positive clone ("pA1-NB-Avr-2") was selected and confirmed by DNA sequencing across the linker/adapter region.

A PCR fragment (411 bp) representing the 5' end of the *Tth dnaE* gene was generated using primers (ATG#P69-S561; SEQ ID NO:206) and (ATG#P69-A971; SEQ ID NO:207):
ATG#P69-S561 (SEQ ID NO:206): GAATTCCTAGGCCGCAAACTCCGCTTC
ATG#P69-A971 (SEQ ID NO:207): GTGCTCGCGCAGGATCTCCCGGTCAATC

The product was cleaved with *Avr*II and *Kpn*I*,* and the resulting 320 bp fragment was cloned into the corresponding sites of plasmid pA1-NB-Arv-2. The clones were screened for introduction of a 320 bp *Avr*II/*Kpn*I fragment. One positive clone ("pAl-NB-TE(5')") was selected, and the sequence across the PCR fragment was confirmed by DNA sequencing.

A 3454 bp *Kpn*I/*Fse*I fragment representing the 3'-end of the *Tth dna*E gene was isolated from the 4.9 kb *Bam*HI fragment cloned into "pBSIIKS+" (*See,* Example 5, part G), cloned into the corresponding sites of plasmid pA1-TE(5'), and screened for the 3454 bp *Kpn*I/*Fse*I fragment. Additional confirmation of the plasmid structure was obtained by analysis of *Nde*I/*Spe*I*, Pst*I*,* and *Avr*II/*Fse*I digests. Plasmid preparation from an 80 ml culture of pA1-NB-TE, yielded approximately 130 µg of DNA. The plasmid pA1-NB-TE was transformed into *E. coli* strain MGC 1030, individual colonies were selected, and screened for expression.

### 5. Verification of Expression of Protein Containing an Amino-Terminal Biotinylated Peptide Tag Fused to Tth DNA Polymerase III α Subunit Expressed by pA1-NB-TE/MGC 1030

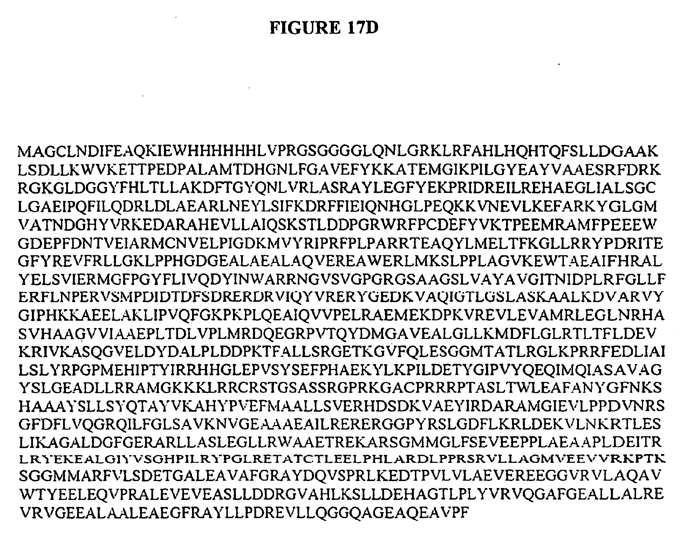

An overnight culture of three suspected biotinylated *Tth* α-expressing plasmids was inoculated 1:50 into 2x YT culture medium containing 100 µg/ml ampicillin, and grown at 30°C, with shaking, until the OD₆₀₀ reached approximately 0.6. Protein expression was then induced with IPTG, at a final concentration of 1 mM, followed by the addition of biotin at a final concentration of 10 µM. The control culture contained biotin, but was not induced with IPTG. After 3 hours of induction, the OD₆₀₀ of the culture was determined and the cells were harvested by centrifugation. Cell pellets were resuspended in 2x Laemelli sample buffer at 70 µl/OD₆₀₀, and sonicated to shear the DNA. Samples were heated for 10 minutes at 90-100°C, and centrifuged to remove cell debris. A 3 µl aliquot (corresponding to 0.0429 OD₆₀₀ units) was loaded onto an SDS-PAGE gel, electrophoresed, and stained with Coomassie blue. An intensely staining band representing at least 3% of total *E. coli* protein was observed. This band, which was not present in the uninduced control, migrated between the β/β' subunits of RNA polymerase and the high molecular weight standard of the Gibco 10 kDa protein ladder (120 kDa), consistent with the expected molecular weight (140,897 Da). The expected sequence of the expressed protein is shown in Figure 17D.

Biotin blots (*See,* Example 8, and Kim and McHenry, J. Biol. Chem., *supra*) were also prepared, in order to confirm the presence of a biotinylated tag on the expressed protein from pA1-NB-TE. Separated proteins from the SDS-PAGE gel described above were transferred to a nitrocellulose membrane at 30 volts, in 12 mM Tris base, 96 mM glycine, 0.01% SDS, and 20% methanol, for 60 minutes at room temperature. Each lane of the gel contained 1 µl of supernatant, corresponding to 0.0143 OD₆₀₀ units of the culture material. In all three cultures tested that expressed a novel protein of the expected molecular weight, a strong biotinylated band was detected that was at least as intense as the endogenous E. *coli* biotin carrier protein band at approximately 20 kDa.

### 6. Large-Scale Growth of pA1-NB-TE/MGC 1030

Strain MGC 1030 (pA1-NB-TE) was grown in a 250 L fermentor, to produce cells for purification of peptide-tagged *Tth* α. F-medium (1.4% yeast extract, 0.8% tryptone, 1.2% K₂HPO₄, and 0.12% KH₂PO₄) was sterilized, and ampicillin (100 mg/L) and kanamycin (35 mg/L) were added. A large-scale inoculum was initiated from 1 ml of DMSO stock culture *(i.e.,* culture stored in DMSO) (to 28 L), and grown overnight at 37°C. The inoculum was transferred (approximately 16.7 L) to the 250 L fermentor (starting OD₆₀₀ of 0.05), containing F-medium with 1% glucose, and 100 mg/L ampicillin. The culture was incubated at 37°C, with 40 LPM aeration, and 20 rpm. Expression of biotin-tagged *Tth* DnaE was induced when the culture reached an OD₆₀₀ of 0.71. Additional ampicillin (100 mg/L) and biotin (10 µM) were added at induction with 1 mM IPTG. Additional ampicillin (100 mg/L) was added after 1 hour of induction. Cell harvest was initiated 3 hours after induction, and the cells were chilled to 19°C during harvest. The harvest volume was approximately 182 L, and the final harvest weight was approximately 2.35 kg of cell paste. An equal amount (w/w) of 50 mM Tris (pH 7.5) and 10% sucrose solution was added to the cell paste. Quality control results showed 5 out of 10 positive colonies on ampicillin-containing medium before induction and 5 out of 10 positive colonies post-induction. Cells were frozen by pouring the cell suspension into liquid nitrogen, and stored at -80°C, until processed. Figure 17D shows the deduced amino acid sequence (SEQ ID NO:223) of *Tth* DnaE, containing a biotin/hexahis tag on the amino terminus.

### 7. Cell Lysis of pA1-NB-TE/MGC 1030 and Determination of Optimal Ammonium Sulfate Precipitation Conditions for Biotinylation/Hexahis Tagging of Tth α (DnaE)

Cells (100 g) obtained from the fermentation described above, were subjected to a lysis procedure equivalent to that described for cells expressing biotinylated *Tth* τ (DnaX), in Example 8. The lysate supernatant (400 ml) was found to contain 9.2 g total protein. The supernatant was divided into 6 separate 60 ml aliquots. Based on this protein concentration, each aliquot originally contained 1,370 mg total protein. Ammonium sulfate sufficient to achieve 35, 40, 45, 50, 60, and 70% saturation at 0°C, was added to each of the cultures. After dissolving the ammonium sulfate, each aliquot was stored on ice for 30 minutes. Precipitated protein was then recovered by centrifugation (12,000 rpm in Sorvall GSA rotor, at 0°C). A separate (*i.e.*, additional) aliquot (0.5 ml) was also taken for analysis, and the pellet recovered by centrifugation in a microfuge (5 min at 14,000 rpm, at 4°C). Two volumes of saturated ammonium sulfate were then added to the recovered supernatants. After storage on ice, the precipitated protein pellets were recovered as described above. This procedure permitted the estimation of the quantity of biotinylated α subunit in both the supernatant and the pellet after the initial precipitation.

Pellets from each of the aliquots (both pellets and supernatants) were taken and dissolved in the original volume with buffer T+25 (containing 50 mM Tris-HCl (pH 7.5), 20% glycerol, 5 mM DTT, 0.1 mM EDTA, and 25 mM NaCl), and the total protein determined using the Pierce's Bradford reagent and method, with BSA used as a standard. Based on this value, 24, 54, 95, 439, 1280, and 1230 mg protein were recovered from the 35, 40, 45, 50, 60, and 70% ammonium sulfate pellets, respectively.

Each pellet was then analyzed for the presence of biotinylated *Tth* DNA polymerase III α subunit by biotin blot analysis, as described in Example 8, as well as by SDS-PAGE analysis and Coomassie Blue staining. For these analyses, 10 µg of each aliquot were used and either stained with Coomassie Blue, or transferred to a nitrocellulose membrane for biotin blotting. Based on these results, it was apparent that significant levels of *Tth* α precipitated even in the lowest (35% saturation) ammonium sulfate concentration, and that no biotinylated *Tth* α was detectable in the supernatant of the 50% saturated ammonium sulfate supernatant. The ratio of biotinylated *Tth* α total protein was highest in the 35% pellet, and was decreased by approximately 3-fold in the 45% pellet. Precipitation with 45% ammonium sulfate was selected as the precipitation condition to provide material for development of additional purification methods.

### 8. Purification of N-Terminal Biotin/Hexahis Tagged Tth α

A 45% saturated ammonium sulfate precipitate obtained from 30 g of cells was prepared by the procedure described above. The resulting pellet contained 141 mg protein. The pellet was dissolved in 10 ml buffer EB (50 mM sodium phosphate (pH 7.6), 300 mM NaCl, 5 mM 2-mercaptoethanol), and applied to a 1.1 ml Qiagen Ni-NTA agarose column equilibrated in buffer EB. The column was washed with 24 ml buffer EB containing 1 mM imidazole, and was eluted with a 12-column volume gradient, in buffer EB with the imidazole concentration ranging from 1 to 100 mM. Activity, as measured by the gap filling assay at 30°C, eluted with a peak at approximately tube 22 of 26 (each tube contained 0.5 ml) collected. The pooled peak (fractions 17-23) contained 2.2 mg protein, and 6.5 x 10⁵ units of gap-filling activity, of the 1.4 x 10⁶ gap filling units applied to the column.

### 9. Analysis of Molecular Weight and Purity of Purified Biotin/Hexahis Tagged Tth Alpha

Samples of the peak fractions obtained from the Ni-NTA column described above, was subjected to SDS-PAGE, using two SDS-PAGE gels, along with molecular weight standards (NEB). One gel was stained with Coomassie Blue, and the proteins present in the other gel were transferred to a nitrocellulose membrane for biotin blotting. Purified *Tth* biotin/hexahis tagged α, detected by biotin blot, migrated 3 mm farther than a biotinylated 165,000 fusion of the maltose binding protein and β-galactosidase, and 7 mm less than biotinylated rabbit muscle phosphorylase, indicating a molecular weight near 147,000 Da. This value is consistent within the error of the procedure with an expected value of approximately 141,000 Da. Tagged *Tth* α was found to migrate more slowly than its *E. coli* counterpart.

On Coomassie-stained SDS-PAGE gels, the major band corresponding to both the band induced with IPTG detectable from the crude extracts, and the biotinylated band from crude and purified protein, represented greater than 30% of the total protein on the gel, and was at least 6-fold more intense than the most abundant contaminants.

### 10. Determination of Temperature Optimum for Catalytic Activity of Purified Tth α Subunit

This section describes the determination of the temperature optimum for DNA synthesis on a gapped template by purified Tth N-terminal Biotin/Hexahis tagged DnaE (α).

A gap filling assay on nuclease-activated DNA was performed at varying temperatures to determine the temperate optimum for the purified *Tth* α subunit of DNA polymerase III. Biotin/hexahis tagged α obtained from Ni++-NTA chromatography described in Example 5, Section H, Subpart 8, was diluted 50-fold in EDB (enzyme dilution buffer) (50 mM HEPES (pH 7.5), 20% glycerol, 0.02% NP40, 0.2 mg/ml BSA) on ice and 0.5, 1 and 2 µl were pipetted into separate tubes and prewarmed for 3 minutes to the specified temperature. A premix of assay solution was made by pipetting (per tube in assay set) 18 µl EDB, 3 µl dNTPs (400 µM dCTP, dATP, dGTP, 150 µM [³H]dTTP (96 cpm/pmol total nucleotide), 1 µl 250 mM MgCl₂, 1 µl nuclease activated salmon sperm DNA (5 mg/ml) (Enzyco) to each tube. Then, 23 µl of the assay solution was prewarmed to the designated temperature and pipetted into the diluted enzyme solution to initiate the reaction. Incubation was continued for an additional five minutes. Upon completion of the assay, tubes were transferred to ice and 2 drops of (0.2 M sodium pyrophosphate) and 0.5 ml of 10% TCA were added. The resulting suspension was then filtered over GFC filters (Whatman) prewetted with the acid wash solution (1 M HCl, 0.2 M sodium pyrophosphate) and washed with an additional 12 ml of acid solution. The filters were then washed with 4 ml 95% ethanol, dried, and the bound radioactivity determined by liquid scintillation counting. One unit of enzyme is defined as that amount of polymerase that incorporates 1 pmol total nucleotide into acid insoluble DNA/minute.

The same assay was used to monitor purifications described in Example 5, with the exception being that as appropriate, the assays were conducted at 30°C, and the enzyme was diluted to a point where the amount added to the assay gave a response in the linear range (*i.e.*, amount of enzyme where radioactivity incorporated was proportional to amount of enzyme added). In these assays, enzyme was added to the assay mix on ice, and the entire solution was transferred to a 30°C water bath.

Conducting the assay at 30, 40, 50, 52.5, 55, 57.5, 60, 62.5, 65, 70, and 80°C, revealed a temperature optimum of approximately 60°C, a temperature that is clearly higher than that of *E. coli* DNA polymerases. The activity of the enzyme (units/µl) at the above temperatures was 19, 74, 290, 390, 400, 490, 500, 500, 460, 390 and 170, respectively.

### 11. Purification of Tth DNA Polymerase III α Subunit to Homogeneity

The *Tth* DNA polymerase is purified to homogeneity. Ammonium sulfate fractionation experiments, similar to those described above (*See,* section 7, above), with the exception being that the catalytic gap filling assays are conducted at an elevated optimal temperature for *Tth* α (*e.g.,* 60°C). As inactivation of the *E. coli* protein is achieved by adding the protein to be assayed to pre-warmed tubes, an unambiguous determination of the distribution of the *Tth* protein is obtained. The ammonium sulfate concentration required for reproducible precipitation of at least 80% of *Tth* activity is determined. Backwashing procedures are then developed, using approximately 1/20 of the initial lysate volume of ammonium sulfate that results in extraction of the maximal level of contaminants, while leaving most of the *Tth* α subunit in the pellet. The concentration of ammonium sulfate to be used in the backwash solution that yields at least 60% of the initially pelleted *Tth* α activity and optimal purity is then determined. In the situation in which activity does not provide a suitable assay because of low level wild-type α expression or other causes, antibodies directed against the purified tagged α are produced, and Western blots used to quantitate the distribution of *Tth* α, in a manner similar to that used for the biotin blots to monitor biotin-tagged *Tth* α. In some embodiments, particularly in cases where the level of *Tth* α expression is unsatisfactory, up to the first 30 codons of the *Tth* α gene are replaced with AT-rich codons commonly used by *E. coli,* yet coding for the same amino acid sequence (*i.e.*, to improve expression).

Once an optimal method for production of an ammonium sulfate fraction containing wild-type *Tth* α is achieved, the obtained material is redissolved in buffer I (50 mM imidazole-HCl (pH 6.8), 20% glycerol, 5 mM DTT, 0.1 mM EDTA), and dialyzed against buffer I plus 25 mM NaCl. The solution is then applied to a BioRex-70 column (BioRad), equilibrated with buffer I, after further dilution to the conductivity of buffer I plus 25 mM NaCl. Or, other ionic strengths are determined in pilot experiments to result in binding of *Tth* α to the column. The column is then washed with buffer I containing a salt level that does not elute *Tth* α (approximately 25 mM NaCl), and then the activity is eluted with a 10-column volume gradient of from 25 to 300 mM NaCl in buffer I. It is contemplated that in some situations, higher salt concentrations are needed, in order to elute activity. Indeed, it is contemplated that this may be necessary with any of the columns described in these Examples.

The peak is detected by the gap filling assay previously described. Fractions containing the highest specific activity (*i.e.,* no less than half of the specific activity of the most pure fraction) are pooled. In any case, no activity that comprises less than 40% of the tube containing the peak activity is pooled. The pooled activity is then precipitated by the addition of ammonium sulfate to 60% saturation and the precipitated protein is recovered by centrifugation.

*Tth* α is then further purified using Toyo-Pearl Ether chromatography using buffers and conditions similar to those described in Example 2, except that the column is loaded at approximately 4 mg/ml of column packing material, and the divalent ions and ATP may be excluded from the buffers. The same provisions apply to increasing or decreasing the load, as described above for the BioRex-70 column. In cases where *Tth* α does not bind satisfactorily to the column, ToyoPearlPhenyl 650 M columns are substituted. It is also contemplated that similar materials made by other manufacturers (*e.g.*, Pharmacia) will find use in the present invention.

Precipitated material is then subjected to anion exchange chromatography, unless SDS-PAGE gels indicate that the protein is already greater than 95% pure. The redissolved pellet is dialyzed in buffer I, and applied to a Q-Sepharose column equilibrated with buffer I, at a load of approximately 4 mg/ml resin, with the same provisions as indicated above. The column is then washed with buffer I containing a salt concentration that does not elute *Tth* α (*e.g.,* approximately 50 mM), and the column is eluted with a gradient starting with the wash buffer and ending with 300 mM NaCl. The peak fractions are selected and precipitated by addition of ammonium sulfate to 60% saturation, and the pellet is recovered by centrifugation.

The final step in the purification involves Sephacryl S-300 chromatography. A Sephacryl S-300 column is equilibrated in 20 mM potassium phosphate (pH 6.5), 0.1 mM EDTA, 5 mM DTT, 20% glycerol, and 100 mM KCl). *Tth* α is then dissolved in 5-20 mg/ml of the same buffer and 1-2% of the total column volume. The column is eluted with the equilibration buffer. Pooled peak fractions are rapidly frozen in liquid nitrogen and stored at -80°C for further use.

### EXAMPLE 6

### Cloning and Sequencing the T. Thermophilus dnaA Gene

### A. Design of PCR Primers

The PCR primers for *dna*A were designed by using highly conserved amino acid sequences from regions of the dnaA gene in a variety of bacteria. For the design of the forward primers, the consensus sequence (SEQ ID NO:118) was derived from the following regions of homology (SEQ ID NOS:119-132):

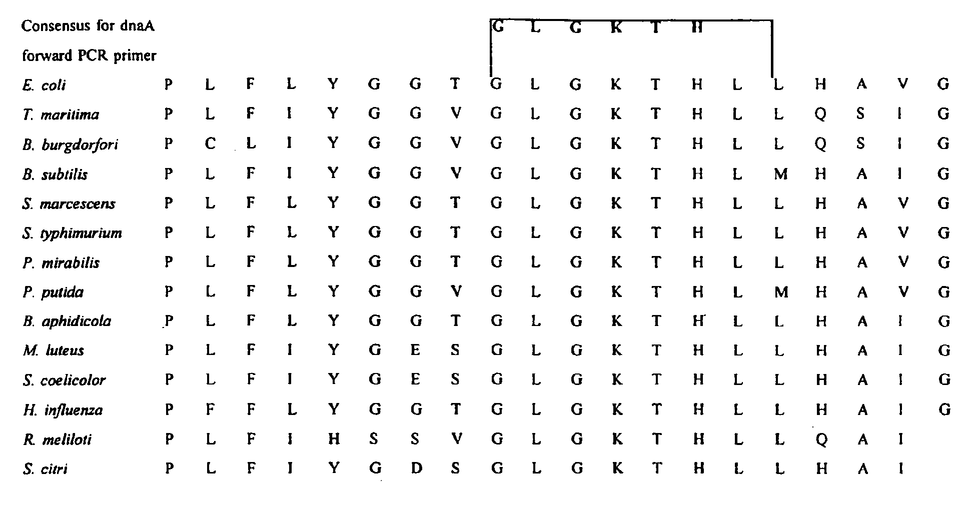

The amino acid sequence used for the design of dnaA forward primers was Gly Leu Gly Lys Thr His (SEQ ID NO:133). The Forward Primer A177Fa had the sequence 5'-GGNYTNGGNAARACSCAT- 3' (SEQ ID NO:134); the Forward Primer A177Fb had the sequence 5'-GGNYTNGGNAARACSCAC-3' (SEQ ID NO:135); the Forward Primer A177Fc had the sequence 5'-GGNYTNGGNAARACWCAT-3' (SEQ ID NO:136); while the Forward Primer A177Fc had the sequence 5'-GGNYTNGGNAARACWCAC-3' (SEQ ID NO:137). Four primers were used to keep degeneracy at or under 512-fold. They varied in codon 5 and 6.

For the design of the reverse primers, the consensus sequence (SEQ ID NO:138) was derived from the following regions of homology (SEQ ID NOS:139-154):

| | | | | | | | | | | | | | | | | | | | | | | | |
|---|---|---|---|---|---|---|---|---|---|---|---|---|---|---|---|---|---|---|---|---|---|---|---|
| Consensus Sequence | | | | | | E | L/ | F | H | T | F | N | | | | | | | | | | | |
| for dnaA reverse | | | | | | | F | | | | | | | | | | | | | | | | |
| PCR primer | | | | | | | | | | | | | | | | | | | | | | | |
| *E. coli* | | | | Q | E | E | F | F | H | T | F | N | A | L | L | E | G | N | Q | Q | I | I | L |
| *T. maritima* | T | G | V | Q | T | E | L | F | H | T | F | N | E | L | H | D | S | G | K | Q | I | V | I |
| *M. leprae* | | G | I | Q | E | E | F | F | H | T | F | N | T | L | H | N | A | N | K | Q | I | V | I |
| *B. subtilis* | | | | Q | E | E | F | F | H | T | F | N | T | L | H | E | E | S | K | Q | I | V | I |
| *B. burgdorferi* | | G | I | Q | E | E | L | F | H | T | F | N | A | L | Y | E | D | N | K | Q | L | V | |
| *S. coelicolor* | | | | Q | E | E | F | F | H | T | F | N | T | L | H | N | A | N | K | Q | I | V | L |
| *M. luteus* | | | | | | E | F | F | H | T | F | N | T | L | Y | N | N | N | K | Q | V | V | I |
| *H. influenza* | | | | Q | E | E | F | F | H | I | F | N | S | L | F | E | T | G | R | Q | I | I | L |
| *P. putida* | | | | Q | E | E | F | F | H | T | F | N | A | L | L | E | E | G | Q | Q | V | I | L |
| *B. aphidicola* | | | | Q | E | E | F | F | H | T | F | N | A | L | L | E | G | N | Q | Q | I | I | L |
| *S. marcescens* | | | | Q | E | E | F | F | H | T | F | N | A | L | L | E | G | N | Q | Q | I | I | L |
| *S. typhimurium* | | | | Q | E | E | F | F | H | T | F | N | A | L | L | E | G | N | Q | Q | I | I | L |
| *P. mirabilis* | | | | Q | E | E | F | F | H | T | F | N | A | L | L | E | G | N | Q | Q | I | I | L |
| *Wolbachia* | | | | | | E | F | F | K | T | F | N | A | L | I | D | Q | N | K | Q | L | V | I |
| *R. meliloti* | | | I | Q | H | E | F | C | H | L | L | N | M | L | L | D | S | A | K | Q | V | V | V |
| *S. citri* | | | | | | | | F | H | I | F | N | S | Y | I | E | K | N | K | Q | I | V | I |

The amino acid sequence for dnaA reverse primers was Glu Leu/Phe Phe His Thr Phe Asn (SEQ ID NO:155). The reverse PCR primers for dnaA were reverse primer A251Ra [5'-TTRAANGTRTGRAANAAYTC-3' (SEQ ID NO:156)]; reverse primer A251Rb [5'-TTRAANGTRTGRAANAGYTC-3' (SEQ ID NO:157)] (SEQ ID NO:67).

### B. PCR Amplification and Cloning of T. Thermophilus dnaA Probe

PCR amplification of dnaA was carried out using the Boehringer Mannheim Expand™ long template PCR system as described above except that the following conditions were used: The annealing steps were conducted at 55°C, elongation at 68°C and the melting step at 94°C; 26 total cycles were run. PCR-amplified *dna*A probe was separated on 2% FMC Metaphor agarose gels, visualized by Sybr green-1 staining, cloned into vector pCRII (Invitrogen), and sequenced as described above. The primer pair that yielded product of the expected length (237 bp) was A177 Fb and A 251Rb.

A roughly 237 bp and a 210 bp band were excised from the gel and cloned into pCrII (Invitrogen) as previously described for dnaX and dnaE. Five colonies from each of the 2 ligations were tested for the presence of insert by digestion with *Eco*RI*,* which releases cloned inserts from the pCRII vector. All five of the dnaA clones from the 237 bp DNA ligation had inserts. Four out of 5 clones using the 210 bp DNA had inserts. Two colonies from the 237 bp insert clones were chosen at random, clones (A237.A and clone A237.E). These clones were grown up for plasmid purification using the Qiagen procedure (described previously). Plasmid pA237.A (DMSO dnaA L-A) was sent for sequencing (Fort Collins) and shown by sequence to have homology to dnaA.

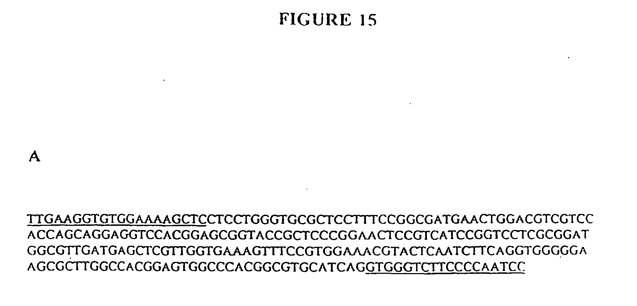

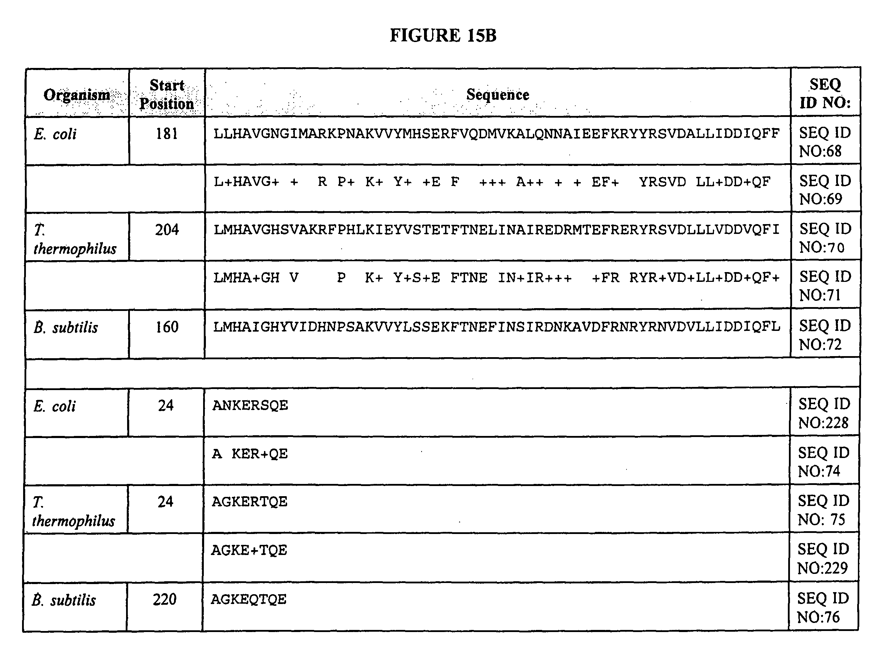

The results are shown in Figure 15. Figure 15A shows the deduced nucleotide sequence of a portion of *T. thermophilus dnaA* (SEQ ID NO:65). In this Figure, the sequences corresponding to primers are underlined (SEQ ID NOS:66 and 67). The DNA sequence in Figure 15A was found to be equivalent to the message. A BLAST search of the sequence between the regions corresponding to the primers was conducted. These results revealed a strong homology to the structural genes encoding bacterial DnaA origin binding protein. The corresponding segment of *B. subtilis dna*A showed 58% identity over a 68 amino acid stretch. The corresponding *E. coli dna*A showed 45% identity over 68 residues. As with other Figures, the intervening designations indicate identical residues. Figure 15B shows the alignment results for *T*. *thermophilus, E. coli,* and *B. subtilis.* In Figure 15B, the numbers refer to *B. subtilis* (SEQ ID NOS:72 and 76) and *E. coli* (SEQ ID NOS:68 and 73) amino acid residues, as appropriate; the numbers for *T. thermophilus* (SEQ ID NOS:70 and 75) refer to the first base of the anticodon for the shown amino acid residue to the left of the corresponding primer sequence near the 3' end of the shown sequence. In Figure 15, homologous sequences are indicated (SEQ ID NOS:69, 71, and 74).

### C. Identification of the T. thermophilus dnaA Gene

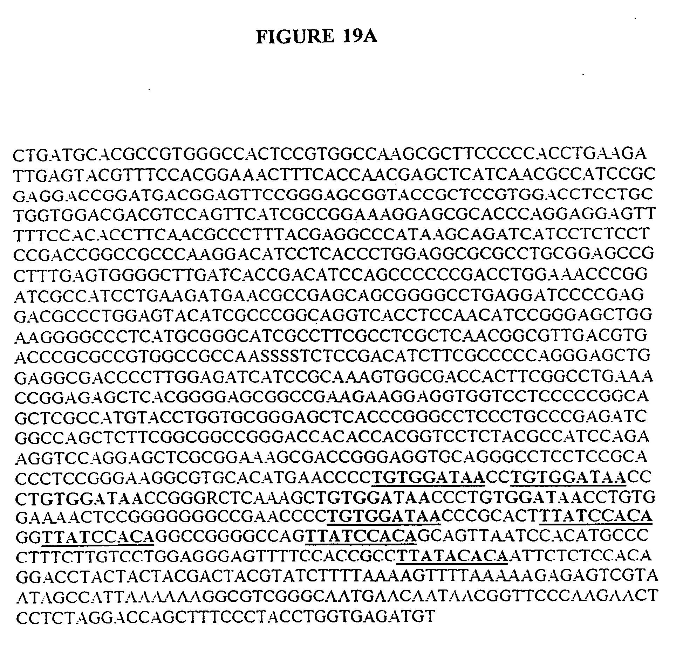

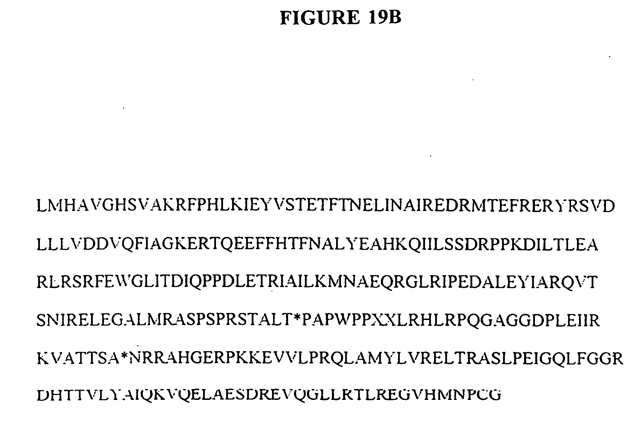

Probe A237 was also used to screen a lambda library using the methods described above for *dna*E (*i.e.,* probe isolated and labeled as for the other oligonucleotides). Over 100 candidate positive clones were identified; two were grown up and the DNA purified as described for dnaE. The first clone (probel-cl#3.1) was sequenced using a primer designed from the sequence of plasmid pA237.A *(See,* Figure 15A). It was discovered that the clone terminated and vector sequence was encountered before the carboxyl terminus of dnaA was reached. The second clone (probe 1-cl#8.1) was sequenced to the end of the gene. Together with merged sequence obtained from the initial PCR probe (Figure 15A), a sequence of 1229 bp (SEQ ID NO:221) was obtained (*See*, Figure 19A). The 5' portion of this sequence translated to an open reading frame, with the exception of stop codons at amino acid positions 174 and 208 (SEQ ID NO:222) (Figure 19B). Performance of a BLAST search substituting "X" (*i.e.,* indicating an unknown amino acid for these positions), indicated a high level of homology with eubacterial dnaA replication origin binding proteins. A gap in the alignment was identified between positions 162 and 219. This information coupled with the stop codons at positions 174 and 208 indicate an error in reading frame for this region. Nevertheless, this information was sufficient for unambiguous identification of the gene.

Following the *Tth dna*A gene, at least 10 copies of the DnaA protein binding site (TTAT(C/A)CACA, and reverse complement; shown in bold and underlined in Figure 19A), appear to be be followed on the 3' end by AT-rich sequences, a feature common to eubacterial replication origins. Thus, this region probably represents the *Tth* chromosomal replication origin. Within the AT-rich segment were two *Dra*I restriction sites, sites that are expected to be rare in GC-rich organisms such as thermophiles. These sites were exploited in a Southern blot, to determine the structure of the downstream sequences.

Lambda DNA carrying the *Tth dna*A gene and downstream sequences (probe 1-cl#8.1) was digested with *Dra*I and a second restriction enzyme and subjected to Southern analysis using an oligonucleotide selected from sequences downstream of the second *Dra*I site. A *Dra*I*-Eco*RI digest yielded and approximately 2.2 kb fragment, indicating that at least 2.2 kb of *Tth* DNA remained downstream before the lambda cloning vector polylinker region. Digestion with *Dra*I and *Bam*HI yielded an approximately 900 bp fragment. This information was used for obtaining the *Tth dnaN* gene as described in Example 9.

### EXAMPLE 7

### Cloning and Sequencing of the T. Thermophilus dnaQ Gene

### A. Design of PCR Primers

Primers for *dna*Q were designed from sequences conserved in the epsilon subunits of bacteria and phage.

**TABLE 3. Primers for dnaQ**

| **CONSENSUS** | **D T/I/V E T T G** | **H N A A/S F D** |
|---|---|---|
| *E. coli* | IVLDTETTGMNQI (SEQ ID NO: 158) | LVIHNAA-FDIGFM (SEQ ID NO: 159) |
| *H. haemolyticus* | IVLDTETTGMNQI (SEQ ID NO: 160) | LVIHNAP-FDIGFM (SEQ ID NO:161) |
| *B. aphidicola* | IVLDTETTGMNSV (SEQ ID NO: 162) | LVIHNAS-FDVGFI (SEQ ID NO:163) |
| *B. subtilis* | VVFDVETTGLSAV (SEQ ID NO: 164) | LVIHNAA-FDMG (SEQ ID NO: 165) |
| *M. genitalium* | VIFDIETTGLHGR (SEQ ID NO: 166) | MVAHNGINFDLPFL (SEQ ID NO: 167) |
| *M. pulmonis* | VVYDIETTGLSPM (SEQ ID NO:168) | MVAHNAA-FDHNFL (SEQ ID NO: 169) |
| *S. aureus* | VVFDVETTGLSNQ (SEQ ID NO:170) | FVAHNAS-FDMGFI (SEQ ID NO:171) |

Two forward primers were designed for each forward and reverse sequence for *dna*Q in order to reduce degeneracy. The amino acid sequence used for the design of forward primers was Asp Thr/Ile/Val Glu Thr Thr Gly ((SEQ ID NO:172). The first forward primer (Q12Fa) had the sequence 5'-GAYACNGARACNACNGG-3' (SEQ ID NO:173), while the second forward primer (Q12Fb) had the sequence 5'-GAYRTNGARACNACNGG-3' (SEQ ID NO:174). Both forward primers were equivalent except for the second codon which encodes Thr, in order to keep the degeneracy below 512-fold, since Thr was found in the gram negative bacteria (primer Q12Fa), while Ile or Val were found in some gram positive bacteria (primer Q12Fb). The amino acid sequence for the reverse primers was His Asn Ala Ala/Ser Phe Asp (SEQ ID NO:175). The first reverse primer (Q98Ra) had the sequence 5'-TCRAANGCNGCRTTRTG-3' (SEQ ID NO:176), while the second reverse primer (Q98Rb) had the sequence 5'-TCRAANSWNGCRTTRTG-3' (SEQ ID NO:177). Both the reverse primers were equivalent except for the fourth codon (from the 3' end) which encoded Ala (primer Q98Ra) and Ser (primer Q98Rb).

### B. PCR Amplification of T. Thermophilus dnaQ probe

PCR amplification, gel analysis and sequencing of a *dna*Q probe was carried out as described above for the *dnaA probe.* Only primer combinations Q12Fa and Q98Ra gave a PCR product of the expected size. The Q12Fa/98Ra primer combination gave a single intense sharp band of approximately 270 bp and 8 additional bands of high molecular weight (6 to 14 kb).

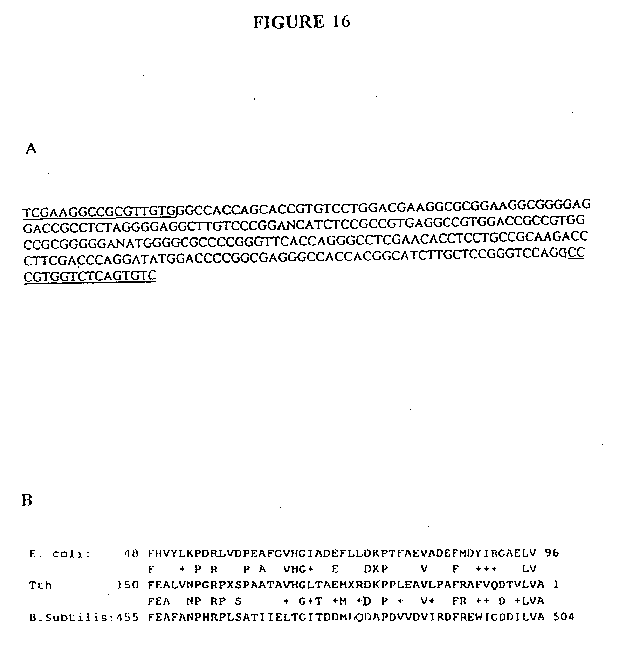

An approximately 264 bp band resulting from amplification of *T*. *thermophilus* chromosomal DNA preparation B using primers Q12Fa and Q98Ra was excised from a 2% Metaphor agarose (1x TAE) and cloned as described for the PCR probes for dnaE and dnaX. Five colonies from the clones were chosen and plasmid isolated using the Promega plus Minipreps. The plasmid DNA from the five colonies were tested for the presence of insert by digestion with restriction endonuclease *Eco*RI, which releases cloned inserts from the pCRII vector. Four out of five showed the presence of insert. Two clones, named plasmid pMGC/QFA11A and QFA11E were chosen for further analysis. Plasmid pMGC/QFA11A (DMSO 1329) was sent was sent for sequencing (Fort Collins) and was shown to have sequence homology to dnaQ (Figure 16).

The results are shown in Figure 16A (SEQ ID NO:77). In this Figure, sequences corresponding to primers are underlined (SEQ ID NOS:78 and 79); the sequence shown in this Figure is the complement of the message strand. A BLAST search of the sequence between the regions corresponding to the primers revealed strong homology to the structural genes encoding bacterial proofreading exonucleases. The exonuclease domain of *B. subtilis* DNA polymerase III (SEQ ID NO:84) showed 40% identity over a 50 amino acid stretch. The epsilon proofreading subunit (ε) of the *E. coli* DNA polymerase III holoenzyme (*dna*Q) (SEQ ID NO:80), showed 32% identity over 49 amino acid residues. The amino acid sequences are shown in Figure 16B. In Figure 16B, for *B. subtilis* and *E. coli,* the numbers refer to amino acid residues, while for *T. thermophilus* (SEQ ID NO:82), the numbers refer to the first base of the anticodon for the shown amino acid residue to the left of the corresponding primer sequence near the 3' end of the shown sequence. As with other Figures, the intervening designations indicate identical residues (SEQ ID NOS:81 and 83).

Southern Blots were also conducted as described above, using above probe and digest of *T. thermophilus* DNA preparation B.

| | |
|---|---|
| *Hind*III | 7.55 Kb |
| *Hind*III/*Ehe*I | 2.25 kb |
| *Ehe*I | 3.4 kb |
| *Apa*LI | Very high molecular weight |
| *Apa*LI/ *Hind*III | 1.1 kb |
| *Apa*LI/*Ehe*I | 2.2 kb |

Plasmid pMGC/QFA11A (DMSO 1329) was also used as a probe to screen a lambda library using the same techniques described for *dnaE.* However, experiments using this probe failed to produce positive colonies as it had for *dnaE* and *dnaA.* Next, an oligonucleotide probe was designed, based on the sequence shown in Figure 16. With this probe (5'-CCT CGA ACA CCT CCT GCC GCA AGA CCC TTC GAC CCA-3'; SEQ ID NO:209), over 100 strong positive plaques were identified and verified by replating. Three were grown up and the DNA purified as described for *dnaE.*

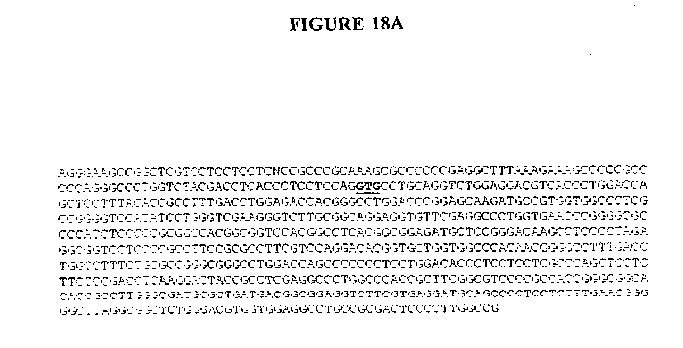

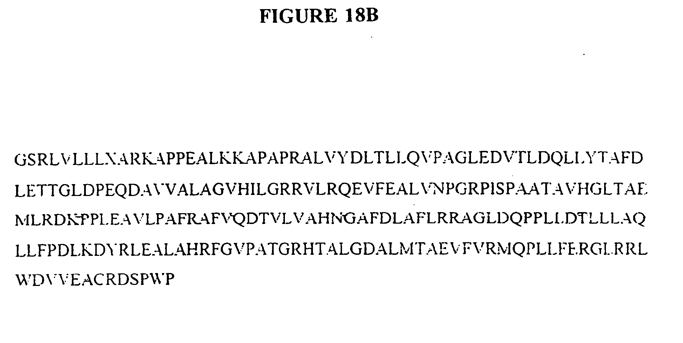

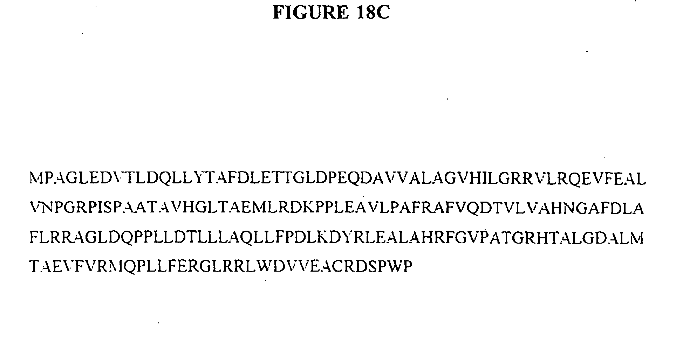

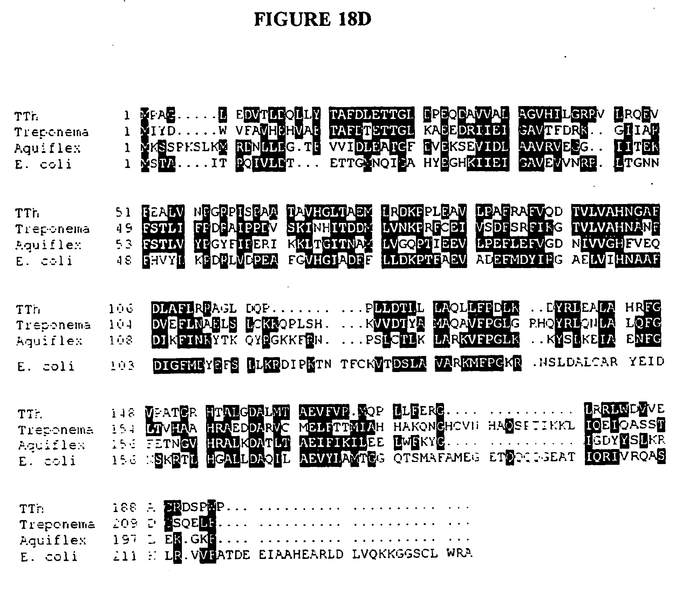

One (probe3-cl#5.1.1) was selected for further sequencing. The sequence (Fig. 18A) of a major portion of the gene was obtained by direct sequencing of the insert in the isolated lambda DNA using sequences selected from the PCR product (Fig. 18A; SEQ ID NO:214) to initiate sequencing. Upon preliminary examination of the sequence, it was found to encode one continuous open reading frame (Figure 18B; SEQ ID NO:215), that showed significant homology to other DNA polymerase III ε subunits from other bacteria (based on a BLAST search). However, alignment of the open reading frame with known protein sequences revealed no homology to the first thirty five (35) amino acids. No candidate ATG start sites were present before regions of homology were encountered. However, if the GTG found as the 36th codon was the initiating codon, a protein would be expressed (Figure 18C; SEQ ID NO:216), that gives good alignment with approximately the same distance from the initiating codon and regions of strong sequence conservation with other eubacterial ε subunits (Figure 18D). Interestingly, the carboxyl-terminus aligns with the carboxyl-terminal residue of the most closely homologous dnaQ genes from *Treponema pallidum* (SEQ ID NO:218) and *Aquiflex aeolicus* (SEQ ID NO:219), potentially indicating that all or almost all of the relevant sequence has been identified. The alignment also includes homologous sequence from *E. coli* (SEQ ID NO:220). A strong secondary structure or other block prevented obtaining more 5' sequence of the *Tth dna*Q gene.

To overcome this problem, a minimal fragment is subcloned into a plasmid and the plasmid sequenced, coming in from both the 5' and the 3' ends of the unknown sequence. To identify a suitable fragment for subcloning, a purified lambda DNA carrying the gene is digested with *Spf*I, to cleave the third codon of the gene. This digested DNA is then divided into a series of aliquots and a second digestion is then performed with one additional restriction enzyme (*Avr*II, *Cla*I*, Kpn*I*, Nco*I*, Nhe*I*, Rsa*I*, Sph*I*, Spl*I*, Spe*I*,* or *Acc*I). A control tube is mock-digested without a second enzyme. These digested DNAs are then subjected to SDS-PAGE and a Southern blot is performed, using a synthetic oligonucleotide selected from the known *Tth dna*Q coding sequence. Useful candidates for subcloning and sequencing are selected from those enzymes that yield products of between 700 and 2000 bps. Selected fragments are subcloned and sequenced, until the 5' end of the *Tth dna*Q gene is determined.

Upon determination of the entire sequence of the *Tth dna*Q gene, a vector is constructed that expresses the candidate *Tth* ε subunit fused by its amino-terminus to the biotin/hexahis tag (*i.e.*, as used in conjunction with the α subunit of *Tth* DNA polymerase III, as described in Example 5). This is accomplished by replacing the polylinker of pA1-NB-Arv-2 with a synthetic oligonucleotide containing, minimally, an *Arv*II sticky end, followed by an *Sbf*I site, to reconstruct the second and third codons of *Tth dna*Q*,* followed by a spacer and a site for an enzyme that cuts downstream of *dna*Q*,* as determined by Southern blotting (above). This site is followed by the spacer and the *Fse*I site and the sequence that follows *Fse*I found in the starting vector. The cloned *Tth dna*Q gene is cleaved with *Sbf*I and the selected downstream enzyme, and then cloned into the corresponding sites of the expression vector.

The *Tth* ε-fusion protein is expressed and purified by lysis, ammonium sulfate fractionation, and Ni-NTA chromatography using methods described in Examples 5 and 8. In the situation where the ε fusion protein is insoluble, it is solubilized in urea (6 M or higher), and chromatographed in the denatured state in the presence of urea on the Ni-NTA column. A second chromatographic step using soft-release avidin (*See*, Kim and McHenry, J. Biol. Chem., *supra*), is used as an antigen to obtain polyclonal or monoclonal antibodies that are used to purify the wild-type *Tth* ε subunit from *Tth* cells by immunoprecipitation or column immunoaffinity procedures, as known in the art. The isolated protein is further purified. Then, the amino-terminus and denatured molecular weight are determined by procedures described in other Examples, to confirm that the correct protein is expressed.

In the case that the amino terminus is generated from the proposed GTG start site, a vector is constructed that expresses wild-type ε by amplifying *Tth dna*Q in modified form with PCR primers that replace the initiating GTG with ATG, and precede the sequence with sequence that corresponds to a *Cla*I site. The 3' end PCR primer reproduces the translational termination site, followed by either *Bam*HI, *Xho*I, or *Xba*I, depending upon which site is absent from the *Tth dna*Q open reading frame. The PCR fragment is cleaved with restriction enzymes that recognize the terminal noncomplementarity sites used for PCR, and cloned into the corresponding sites of pA1-CB-ClaI (*See*, Example 8).

In the case that the analysis of the wild-type epsilon indicates more extensive sequence on the amino-terminal end, a Southern analysis is performed on the DNA digest described above using an oligonucleotide probe selected from the 5' side of the *Sbf*I site. Hybridizing fragments of increasing size are subcloned until a sequence is revealed that matches the true experimentally determined terminus of the *Tth dna*Q gene. This wild-type *Tth* ε is then expressed, using modifications of the procedures described above.

In either case, standard lysis procedures as described in Example 5, and optimized ammonium sulfate fractionation procedures as described in Examples 5 and 8, will be used, with an antibody directed against a portion of *Tth* epsilon, in order to maximize purification, with retention of at least half of the *Tth* epsilon protein. *Tth* epsilon is purified by cation, anion, hydrophobic, and gel filtration chromatographic procedures until homogenous. The purification is monitored by quantitative immunoblotting procedures and 3' to 5' exonuclease assays (*See,* Griep et al., Biochem., 29:9006-9014 [1990]), to ensure selection of procedures that provide good yields of active epsilon, and for selection of the most pure fractions, to provide material for additional purification steps. Native DnaQ (*Tth* epsilon) is used for the reconstitution of DNA polymerase III holoenzyme, and developed as an additive to improve the fidelity of thermophilic polymerases that do not contain proofreading exonucleases, and as an additive to remove unincorporated bases, permitting long PCR reactions to be performed with a variety of thermophilic polymerases.

The amino-terminal ε fusion proteins are also used to prepare affinity columns for the isolation of novel proteins that bind to DnaQ (α subunit) alone, as well as DnaQ present in a complex with the isolated DnaE protein. The structural genes for novel proteins found by this method are isolated, sequenced, expressed, purified, and used to determine whether they make contributions to the functional activity of the *T. thermophilus* DNA polymerase III holoenzyme.

### EXAMPLE 8

### Construction of Vectors Expressing Native T. thermophilus τ and γ Subunits

As the sequence of the *T. thermophilus dna*X gene is complete *(See,* Figure 9A), vectors were constructed that overproduce both the τ and γ subunits of the *T. thermophilus* DNA polymerase III holoenzyme in *E. coli.* Methods used to construct these vectors are similar to those followed previously for the corresponding *E. coli* subunits to overproduce native τ and γ (*See e.g.,* Dallmann et al., J. Biol. Chem., 270:29555-29562 [1995]).

### 1. Construction of the Starting Vectors

First, a vector, pDRK-C, (*See,* Kim and McHenry, J. Biol. Chem., 271: 20690-20698 [1996]) containing a pBR322 origin of replication, a gene expressing the lac I^{Q} repressor protein, and a semisynthetic *E. coli* promoter (pA1) that is repressed by the lacI repressor was modified. Plasmid pDRKC DNA was prepared and digested with *Bam*HI; the resulting 3' ends were filled in to the end of the coresponding template strand with the Klenow fragment of DNA pol I in the presence of Mg⁺⁺ and the four dNTPs (ATP, GTP, TTP, and CTP), and resealed with T4 DNA ligase, in the presence of 1 mM ATP. Plasmids were transformed into *E. coli,* plasmid-containing colonies were selected by ampicillin resistance, and the plasmds were prepared and screened for loss of the *Bam*HI site One of the colonies that contained plasmid that had not been cleaved by *Bam*HI was selected, grown, and used for preparation of the resulting plasmid pDRKC-Bam^{minus}.

pDRKC-Bam^{minus} was prepared and digested with *Xba*I and *Dra*III to remove a small polylinker (this removed polylinker contains *Xba*I*-Nco*I*-Not*I*-Dra*III sites). The following oligonucleotide was synthesized and inserted into the digested plasmid:

The conversion of the *Bam*HI site to GGATCGATCC, and the replacement of the original polylinker with SEQ ID NO:178 was confirmed by DNA sequencing, using methods known in the art. Creation of the filled-in *Bam*HI site was found to have created a *Cla*I site, but it is not cleaved if plasmid is puriifed from methylase-proficient *E. coli* strains.

The resulting plasmid pA1-CB-Cla-1 (previously referred to as pA1-CB-EBXXDS) contained the restriction sites within a polylinker to enable the following cloning steps. The following is a reproduction of the above oligonucletotides with the relevant sequences annotated:
5'CT*AGGAGG*TTTTAATC*GATG***CGGCC**GGATCC**TCGAG**TCTAGAC**ACTGG** ----CTCCAAAATTAGCTACGCCGGCCTAGGAGCTCAGATCTGTG-5' (SEQ ID NO:179). In this sequence, the following annotations apply:

CTAG--Sticky end for *Xba*I, but destroys site, so it is not recleaved
*AGGAGG* = rbs
ATCG*AT = Cla*I site
ATG = initiation codon
**CGGCCG** *= Eag*I site
GGATCC = *Bam*HI site
**CTCGAG** *= Xho*I site
TCTAGA *= Xba*I site
**CACTGG** = 3'-overhang to regenerate *Dra*III site

In parallel, a T7 promoter cloning vector was developed, such that the determination of which (*i.e.,* T7 or pA1) provided the best levels of soluble protein in a form amenable to further purification. The starting vector, pET11-KC (Kim and McHenry, J. Biol. Chem., 271:20690-20698 [1996]) contains a pBR322 replication origin, a copy of lacI and a T7 promoter. A *Cla*I site in the vector was destroyed and the polylinker was replaced with a synthetic one to enable further cloning steps. PET11-KC was prepared and cut with *Cla*I, then filled in and religated to destroy the site. The resulting plasmids were transformed into *E. coli,* and individual colonies were screened for plasmid that had lost the ability to be cleaved by *Cla*I. One colony was selected and used to prepare plasmid DNA. The resulting DNA, pET11-KC-Cla^{minus}, was then cut with *Xba*I and *Dra*III, and the same duplex replacement oligonucleotide described above are cloned into it, resulting in plasmid pET-CB-Cla-1 (previously referred to a pET-CB-CEBXXDS). The sequence of pET-CB-Cla-1 was confirmed by DNA sequencing, using methods known in the art.

### 2. Construction of Plasmids that Overexpress T. thermophilus τ and γ From the pA1 Promoter

Plasmid "pAX2S" (also known as "pUNC01") DNA was prepared from the stock strain DMSO 1386. A 465 bp segment corresponding to the amino-terminal end of *T. thermophilus* DnaX was PCR-amplified using primers P38-S1587 (TATCGATGAGCGCCCTCTACCG; SEQ ID NO:210)) and P38-A2050 (CGGTGGTGGCGAAGACGAAGAG; SEQ ID NO:211). The forward primer (P38-S 1587) added a *Cla*I site overlapping the initiation codon and changed the initiator TGT to ATG at the 5'-end of *dna*X. Addition of 5% DMSO was required for efficient amplification of the GC-rich template. The resulting PCR product was cloned into the pGEM-T Easy vector (Promega) using the pGEM-T Easy vector kits. This vector is supplied with a T-overhang for direct cloning of PCR fragments containing a nontemplated A-overhang from Taq polymerase. A clone was selected that carried a plasmid with an approximately 319 bp insert that could be removed by digestion with *Cla*I and *Bam*HI*.* The sequence of the DNA was verified through the region to be subcloned, and the approximately 319 bp *Cla*I*lBam*HI fragment from the PCR clone was subcloned into the corresponding sites of pA1-CB-Cla-1 resulting in the plasmid designated as "pA1-5'GX."

The C-terminal-coding portion of *T. thermophilus dna*X was removed from pAX2.S by cleavage with *Bam*HI and *Xba*II*,* and the resulting 1558 bp fragment was cloned into the corresponding sites of pAl-5'-GX, generating plasmid "pA1-TX" that should express *T. thermophilus* τ and γ. Plasmid was transformed into *E. coli,* and colonies were screened for the approximately 1559 bp fragment, as well as integrity of the *Spe*I and *Afl*II sites. One clone, "pAl-CB-TX" was selected. The DNA sequence across the 5'-cloning sites was verified by DNA sequencing.

### 3. Construction of pA1 Promoter-Containing Plasmids that Overexpress T. thermophilus τ Fused to a Carboxyl-Terminal Peptide That Contains Hexahistidine and a Biotinylation Site

The present invention also provides methods and compositions for expression *T. thermophilus* τ fused on its carboxyl-terminus to tagged peptides. This permits rapid purification and oriented immobilization to create an affinity column for isolation of additional *T. thermophilus* proteins that bind τ. During the development of the present invention, it was determined that *E. coli* τ tolerates fusion of foreign proteins to its C-terminus with preservation of activity. These observations were utilized to produce the fused *T. thermophilus* τ. In particular, the present invention provides methods and compositions for expression of *T. thermophilus* τ fused on its carboxyl-terminus to tagged peptides containing hexahistidine and a site that is biotinylated *in vivo* by the *E. coli* biotinylation enzyme. This permits rapid purification and oriented immobilization to create an affinity column for isolation of additional *T. thermophilus* proteins that bind τ.

In these experiments, the vector pDRK-C described above encodes a 30-residue peptide that is brought into frame with the C-terminus of *dna*X*.* This was accomplished by engineering a PCR product to contain a properly phased *Spe*I site in place of the normal termination codon of *T. thermophilus dna*X*.* PCR was then conducted using the cloned *T. thermophilus dna*X gene as a template. One PCR primer was internal to the *dnaX Afl*II site, while the second primer contained a cleavable *Spe*I site that is preceeded by the final 8 codons of *T. thermophilus* dnaX (excluding the stop codon). The PCR product was then cleaved with *Spe*I and *Afl*I*,* and inserted into the corresponding sites of the vector to generate plasmid "pA1-CB-Tth-dnaX" (also known as "pA1-CB-TX").

Specifically, a PCR reaction was conducted using primers P38-S2513 (forward primer; 5'-CCGCCATGACCGCCCTGGAC; SEQ ID NO:212) and P38-A3183 (reverse primer: 5'-ACTAGTTATACCAGTACCCCCTAT; SEQ ID NO:213). It was necessary to add DMSO to 5% final concentration to obtain amplification of the desired product from the GC-rich template. The PCR fragment was cloned into pGEM-T Easy, generating the plasmid "pT-geneX3'." Plasmids were transformed into *E. coli,* a plasmid containing colony selected and the DNA sequence of the cloned PCR product was confirmed. The approximately 610 *Afl*II*-Spe*I fragment was removed and cloned into the corresponding sites of pAl-TX, replacing the 3'-region of *T. thermophilus dna*X and bringing the C-terminus of *dna*X into frame with the desired fusion peptide. The plasmid was then transformed into *E. coli,* and colonies were screened for those that contained an approximately 610 bp *Afl*II*-Spe*I fragment and an approximately 1600 bp *Cla*I*-Spe*I fragment. The resulting plasmid was designated "pA1-CB-TX."

### 4. Placing the Sequences Expressing T. thermophilus τ and τ-Biotin/Hexahistidine Polypeptide Under Control of the T7 Promoter

Next, the *Cla*I*-Spe*I fragment containing the entire coding region of the *T. thermophilus dnaX* gene was removed from the two pA1 promoter driven expression vectors, pA1-TX and pA1-CB-TX, and placed into the corresponding sites of the T7 promoter driven expression vector pET-CB-Cla-1 to generate plasmids pET-TX and pET-CB-TX that express the wild type *T. thermophilus* DnaX proteins and DnaX protein fused to the described hexahistidine-biotinylation peptide respectively.

### Construction of pET-TX

An approximately 1.9 Kb *Cla*I*-Spe*I fragment was removed from pA1-TX and cloned into pET-CB-Cla-1 that had been cleaved with *Cla*I and *Spe*I. Plasmid was transformed into *E. coli,* and individual colonies were screened for those that contained plasmid carrying a 1.9 Kb *Cla*I*-Spe*I fragment and the expected *Spe*I, *Cla*I and *Bam*HI sites. A colony was selected and the plasmid it contained was named "pET-TX." This vector expresses the wild type *T. thermophilus dna*X gene under the control of a T7 promoter.

### Construction of pET CB-TX

An approximately 1.6 Kb *Cla*I-*Spe*I fragment was removed from pA1-CB-TX and cloned into pET-CB-Cla-1 that had been cleaved with *Cla*I and *Spe*I. Plasmid was transformed into *E. coli,* and individual colonies were screened for those that contained plasmid carrying a 1.6 Kb *Cla*I*-Spe*I fragment. A colony was selected and the plasmid it contained was named "pET-CB-TX." This vector expresses *T. thermophilus dnaX* fused on its carboxyl-terminus to the described hexahistidine-biotinylated peptide under the control of a T7 promoter.

### 5. Comparing Overproduction of Soluble T. thermophilus dnaX Protein From pA1-TX, pA1-CB-TX, pET-TX and pET-CB-TX

Next, DNA from the plasmids pA1-CB-TX and pET-CB-TX was prepared and transformed into strain MGC-1030 (mcrA, mcrB, lambda-, lexA3, uvrD::Tc, OmpT::Kn) and BL21 (DE3) (F⁻, ompT hsdS, gal, lysogen of lambda DE3 [carries gene for T7 RNA polymerase under the control of the lac uv5 promoter]; available from Enzyco) to generate a strain suitable for overproduction of *T. thermophilus* τ-fusion protein in *E. coli.*

To permit comparison of the levels of expression, these strains were first grown as overnight cultures in 2x YT medium (16 g tryptone, 10 g yeast extract, and 5 g NaCl per liter) with and without ampicillin. Control strains (MGC-1030 and BL21(DE3)) without plasmids and an overproducer of the *E. coli* τ fused at its C-terminus to the described hexahistidine/biotinylated peptide were also grown. After overnight incubation, the cultures were inoculated at a 1:50 dilution into 3 ml of like media and were allowed to grow until the cell density (measured as OD₆₀₀) reached approximately 0.6. The cells were induced with 1 mM IPTG, followed by addition of biotin to 10 µM. The cells were allowed to grow for 3 h post-induction before harvesting. The final OD of the cells at harvest was between 1.0 and 1.5.

The cells were lysed in reducing sample buffer, sonicated and heated to 100°C for 5 min, and the cell debris was removed by centrifugation immediately prior to loading on an SDS-PAGE gel. Cells (pellet resulting from 2.5 ml cell culture) were suspended in 2x Novex SB (sample buffer; #LC2678) (170-400 µl depending on OD of harvested cells, with 70 µl sample buffer/OD₆₀₀/ml), boiled for 5 min and immediately loaded onto a 4-20% gradient SDS-PAGE gel, and run at 135 constant volts at room temperature in Tris/glycine buffer. For the Coomassie-stained gels, 0.017 OD₆₀₀ units were loaded/lane; for the biotin-blot gels, 0.006 OD₆₀₀ units were loaded/lane. Proteins from the resolved gels were transferred to membranes and detected with streptavidin-alkaline phosphatase as described in Kim and McHenry (Kim and McHenry, J. Biol. Chem., 271: 20690 [1996]). By visual inspection, the blots showed that vector pAl-CB-TX expressed approximately 3-fold more *T. thermophilus* DnaX fusion protein than pET-CB-TX, but expressed approximately 20-fold less that the control *E. coli* τ fusion protein. The density of the pAl-CB-TX expressed τ fusion protein was approximately 7-fold less than the levels of the endogenous *E. coli* biotin-carrier protein. Accordingly, pAl-CB-TX was used for further work where a fusion protein was required.

### 6. Purification of T. thermophilus τ Subunit Fusion with a Biotinylated Peptide Containing Hexahistidine

In these experiments, all purification procedures (unless otherwise specified) are generally conducted at 4°C. However, in cases where the protein dissociates or is judged to have lost activity in this or any subsequent purifications, room temperature may be used. Cells are grown and lysed as described with modifications made in the growth conditions to yield optimal levels of soluble undegraded protein as determined in section 5 above. DnaX protein containing in the cleared lysate is precipitated using ammonium sulfate added to 60% saturation, or higher, as necessary to precipitate all of the DnaX protein. The *T. thermophilus* τ protein fused to a C-terminal biotinylated hexahistidine-containing peptide is purified by ammonium sulfate fractionation, chromatography on Ni⁺⁺-NTA ion chelating chromatography much as described above. If necessary, additional purification can be achieved by affinity chromatography on monomeric avidin affinity columns as known in the art.

The purified protein is used to generate a battery of monoclonal antibodies that react with it by the procedures described in Example 2, above. Antibody producing cell lines are selected that express antibody that reacts strongly with *T. thermophilus* τ fusion protein as shown in ELISA assays and Western blots, as known in the art and described in previous Examples. The latter assay system is used to distinguish antibodies that react with contaminants present in the *T. thermophilus* τ fusion protein preparation. As a control, the *E. coli* α subunit that has the same fusion peptide is included in the screen, in order to eliminate antibodies that are directed against the fusion peptide. Selected hybridomas are grown up at the 3 liter level to produce an abundant quantity of antibody.

### 7. Large Scale Production of Cells Expressing T. thermophilus τ-Hexahistidine-Biotin Fusion Protein

Strain MGC1030 (pA1-CB-TX) was grown in a 250 L fermentor to produce cells for purification of biotin peptide-tagged *Tth* τ. F- media (yeast extract (1.4%), tryptone (0.8%), K₂HPO₄ (1.2%), and KH₂PO₄ (0.12%)) was sterilized and ampicillin (100 mg/L) and kanamycin (35 mg/L) added. A large scale inoculum was initiated from 1 mL of DMSO stock (to 28 L) and grown overnight at 37°C. The inoculum was transferred (3.5 L) to the 250 L fermentor (starting OD₆₀₀= 0.046) containing F-media + 1% glucose (sterilized separately and added after sterilization of F-media) and ampicillin at 100 mg/L. Temperature was set at 37°C, aeration was set at 40 LPM, and agitation set at 200 rpm. A pre-induction sample of 1 liter was collected through the sample valve just prior induction, stored at 4°C, and spun down into a pellet the next day. Expression of biotin-tagged *Tth* dnaX was induced when the culture reached OD₆₀₀= 0.794 with IPTG at 1 mM. Additional ampicillin (100 mg/L) and biotin (10 µM) were added at the time of induction with 1 mM IPTG Additional ampicillin (100 mg/L) was added 1 hour post induction. Harvest was initiated 3 hours post induction; cells were chilled to 14°C during harvest. The harvest volume was 170 L and final harvest weight was 2.22 g of cell paste. An equal amount (w/w) of 50 mM Tris (pH 7.5)/10% sucrose solution was added to the cell paste. Quality control results showed 8/10 colonies on ampicillin-containing plates before induction and 7/10 colonies post induction. Cells were frozen by pouring into liquid nitrogen and stored at -80°C until further processed.

### 8. Purification of T. thermophilus τ-Hexahistidine-Biotin Fusion Protein

First, 1650 ml of Tris-sucrose prewarmed to 45°C were added to a 1200 g cell suspension (600 g cells) of MGC 1030 (pAl-CB-TX) cells in Tris-sucrose. Then, 30 ml of 0.5 M DTT was added, followed by 150 ml lysis solution (0.3 spermidine-HCl (pH 7.5), 10% sucrose, 2 M NaCl), and 30 ml 0.5 M EDTA. The pH was adjusted to pH 8.2, by the addition of 25 ml 2 M Tris base. Lysozyme (600 mg) was dissolved in 25 ml Tris-sucrose. After 5 minutes of mixing by stirring at 4°C, the slurry was poured into GSA bottles, and placed on ice for 1 hour. The bottles were then swirled in a 37°C water bath for 4 min., gently inverting every 30 seconds. The bottles were then centrifuged at 12,000 rpm, in a Sorvall GSA rotor for 1 hour at 0°C. The supernatant (Fr I; 2250 ml) contained approximately 34.7 g protein Then, 0.390 g ammonium sulfate was added for each ml of Fr I, to achieve 60% saturation at 4°C. The pellet was collected by centrifugation at 12,000 rpm for 30 min., at 0°C in a Sorvall GSA rotor. The pellet (Fr II; 25.9 g protein) was redissolved in 770 ml of Buffer N, and dialyzed overnight versus buffer N (50 mM sodium phosphate (pH 7.8), 500 mM NaCl, 10% glycerol, 0.5 mM DTT, 0.1 mM PMSF, 1 mM imidazole), and was then applied to a 15 ml Ni-NTA agarose column (Qiagen). The flow-through was collected and reapplied to the column. The column was then washed with 20 column volumes of buffer containing 50 mM sodium phosphate (pH 7.8), 500 mM NaCl, 10% glycerol, 0.5 mM DTT, and 10 mM imidazole. Then, a 12 column volume gradient was run, with an increase in imidazole concentration from 10 to 300 mM.

Biotin blots performed on the fractions indicated that the majority of the tagged *Tth* DnaX protein eluted with the 10 mM imidazole-containing wash, demonstrating a weaker affinity for the column than was expected. Nevertheless, the weak binding permitted separation from the majority of protein that flowed through; minimally, a 10-fold purification over the applied fraction was achieved. The wash fraction (Fr III, 350 mg protein) was precipitated by addition of an equal volume of saturated ammonium sulfate to produce two equal volume pellets were collected by centrifugation.

One of the two Fr III pellets was redissolved in 30 ml of dialysis buffer, and dialyzed overnight versus dialysis buffer (50 mM Tris-HCl (pH 7.5), 10% glycerol, and 5 mM DTT). The dialysate was applied to a 30 ml SP Sepharose column equilibrated in the same buffer as used for the dialysis step. The column was washed with one column volume, and proteins were eluted with a 10 column volume gradient ranging from 0 to 400 mM NaCl in dialysis buffer. The peak of eluted *Tth* τ was estimated based on biotin blot results. Fractions 26-36 (52 ml) from the 56 fractions collected from the gradient were pooled, resulting in approximately 1560 µg total protein (Fr. IV).

Fr. IV was concentrated using two methods. One-half of the fraction was precipitated by addition of ammonium sulfate to 80% saturation. The precipitate was recovered by centrifugation at 13,000 rpm for 2 hours in an HB4 swinging bucket rotor (Sorvall) at 4°C, resulting in recovery of 460 µg protein. The other half of Fr IV was concentrated using an Amicon concentrator with a YM10 (25 mm) membrane, with stirring at 4°C, resulting in recovery of 560 µg protein. Biotin blots indicated that a better specific recovery of biotinylated *Tth* τ (DnaX protein) was achieved using the Amicon membrane than by ammonium sulfate precipitation.

An SDS-PAGE gel was run and the proteins were transferred to a nitrocellulose membrane, and biotin blots were prepared. Comparison of the biotin blotted band with protein standards indicated that protein had migrated 3 mm farther than carboxyl-terminal tagged *E. coli* τ protein (approximately 51,000 Da), indicating that the protein had a molecular weight of approximately 69,000 Da, which is consistent within experimental error, and the variability of the technique with variances in primary sequence, of the expected molecular weight of approximately 61,000 Da.

### 9. Improvement in Expression Level of Tth τ and C-Terminal Biotin/Hexahis Tagged τ

The low level of *Tth* τ expression was thought likely to be due to the abundance of GC rich codons, especially near the initiating ATG. To minimize this effect, the initial portion of the gene is resynthesized, replacing the GC rich codons with degenerate codons that are common in *E. coli,* yet encode the same amino acid.

In specific, plasmids pA1-TX and pAl-CB -TX are cleaved with *Cla*I (*i.e.,* which cleaves just before to initiating ATG), and with *Pml*I, and replace the intervening sequence with a synthesized and annealed oligonucleotides containing commonly used *E. coli* codons. The sequence of these oligonucleotides are: and

The resulting biotin peptide-tagged expression vectors are grown up, induced, analyzed by biotin blotting methods, and compared with the expression obtained with the existing biotin-tagged *Tth* τ expressing vector, and the best expressing vector chosen for further experimentation.

### 10. Purification of T. thermophilus τ Subunit with a Biotinylated Peptide-Containing Fusion

The optimally expressed C-terminal biotin-tagged *Tth* τ protein is purified using modifications of the methods described for partial purification (*i.e.,* methods in the present Example). The ammonium sulfate precipitation methods are optimized, using the methods described for tagged *Tth* α in Example 5. The resulting ammonium sulfate pellet is then subjected to Ni-NTA chromatography, using methods similar to those described in Example 5 for *Tth* α, with the exception being that the column is washed with 5 column volumes of buffer containing 1 mM imidazole prior to beginning the gradient. Once the results of the Ni-NTA chromatography are analyzed, the washing procedure is altered, so that as high a concentration of imidazole can be used without elution of tagged *Tth* τ; the gradient is also optimized, so that *Tth* biotin-tagged τ elutes approximately one half way through the gradient.

The purified protein is used to generate a battery of monoclonal antibodies that react with it by the procedures described in Example 2, above. Antibody producing cell lines are selected that express antibody that reacts strongly with *T. thermophilus* τ fusion protein as shown in ELISA assays and Western blots, as known in the art and described in previous Examples. The latter assay system is used to distinguish antibodies that react with contaminants present in the *T. thermophilus* τ fusion protein preparation. As a control, the *E. coli* α subunit that has the same fusion peptide is included in the screen, in order to eliminate antibodies that are directed against the fusion peptide. Selected hybridomas are grown up at the 3 liter level to produce an abundant quantity of antibody.

### 11. Purification of Natural T. thermophilus τ and γ Subunits

Natural τ and γ subunits expressed from the modified *T. thermophilus* dnaX gene in *E. coli* are purified by column chromatographic procedures and assayed by SDS-PAGE, with confirmation of the identity of the authentic τ protein by Western blots using the above monoclonal antibodies. Cell growth and lysis are conducted as described above (*See e.g.,* Dallmann *et al.,supra*) with modifications in growth conditions to yield optimal levels of soluble undegraded protein as determined in section 5 above. The ammonium sulfate precipitation conditions are optimized in order to maximize the amount of *T. thermophilus* DnaX protein precipitated and minimize the total level of protein precipitated. Protein determinations are conducted by the method of Bradford with modifications following the instructions that come with the reagent supplied by Pierce. The level of *T. thermophilus* DnaX protein is determined by quantitative Western blots using methods as described in previous Examples (*e.g.*, those used to monitor the levels of *T. thermophilus* DnaE protein), with the exception being that antibodies to DnaX prepared as described above will be used. While the optimum level of ammonium sulfate determined by experiment is used, the following provides a representative procedure: Fr. I (cleared lysate) is precipitated with 107 g of ammonium sulfate (0.226 g for each mL of Fr. I, 40 % saturation) and centrifuged at 22,000 x g for approximately 30 min. Pellets are backwashed by resuspension in a Dounce homogenizer with 100 mL of Buffer TBP containing 0.1 M NaCl, and 0.2 g/mL ammonium sulfate (35 % saturation), and re-centrifuged. The final pellets are stored at 4 °C until used and referred to as Fr. II.

The chromatography columns (*e.g.*, hydrophobic, ion exchange, and/or sizing columns) are chosen so as to yield maximally pure protein and provide the highest yield. Portions of Fr II are dissolved in Buffer SP (50 mM Tris-HCl (pH 7.5), 10% (w/v) glycerol, 5 mM dithiothreitol) (approximately 7 mg total protein/ml final), clarified by centrifugation (28,000 x g, 30 min), and diluted with the same buffer to a conductivity equivalent to 50 mM NaCl. In experiments where the desired DnaX protein does not remain soluble as judged by remaining in the supernatant after gentle centrifugation, it is dissolved in larger quantities of buffer, adding additional salt and diluting just before application to the column, and/or adding low levels of various detergents (*i.e*., 0.02% NP-40).

This material is loaded onto a Q Sepharose (Pharmacia; 2 mg protein/ml resin) column equilibrated in buffer SP. After loading, the column is washed with one column volume of Buffer SP, then developed with a 12 column volume gradient of 50 to 600 mM NaCl in Buffer SP at a flow rate of 1 column volume/h, and 100 fractions are collected. The elution position of *T. thermophilus* γ and τ are determined by quantitative Western blots, and total protein determined by the method of Bradford. Fractions with the highest ratio of DnaX protein to total protein are pooled. Generally, this results in pooling fractions of ½ peak height or greater. In the situation where the protein fails to bind to the column, buffers with lower salt (down to O NaCl with dialysis to decrease endogenous levels of salt) are used, and, if that fails, buffers with higher pH are used. In the situation where the protein fails to elute, the ionic strength of the gradient is increased until it does so. Once the elution position is determined, the gradient is optimized, so that the total ionic strength change is not more than what is needed (generally no more than a 400 mM NaCl change) and the dnaX protein elutes ½ way through the gradient. The pooled peak is then precipitated by the addition of sufficient ammonium sulfate to precipitate all protein (generally 60% saturation) and the pellet collected by centrifugation (28,000 x g for 30 min) to yield Fr. III.

The Fr. III pellet is the dissolved in Buffer SP to a concentration of approximately 2 mg/ml, and centrifuged (28,000 x g, 30 min) to clarify. Fr. III is then be loaded onto a SP Sepharose (Pharmacia; approximately 5 mg protein/ml resin) column equilibrated with buffer SP. After loading, the column is washed with one column volume of Buffer SP, and developed with a 12 column volume gradient of 50 to 600 mM NaCl in Buffer SP at a flow rate of 1 column volume/h; approximately 100 fractions are collected. Chromatography is quantitated and optimized as described for the Q-Sepharose column. Additionally, this column resolves τ and γ, and conditions are optimized to enable this resolution. In situation where τ and γ are not separated by this procedure or the preceding Q Sepharose procedure, hydrophobic chromatography using commercially available resins is conducted. The resulting pooled fractions of γ and τ are precipitated with ammonium sulfate as described to Q-Sepharose resulting in Fr. IV.

Fr IV is dissolved in Buffer H (25 mM Hepes-KOH (pH 7.5), 25 mM NaCl, 5% glycerol, 0.1 mM EDTA) (approximately 7 mg protein/ml), and applied to a S-400 HR (Pharmacia) column (44:1 height:diameter ratio, total column volume 50-times sample volume) equilibrated with Buffer H. The column is developed in the same buffer at a flow rate of 1 column volume/day, and 100 fractions collected. DnaX protein (τ and γ run on separate columns since resolved in a preceding column) are quantitated as described for the Q-Sepharose column These fractions are pooled, and distributed in aliquots, which are then flash-frozen in liquid nitrogen and stored at -80°C as Fr. IV. This material provides reagents for reconstituted *T. thermophilus* DNA polymerase III holoenzyme.

### EXAMPLE 9

### Isolation of the T. thermophilus DnaN Protein

The sequence downstream from the *T. thermophilus* dnaA gene is determined and, if homologous to *dna*N genes of other eubacteria, used to express *T. thermophilus* β subunit. The *dna*N gene resides downstream of the *dna*A gene in most, but not all, eubacteria.

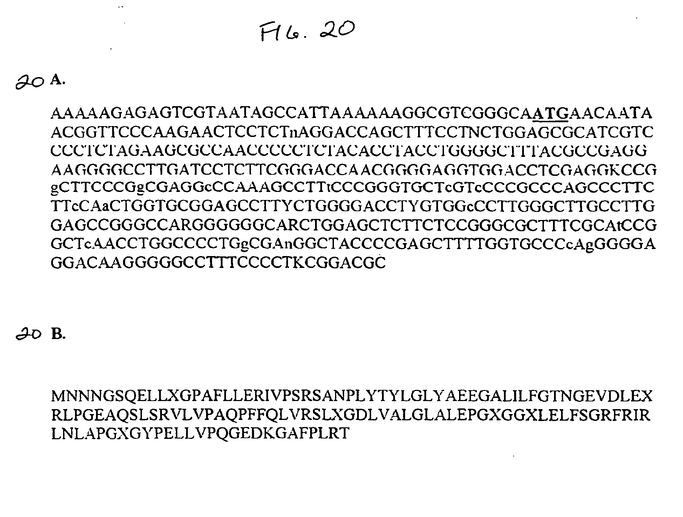

A large scale lambda vector preparation was made, the DNA extracted, purified, and digested with *Dra*I and *EcoRI* to obtain *dna*N sequence. The resulting 2.2 kb fragment was separated from other fragments by electrophoresis, and then eluted in water for direct sequence analysis. Primers for sequence analysis were selected from the available sequence downstream of the second *Dra*I site (*See,* Figure 19A). The resulting sequence (Figure 20A) is preliminary and likely to contain errors, as it has not been processed with sequencing in the second direct yet. Nonetheless, the sequence information was sufficient for the identification of the encoded gene.

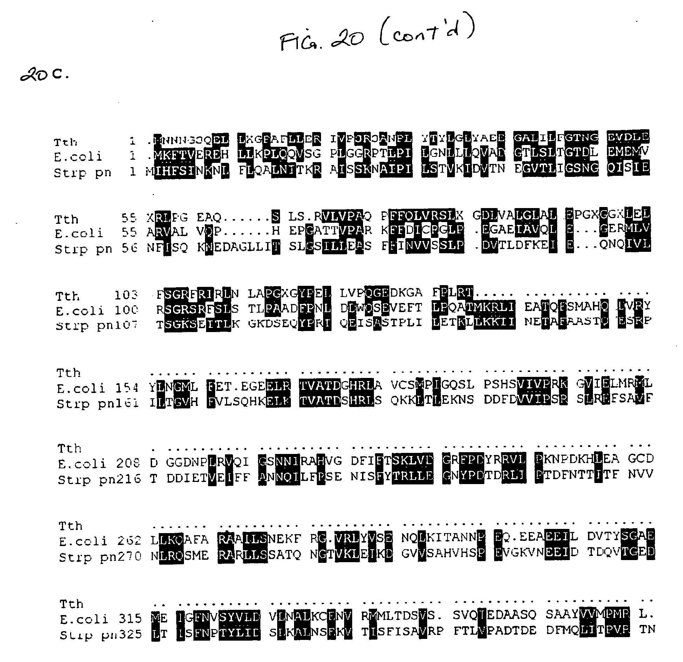

Upon subjecting the DNA sequence to ORF finder (NIH), an open reading frame of 137 amino acids initiating at the underlined ATG in Figure 20A was obtained. This amino acid sequence is shown in Figure 20B. Using the BLAST program linked to ORF finder, the open reading frame was compared with all known and deduced protein sequences. Nine eubacterial beta polymerase sequences were identified as the best matches. The probability of the best match (with *Salmonella* sequence that is nearly identical to *E. coli* sequence) yielded and E value of 0.007, indicating a 670-fold higher probability of a match than the first non-beta match (E=4.7), that was not even from an eubacterium. Alignment of the *Tth* amino acid beta sequence (SEQ ID NO:232) was compared with *E. coli* (SEQ ID NO:233) and *S. pneumoniae* (SEQ ID NO:234) β sequences. This alignment is shown in Figure 20C. As indicated, this alignment shows that the best alignment was obtained with the amino-terminus of both (*E. coli* and *S. pneumoniae*) β sequences. Matches with sequence were approximately equally good between all three divergent sequences, indicating that *Tth* is approximately as close to both *E. coli* and *S. pneumoniae* betas as they are to one another. Thus, there is strong evidence that the sequence identified in these experiments is the amino-terminus of *Tth* DnaN.

Full-length sequence of *Tth dna*N is obtained by sequencing the isolated *Dra*I-*EcoRI* fragment, as well as the isolated 900 bp *Dra*I*-Bam*HI fragment described above, if needed, until full and accurate sequence is obtained. The *Dra*I*-Exo*RI and *Dra*I-*Bam*HI fragments or subfragments of these fragments are used for cloning and to enable sequencing from both sides, if difficulties in sequencing arise (*e.g*., due to secondary structure barriers).

Upon obtaining full-length sequence, PCR is used to construct a vector. In this process a primer containing a non-complementary *Cla*I site immediately preceding the region of complementarity starting at the initiating ATG is used. A 3' terminal primer containing an *Spe*I site (non-complementary) immediately after the complementary portion of the oligonucleotide that will end at the stop codon found after the last codon for *Tth dna*N is also used. Upon obtaining the terminal sequences with certainty, this procedure is conducted (*i.e.,* it is not contemplated that the internal sequences will be necessary in these steps). The resulting fragment is cleaved with *Cla*I and *Spe*I*,* and cloned into the corresponding sites of vector pAl-CB-Cla-1 (*See,* Example 8), and the sequence is verified by DNA sequencing as known in the art. A C-terminal biotin/hexahis tagged *Tth* β expressing vector is constructed by appropriate modifications of the procedures described for making C-terminal tagged *Tth* τ in Example 8.

It is contemplated that in the situation where an internal *Spe*I site is identified within in the *Tth dnaN* gene, an alternative approach is used. In this alternative approach, an *Xba*I or *Dra*III site also present in the vector is substituted. In an another alternative, where a *Cla*I site is present within *Tth dnaN,* precluding its use for cloning the initiating ATG in front of the vector ribosome binding site, vectors presently available that have the *Cla*I site substituted with *Nde*I*, Swa*I or *Nsi*I are used.

Expressed C-terminal tagged β is purified first, using ammonium sulfate precipitation optimization and Ni⁺⁺-NTA agarose chromatography, and if necessary, soft-release avidin chromatography as described for *Tth* DnaE (α) in Example 5. Polyclonal and/or monoclonal antibodies directed against β (*i. e.,* as the antigen) are prepared as described in Examples 5 and 8. These antibodies are then used to optimize purification of wild-type *Tth* β.

An optimized *Tth* β ammonium sulfate pellet (optimized and obtained as described in Examples 5 and 8), is further purified by chromatography on Q-Sepharose, S or SP Sepharose, hydrophobic chromatography, and gel filtration chromatography as described in Examples 5-8. Ionic strength and pH of column buffers are optimized, in order to permit sticking of the β protein to the column resins, as well as elutions near the middle of the gradient which produce good yields.

The purified β is then used in conjunction with *Tth* DNA polymerase III (α or an α and ε complex) to improve the processivity of DNA polymerase III in PCR or other applications involving DNA synthesis. In this simple assay format, β levels will be in 100-fold stoichiometric excess of DNA polymerase III, and reactions conducted using linear templates. In yet other applications, β is combined with DNA polymerase III and *Tth* DnaX complex and SSB, on primed templates in the presence of ATP (1 mM) and 10 mM MgCl₂. This enables efficient assembly of the polymerase on DNA with β. Elongation proceeds with the addition of dNTPs to 1 mM concentrations. Reactions are conducted at the optimal temperature of the reconstituted reaction (approximately 60°C) or higher. β allows *Tth* DNA polymerase to replicate denatured and single-stranded DNA molecules of up to approximately 1 megabase. In these reactions, the DNA template is varied from 2.5 to 250 fmoles. Other reaction components and solution conditions used are those described for the gap-frlling assay in Example 5, with the exception being that the pH may be varied to the optimal point (*e.g.*, between pH 6 and 9), and potassium glutamate may be added to an optimal level (*e.g.*, 50-200 mM). Primers are varied from a 1:1 ratio relative to template, to a 10,000-fold excess over template, depending upon the level of amplification desired. Other components are also included, as described in other Examples.

### EXAMPLE 10

### Isolation of T. thermophilus SSB and Expression of Purification of SSB

*T. thermophilus* SSB is isolated by chromatography of DNA cellulose columns using modifications of the methods of Molineux *et al.* (Molineux et al., J. Biol. Chem., 249 6090-6098 [1974]). Lysates (Fraction I) are prepared by the methods described in Example 2. Fraction II is prepared by addition of 0.24 g to each ml of Fraction I and precipitates collected as described in Example 2. The collected ammonium sulfate precipitate is dissolved in 20 mM Tris-HCl (pH 7.5), 50 mM NaCl, 10% glycerol, 5 mM β-mercaptoethanol at a concentration of 1 mg protein/ml and applied to a denatured DNA cellulose column equilibrated in the same buffer. A 1.5 x 13 cm column is run for each 100 g of *T. thermophilus* used as starting material. The column is then washed successively with two column volumes of equilibration buffer at 0.5 column volume/h containing 100, 200, 400, 800, 1600 and 2000 mM NaCl respectively. Fractions eluting from the column are monitored by SDS-PAGE, and the tightest binding fractions that contain proteins between 15,000 Da and 30,000 Da are pooled individually for each protein and subjected to further purification by Q-Sepharose (Pharmacia) chromatography. The pooled fractions are dialyzed against 20 mM Tris-HCl (pH 7.5), 10% glycerol, 5 mM β-mercaptoethanol, and then applied to a Q Sepharose column equilibrated in the same buffer at a load ratio of 2 mg protein/ml resin. The column is eluted with a 20-column gradient from 0-1 M NaCl in the equilibration buffer and the fractions containing the sought protein selected by SDS-PAGE in the preceding step pooled. This procedure is repeated for each major candidate.

Each of the pooled candidate fractions are concentrated by ammonium sulfate precipitation (60% saturation) and collected as described under Example 2. The protein is as concentrated as possible (*e.g.,* consistent with its being soluble after centrifugation in a bench top microcentrifuge for 2 minutes as judged by protein determination with the Bradford reagent as described in the above Examples), dialyzed against the Q-Sepharose equilibration buffer, and then subjected to SDS-PAGE, and blotted onto a membrane.

Both N-terminal and internal peptide sequencing are conducted as described above for DnaX and DnaE. For the protein that contains sequences that show the best homology to SSB from other eubacteria (*i.e.*, as judged by the most favorable score using the NIH's BLAST server), oligonucleotides are designed for obtaining a fragment of the *T. thermophilus* ssb gene using methods described for dnaX, E, Q and A. Success in obtaining a fragment of the sought ssb gene is judged by a favorable score of a sequence obtained by DNA sequencing the cloned PCR products (i.e., better than 1 x 10⁻²) in a BLAST search of the sequences between primers against the nr database at NIH's server. This fragment is used to probe a lambda library for a full length clone as described for DnaE.

The candidate full length fragment is subjected to DNA sequencing and its identity confirmed by recognizable homology to other eubacterial ssbs using the default parameters in the NIH BLAST server. The full length gene is modified and overexpressed using the strategies outlined for DnaX above. The isolated homogeneous protein (*e.g*., either from purification from an overproducing strain or frame non-overproducing *T. thermophilus*) is then used to support reconstituted *T. thermophilus* replication systems.

### EXAMPLE 11

### Use of T. thermophilus DnaX Proteins to Obtain Additional Components of the DnaX Complex and Reconstitution of T. thermophilus DnaX Complex

In other cellular systems examined to date, the ATPase that transfers the sliding clamp processivity factor (*i.e.,* β₂ in *E. coli,* PCNA in eukaryotes) contains five different proteins that are tightly and cooperatively bound in a complex. In this Example, *T. thermophilus* DnaX protein is immobilized on a column and cleared *T. thermophilus* lysates are passed over the column, in order to permit subunit exchange and assembly of a full DnaX complex on the immobilized protein. Contaminants are washed away, and the specifically bound proteins eluted, separated by SDS-PAGE, transferred to a membrane, and both amino-terminal and internal peptide sequences determined, using methods known in the art. These sequences are used to isolate the structural gene for the isolated proteins. These proteins are expressed and purified, their ability to form a specific complex with *T. thermophilus* DnaX confirmed, and used to reconstitute *T. thermophilus* DnaX complex to provide a functional *T. thermophilus* DNA polymerase III holoenzyme.

Alternatively, if the *Tth* complex is so stable that significant subunit exchange does not occur to permit success with the procedure described in the preceding paragraph, the biotin/hexahis vector is modified by moving the modified *Tth dnaX* gene from pA1-CB-TX to a *Tth* expression vector, transfecting the vector into *T. thermophilus* and express the tagged protein in *T. thermophilus.* It is contemplated that this will allow assembly of the tagged *Tth* DnaX protein with the other components of the *Tth* DnaX complex as they are synthesized.

The *in vivo* assembled complex is isolated by Ni⁺⁺-NTA chromatography as described herein. The associated proteins are then characterized, and used to isolate the structural gene, as well as construct expression systems as described above. If the tagged DnaX protein synthesized in *T. thermophilus* is biotinylated, as revealed by biotin blots as done for biotinylated *Tth* τ expressed in *E. coli,* soft release avidin, avidin or streptavidin chromatography are used to isolate the complex. It is contemplated that if necessary, the *Tth* complex will be biotinylated by treatment with the *E. coli* biotinylation enzyme.

If both of the above methods are determined to be unsatisfactory, the DnaX accessory proteins are purified biochemically monitoring their position of elution during chromatographic procedures, based upon their requirement to be added back to purified DnaE, DnaQ, DnaX and DnaN proteins (α, ε, τ, γ and β, respectively), and, if necessary SSB, to reconstitute replication on a long single-stranded DNA template containing a single oligonucleotide primer.

Also, an assay is set up that employs a long single stranded template and a single primer. This assay system is used to assay for an activity in *T. thermophilus* extracts (or ammonium sulfate or column fractions, as appropriate) that stimulates replication by limiting levels of DNA polymerase III (α and ε) in the presence of DnaN (β) and, if necessary, SSB. This assay is used to guide purification of *T. thermophilus* DnaX auxiliary factors. The isolated proteins are partially sequenced (*e.g*., N-terminus and internal sequences), a fragment isolated by PCR, the entire full length gene isolated from chromosomal libraries in lambda vectors as described for DnaE, the corresponding structural genes sequenced, expressed and the resulting proteins purified by ammonium sulfate fractionation and chromatographic procedures. Then, conditions for reconstitution of DNA polymerase III holoenzyme activity on long single-stranded templates are optimized.

### EXAMPLE 12

### Additional Assay Systems

In this Example, methods that assay specifically for processive DNA replication on long single-stranded templates are described. These assays are useful, as they permit further optimization of the systems described above to maximize processive synthesis. These steps include the assaying cleared lysates, ammonium sulfate and chromatographic fractions for factors that further increase the yield of long replication products. Once identified, these factors are purified, their structural genes isolated and expression vectors constructed to provide larger quantities of these stimulatory factors.

In addition, the processive assays are modified to include regions of high secondary structure at a defined point in the template. In a second assay, long single-stranded DNA that contains extensive complementarity to internal regions ranging from 100-1000 bases are added. Conditions that increase readthrough of these regions including the assaying for *T. thermophilus* protein factors that enable this readthrough

Furthermore, the initial optimizations is conducted on lambda clones containing inserts up to 23 Kb. Primers are selected from insert sequence or flanking lambda sequences. Optimum protocols to amplify DNA to provide optimal yields are developed.

In other assays, construct templates with extensive single-stranded regions in front of the primer are constructed. The duplex region is either blunt or contains extensive 5'-noncomplementary tails. Among the factors that stimulate these assays, it is contemplated that helicases and helicase-associated factors are required for the assembly of the helicases onto DNA or for their maximal activity once associated. Helicases that can specifically interact with the DNA polymerase III holoenzyme are favored in this type of approach, since they provide the most striking stimulation of assays using the DNA polymerase III holoenzyme as a polymerase.

It is also contemplated that DNA polymerase III holoenzyme-associating helicases will be isolated using some of the protein affinity column approaches described herein (*i.e.,* DnaE or DnaX and associating components (or both) immobilized on a streptavidin or avidin column).

It is contemplated that other routes toward stimulating helicases will be more directed. For example, it is contemplated that searches be conducted for Tth homologs of DnaB, uvrD and rep (*i.e.,* helicases that have been found to be associated with DNA polymerase III holoenzyme from E. *coli* and/or work in conjunction with it for the processive replication of duplex DNA). These helicases are isolated by alignment with homologs, selection of conserved regions and synthesis of degererate PCR primers, isolation of a portion of the gene by PCR, and isolation of the full length gene from *Tth*-lambda libraries as described herein for dnaA and dnaQ.

Once these helicases are in hand, it is contemplated that they be added to assays, to identify for additional assembly factors required for their function. It is also contemplated that this type of procedure will function in the isothermal amplification of DNA without the requirement for thermal cycling. It is further contemplated that it will be advangageous to add polymerases with more potent strand displacement activity, even if their overall processivity and ability to function on long templates is limited, to assist the polymerase through regions of difficult secondary structure.

Once optimization has been performed on lambda isolates and on duplex containing duplex regions in front of the primer, the procedure is extended to 100 Kb or longer isolates using longer vectors. The optimal conditions are determined for these vectors, and the methodology is extended to isolated chromosomal DNA to determine the practical limitations of these methods.

Optimal elongation protocols are developed, and cycling protocols that enable reconstituted *T. thermophilus* DNA polymerase III holoenzyme plus isolated accessory factors used in PCR methods using substrates (*i.e.*, targets) beyond 10 kb, 50 kb, and 200 kb, and larger, are also established. Conditions that permit the component proteins to remain stable during repeated denaturation steps to approximately 95°C, in standard protocols as known in the art are also established. In the alternative, capillary technology requiring very short denaturation times (*i.e.,* a few seconds at approximately 95°C) are developed. In addition, methods and conditions for isothermal amplification, wherein the polymerase is coupled to the action of thermophilic helicase and associated assembly factors are also obtained.

From the above, it is clear that the present invention provides novel thermophilic DNA polymerases and preparations from *T*. *thermophilus.* In particular, the present invention provides *T. thermophilus* polymerase III preparations and means to identify polymerase IIIs present in other species.

## Claims

1. An isolated nucleotide sequence consisting of the sequence set forth in SEQ ID NO:196.

2. The isolated nucleotide sequence of Claim 1, wherein said nucleotide sequence further comprises 5' and 3' flanking regions.

3. A recombinant DNA vector comprising the nucleotide of Claim 1.

4. A host cell containing the recombinant DNA vector of Claim 3.

5. A purified DnaE protein comprising the amino acid sequence set forth in SEQ ID NO:197.

6. A fusion protein comprising at least a portion of the DnaE protein of Claim 5, and a non-dnaE protein sequence.

7. An isolated nucleotide sequence as set forth in SEQ ID NO:214.

8. The nucleotide sequence of Claim 7, wherein said nucleotide sequence further comprises 5' and 3' flanking regions.

9. A recombinant DNA vector comprising the nucleotide of Claim 7.

10. A host cell containing the recombinant DNA vector of Claim 9.

11. A purified DnaQ protein comprising an amino acid sequence selected from the group consisting of SEQ ID NOS:215 and 216.

12. A fusion protein comprising the DnaQ protein of Claim 11, and a non-dnaQ protein sequence.

13. The isolated nucleotide sequence set forth in SEQ ID NO:221.

14. The isolated nucleotide sequence of Claim 13, wherein said nucleotide sequence further comprises 5' and 3' flanking regions.

15. A recombinant DNA vector comprising the nucleotide of Claim 13.

16. A host cell containing the recombinant DNA vector of Claim 15.

17. A purified DnaA protein comprising the amino acid sequence set forth in SEQ ID NO:222.

18. A fusion protein comprising the DnaA protein of Claim 17, and a non-DnaA protein sequence.

19. The isolated nucleotide sequence consisting of the sequence set forth in SEQ ID NO:230.

20. The nucleotide sequence of Claim 19, wherein said nucleotide sequence further comprises 5' and 3' flanking regions.

21. A recombinant DNA vector comprising the nucleotide of Claim 19.

22. A host cell containing the recombinant DNA vector of Claim 21.

23. A purified DnaN protein consisting of the amino acid sequence set forth in SEQ ID NO:231.

24. A fusion protein comprising the DnaN protein of Claim 23, and a non-DnaN protein sequence.

25. The isolated nucleotide sequence set forth in SEQ ID NO:7.

26. The isolated nucleotide sequence of Claim 25, wherein said nucleotide sequence further comprises 5' and 3' flanking regions.

27. A recombinant DNA vector comprising the nucleotide of Claim 25.

28. A host cell containing the recombinant DNA vector of Claim 27.

29. A purified dnaX protein comprising the amino acid sequence set forth in SEQ ID NO:9.

30. A fusion protein comprising the dnaX protein of Claim 29, and a non-dnaX protein sequence.

31. An isolated amino acid sequence as set forth in SEQ ID NO:2.

32. An isolated nucleotide sequence encoding the amino acid sequence of Claim 31.

33. The nucleotide sequence of Claim 32, wherein said nucleotide sequence further comprises 5' and 3' flanking regions.

34. A recombinant vector comprising the nucleotide sequence of Claim 32.

35. A host cell containing the recombinant DNA vector of Claim 34.

36. A purified tau protein encoded by a polynucleotide sequence homologous to the coding strand of the nucleotide sequences of Claim 31.

37. The purified protein of Claim 36, wherein said tau protein is from Thermus thermophilus.

38. A fusion protein comprising the tau protein of Claim 36, and a non-tau protein sequence.

39. An isolated amino acid sequence as set forth in SEQ ID NO : 1.

40. An isolated nucleotide sequence encoding the amino acid sequence of Claim 39.

41. A purified gamma protein encoded by a polynucleotide sequence homologous to the coding strand of the nucleotide sequences of Claim 40, wherein the protein is not the Thermus thermophilus DnaX protein.

42. The purified protein of Claim 41, wherein said gamma protein is from Thermus thermophilus.

43. The nucleotide sequence of Claim 40, wherein said nucleotide sequence further comprises 5' and 3' flanking regions.

44. A recombinant vector comprising the nucleotide sequence of Claim 40.

45. A host cell containing the recombinant DNA vector of Claim 44.

46. A fusion protein comprising the gamma protein of Claim 40, and a non-gamma protein sequence.

47. A method for identification of polymerase subunits comprising the steps of:
a) providing a test sample suspected of containing amplifiable nucleic acid encoding at least a portion of a DNA polymerase III subunit protein;
b) isolating said amplifiable nucleic acid from said test sample;
c) combining said amplifiable nucleic acid with amplification reagents, and at least two primers selected from the group consisting of primers having the nucleic acid sequence set forth in SEQ ID NOS:21, 22, 86, 87, 91,97, 110, 111, 112, 113, 114, 115, 116, 134, 135, 136, 137, 156, 157, 173, 174, 176, 177, 208, 209, 210, 211, 212, and 213, to form a reaction mixture; and
d) combining said reaction mixture with an amplification enzyme under conditions wherein said amplifiable nucleic acid is amplified to form amplification product.

48. The method of Claim 47, further comprising the step of detecting said amplification product.

49. The method of Claim 48, in which said detecting is accomplished by hybridization of said amplification product with a probe.

50. The method of Claim 47, wherein said primers are capable of hybridizing to nucleic acid encoding at least one polymerase subunit selected from the group consisting of DnaA, DnaN, DnaE, DnaX, DnaQ, and DnaB.

51. The method of Claim 47, wherein said test sample comprises nucleic acid obtained from a thermophilic organism.

52. The method of Claim 51, wherein said thermophilic organism is a member of the genus Thermus.

## Patentansprüche

1. Isolierte Nukleotidsequenz, bestehend aus der Sequenz wie in der SEQ ID Nr.: 196 beschrieben.

2. Isolierte Nukleotidsequenz nach Anspruch 1, **dadurch gekennzeichnet, dass** die Nukleotidsequenz des Weiteren 5'- und 3'-flankierende Regionen aufweist.

3. Rekombinanter DNA-Vektor, umfassend die Nukleotidsequenz nach Anspruch 1.

4. Wirtszelle, enthaltend den rekombinanten DNA-Vektor nach Anspruch 3.

5. Gereinigtes DnaE-Protein, umfassend die Aminosäurensequenz wie in der SEQ ID Nr.: 197 beschrieben.

6. Fusionsprotein, umfassend mindestens einen Teil des DnaE-Proteins nach Anspruch 5 und eine nicht-DnaE-Proteinsequenz.

7. Isolierte Nukleotidsequenz wie in der SEQ ID Nr.: 214 beschrieben.

8. Nukleotidsequenz nach Anspruch 7, **dadurch gekennzeichnet, dass** die Nukleotidsequenz des Weiteren 5'- und 3'-flankierende Regionen aufweist.

9. Rekombinanter DNA-Vektor, umfassend die Nukleotidsequenz nach Anspruch 7.

10. Wirtszelle, enthaltend den rekombinanten DNA-Vektor nach Anspruch 9.

11. Gereinigtes DnaQ-Protein, umfassend eine Aminosäurensequenz ausgewählt aus der Gruppe bestehend aus den SEQ ID Nr.: 215 und 216.

12. Fusionsprotein, umfassend das DnaQ-Protein nach Anspruch 11 und eine nicht-DnaQ-Proteinsequenz.

13. Isolierte Nukleotidsequenz wie in der SEQ ID Nr.: 221 beschrieben.

14. Isolierte Nukleotidsequenz nach Anspruch 13, **dadurch gekennzeichnet, dass** die Nukleotidsequenz des Weiteren 5'- und 3'-flankierende Regionen aufweist.

15. Rekombinanter DNA-Vektor, umfassend die Nucleotidsequenz nach Anspruch 13.

16. Wirtszelle, enthaltend den rekombinanten DNA-Vektor nach Anspruch 15.

17. Gereinigtes DnaA-Protein, umfassend die Aminosäurensequenz wie in der SEQ ID Nr.: 222 beschrieben.

18. Fusionsprotein, umfassend das DnaA-Protein nach Anspruch 17 und eine nicht-DnaA-Proteinsequenz.

19. Isolierte Nukleotidsequenz, bestehend aus der Sequenz wie in der SEQ ID Nr.: 230 beschrieben.

20. Isolierte Nukleotidsequenz nach Anspruch 19, **dadurch gekennzeichnet, dass** die Nukleotidsequenz des Weiteren 5'- und 3'-flankierende Regionen aufweist.

21. Rekombinanter DNA-Vektor, umfassend die Nukleotidsequenz nach Anspruch 19.

22. Wirtszelle, enthaltend den rekombinanten DNA-Vektor nach Anspruch 21.

23. Gereinigtes DnaN-Protein, bestehend aus der Aminosäurensequenz wie in der SEQ ID Nr.: 231 beschrieben.

24. Fusionsprotein, umfassend das DnaN-Protein nach Anspruch 23 und eine nicht-DnaN-Proteinsequenz.

25. Isolierte Nukleotidsequenz wie in der SEQ ID Nr.: 7 beschrieben.

26. Isolierte Nukleotidsequenz nach Anspruch 25, **dadurch gekennzeichnet, dass** die Nukleotidsequenz des Weiteren 5'- und 3'-flankierende Regionen aufweist.

27. Rekombinanter DNA-Vektor, umfassend die Nukleotidsequenz nach Anspruch 25.

28. Wirtszelle, enthaltend den rekombinanten DNA-Vektor nach Anspruch 27.

29. Gereinigtes DnaX-Protein, umfassend die Aminosäurensequenz wie in der SEQ ID Nr.: 9 beschrieben.

30. Fusionsprotein, umfassend das DnaX-Protein nach Anspruch 29 und eine nicht-DnaX-Proteinsequenz.

31. Isolierte Aminosäurensequenz wie in der SEQ ID Nr.: 2 beschrieben.

32. Isolierte Nukleotidsequenz, kodierend die Aminosäurensequenz nach Anspruch 31.

33. Nukleotidsequenz nach Anspruch 32, **dadurch gekennzeichnet, dass** die Nukleotidsequenz des Weiteren 5'- und 3'-flankierende Regionen aufweist.

34. Rekombinanter Vektor, umfassend die Nukleotidsequenz nach Anspruch 32.

35. Wirtszelle, enthaltend den rekombinanten DNA-Vektor nach Anspruch 34.

36. Gereinigtes tau-Protein, kodiert durch eine Polynukleotidsequenz, die homolog zu dem kodierenden Strang der Nukleotidsequenz nach Anspruch 31 ist.

37. Gereinigtes Protein nach Anspruch 36, **dadurch gekennzeichnet, dass** das tau-Protein von Thermus thermophilus ist.

38. Fusionsprotein, umfassend das tau-Protein nach Anspruch 36 und eine nicht-tau-Proteinsequenz.

39. Isolierte Aminosäurensequenz wie in der SEQ ID Nr.: 1 beschrieben.

40. Isolierte Nukleotidsequenz, kodierend die Aminosäurensequenz nach Anspruch 39.

41. Gereinigtes gamma-Protein, kodiert durch eine Polynukleotidsequenz, die homolog zu dem kodierenden Strang der Nukleotidsequenz nach Anspruch 40 ist, wobei das Protein nicht das Thermus thermophilus DnaX-Protein ist.

42. Gereinigtes Protein nach Anspruch 41, **dadurch gekennzeichnet, dass** das gamma-Protein von Thermus thermophilus ist.

43. Nukleotidsequenz nach Anspruch 40, **dadurch gekennzeichnet, dass** die Nukleotidsequenz des Weiteren 5'- und 3'-flankierende Regionen aufweist.

44. Rekombinanter Vektor, umfassend die Nukleotidsequenz nach Anspruch 40.

45. Wirtszelle, enthaltend den rekombinanten DNA-Vektor nach Anspruch 44.

46. Fusionsprotein, umfassend das gamma-Protein nach Anspruch 40 und eine nicht-gamma-Proteinsequenz.

47. Verfahren zur Identifikation von Polymeraseuntereinheiten, umfassend die Schritte:
a) Bereitstellung einer Testprobe, von der erwartet wird, dass sie amplifizierbare Nukleinsäure enthält, die für zumindest einen Teil eines DNA-Polymerase III-Untereinheit-Proteins kodiert;
b) Isolierung der amplifizierbaren Nukleinsäure aus der Testprobe;
c) Kombination der amplifizierbaren Nukleinsäure mit Amplifikationsreagenzien und mindestens zwei Primern, ausgewählt aus der Gruppe bestehend aus Primern, die die Nukleinsäuresequenz haben, wie sie in den SEQ ID Nr.: 21, 22, 86, 87, 91, 97, 110, 111, 112, 113, 114, 115, 116, 134, 135, 136, 137, 156, 157, 173, 174, 176, 177, 208, 209, 210, 211, 212 und 213 beschrieben sind, um ein Reaktionsgemisch zu bilden, und
d) Kombination des Reaktionsgemisches mit einem Amplifikationsenzym unter Bedingungen, bei denen die amplifizierbare Nukleinsäure amplifiziert wird, um ein Amplifikationsprodukt zu bilden.

48. Verfahren nach Anspruch 47, umfassend den weiteren Schritt der Detektion des Amplifikationsproduktes.

49. Verfahren nach Anspruch 48, in dem die Detektion durch Hybridisierung des Amplifikationsproduktes mit einer Probe ausgeführt wird.

50. Verfahren nach Anspruch 47, **dadurch gekennzeichnet, dass** die Primer in der Lage sind mit einer Nukleinsäure zu hybridisieren, die mindestens eine Polymeraseuntereinheit, ausgewählt aus der Gruppe bestehend aus DnaA, DnaN, DnaE, DnaX, DnaQ und DnaB, kodiert.

51. Verfahren nach Anspruch 47, **dadurch gekennzeichnet, dass** die Testprobe Nukleinsäure umfasst, die von einem thermophilen Organismus erhalten wurde.

52. Verfahren nach Anspruch 51, **dadurch gekennzeichnet, dass** der thermophile Organismus ein Mitglied der Thermus-Gattung ist.

## Revendications

1. Séquence nucléotidique isolée consistant en la séquence décrite dans la SEQ ID NO :196.

2. Séquence nucléotidique isolée selon la revendication 1, dans laquelle ladite séquence nucléotidique comprend en outre des régions flanquantes 5' et 3'.

3. Vecteur d'ADN recombinant comprenant le nucléotide de la revendication 1.

4. Cellule hôte contenant le vecteur d'ADN recombinant de la revendication 3.

5. Protéine DnaE purifiée comprenant la séquence d'acides aminés décrite dans la SEQ ID NO : 197.

6. Protéine de fusion comprenant au moins une partie de la protéine DnaE de la revendication 5, et une séquence de protéine non DnaE.

7. Séquence nucléotidique isolée telle que décrite dans la SEQ ID NO :214.

8. Séquence nucléotidique isolée selon la revendication 7, où ladite séquence nucléotidique comprend en outre des régions flanquantes 5' et 3'.

9. Vecteur d'ADN recombinant comprenant le nucléotide de la revendication 7.

10. Cellule hôte contenant le vecteur d'ADN recombinant de la revendication 9.

11. Protéine DnaQ purifiée comprenant une séquence d'acides aminés sélectionnée dans le groupe consistant en les SEQ ID NO :215 et 216.

12. Protéine de fusion comprenant la protéine DnaQ de la revendication 11, et une séquence de protéine non DnaQ.

13. Séquence nucléotidique isolée décrite dans la SEQ ID NO :221.

14. Séquence nucléotidique isolée selon la revendication 13, dans laquelle ladite séquence nucléotidique comprend en outre des régions flanquantes 5' et 3'.

15. Vecteur d'ADN recombinant comprenant le nucléotide de la revendication 13.

16. Cellule hôte contenant le vecteur d'ADN recombinant de la revendication 15.

17. Protéine DnaA purifiée comprenant la séquence d'acides aminés décrite dans la SEQ ID NO :222.

18. Protéine de fusion comprenant la protéine DnaA de la revendication 17, et une séquence de protéine non DnaA.

19. Séquence nucléotidique isolée consistant en la séquence décrite dans la SEQ ID NO :230.

20. Séquence nucléotidique selon la revendication 19, où ladite séquence nucléotidique comprend en outre des régions flanquantes 5' et 3'.

21. Vecteur d'ADN recombinant comprenant le nucléotide de la revendication 19.

22. Cellule hôte contenant le vecteur d'ADN recombinant de la revendication 21.

23. Protéine DnaN purifiée consistant en la séquence d'acides aminés décrite dans la SEQ ID NO :231.

24. Protéine de fusion comprenant la protéine DnaN de la revendication 23, et une séquence de protéine non DnaN.

25. Séquence nucléotidique isolée décrite dans la SEQ ID NO :7.

26. Séquence nucléotidique isolée selon la revendication 25, où ladite séquence nucléotidique comprend en outre des régions flanquantes 5' et 3'.

27. Vecteur d'ADN recombinant comprenant le nucléotide de la revendication 25.

28. Cellule hôte contenant le vecteur d'ADN recombinant de la revendication 27.

29. Protéine dnaX purifiée comprenant la séquence d'acides aminés décrite dans la SEQ ID NO :9.

30. Protéine de fusion comprenant la protéine dnaX de la revendication 29, et une séquence de protéine non dnaX.

31. Séquence d'acides aminés isolée telle que décrite dans la SEQ ID NO :2.

32. Séquence nucléotidique isolée codant pour la séquence d'acides aminés de la revendication 31.

33. Séquence nucléotidique isolée selon la revendication 32, où ladite séquence nucléotidique comprend en outre des régions flanquantes 5' et 3'.

34. Vecteur recombinant comprenant la séquence nucléotidique de la revendication 32.

35. Cellule hôte contenant le vecteur d'ADN recombinant de la revendication 34.

36. Protéine tau purifiée codée par une séquence polynucléotidique homologue au brin codant des séquences nucléotidique de la revendication 31.

37. Protéine purifiée selon la revendication 36, où ladite protéine tau est issue de Thermus thermophilus.

38. Protéine de fusion comprenant la protéine tau de la revendication 36, et une séquence de protéine non tau.

39. Séquence d'acides aminés isolée telle que décrite dans la SEQ ID NO :1.

40. Séquence nucléotidique isolée codant pour la séquence d'acides aminés de la revendication 39.

41. Protéine gamma purifiée codée par une séquence polynucléotidique homologue au brin codant des séquences nucléotidiques de la revendication 40, dans laquelle la protéine n'est pas la protéine DnaX de Thermus thermophilus.

42. Protéine purifiée selon la revendication 41, dans laquelle ladite protéine gamma est issue de Thermus thermophilus.

43. Séquence nucléotidique selon la revendication 40, dans laquelle ladite séquence nucléotidique comprend en outre des régions flanquantes 5' et 3'.

44. Vecteur recombinant comprenant la séquence nucléotidique de la revendication 40.

45. Cellule hôte contenant le vecteur d'ADN recombinant de la revendication 44.

46. Protéine de fusion comprenant la protéine gamma de la revendication 40, et une séquence de protéine non gamma.

47. Procédé d'identification de sous-unités de polymérase comprenant les étapes consistant à :
a) mettre à disposition un échantillon test suspecté de contenir un acide nucléique amplifiable codant pour au moins une partie d'une protéine de sous-unité d'ADN polymérase III ;
b) isoler ledit acide nucléique amplifiable à partir dudit échantillon de test ;
c) combiner ledit acide nucléique amplifiable avec des réactifs d'amplification, et au moins deux amorces sélectionnées dans le groupe consistant en des amorces ayant la séquence d'acide nucléique décrite dans la SEQ ID NO :21, 22, 86, 87, 91, 97, 110, 111, 112, 113, 114, 115, 116, 134, 135, 136, 137, 156, 157, 173, 174, 176, 177, 208, 209, 210, 211, 212, et 213, afin de former un mélange réactionnel ; et
d) combiner ledit mélange réactionnel avec une enzyme d'amplification dans des conditions où ledit acide nucléique amplifiable est amplifié pour former un produit d'amplification.

48. Procédé selon la revendication 47, comprenant en outre une étape de détection dudit produit d'amplification.

49. Procédé selon la revendication 48, dans lequel ladite détection est réalisée par une hybridation dudit produit d'amplification avec une sonde.

50. Procédé selon la revendication 47, où lesdites amorces sont capables de s'hybrider à un acide nucléique codant pour au moins une sous-unité de polymérase sélectionnée dans le groupe consistant en DnaA, DnaN, DnaE, DnaX, DnaQ, et DnaB.

51. Procédé selon la revendication 47, où ledit échantillon de test comprend un acide nucléique obtenu à partir d'un organisme thermophile.

52. Procédé selon la revendication 51, où ledit organisme thermophile est un membre du genre Thermus.


### SEQUENCE LISTING

<110> Enzyco, Inc.
<120> NOVEL THERMOPHILIC POLYMERASE III HOLOENZYME
<130> EP 98 944 849.3
<140> EP 98 944 849.3
   <141> 1998-09-11
<150> PCT/US98/18946
   <151> 1998-09-11
<150> 09/151,888
   <151> 1998-09-11
<150> 08/928,213
   <151> 1997-09-12
<160> 236
<170> PatentIn Ver. 2.0
<210> 1
   <211> 19
   <212> PRT
   <213> Thermus thermophilus
<400> 1
   Ser Ala Leu Tyr Arg Arg Phe Arg Pro Leu Thr Phe Gln Glu Val Val
     1               5                  10                  15
   Gly Gln Glu
<210> 2
   <211> 20
   <212> PRT
   <213> Thermus thermophilus
<400> 2
   Ser Ala Leu Tyr Arg Arg Phe Arg Pro Leu Thr Phe Gln Glu Val Val
     1               5                  10                  15
   Gly Gln Glu His
                20
<210> 3
   <211> 18
   <212> PRT
   <213> Escherichia coli
<400> 3
   Leu Ala Arg Lys Trp Arg Pro Gln Thr Phe Ala Arg Val Val Gly Gln
   1 5 10 15
   Glu His
<210> 4
   <211> 19
   <212> PRT
   <213> Bacillus subtilis
<400> 4
   Ala Leu Tyr Arg Val Phe Arg Pro Gln Arg Phe Glu Asp Val Val Gly
   1 5 10 15
   Gln Glu His
<210> 5
   <211> 18
   <212> PRT
   <213> Artificial Sequence
<220>
   <221> SITE
   <222> (4)
   <223> The residue at this position is similar but not identical to arginine or lysine
<220>
   <221> SITE
   <222> (5)
   <223> The residue at this position is similar but not identical to phenyalanine or tryptophan
<220>
   <221> SITE
   <222> (12)
   <223> The residue at this position is similar but not identical to glutamic acid or aspartic acid
<220>
   <223> Description of Artificial Sequence: Homology
<220>
   <221> SITE
   <222> (2)
   <223> The residue at this position can be any amino acid.
<220>
   <221> SITE
   <222> (8)
   <223> The residue at this position can be any amino acid.
<220>
   <221> SITE
   <222> (11)
   <223> The residue at this position can be any amino acid.
<400> 5
<210> 6
   <211> 19
   <212> PRT
   <213> Artificial Sequence
<220>
   <221> SITE
   <222> (12)
   <223> The residue at this position is similar but not identical to glutamine and/or glutamic acid
<220>
   <221> SITE
   <222> (13)
   <223> The residue at this position is similar but not identical to aspartic acid and/or glutamic acid
<220>
   <223> Description of Artificial Sequence: Homology
<220>
   <221> SITE
   <222> (5)
   <223> The residue at this position can be any amino acid.
<220>
   <221> SITE
   <222> (9)
   <223> The residue at this position can be any amino acid.
<220>
   <221> SITE
   <222> (10)
   <223> The residue at this position can be any amino acid.
<400> 6
<210> 7
   <211> 2681
   <212> DNA
   <213> Thermus thermophilus
<400> 7
   tccgccaggc gctttcccgt gaggtagagg gccatctcca ccacctcgag ctgctccggg 60
   ggcgtcaccc gaagcccccc cacgaagcgg ctctcgtacc ccaggcgccc aagccaagcc 120
   ccgatctcgg gcccgccccc gtggacgaga accaagggac cagggtaggc ggcaagctcg 180
   tccaataggg cctccgcccc cctaaggctt cctcccacct tcaccaaaag ggcctcactc 240
   aaggtagacc acctcctcgg gcaggccctc ctcctcctcg gcctccagga ggtcggcgaa 300
   gcccagaagg gcgtggtccg ggtggaggcc gaagcgggcg gcgagggccc ggaccgcgtg 360
   ctcgggcggg agggcctggg ccctcgtcag gaggggggcg aggtcggccg gggccttcag 420
   ggccgtccca agctccggac ccgcgcgaag ctccgccctc acctcccccc cctcggccag 480
   gaggaagagg aggtcctcct cgcttagtac caggaaggcc aaggcctgcc ctaggcggga 540
   aagctcccgc aggaaggccc ggatggcctc cgggtcctcc tccgcctcga gggcctcgtg 600
   gtagaggctc gtccaagggg ggtcggggac caggtagacc cccgtccctc ccgtgcgccc 660
   ctcggcccag gccgccaccg cctccagggg ggcctgcacg tgcaaggaga ggaagctccg 720
   caccacgccc tatactagcc cttgtgagcg ccctctaccg ccgcttccgc cccctcacct 780
   tccaggaggt ggtggggcag gagcacgtga aggagcccct cctcaaggcc atccgggagg 840
   ggaggctcgc ccaggcctac ctcttctccg ggcccagggg cgtgggcaag accaccacgg 900
   cgaggctcct cgccatggcg gtggggtgcc agggggaaga ccccccttgc ggggtctgcc 960
   cccactgcca ggcggtgcag aggggcgccc acccggacgt ggtggagatt gacgccgcca 1020
   gcaacaactc cgtggaggac gtgcgggagc tgagggaaag gatccacctc gccccccttt 1080
   ctgcccccag gaaggtcttc atcctggacg aggcccacat gctctccaaa agcgccttca 1140
   acgccctcct caagaccctg gaggagcccc cgccccacgt cctcttcgtc ttcgccacca 1200
   ccgagcccga gaggatgccc cccaccatcc tctcccgcac ccagcacttc cgcttccgcc 1260
   gcctcacgga ggaggagatc gcctttaagc tccggcgcat cctggaggcc gtggggcggg 1320
   aggcggagga ggaggccctc ctcctcctcg cccgcctggc ggacggggcc cttagggacg 1380
   cggaaagcct cctggagcgc ttcctcctcc tggaaggccc cctcacccgg aaggaggtgg 1440
   agcgcgccct aggcctcccc cccagggagg ccctggccga gatcgccgcc tccctcgcga 1500
   gggggaaaac ggcggaggcc ctgggcctcg cccggcgcct ctacggggaa gggtacgccc 1560
   cgaggagcct ggtctcgggc cttttggagg tgttccggga aggcctctac gccgccttcg 1620
   gcctcgcggg aacccccctt cccgccccgc cccaggccct gatcgccgcc atgaccgccc 1680
   tggacgaggc catggagcgc ctcgcccgcc gctccgacgc cttaagcctg gaggtggccc 1740
   tcctggaggc gggaagggcc ctggccgccg aggccctgcc ccagcccacg ggcgctcccc 1800
   ccccagaggt cggccccaag ccggaaagcc ccccggcccc ggaaccccca aggcccgagg 1860
   aggcgcccga cctgcgggag cggtggcggg ccttcctcga ggccctcagg cccaccctac 1920
   gggccttcgt gcgggaggcc cgcccggagg tccgggaagg ccagctctgc ctcgctttcc 1980
   ccgaggacaa ggccttccac taccgcaagg cctcggaaca gaaggcgagg ctcctccccc 2040
   tggcccaggc ccatttcggg gtggaggagg tcgttctcgt cctggaggga gaaaaaaaaa 2100
   gcctgagccc aaggccccgc tcggccccac ctcctgaagc gcccgcaccc ccgggccctc 2160
   ccgaggagga ggtagaggcg gaggaagcgg cggaggaggc cccggaggag gccttgaggc 2220
   gggtggtccg cctcctgggg gggcgggtgc tctgggtgcg gcggcccagg acccgggagg 2280
   cgccggagga ggaacccctg agccaagacg agataggggg tactggtata taatgggggc 2340
   atgacgcgga ccaccgacct cggacaagag accgtggaca acatcctcaa gcgcctccgc 2400
   cgtattgagg gccaggtgcg gggctccaaa aagatggtgg ccgagggccg cccctgcgac 2460
   gaggtcctca cccagatgac cgccaccaag aaggccatgg aggcggcggc caccctgatc 2520
   ctccacgagt tcctgaacgt ctgcgccgcc gaggtctccg agggcaaggt gaaccccaag 2580
   aagcccgagg agatcgccac catgctgaag aagttcatct agatgggtcg gcttcggggg 2640
   cgcctccggc gcctcctccg ggcccttctc gcccaggagg c                     2681
<210> 8
   <211> 528
   <212> PRT
   <213> Thermus thermophilus
<400> 8
   Ser Ala Leu Tyr Arg Arg Phe Arg Pro Leu Thr Phe Gln Glu Val Val
    1               5                  10                  15
   Gly Gln Glu His Val Lys Glu Pro Leu Leu Lys Ala Ile Arg Glu Gly
               20                  25                  30
   Arg Leu Ala Gln Ala Tyr Leu Phe Ser Gly Pro Arg Gly Val Gly Lys
           35                  40                  45
   Thr Thr Thr Ala Arg Leu Leu Ala Met Ala Val Gly Cys Gln Gly Glu
       50                  55                  60
   Asp Pro Pro Cys Gly Val Cys Pro His Cys Gln Ala Val Gln Arg Gly
   65                  70                  75                  80
   Ala His Pro Asp Val Val Glu Ile Asp Ala Ala Ser Asn Asn Ser Val
   85 90 95
   Glu Asp Val Arg Glu Leu Arg Glu Arg Ile His Leu Ala Pro Leu Ser
   100 105 110
   Ala Pro Arg Lys Val Phe Ile Leu Asp Glu Ala His Met Leu Ser Lys
   115 120 125
   Ser Ala Phe Asn Ala Leu Leu Lys Thr Leu Glu Glu Pro Pro Pro His
   130 135 140
   Val Leu Phe Val Phe Ala Thr Thr Glu Pro Glu Arg Met Pro Pro Thr
   145 150 155 160
   Ile Leu Ser Arg Thr Gln His Phe Arg Phe Arg Arg Leu Thr Glu Glu
   165 170 175
   Glu Ile Ala Phe Lys Leu Arg Arg Ile Leu Glu Ala Val Gly Arg Glu
   180 185 190
   Ala Glu Glu Glu Ala Leu Leu Leu Leu Ala Arg Leu Ala Asp Gly Ala
   195 200 205
   Leu Arg Asp Ala Glu Ser Leu Leu Glu Arg Phe Leu Leu Leu Glu Gly
   210 215 220
   Pro Leu Thr Arg Lys Glu Val Glu Arg Ala Leu Gly Leu Pro Pro Arg
   225 230 235 240
   Glu Ala Leu Ala Glu Ile Ala Ala Ser Leu Ala Arg Gly Lys Thr Ala
   245 250 255
   Glu Ala Leu Gly Leu Ala Arg Arg Leu Tyr Gly Glu Gly Tyr Ala Pro
   260 265 270
   Arg Ser Leu Val Ser Gly Leu Leu Glu Val Phe Arg Glu Gly Leu Tyr
   275 280 285
   Ala Ala Phe Gly Leu Ala Gly Thr Pro Leu Pro Ala Pro Pro Gln Ala
   290 295 300
   Leu Ile Ala Ala Met Thr Ala Leu Asp Glu Ala Met Glu Arg Leu Ala
   305 310 315 320
   Arg Arg Ser Asp Ala Leu Ser Leu Glu Val Ala Leu Leu Glu Ala Gly
   325 330 335
   Arg Ala Leu Ala Ala Glu Ala Leu Pro Gln Pro Thr Gly Ala Pro Pro
   340 345 350
   Pro Glu Val Gly Pro Lys Pro Glu Ser Pro Pro Ala Pro Glu Pro Pro
   355 360 365
   Arg Pro Glu Glu Ala Pro Asp Leu Arg Glu Arg Trp Arg Ala Phe Leu
   370 375 380
   Glu Ala Leu Arg Pro Thr Leu Arg Ala Phe Val Arg Glu Ala Arg Pro
   385 390 395 400
   Glu Val Arg Glu Gly Gln Leu Cys Leu Ala Phe Pro Glu Asp Lys Ala
   405 410 415
   Phe His Tyr Arg Lys Ala Ser Glu Gln Lys Ala Arg Leu Leu Pro Leu
   420 425 430
   Ala Gln Ala His Phe Gly Val Glu Glu Val Val Leu Val Leu Glu Gly
   435 440 445
   Glu Lys Lys Ser Leu Ser Pro Arg Pro Arg Ser Ala Pro Pro Pro Glu
   450 455 460
   Ala Pro Ala Pro Pro Gly Pro Pro Glu Glu Glu Val Glu Ala Glu Glu
   465 470 475 480
   Ala Ala Glu Glu Ala Pro Glu Glu Ala Leu Arg Arg Val Val Arg Leu
   485 490 495
   Leu Gly Gly Arg Val Leu Trp Val Arg Arg Pro Arg Thr Arg Glu Ala
   500 505 510
   Pro Glu Glu Glu Pro Leu Ser Gln Asp Glu Ile Gly Gly Thr Gly Ile
   515 520 525
<210> 9
   <211> 273
   <212> PRT
   <213> Thermus thermophilus
<220>
   <221> SITE
   <222> (1)..(6)
   <223> The residues at these positions can be any amino acid.
<220>
   <221> SITE
   <222> (70) .. (75)
   <223> The residues at these positions can be any amino acid.
<220>
   <221> SITE
   <222> (145)
   <223> The residue at this position can be any amino acid.
<220>
   <221> SITE
   <222> (155)..(156)
   <223> The residues at these positions can be any amino acid.
<220>
   <221> SITE
   <222> (185)..(188)
   <223> The residues at these positions can be amino acid.
<220>
   <221> SITE
   <222> (242) .. (294)
   <223> The residues at these positions can be any amino acid.
<220>
   <221> SITE
   <222> (263)..(273)
   <223> The residues at these positions can be amino acid.
<400> 9
<210> 10
   <211> 273
   <212> PRT
   <213> Escherichia coli
<220>
   <221> SITE
   <222> (1)..(3)
   <223> The residues at these positions can be any amino acid.
<220>
   <221> SITE
   <222> (71)..(74)
   <223> The residues at these positions can be any amino acid.
<220>
   <221> SITE
   <222> (145)
   <223> The residue at this position can be any amino acid.
<220>
   <221> SITE
   <222> (155)..(156)
   <223> The residues at these positions can be any amino acid.
<220>
   <221> SITE
   <222> (185)..(188)
   <223> The residues at these positions can be any amino acid.
<220>
   <221> SITE
   <222> (243)..(294)
   <223> The residues at these positions can be any amino acid.
<400> 10
<210> 11
   <211> 273
   <212> PRT
   <213> Bacillus subtilis
<220>
   <221> SITE
   <222> (1)..(3)
   <223> The residues at these positions can be any amino acid.
<220>
   <221> SITE
   <222> (71)..(74)
   <223> The residues at these positions can be any amino acid.
<220>
   <221> SITE
   <222> (145)
   <223> The residue at this position can be any amino acid.
<220>
   <221> SITE
   <222> (155)..(156)
   <223> The residues at these positions can be any amino acid.
<220>
   <221> SITE
   <222> (185)..(188)
   <223> The residues at these positions can be any amino acid.
<220>
   <221> SITE
   <222> (243)..(244)
   <223> The residues at these positions can be any amino acid.
<220>
   <221> SITE
   <222> (263)..(273)
   <223> The residues at these positions can be any amino acid.
<400> 11
<210> 12
   <211> 273
   <212> PRT
   <213> Mycoplasma pneumoniae
<220>
   <221> SITE
   <222> (1)..(4)
   <223> The residues at these positions can be any amino acid.
<220>
   <221> SITE
   <222> (70)..(74)
   <223> The residues at these positions can be any amino acid.
<220>
   <221> SITE
   <222> (145)
   <223> The residue at this position can be any amino acid.
<220>
   <221> SITE
   <222> (155)..(156)
   <223> The residues at these positions can be any amino acid.
<220>
   <221> SITE
   <222> (185)..(188)
   <223> The residues at these positions can be any amino acid.
<220>
   <221> SITE
   <222> (244)
   <223> The residue at this position can be any amino acid.
<220>
   <221> SITE
   <222> (265)..(271)
   <223> The residues at these positions can be any amino acid.
<400> 12

<210> 13
   <211> 273
   <212> PRT
   <213> Caulobacter crescentus
<220>
   <221> SITE
   <222> (145)
   <223> The residue at this position can be any amino acid.
<220>
   <221> SITE
   <222> (155)..(156)
   <223> The residues at these positions can be any amino acid.
<220>
   <221> SITE
   <222> (185)..(188)
   <223> The residues at these positions can be any amino acid.
<220>
   <221> SITE
   <222> (263)..(273)
   <223> The residues at these positions can be any amino acid.
<400> 13
<210> 14
   <211> 273
   <212> PRT
   <213> Synechocystis sp.
<220>
   <221> SITE
   <222> (1)..(3)
   <223> The residues at these positions can be any amino acid.
<220>
   <221> SITE
   <222> (73)..(74)
   <223> The residues at these positions can be any amino acid.
<220>
   <221> SITE
   <222> (244)
   <223> The residue at this position can be any amino acid.
<400> 14
<210> 15
   <211> 273
   <212> PRT
   <213> Artificial Sequence
<220>
   <223> Description of Artificial Sequence: Consensus
<220>
   <221> SITE
   <222> (1)..(5)
   <223> The residues at these positions can be any amino acid.
<220>
   <221> SITE
   <222> (6)..(7)
   <223> The residues at these positions can be any amino acid.
<220>
   <221> SITE
   <222> (10)
   <223> The residue at this position can be any amino acid.
<220>
   <221> SITE
   <222> (16)
   <223> The residue at this position can be any amino acid.
<220>
   <221> SITE
   <222> (19)
   <223> The residue at this position can be any amino acid.
<220>
   <221> SITE
   <222> (26)
   <223> The residue at this position can be any amino acid.
<220>
   <221> SITE
   <222> (28)..(30)
   <223> The residues at these positions can be any amino acid.
<220>
   <221> SITE
   <222> (32)
   <223> The residue at this position can be any amino acid.
<220>
   <221> SIMILAR
   <222> (36)..(38)
   <223> The residues at these positions can be any amino acid.
<220>
   <221> SITE
   <222> (41)
   <223> The residue at this position can be any amino acid.
<220>
   <221> SITE
   <222> (49)
   <223> The residue at this position can be any amino acid.
<220>
   <221> SITE
   <222> (52)
   <223> The residue at this position can be any amino acid.
<220>
   <221> SITE
   <222> (57)
   <223> The residue at this position can be any amino acid.
<220>
   <221> SITE
   <222> (68)..(78)
   <223> The residues at these positions can be any amino acid.
<220>
   <221> SITE
   <222> (81)..(85)
   <223> The residues at these positions can be any amino acid.
<220>
   <221> SITE
   <222> (90)..(91)
   <223> The residues at these positions can be any amino acid.
<220>
   <221> SITE
   <222> (93)..(99)
   <223> The residues at these positions can be any amino acid.
<220>
   <221> SITE
   <222> (105)..(107)
   <223> The residues at these positions can be any amino acid.
<220>
   <221> SITE
   <222> (115)
   <223> The residue at this position can be any amino acid.
<220>
   <221> SITE
   <222> (117)
   <223> The residue at this position can be any amino acid.
<220>
   <221> SITE
   <222> (119)
   <223> The residue at this position can be any amino acid.
<220>
   <221> SITE
   <222> (123)..(125)
   <223> The residues at these positions can be any amino acid.
<220>
   <221> SITE
   <222> (135)
   <223> The residue at this position can be any amino acid.
<220>
   <221> SITE
   <222> (140)..(142)
   <223> The residues at these positions can be any amino acid.
<220>
   <221> SITE
   <222> (145)
   <223> The residue at this position can be any amino acid.
<220>
   <221> SITE
   <222> (155)..(156)
   <223> The residues at these positions can be any amino acid.
<220>
   <221> SITE
   <222> (158)
   <223> The residue at this position can be any amino acid.
<220>
   <221> SITE
   <222> (160)
   <223> The residue at this position can be any amino acid.
<220>
   <221> SITE
   <222> (169)..(170)
   <223> The residues at these positions can be any amino acid.
<220>
   <221> SITE
   <222> (184)..(189)
   <223> The residues at these positions can be any amino acid.
<220>
   <221> SITE
   <222> (193)..(194)
   <223> The residues at these positions can be any amino acid.
<220>
   <221> SITE
   <222> (196)
   <223> The residue at this position can be any amino acid.
<220>
   <221> SITE
   <222> (198)..(199)
   <223> The residues at these positions can be any amino acid.
<220>
   <221> SITE
   <222> (202)
   <223> The residue at this position can be any amino acid.
<220>
   <221> SITE
   <222> (206)..(207)
   <223> The residues at these positions can be any amino acid.
<220>
   <221> SITE
   <222> (209)..(212)
   <223> The residues at these positions can be any amino acid.
<220>
   <221> SITE
   <222> (214)
   <223> The residue at this position can be any amino acid.
<220>
   <221> SITE
   <222> (218)
   <223> The residue at this position can be any amino acid.
<220>
   <221> SITE
   <222> (223)
   <223> The residue at this position can be any amino acid.
<220>
   <221> SITE
   <222> (227)
   <223> The residue at this position can be any amino acid.
<220>
   <221> SITE
   <222> (238)
   <223> The residue at this position can be any amino acid.
<220>
   <221> SITE
   <222> (240)..(297)
   <223> The residues at these positions can be any amino acid.
<220>
   <221> SITE
   <222> (250)..(252)
   <223> The residues at these positions can be any amino acid.
<220>
   <221> SITE
   <222> (259)..(256)
   <223> The residues at these positions can be any amino acid.
<220>
   <221> SITE
   <222> (259)..(261)
   <223> The residues at these positions can be any amino acid.
<220>
   <221> SITE
   <222> (263)..(273)
   <223> The residues at these positions can be any amino acid.
<400> 15
<210> 16
   <211> 47
   <212> DNA
   <213> Thermus thermophilus
<400> 16
   gaaaaaaaaa gcctgagccc aaggccccgc tcggccccac ctcctga 47
<210> 17
   <211> 15
   <212> PRT
   <213> Thermus thermophilus
<400> 17
   Glu Lys Lys Ser Leu Ser Pro Arg Pro Arg Ser Ala Pro Pro Pro
   1 5 10 15
<210> 18
   <211> 15
   <212> PRT
   <213> Thermus thermophilus
<400> 18
   Glu Lys Lys Lys Pro Glu Pro Lys Ala Pro Leu Gly Pro Thr Ser
   1 5 10 15
<210> 19
   <211> 4
   <212> PRT
   <213> Thermus thermophilus
<400> 19
   Glu Lys Lys Ala
    1
<210> 20
   <211> 371
   <212> DNA
   <213> Thermus thermophilus
<400> 20
   ggttcctcaa ggtcttgagg agggcgttga aggcgctttt ggaaacatgt gggcctcgtc 60
   cagataaaac cttcctgggg gcaaaagggg ggcgaggtgg atcctttccc tcagctcccg 120
   cactcctcca cggattgttg ctggcggcgt caatctccac cacgtccggg tgggcgcccc 180
   tctgcaccgc ctggcagtgg gggcaacccc gcaagggggg tcttccccct ggcaccccac 240
   cgccatggca gacctcccgt ggtggtcttg cccaccccct gggcccggaa aaaaagtgcc 300
   tgggcgaacc tcccctcccg gatggccttg agagggctcc ttcactgctc ctgacccaca 360
   acctcttgaa a                                                       371
<210> 21
   <211> 20
   <212> DNA
   <213> Artificial Sequence
<220>
   <223> Description of Artificial Sequence: Primer X1FA
<220>
   <221> misc_feature
   <222> (12)
   <223> The residue at this position can be any nucleotide.
<220>
   <221> misc_feature
   <222> (15)
   <223> The residue at this position can be any nucleotide.
<400> 21
   ttycargarg tngtnggwca 20
<210> 22
   <211> 17
   <212> DNA
   <213> Artificial Sequence
<220>
   <223> Description of Artificial Sequence: Primer X139R
<220>
   <221> misc_feature
   <222> (9)
   <223> The residue at this position can be any nucleotide.
<220>
   <221> misc_feature
   <222> (12)
   <223> The residue at this position can be any nucleotide.
<400> 22
   ggytcytcna rngtytt 17

<210> 23
   <211> 71
   <212> PRT
   <213> Thermus thermophilus
<400> 23
<210> 24
   <211> 71
   <212> PRT
   <213> Bacillus subtilis
<400> 24
   Asp Glu Pro Cys Asn Glu Cys Ala Ala Cys Lys Gly Ile Thr Asn Gly
    1               5                  10                  15
   Ser Ile Ser Asp Val Ile Glu Ile Asp Ala Ala Ser Asn Asn Gly Val
               20                  25                  30
   Asp Glu Ile Arg Asp Ile Arg Asp Lys Val Lys Phe Ala Pro Ser Ala
           35                  40                  45
   Val Thr Tyr Lys Val Tyr Ile Ile Asp Glu Val His Met Leu Ser Ile
       50                  55                  60
   Gly Ala Phe Asn Ala Leu Leu
   65                  70
<210> 25
   <211> 71
   <212> PRT
   <213> Artificial Sequence
<220>
   <221> SITE
   <222> (11)
   <223> The residue at this position is similar but not identical to glutamine and/or lysine
<220>
   <221> SITE
   <222> (13)
   <223> The residue at this position is similar but not identical to valine and/or isoleucine
<220>
   <221> SITE
   <222> (17)
   <223> The residue at this position is similar but not identical to serine and/or alanine
<220>
   <221> SITE
   <222> (22)
   <223> The residue at this position is similar but not identical to isoleucine and/or valine
<220>
   <221> SITE
   <222> (42)
   <223> The residue at this position is similar but not identical to isoleucine and/or valine
<220>
   <221> SITE
   <222> (56)
   <223> The residue at this position is similar but not identical to leucine and/or isoleucine
<220>
   <223> Description of Artificial Sequence: Consensus
<400> 25
<210> 26
   <211> 10
   <212> PRT
   <213> Thermus thermophilus
<400> 26
   Arg Phe Ala His Leu His Gln His Thr Gln
   1 5 10
<210> 27
   <211> 9
   <212> PRT
   <213> Mycobacterium tuberculosis
<400> 27
   Phe Val His Leu His Asn His Thr Glu
   1 5
<210> 28
   <211> 10
   <212> PRT
   <213> Haemophilus influenzae
<400> 28
   Arg Phe Ile His Leu Arg Thr His Thr Asp
   1 5 10
<210> 29
   <211> 9
   <212> PRT
   <213> Artificial Sequence
<220>
   <221> SITE
   <222> (9)
   <223> The residue at this position is similar but not identical to glutamic acid, glutamine and/or aspartic acid
<220>
   <223> Description of Artificial Sequence: Homology
<400> 29
<210> 30
   <211> 10
   <212> PRT
   <213> Artificial Sequence
<220>
   <223> Description of Artificial Sequence: Homology
<400> 30
<210> 31
   <211> 23
   <212> PRT
   <213> Thermus thermophilus
<400> 31
<210> 32
   <211> 18
   <212> PRT
   <213> Escherichia coli
<400> 32
   Thr Gly Tyr Gln Asn Leu Thr Leu Leu Ile Ser Lys Ala Tyr Gln Arg
    1              5                   10                  15
   Gly Tyr
<210> 33
   <211> 19
   <212> PRT
   <213> Artificial Sequence
<220>
   <221> SITE
   <222> (13)
   <223> The residue at this position is similar but not identical to arginine and/or lysine
<220>
   <221> SITE
   <222> (19)
   <223> The residue at this position is similar but not identical to phenyalanine and/or tyrosine
<220>
   <223> Description of Artificial Sequence: Homology
<400> 33
<210> 34
   <211> 26
   <212> PRT
   <213> Thermus thermophilus
<220>
   <221> SITE
   <222> (1)
   <223> The residue at this position is uncertain but could be either Gly, Trp or Pro
<220>
   <221> UNSURE
   <222> (16)
   <223> The residue at this location is uncertain but could be Gln
<220>
   <221> UNSURE
   <222> (19)
   <223> The residue at this position is uncertain but could be Ile or Ala
<220>
   <221> SITE
   <222> (21)
   <223> The residue at this position is uncertain but could be either Ala or Ser
<220>
   <221> SITE
   <222> (24)
   <223> The residue at this position is uncertain but could be either Gly or Ala
<220>
   <221> UNSURE
   <222> (26)
   <223> The residue at this location is uncertain
<220>
   <221> SITE
   <222> (22)
   <223> The residue at this position is uncertain, but could be Ala.
<220>
   <221> SITE
   <222> (23)
   <223> The residue at this position is uncertain but could be Val
<400> 34
<210> 35
   <211> 19
   <212> PRT
   <213> Escherichia coli
<400> 35
   Val Leu Glu Pro Thr Tyr Gly Ile Ile Leu Tyr Gln Glu Gln Val Met
   1 5 10 15
   Gln Ile Ala
<210> 36
   <211> 19
   <212> PRT
   <213> Artificial Sequence
<220>
   <221> SITE
   <222> (1)
   <223> The residue at this position is similar but not identical to Gly, Trp, Pro or Val.
<220>
   <221> SITE
   <222> (3)
   <223> The residue at this position is similar but not identical to Leucine and/or glutamic
<220>
   <221> SITE
   <222> (10)
   <223> The residue at this position is similar but not identical to proline and/or leucine
<220>
   <223> Description of Artificial Sequence: Homology
<400> 36
<210> 37
   <211> 13
   <212> PRT
   <213> Thermus thermophilus
<220>
   <221> UNSURE
   <222> (11)
   <223> The residue at this position is uncertain buy may be leucine
<220>
   <221> UNSURE
   <222> (12)
   <223> The residue at this position is uncertain but may be phenylalanine
<220>
   <221> UNSURE
   <222> (13)
   <223> The residue at this position is uncertain, but probably Asp or Ala
<400> 37
   Gly Leu Asp Gly Gly Tyr Phe His Leu Thr Leu Phe Asp
    1               5                   10
<210> 38
   <211> 13
   <212> PRT
   <213> Escherichia coli
<400> 38
<210> 39
   <211> 11
   <212> PRT
   <213> Artificial Sequence
<220>
   <221> SITE
   <222> (1)
   <223> The residue at this position is similar but not identical to aspartic acid and/or lysine
<220>
   <221> SITE
   <222> (6)
   <223> The residue at this position is similar but not identical to histidine and/or arginine
<220>
   <223> Description of Artificial Sequence: Homology
<220>
   <221> UNSURE
   <222> (9)
   <223> The residue at this position is uncertain, but may be leucine.
<400> 39
<210> 40
   <211> 348
   <212> DNA
   <213> Thermus thermophilus
<400> 40
   catcttcacc agcacaccca gttctccctc ctggacgggg cggcgaarct ttccracctc 60
   ctcaaktggg tcaaggagac gaccccccga ggaccccgcc ttggccatga ccgaccacgg 120
   caacttcttc ggggccgkgg akttctacaa gaaggccacc gaaatgggca tcaagcccat 180
   cytgggctac gaggcctamg tggcggcgga aagccgcttt gaccgcaagc ggggaaaggg 240
   cytagacggg ggctactttc acytcaccyt cytcgccaag gacttcacgg ggtaccagaa 300
   cctggtgcgc ctggcgagcc gggcttacct ggaggggttt tacgaaaa             348
<210> 41
   <211> 20
   <212> DNA
   <213> Artificial Sequence
<220>
   <223> Description of Artificial Sequence: Primer
<400> 41
   catcttcacc agcacaccca 20
<210> 42
   <211> 17
   <212> DNA
   <213> Artificial Sequence
<220>
   <223> Description of Artificial Sequence: Primer
<400> 42
   gaggggtttt acgaaaa 17
<210> 43
   <211> 14
   <212> PRT
   <213> Synechocystis sp.
<400> 43
   Tyr Ser Leu Leu Asp Gly Ala Ser Gln Leu Pro Ala Leu Ile
    1               5                  10
<210> 44
   <211> 14
   <212> PRT
   <213> Artificial Sequence
<220>
   <221> SITE
   <222> (1)
   <223> The residue at this position is similar but not identical to tyrosine and/or phenyalanine
<220>
   <221> SITE
   <222> (8)
   <223> The residue at this position is similar but not identical to alanine and/or serine
<220>
   <221> SITE
   <222> (9)
   <223> The residue at this position is similar but not identical to leucine and/or isoleucine
<220>
   <221> SITE
   <222> (14)
   <223> The residue at this position is similar but not identical to isoleucine and/or lysine
<220>
   <223> Description of Artificial Sequence: Homology
<400> 44
<210> 45
   <211> 18
   <212> PRT
   <213> Thermus thermophilus
<400> 45
<210> 46
   <211> 18
   <212> PRT
   <213> Artificial Sequence
<220>
   <221> SITE
   <222> (1)
   <223> The residue at this position is similar but not identical to phenyalanine and/or tyrosine
<220>
   <221> SITE
   <222> (3)
   <223> The residue at this position is similar but not identical to leucine and/or methionine
<220>
   <221> SITE
   <222> (10)
   <223> The residue at this position is similar but not identical to leucine and/or isoleucine
<220>
   <221> SITE
   <222> (11)
   <223> The residue at this position is similar but not identical to proline, serine and/or threonine
<220>
   <221> SITE
   <222> (13)
   <223> The residue at this position is similar but not identical to leucine and/or methionine
<220>
   <221> SITE
   <222> (18)
   <223> The residue at this position is similar but not identical to lysine and/or glutamic acid
<220>
   <223> Description of Artificial Sequence: Homology
<400> 46
<210> 47
   <211> 18
   <212> PRT
   <213> Mycobacterium tuberculosis
<400> 47
   Tyr Ser Met Leu Asp Gly Ala Ala Lys Ile Thr Pro Met Leu Ala Glu
    1               5                  10                  15
   Val Glu
<210> 48
   <211> 36
   <212> PRT
   <213> Synechocystis sp.
<400> 48
   Pro Ala Ile Ala Leu Thr Asp His Gly Val Met Tyr Gly Ala Val Glu
    1               5                  10                  15
   Leu Leu Lys Val Cys Arg Gly Lys Pro Ile Lys Pro Ile Ile Gly Asn
               20                  25                  30
   Glu Met Tyr Val
           35
<210> 49
   <211> 36
   <212> PRT
   <213> Artificial Sequence
<220>
   <221> SITE
   <222> (3)
   <223> The residue at this position is similar but not identical to isoleucine, leucine and/or valine
<220>
   <221> SITE
   <222> (5)
   <223> The residue at this position is similar but not identical to leucine and/or methionine
<220>
   <221> SITE
   <222> (12)
   <223> The residue at this position is similar but not identical to tyrosine and/or phenyalanine
<220>
   <221> SITE
   <222> (30)
   <223> The residue at this position is similar but not identical to isoleucine and/or leucine
<220>
   <223> Description of Artificial Sequence: Homology
<220>
   <221> SITE
   <222> (10)..(11)
   <223> The residues at these positions can be any amino acid.
<220>
   <221> SITE
   <222> (15)..(18)
   <223> The residues at these positions can be any amino acid.
<220>
   <221> SITE
   <222> (20)..(25)
   <223> The residues at these positions can be any amino acid.
<220>
   <221> SITE
   <222> (32)
   <223> The residue at this position can be any amino acid.
<220>
   <221> SITE
   <222> (34)..(35)
   <223> The residues at these positions can be any amino acid.
<400> 49

<210> 50
   <211> 46
   <212> PRT
   <213> Thermus thermophilus
<220>
   <221> SITE
   <222> (15)..(16)
   <223> The residues at these positions can be any amino acid.
<220>
   <221> SITE
   <222> (35)
   <223> The residue at this position can be any amino acid.
<400> 50
<210> 51
   <211> 46
   <212> PRT
   <213> Artificial Sequence
<220>
   <221> SITE
   <222> (3)
   <223> The residue at this position is similar but not identical to isoleucine, leucine and/or valine
<220>
   <221> SITE
   <222> (23)
   <223> The residue at this position is similar but not identical to glycine, glutamic acid and/or lysine
<220>
   <221> SITE
   <222> (30)
   <223> The residue at this position is similar but not identical to isoleucine and/or leucine
<220>
   <221> SITE
   <222> (36)
   <223> The residue at this position is similar but not identical to isoleucine and/or valine
<220>
   <221> SITE
   <222> (45)
   <223> The residue at this position is similar but not identical to arginine and/or lysine
<220>
   <223> Description of Artificial Sequence: Homology
<220>
   <221> SITE
   <222> (4)
   <223> The residue at this position can be any amino acid.
<220>
   <221> SITE
   <222> (11)
   <223> The residue at this position can be any amino acid.
<220>
   <221> SITE
   <222> (15)..(16)
   <223> The residues at these positions can be any amino acid.
<220>
   <221> SITE
   <222> (19)..(20)
   <223> The residues at these positions can be any amino acid.
<220>
   <221> SITE
   <222> (24)
   <223> The residue at this position can be any amino acid.
<220>
   <221> SITE
   <222> (32)
   <223> The residue at this position can be any amino acid.
<220>
   <221> SITE
   <222> (35)
   <223> The residue at this position can be any amino acid.
<220>
   <221> SITE
   <222> (38)..(39)
   <223> The residues at these positions can be any amino acid.
<220>
   <221> SITE
   <222> (44)
   <223> The residue at this position can be any amino acid.
<400> 51
<210> 52
   <211> 46
   <212> PRT
   <213> Mycobacterium tuberculosis
<400> 52
   Pro Ala Val Gly Met Thr Asp His Gly Asn Met Phe Gly Ala Ser Glu
    1               5                  10                  15
   Phe Tyr Asn Ser Ala Thr Lys Ala Gly Ile Lys Pro Ile Ile Gly Val
               20                  25                  30
   Glu Ala Tyr Ile Ala Pro Gly Ser Arg Phe Asp Thr Arg Arg
           35                  40                  45
<210> 53
   <211> 21
   <212> PRT
   <213> Synechocystis sp.
<400> 53
   Phe His Gln Val Val Leu Ala Lys Asn Asn Gln Gly Tyr Arg Asn Leu
    1               5                  10                  15
   Val Lys Leu Thr Thr
               20
<210> 54
   <211> 21
   <212> PRT
   <213> Artificial Sequence
<220>
   <221> SITE
   <222> (9)
   <223> The residue at this position is similar but not identical to asparagine and/or aspartic acid
<220>
   <221> SITE
   <222> (14)
   <223> The residue at this position is similar but not identical to arginine and/or glutamine
<220>
   <221> SITE
   <222> (18)
   <223> The residue at this position is similar but not identical to lysine and/or arginine
<220>
   <221> SITE
   <222> (21)
   <223> The residue at this position is similar but not identical to threonine and/or serine
<220>
   <223> Description of Artificial Sequence: Homology
<220>
   <221> SITE
   <222> (3)..(6)
   <223> The residues at these positions can be any amino acid.
<220>
   <221> SITE
   <222> (10)..(11)
   <223> The residues at these positions can be any amino acid.
<220>
   <221> SITE
   <222> (20)
   <223> The residue at this position can be any amino acid.
<400> 54
<210> 55
   <211> 26
   <212> PRT
   <213> Thermus thermophilus
<220>
   <221> SITE
   <222> (6)
   <223> The residue at this position can be any amino acid.
<220>
   <221> SITE
   <222> (8)..(9)
   <223> The residues at these positions can be any amino acid.
<400> 55
<210> 56
   <211> 26
   <212> PRT
   <213> Artificial Sequence
<220>
   <221> SITE
   <222> (11)
   <223> The residue at this position is similar but not identical to lysine and/or glutamic acid
<220>
   <221> SITE
   <222> (12)
   <223> The residue at this position is similar but not identical to asparagine and/or aspartic acid
<220>
   <221> SITE
   <222> (17)
   <223> The residue at this position is similar but not identical to arginine and/or glutamine
<220>
   <221> SITE
   <222> (21)
   <223> The residue at this position is similar but not identical to lysine and/or arginine
<220>
   <221> SITE
   <222> (23)
   <223> The residue at this position is similar but not identical to serine, alanine and/or threonine
<220>
   <223> Description of Artificial Sequence: Homology
<220>
   <221> SITE
   <222> (2)
   <223> The residue at this position can be any amino acid.
<220>
   <221> SITE
   <222> (4)
   <223> The residue at this position can be any amino acid.
<220>
   <221> SITE
   <222> (6)
   <223> The residue at this position can be any amino acid.
<220>
   <221> SITE
   <222> (8)..(9)
   <223> The residue at this position can be any amino acid.
<220>
   <221> SITE
   <222> (13)
   <223> The residue at this position can be any amino acid.
<220>
   <221> SITE
   <222> (16)
   <223> The residue at this position can be any amino acid.
<220>
   <221> SITE
   <222> (20)
   <223> The residue at this position can be any amino acid.
<220>
   <221> SITE
   <222> (25)
   <223> The residue at this position can be any amino acid.
<400> 56
<210> 57
   <211> 26
   <212> PRT
   <213> Mycobacterium tuberculosis
<400> 57
   Gly Ser Tyr Thr His Leu Thr Met Met Ala Glu Asn Ala Thr Gly Leu
    1               5                  10                  15
   Arg Asn Leu Phe Lys Leu Ser Ser His Ala
               20                  25
<210> 58
   <211> 1109
   <212> DNA
   <213> Thermus thermophilus
<220>
   <221> misc_feature
   <222> (558)
   <223> The residue at this position can be any nucleotide.
<220>
   <221> misc_feature
   <222> (610)
   <223> The residue at this position can be any nucleotide.
<220>
   <221> misc_feature
   <222> (751)
   <223> The residue at this position can be any nucleotide.
<220>
   <221> misc_feature
   <222> (769)
   <223> The residue at this position can be any nucleotide.
<220>
   <221> misc_feature
   <222> (951)
   <223> The residue at this position can be any nucleotide.
<400> 58
   atgggccgca aactccgctt cgcccacctc caccagcaca cccagttctc cctcctggac 60
   ggggcggcga arctttccra cctcctcaad tgggtcaagg agacgacccc ccgaggaccc 120
   cgccttggcc atgaccgacc acggcaactt cttcggggcc gkggakttct acaagaaggc 180
   caccgaaatg ggcatcaagc ccatcytggg ctacgaggcc tamgtggcgg cggaaagccg 240
   ctttgaccgc aagcggggaa agggcytaga cgggggctac tttcacytca ccytcytcgc 300
   caaggacttc acggggtacc agaacctggt gcgcctggcg agccgggctt acctggaggg 360
   gttttacgaa aagccccgga ttgaccggga gatcaycctg cgcgagcacg ccgagggcct 420
   catcgccctc tcggggtgcc tcggggcgga gatcccccag ttcatcctcc aggaccgtct 480
   ggacctggcc gaggcccggc tcaacgagta cctctccaty ttcaaggacc gcttcttcat 540
   tgaratccag aaccacggcc tccccgagca gaaaaaggtc aacgaggtcc tcaaggantt 600
   cgcccgaaan tacggcctgg ggatggtggc caccaacgac ggccattacg tgaggaagga 660
   ggacgcccgg gcccacgagg tcctcctcgc catccagtcc aagagtaccy tggacgaccc 720
   cgggcgctgg cgcttcccct gcgacgagtt ntacgtgaag accccsgang agatgcgggc 780
   catgttcccc cgaggaggag tggggggacg agccctttga caacaccgtg gaagatcgcc 840
   cgcatgtgca acgtggagct gcccatcggg gggacaagat ggtctaccgc atcccccgct 900
   tccccytccc cgcccgtcgg amcgargccc agttacttca tggagctcac ntttaagggg 960
   ctcctccgcc gctacccgga ccggatcacc gagggcttct accgggaggt cttccgcctt 1020
   ttggggaagc ttccccccca cggggacggg gaggccctgg ccgaggcctt ggcccaggtt 1080
   ggagcgggag gcttgggaga agctcattg                                   1109
<210> 59
   <211> 366
   <212> PRT
   <213> Thermus thermophilus
<400> 59
   Arg Lys Leu Arg Phe Ala His Leu His Gln His Thr Gln Phe Ser Leu
   1               5                   10                  15
   Leu Asp Gly Ala Ala Lys Leu Ser Asp Leu Ile Asn Trp Val Lys Glu
               20                  25                  30
   Thr Thr Pro Glu Asp Pro Ala Leu Ala Met Thr Asp His Gly Asn Phe
           35                  40                  45
   Phe Gly Ala Val Asp Phe Tyr Lys Lys Ala Thr Glu Met Gly Ile Lys
       50                  55                  60
   Pro Ile Leu Gly Tyr Glu Ala Tyr Val Ala Ala Glu Ser Arg Phe Asp
   65                  70                  75                  80
   Arg Lys Arg Gly Lys Gly Leu Asp Gly Gly Tyr Phe His Phe Thr Leu
                   85                  90                  95
   Leu Ala Lys Asp Phe Thr Gly Tyr Gln Asn Leu Val Arg Leu Ala Ser
               100                 105                 110
   Arg Ala Tyr Leu Glu Gly Phe Tyr Glu Lys Pro Arg Ile Asp Arg Glu
           115                 120                 125
   Ile Thr Leu Arg Glu His Ala Glu Gly Leu Ile Ala Leu Ser Gly Cys
       130                 135                 140
   Leu Gly Ala Glu Ile Pro Gln Phe Ile Leu Gln Asp Arg Leu Asp Leu
   145                 150                 155                 160
   Ala Glu Ala Arg Leu Asn Glu Tyr Leu Ser Ile Phe Lys Asp Arg Phe
                   165                 170                 175
   Phe Ile Glu Ile Gln Asn His Gly Leu Pro Glu Gln Lys Lys Val Asn
               180                 185                 190
   Glu Val Leu Lys Asp Phe Ala Arg Lys Tyr Gly Leu Gly Met Val Ala
           195                 200                 205
   Thr Asn Asp Gly His Tyr Val Arg Lys Glu Asp Ala Arg Ala His Glu
       210                 215                 220
   Val Leu Leu Ala Ile Gln Ser Lys Ser Thr Leu Asp Asp Pro Gly Arg
   225                 230                 235                 240
   Trp Arg Phe Pro Cys Asp Glu Phe Tyr Val Lys Thr Pro Asp Glu Met
                   245                 250                 255
   Arg Ala Met Phe Pro Pro Arg Arg Ser Gly Gly Thr Ser Pro Leu Thr
               260                 265                 270
   Thr Pro Trp Lys Ile Ala Arg Met Cys Asn Val Glu Leu Pro Ile Gly
   275 280 285
   Gly Thr Arg Trp Ser Thr Ala Ser Pro Ala Ser Pro Ser Pro Pro Val
   290 295 300
   Gly Pro Lys Pro Ser Tyr Phe Met Glu Leu Thr Phe Lys Gly Leu Leu
   305 310 315 320
   Arg Arg Tyr Pro Asp Arg Ile Thr Glu Gly Phe Tyr Arg Glu Val Phe
   325 330 335
   Arg Leu Leu Gly Lys Leu Pro Pro His Gly Asp Gly Glu Ala Leu Ala
   340 345 350
   Glu Ala Leu Ala Gln Val Gly Ala Gly Gly Leu Gly Glu Ala
   355 360 365
<210> 60
   <211> 219
   <212> PRT
   <213> Mycobacterium tuberculosis
<220>
   <221> SITE
   <222> (40)..(41)
   <223> The residue at this position can be any amino acid.
<220>
   <221> SITE
   <222> (139)..(140)
   <223> The residue at this position can be any amino acid.
<220>
   <221> SITE
   <222> (144)..(145)
   <223> The residue at this position can be any amino acid.
<220>
   <221> SITE
   <222> (151)
   <223> The residue at this position can be any amino acid.
<400> 60
<210> 61
   <211> 219
   <212> PRT
   <213> Thermus thermophilus
<220>
   <221> SITE
   <222> (1)..(5)
   <223> The residue at this position can be any amino acid.
<220>
   <221> SITE
   <222> (86)..(96)
   <223> The residue at this position can be any amino acid.
<220>
   <221> SITE
   <222> (139)..(141)
   <223> The residue at this position can be any amino acid.
<220>
   <221> SITE
   <222> (144)..(145)
   <223> The residue at this position can be any amino acid.
<220>
   <221> SITE
   <222> (178)
   <223> The residue at this position can be any amino acid.
<400> 61
<210> 62
   <211> 219
   <212> PRT
   <213> Synechocystis sp.
<220>
   <221> SITE
   <222> (1)..(7)
   <223> The residue at this position can be any amino acid.
<220>
   <221> SITE
   <222> (40)..(41)
   <223> The residue at this position can be any amino acid.
<220>
   <221> SITE
   <222> (82)..(97)
   <223> The residue at this position can be any amino acid.
<220>
   <221> SITE
   <222> (151)
   <223> The residue at this position can be any amino acid.
<220>
   <221> SITE
   <222> (178)
   <223> The residue at this position can be any amino acid.
<400> 62
<210> 63
   <211> 473
   <212> DNA
   <213> Thermus thermophilus
<220>
   <221> misc_feature
   <222> (411)
   <223> The residue at this position can be any nucleotide.
<220>
   <221> misc_feature
   <222> (453)
   <223> The residue at this position can be any nucleotide.
<400> 63
   cctagttctc cctcctggac ggggcggcga agctttccga cctcctcaag tgggtcaagg 60
   aaacgacccc cgagggaccc cgccttggcc atgaccgacc acggcaacct cttcggggcc 120
   gtggagttct acaagaaggc caccgaaatg ggcatcaagc ccatcctggg ctacgaggcc 180
   tacgtggcgg cggaaagccg ctttgaccgc aagcggggga aagggcctag gacgggggct 240
   actttcacct caccctcctc gccaaggact tcacggggta ccagaacctg gtgcgcctgg 300
   cgagccgggc ttacctggag gggttttacg aaaagccccg gattgaccgg ggagatcctg 360
   cgcgagcacg ccgagggcct catcgccctc tcggggtgcc tcggggcgga natcccccag 420
   ttcatcctcc aggaccgtct ggacctggcc gangcccggc tcaacaagta cct        473
<210> 64
   <211> 21
   <212> DNA
   <213> Artificial Sequence
<220>
   <223> Description of Artificial Sequence: Primer
<400> 64
   cctagttctc cctcctggac g 21
<210> 65
   <211> 242
   <212> DNA
   <213> Thermus thermophilus
<400> 65
   ttgaaggtgt ggaaaagctc ctcctgggtg cgctcctttc cggcgatgaa ctggacgtcg 60
   tccaccagca ggaggtccac ggagcggtac cgctcccgga actccgtcat ccggtcctcg 120
   cggatggcgt tgatgagctc gttggtgaaa gtttccgtgg aaacgtactc aatcttcagg 180
   tgggggaagc gcttggccac ggagtggccc acggcgtgca tcaggtgggt cttccccaat 240
   cc                                                                242
<210> 66
   <211> 20
   <212> DNA
   <213> Artificial Sequence
<220>
   <223> Description of Artificial Sequence: Primer
<400> 66
   ttgaaggtgt ggaaaagctc 20
<210> 67
   <211> 18
   <212> DNA
   <213> Artificial Sequence
<220>
   <223> Description of Artificial Sequence: Primer
<400> 67
   gtgggtcttc cccaatcc 18
<210> 68
   <211> 60
   <212> PRT
   <213> Escherichia coli
<400> 68
   Leu Leu His Ala Val Gly Asn Gly Ile Met Ala Arg Lys Pro Asn Ala
   1               5                   10                  15
   Lys Val Val Tyr Met His Ser Glu Arg Phe Val Gln Asp Met Val Lys
               20                  25                  30
   Ala Leu Gln Asn Asn Ala Ile Glu Glu Phe Lys Arg Tyr Tyr Arg Ser
           35                  40                  45
   Val Asp Ala Leu Leu Ile Asp Asp Ile Gln Phe Phe
       50                  55                  60
<210> 69
   <211> 60
   <212> PRT
   <213> Artificial Sequence
<220>
   <221> SITE
   <222> (2)
   <223> The residue at this position is similar but not identical to leucine and/or methionine
<220>
   <221> SITE
   <222> (7)
   <223> The residue at this position is similar but not identical to asparagine and/or histidine
<220>
   <221> SITE
   <222> (9)
   <223> The residue at this position is similar but not identical to isoleucine and/or valine
<220>
   <221> SITE
   <222> (15)
   <223> The residue at this position is similar but not identical to asparagine, histidine and/or serine

<220>
   <221> SITE
   <222> (18)
   <223> The residue at this position is similar but not identical to valine and/or isoleucine
<220>
   <221> SITE
   <222> (21)
   <223> The residue at this position is similar but not identical to methionine, valine and/or leucine
<220>
   <221> SITE
   <222> (23)
   <223> The residue at this position is similar but not identical to serine and/or threonine
<220>
   <221> SITE
   <222> (29)
   <223> The residue at this position is similar but not identical to aspartic acid and/or glutamic acid
<220>
   <221> SITE
   <222> (30)
   <223> The residue at this position is similar but not identical to methionine, leucine and/or phenyalanine
<220>
   <221> SITE
   <222> (31)
   <223> The residue at this position is similar but not identical to valine and/or isoleucine
<220>
   <221> SITE
   <222> (34)
   <223> The residue at this position is similar but not identical to leucine and/or isoleucine
<220>
   <221> SITE
   <222> (35)
   <223> The residue at this position is similar but not identical to glutamine and/or arginine
<220>
   <221> SITE
   <222> (37)
   <223> The residue at this position is similar but not identical to asparagine and/or aspartic acid
<220>
   <221> SITE
   <222> (39)
   <223> The residue at this position is similar but not identical to isoleucine, methionine and/or alanine
<220>
   <221> SITE
   <222> (43)
   <223> The residue at this position is similar but not identical to lysine and/or arginine
<220>
   <221> SITE
   <222> (54)
   <223>. The residue at this position is similar but not identical to isoleucine and/or valine
<220>
   <221> SITE
   <222> (57)
   <223> The residue at this position is similar but not identical to isoleucine and/or valine
<220>
   <223> Description of Artificial Sequence: Homology
<220>
   <221> SITE
   <222> (8)
   <223> The residue at this position can be any amino acid.
<220>
   <221> SITE
   <222> (10)..(11)
   <223> The residue at this position can be any amino acid.
<220>
   <221> SITE
   <222> (13)
   <223> The residue at this position can be any amino acid.
<220>
   <221> SITE
   <222> (16)
   <223> The residue at this position can be any amino acid.
<220>
   <221> SITE
   <222> (19)
   <223> The residue at this position can be any amino acid.
<220>
   <221> SITE
   <222> (22)
   <223> The residue at this position can be any amino acid.
<220>
   <221> SITE
   <222> (25)
   <223> The residue at this position can be any amino acid.
<220>
   <221> SITE
   <222> (27)..(28)
   <223> The residue at this position can be any amino acid.
<220>
   <221> SITE
   <222> (32)
   <223> The residue at this position can be any amino acid.
<220>
   <221> SITE
   <222> (36)
   <223> The residue at this position can be any amino acid.
<220>
   <221> SITE
   <222> (38)
   <223> The residue at this position can be any amino acid.
<220>
   <221> SITE
   <222> (40)
   <223> The residue at this position can be any amino acid.
<220>
   <221> SITE
   <222> (44)..(45)
   <223> The residue at this position can be any amino acid.
<220>
   <221> SITE
   <222> (51)
   <223> The residue at this position can be any amino acid.
<220>
   <221> SITE
   <222> (60)
   <223> The residue at this position can be any amino acid.
<400> 69
<210> 70
   <211> 60
   <212> PRT
   <213> Thermus thermophilus
<400> 70
   Leu Met His Ala Val Gly His Ser Val Ala Lys Arg Phe Pro His Leu
     1               5                  10                  15
   Lys Ile Glu Tyr Val Ser Thr Glu Thr Phe Thr Asn Glu Leu Ile Asn
                20                  25                  30
   Ala Ile Arg Glu Asp Arg Met Thr Glu Phe Arg Glu Arg Tyr Arg Ser
           35                  40                  45
   Val Asp Leu Leu Leu Val Asp Asp Val Gln Phe Ile
        50                  55                  60
<210> 71
   <211> 60
   <212> PRT
   <213> Artificial Sequence
<220>
   <221> SITE
   <222> (5)
   <223> The residue at this position is similar but not identical to valine and/or isoleucine
<220>
   <221> SITE
   <222> (18)
   <223> The residue at this position is similar but not identical to valine and/or isoleucine
<220>
   <221> SITE
   <222> (21)
   <223> The residue at this position is similar but not identical to methionine, valine and/or leucine
<220>
   <221> SITE
   <222> (23)
   <223> The residue at this position is similar but not identical to serine and/or threonine
<220>
   <221> SITE
   <222> (33)
   <223> The residue at this position is similar but not identical to serine and/or alanine
<220>
   <221> SITE
   <222> (36)
   <223> The residue at this position is similar but not identical to aspartic acid, glutamic acid and/or asparagine
<220>
   <221> SITE
   <222> (37)
   <223> The residue at this position is similar but not identical to aspartic acid and/or asparagine
<220>
   <221> SITE
   <222> (38)
   <223> The residue at this position is similar but not identical to alanine, arginine and/or lysine
<220>
   <221> SITE
   <222> (41)
   <223> The residue at this position is similar but not identical to aspartic acid and/or glutamic acid
<220>
   <221> SITE
   <222> (48)
   <223> The residue at this position is similar but not identical to asparagine and/or serine
<220>
   <221> SITE
   <222> (51)
   <223> The residue at this position is similar but not identical to valine, leucine and/or alanine
<220>
   <221> SITE
   <222> (54)
   <223> The residue at this position is similar but not identical to valine and/or isoleucine
<220>
   <221> SITE
   <222> (57)
   <223> The residue at this position is similar but not identical to valine and/or isoleucine
<220>
   <221> SITE
   <222> (60)
   <223> The residue at this position is similar but not identical to phenyalanine, isoleucine and/or leucine
<220>
   <223> Description of Artificial Sequence: Homology
<220>
   <221> SITE
   <222> (8)
   <223> The residue at this position can be any amino acid.
<220>
   <221> SITE
   <222> (10)..(13)
   <223> The residue at this position can be any amino acid.
<220>
   <221> SITE
   <222> (15)..(16)
   <223> The residue at this position can be any amino acid.
<220>
   <221> SITE
   <222> (19)
   <223> The residue at this position can be any amino acid.
<220>
   <221> SITE
   <222> (25)
   <223> The residue at this position can be any amino acid.
<220>
   <221> SITE
   <222> (30)
   <223> The residue at this position can be any amino acid.
<220>
   <221> SITE
   <222> (39)
   <223> The residue at this position can be any amino acid.
<220>
   <221> SITE
   <222> (40)
   <223> The residue at this position can be any amino acid.
<400> 71
<210> 72
   <211> 60
   <212> PRT
   <213> Bacillus subtilis
<400> 72
   Leu Met His Ala Ile Gly His Tyr Val Ile Asp His Asn Pro Ser Ala
    1               5                  10                  15
   Lys Val Val Tyr Leu Ser Ser Glu Lys Phe Thr Asn Glu Phe Ile Asn
               20                  25                  30
   Ser Ile Arg Asp Asn Lys Ala Val Asp Phe Arg Asn Arg Tyr Arg Asn
           35                  40                  45
   Val Asp Val Leu Leu Ile Asp Asp Ile Gln Phe Leu
       50                  55                  60
<210> 73
   <211> 8
   <212> PRT
   <213> Escherichia coli
<400> 73
   Ala Asn Lys Glu Arg Ser Gln Glu
   1 5
<210> 74
   <211> 8
   <212> PRT
   <213> Artificial Sequence
<220>
   <223> Description of Artificial Sequence: Homology
<220>
   <221> SITE
   <222> (6)
   <223> The residue at this position is similar but not identical to Threonine or Serine.
<220>
   <221> SITE
   <222> (2)
   <223> The residue at this position can be any amino acid.
<400> 74
<210> 75
   <211> 8
   <212> PRT
   <213> Thermus thermophilus
<400> 75
   Ala Gly Lys Glu Arg Thr Gln Glu
   1 5
<210> 76
   <211> 8
   <212> PRT
   <213> Bacillus subtilis
<400> 76
   Ala Gly Lys Glu Gln Thr Gln Glu
   1 5
<210> 77
   <211> 263
   <212> DNA
   <213> Thermus thermophilus
<220>
   <221> misc_feature
   <222> (92)
   <223> The residue at this position can be any nucleotide.
<220>
   <221> misc_feature
   <222> (135)
   <223> The residue at this position can be any nucleotide.
<400> 77
   tcgaaggccg cgttgtgggc caccagcacc gtgtcctgga cgaaggcgcg gaaggcgggg 60
   aggaccgcct ctaggggagg cttgtcccgg ancatctccg ccgtgaggcc gtggaccgcc 120
   gtggccgcgg ggganatggg gcgccccggg ttcaccaggg cctcgaacac ctcctgccgc 180
   aagacccttc gacccaggat atggaccccg gcgagggcca ccacggcatc ttgctccggg 240
   tccaggcccg tggtctcagt gtc                                         263
<210> 78
   <211> 17
   <212> DNA
   <213> Artificial Sequence
<220>
   <223> Description of Artificial Sequence: Primer Q12F
<400> 78
   tcgaaggccg cgttgtg 17
<210> 79
   <211> 17
   <212> DNA
   <213> Artificial Sequence
<220>
   <223> Description of Artificial Sequence: Primer Q98R
<400> 79
   cccgtggtct cagtgtc 17
<210> 80
   <211> 49
   <212> PRT
   <213> Escherichia coli
<400> 80
   Phe His Val Tyr Leu Lys Pro Asp Arg Leu Val Asp Pro Glu Ala Phe
   1               5                   10                  15
   Gly Val His Gly Ile Ala Asp Glu Phe Leu Leu Asp Lys Pro Thr Phe
               20                  25                  30
   Ala Glu Val Ala Asp Glu Phe Met Asp Tyr Ile Arg Gly Ala Glu Leu
           35                  40                  45
   Val
<210> 81
   <211> 49
   <212> PRT
   <213> Artificial Sequence
<220>
   <221> SITE
   <222> (5)
   <223> The residue at this position is similar but not identical to leucine, valine and/or alanine
<220>
   <221> SITE
   <222> (21)
   <223> The residue at this position is similar but not identical to isoleucine and/or leucine
<220>
   <221> SITE
   <222> (42)
   <223> The residue at this position is similar but not identical to tyrosine, phenyalanine and/or tyrptophan
<220>
   <221> SITE
   <222> (43)
   <223> The residue at this position is similar but not identical to isoleucine and/or valine
<220>
   <221> SITE
   <222> (44)
   <223> The residue at this position is similar but not identical to arginine, glutamine and/or glycine
<220>
   <223> Description of Artificial Sequence: Homology
<220>
   <221> SITE
   <222> (2)..(4)
   <223> The residue at this position can be any amino acid.
<220>
   <221> SITE
   <222> (6)
   <223> The residue at this position can be any amino acid.
<220>
   <221> SITE
   <222> (8)
   <223> The residue at this position can be any amino acid.
<220>
   <221> SITE
   <222> (10)..(12)
   <223> The residue at this position can be any amino acid.
<220>
   <221> SITE
   <222> (14)
   <223> The residue at this position can be any amino acid.
<220>
   <221> SITE
   <222> (16).. (17)
   <223> The residue at this position can be any amino acid.
<220>
   <221> SITE
   <222> (22).. (23)
   <223> The residue at this position can be any amino acid.
<220>
   <221> SITE
   <222> (25)..(27)
   <223> The residue at this position can be any amino acid.
<220>
   <221> SITE
   <222> (31) .. (34)
   <223> The residue at this position can be any amino acid.
<220>
   <221> SITE
   <222> (36)..(38)
   <223> The residue at this position can be any amino acid.
<220>
   <221> SITE
   <222> (90) .. (41)
   <223> The residue at this position can be any amino acid.
<220>
   <221> SITE
   <222> (45)..(47)
   <223> The residue at this position can be any amino acid.
<400> 81
<210> 82
   <211> 50
   <212> PRT
   <213> Thermus thermophilus
<220>
   <221> SITE
   <222> (11)
   <223> The residue at this position can be any amino acid.
<220>
   <221> SITE
   <222> (26)
   <223> The residue at this position can be any amino acid.
<400> 82
<210> 83
   <211> 50
   <212> PRT
   <213> Artificial Sequence
<220>
   <221> SITE
   <222> (18)
   <223> The residue at this position is similar but not identical to valine and/or leucine
<220>
   <221> SITE
   <222> (24)
   <223> The residue at this position is similar but not identical to glutamic acid and/or aspartic acid
<220>
   <221> SITE
   <222> (27)
   <223> The residue at this position is similar but not identical to leucine, arginine and/or glutamine
<220>
   <221> SITE
   <222> (32)
   <223> The residue at this position is similar but not identical to phenyalanine, leucine and/or valine
<220>
   <221> SITE
   <222> (36)
   <223> The residue at this position is similar but not identical to alanine, leucine and/or isoleucine
<220>
   <221> SITE
   <222> (42)
   <223> The residue at this position is similar but not identical to tyrosine, phenyalanine and/or tryptophan
<220>
   <221> SITE
   <222> (43)
   <223> The residue at this position is similar but not identical to isoleucine and/or valine
<220> '
   <221> SITE
   <222> (47)
   <223> The residue at this position is similar but not identical to glutamic acid, valine and/or isoleucine
<220>
   <223> Description of Artificial Sequence: Homology
<220>
   <221> SITE
   <222> (21)
   <223> The residue at this position is similar but not identical to isoleucine and/or leucine.
<220>
   <221> SITE
   <222> (4)..(5)
   <223> The residue at this position can be any amino acid.
<220>
   <221> SITE
   <222> (8)
   <223> The residue at this position can be any amino acid.
<220>
   <221> SITE
   <222> (11)
   <223> The residue at this position can be any amino acid.
<220>
   <221> SITE
   <222> (13)..(17)
   <223> The residue at this position can be any amino acid.
<220>
   <221> SITE
   <222> (19)
   <223> The residue at this position can be any amino acid.
<220>
   <221> SITE
   <222> (23)
   <223> The residue at this position can be any amino acid.
<220>
   <221> SITE
   <222> (26)
   <223> The residue at this position can be any amino acid.
<220>
   <221> SITE
   <222> (29)
   <223> The residue at this position can be any amino acid.
<220>
   <221> SITE
   <222> (31)
   <223> The residue at this position can be any amino acid.
<220>
   <221> SITE
   <222> (33)..(34)
   <223> The residue at this position can be any amino acid.
<220>
   <221> SITE
   <222> (37)..(38)
   <223> The residue at this position can be any amino acid.
<220>
   <221> SITE
   <222> (41)
   <223> The residue at this position can be any amino acid.
<220>
   <221> SITE
   <222> (44)
   <223> The residue at this position can be any amino acid.
<220>
   <221> SITE
   <222> (46)
   <223> The residue at this position can be any amino acid.
<400> 83
<210> 84
   <211> 50
   <212> PRT
   <213> Bacillus subtilis
<400> 84
   Phe Glu Ala Phe Ala Asn Pro His Arg Pro Leu Ser Ala Thr Ile Ile
     1               5                  10                  15
   Glu Leu Thr Gly Ile Thr Asp Asp Met Leu Gln Asp Ala Pro Asp Val
               20                  25                  30
   Val Asp Val Ile Arg Asp Phe Arg Glu Trp Ile Gly Asp Asp Ile Leu
           35                  40                  45
   Val Ala
       50
<210> 85
   <211> 20
   <212> DNA
   <213> Artificial Sequence
<220>
   <223> Description of Artificial Sequence: Primer X139R
<400> 85
   tgacccacaa cctcttgaaa 20
<210> 86
   <211> 20
   <212> DNA
   <213> Artificial Sequence
<220>
   <223> Description of Artificial Sequence: Primer E1F
<220>
   <221> misc_feature
   <222> (6)
   <223> The residue at this position can be any nucleotide.
<220>
   <221> misc_feature
   <222> (18)
   <223> The residue at this position can be any nucleotide.
<400> 86
   cayytncayc arcayacnca 20
<210> 87
   <211> 17
   <212> DNA
   <213> Artificial Sequence
<220>
   <223> Description of Artificial Sequence: Primer E91F
<220>
   <221> misc_feature
   <222> (6)
   <223> The residue at this position can be any nucleotide.
<400> 87
   garggnttyt aygaraa 17
<210> 88
   <211> 23
   <212> PRT
   <213> Thermus thermophilus
<400> 88
   Ala Arg Leu Leu Pro Leu Ala Gln Ala His Phe Gly Val Glu Glu Val
    1               5                  10                  15
   Val Leu Val Leu Glu Gly Glu
               20
<210> 89
   <211> 7
   <212> PRT
   <213> Thermus thermophilus
<400> 89
   Phe Gln Glu Val Val Gly Gln
    1               5
<210> 90
   <211> 6
   <212> PRT
   <213> Artificial Sequence
<220>
   <223> Description of Artificial Sequence: Primer
<400> 90
   Lys Thr Leu Glu Glu Pro
    1               5

<210> 91
   <211> 20
   <212> DNA
   <213> Artificial Sequence
<220>
   <223> Description of Artificial Sequence: Primer X1Fb
<220>
   <221> misc_feature
   <222> (12)
   <223> The residue at this position can be any nucleotide.
<220>
   <221> misc_feature
   <222> (15)
   <223> The residue at this position can be any nucleotide.
<400> 91
   ttycargarg tngtnggsca 20
<210> 92
   <211> 16
   <212> PRT
   <213> Escherichia coli
<400> 92
   Lys Val Tyr Leu Ile Asp Glu Val His Met Leu Ser Arg His Ser Phe
   1 5 10 15
<210> 93
   <211> 16
   <212> PRT
   <213> Bacillus subtilis
<400> 93
   Lys Val Tyr Ile Ile Asp Glu Val His Met Leu Ser Ile Gly Ala Phe
   1 5 10 15
<210> 94
   <211> 16
   <212> PRT
   <213> Haemophilus influenzae
<400> 94
   Lys Val Tyr Leu Ile Asp Glu Val His Met Leu Ser Arg His Ser Phe
   1 5 10 15
<210> 95
   <211> 16
   <212> PRT
   <213> Homo sapiens
<400> 95
   Lys Ile Ile Ile Leu Asp Glu Ala Asp Ser Met Thr Asp Gly Ala Gln
   1 5 10 15
<210> 96
   <211> 6
   <212> PRT
   <213> Artificial Sequence
<220>
   <221> SITE
   <222> (3)
   <223> The residue at this position could be either Ala or Val
<220>
   <223> Description of Artificial Sequence: Consensus
<400> 96
   Asp Glu Ala His Met Leu
   1 5
<210> 97
   <211> 17
   <212> DNA
   <213> Artificial Sequence
<220>
   <223> Description of Artificial Sequence: Primer X126R
<220>
   <221> misc_feature
   <222> (9)
   <223> The residue at this position can be any nucleotide.
<400> 97
   arcatrtgnr cytcrtc 17
<210> 98
   <211> 16
   <212> PRT
   <213> Escherichia coli
<400> 98
   Ser Phe Asn Ala Leu Leu Lys Thr Leu Glu Glu Pro Pro Glu His Val
   1 5 10 15
<210> 99
   <211> 16
   <212> PRT
   <213> Bacillus subtilis
<400> 99
   Ala Phe Asn Ala Leu Leu Lys Thr Leu Glu Glu Pro Pro Glu His Cys
   1 5 10 15
<210> 100
   <211> 16
   <212> PRT
   <213> Haemophilus influenzae
<400> 100
   Ala Phe Asn Ala Leu Leu Lys Thr Leu Glu Glu Pro Pro Glu Tyr Val
   1 5 10 15
<210> 101
   <211> 6
   <212> PRT
   <213> Artificial Sequence
<220>
   <223> Description of Artificial Sequence: Consensus
<400> 101
   Lys Thr Leu Glu Glu Pro
   1 5
<210> 102
   <211> 19
   <212> PRT
   <213> Artificial Sequence
<220>
   <223> Description of Artificial Sequence: Homology
<400> 102
   Ser Ala Leu Tyr Arg Arg Phe Arg Pro Leu Thr Phe Gln Glu Val Val
   1 5 10 15
   Gly Gln Glu
<210> 103
   <211> 56
   <212> DNA
   <213> Artificial Sequence
<220>
   <223> Description of Artificial Sequence: Polylinker
<400> 103
   ggccgcaatt gcacgcgttc gaattccatg acgtcttcca gtgcactggt taatta 56
<210> 104
   <211> 58
   <212> DNA
   <213> Artificial Sequence
<220>
   <223> Description of Artificial Sequence: Polylinker
<400> 104
   ctagttaatt aaccagtgca ctggaagacg tcatggaatt cgaacgcgtg caattgcc 58
<210> 105
   <211> 7
   <212> PRT
   <213> Thermus thermophilus
<400> 105
   Gly Val Gly Lys Thr Thr Thr
   1 5
<210> 106
   <211> 6
   <212> PRT
   <213> Thermus thermophilus
<400> 106
   Glu Ile Asp Ala Ala Ser
   1 5
<210> 107
   <211> 11
   <212> PRT
   <213> Thermus thermophilus
<400> 107
   Phe Asn Ala Leu Leu Lys Thr Leu Glu Glu Pro
   1 5 10
<210> 108
   <211> 23
   <212> PRT
   <213> Thermus thermophilus
<400> 108
   Ala Arg Leu Leu Pro Leu Ala Gln Ala His Phe Gly Val Glu Glu Val
   1 5 10 15
   Val Leu Val Leu Glu Gly Glu
   20
<210> 109
   <211> 13
   <212> PRT
   <213> Thermus thermophilus
<400> 109
   Arg Lys Leu Arg Phe Ala His Leu His Gln His Thr Gln
   1 5 10
<210> 110
   <211> 20
   <212> DNA
   <213> Artificial Sequence
<220>
   <223> Description of Artificial Sequence: Primer 1F
<220>
   <221> misc_feature
   <222> (6)
   <223> The residue at this position can be any nucleotide.
<220>
   <221> misc_feature
   <222> (18)
   <223> The residue at this position can be any nucleotide.
<400> 110
   cayytncayc arcayacnca 20
<210> 111
   <211> 17
   <212> DNA
   <213> Artificial Sequence
<220>
   <223> Description of Artificial Sequence: Primer 91F
<220>
   <221> misc_feature
   <222> (6)
   <223> The residue at this position can be any nucleotide.
<400> 111
   garggnttyt aygaraa 17
<210> 112
   <211> 17
   <212> DNA
   <213> Artificial Sequence
<220>
   <223> Description of Artificial Sequence: Primer 91R
<220>
   <221> misc_feature
   <222> (12)
   <223> The residue at this position can be any nucleotide.
<400> 112
   ttytcrtara anccytc 17
<210> 113
   <211> 20
   <212> DNA
   <213> Artificial Sequence
<220>
   <223> Description of Artificial Sequence: Primer 676F
<400> 113
   taycargarc arcaratgca 20
<210> 114
   <211> 17
   <212> DNA
   <213> Artificial Sequence
<220>
   <223> Description of Artificial Sequence: Primer 676R
<400> 114
   tgcatytgyt cytgrta 17
<210> 115
   <211> 17
   <212> DNA
   <213> Artificial Sequence
<220>
   <223> Description of Artificial Sequence: Primer 853R
<220>
   <221> misc_feature
   <222> (9)
   <223> The residue at this position can be any nucleotide.
<220>
   <221> misc_feature
   <222> (12)
   <223> The residue at this position can be any nucleotide.
<400> 115
   tgraartanc cnccrtc 17
<210> 116
   <211> 35
   <212> DNA
   <213> Artificial Sequence
<220>
   <223> Description of Artificial Sequence: Biotin Primer
<400> 116
   ccgcgcttaa ttaacccagt tctccctcct ggacg 35
<210> 117
   <211> 4
   <212> PRT
   <213> Artificial Sequence
<220>
   <223> Description of Artificial Sequence: Synthetic
<220>
   <221> SITE
   <222> ()
   <223> The residue at this position can be any amino acid.
<400> 117
<210> 118
   <211> 6
   <212> PRT
   <213> Artificial Sequence
<220>
   <223> Description of Artificial Sequence: Consensus
<400> 118
   Gly Leu Gly Lys Thr His
   1 5
<210> 119
   <211> 20
   <212> PRT
   <213> Escherichia coli
<400> 119
   Pro Leu Phe Leu Tyr Gly Gly Thr Gly Leu Gly Lys Thr His Leu Leu
   1 5 10 15
   His Ala Val Gly
   20
<210> 120
   <211> 20
   <212> PRT
   <213> Thermotoga maritima
<400> 120
   Pro Leu Phe Ile Tyr Gly Gly Val Gly Leu Gly Lys Thr His Leu Leu
   1 5 10 15
   Gln Ser Ile Gly
   20
<210> 121
   <211> 20
   <212> PRT
   <213> Borrelia burgdorferi
<400> 121
   Pro Cys Leu Ile Tyr Gly Gly Val Gly Leu Gly Lys Thr His Leu Leu
   1 5 10 15
   Gln Ser Ile Gly
   20
<210> 122
   <211> 20
   <212> PRT
   <213> Bacillus subtilis
<400> 122
   Pro Leu Phe Ile Tyr Gly Gly Val Gly Leu Gly Lys Thr His Leu Met
   1 5 10 15
   His Ala Ile Gly
<210> 123
   <211> 20
   <212> PRT
   <213> Serratia marcescens
<400> 123
   Pro Leu Phe Leu Tyr Gly Gly Thr Gly Leu Gly Lys Thr His Leu Leu
   1 5 10 15
   His Ala Val Gly
   20
<210> 124
   <211> 20
   <212> PRT
   <213> Salmonella typhimurium
<400> 124
   Pro Leu Phe Leu Tyr Gly Gly Thr Gly Leu Gly Lys Thr His Leu Leu
   1 5 10 15
   His Ala Val Gly
   20
<210> 125
   <211> 20
   <212> PRT
   <213> Proteus mirabilis
<400> 125
   Pro Leu Phe Leu Tyr Gly Gly Thr Gly Leu Gly Lys Thr His Leu Leu
   1 5 10 15
   His Ala Val Gly
   20
<210> 126
   <211> 20
   <212> PRT
   <213> Buchnera aphidicola
<400> 126
   Pro Leu Phe Leu Tyr Gly Gly Val Gly Leu Gly Lys Thr His Leu Met
   1 5 10 15
   His Ala Val Gly
   20
<210> 127
   <211> 20
   <212> PRT
   <213> Buchnera aphidicola
<400> 127
   Pro Leu Phe Leu Tyr Gly Gly Thr Gly Leu Gly Lys Thr His Leu Leu
   1 5 10 15
   His Ala Ile Gly
<210> 128
   <211> 20
   <212> PRT
   <213> Micrococcus luteus
<400> 128
   Pro Leu Phe Ile Tyr Gly Glu Ser Gly Leu Gly Lys Thr His Leu Leu
   1 5 10 15
   His Ala Ile Gly
   20
<210> 129
   <211> 20
   <212> PRT
   <213> Streptomyces coelicolor
<400> 129
   Pro Leu Phe Ile Tyr Gly Glu Ser Gly Leu Gly Lys Thr His Leu Leu
   1 5 10 15
   His Ala Ile Gly
   20
<210> 130
   <211> 20
   <212> PRT
   <213> Haemophilus influenzae
<400> 130
   Pro Phe Phe Leu Tyr Gly Gly Thr Gly Leu Gly Lys Thr His Leu Leu
   1 5 10 15
   His Ala Ile Gly
   20
<210> 131
   <211> 19
   <212> PRT
   <213> Rhizobium meliloti
<400> 131
   Pro Leu Phe Ile His Ser Ser Val Gly Leu Gly Lys Thr His Leu Leu
   1 5 10 15
   Gln Ala Ile
<210> 132
   <211> 19
   <212> PRT
   <213> Spiroplasma citri
<400> 132
   Pro Leu Phe Ile Tyr Gly Asp Ser Gly Leu Gly Lys Thr His Leu Leu
   1 5 10 15
   His Ala Ile
<210> 133
   <211> 6
   <212> PRT
   <213> Artificial Sequence
<220>
   <223> Description of Artificial Sequence: Primer
<400> 133
   Gly Leu Gly Lys Thr His
   1 5
<210> 134
   <211> 18
   <212> DNA
   <213> Artificial Sequence
<220>
   <223> Description of Artificial Sequence: Primer A177Fa
<220>
   <221> misc_feature
   <222> (3)
   <223> The residue at this position can be any nucleotide.
<220>
   <221> misc_feature
   <222> (6)
   <223> The residue at this position can be any nucleotide.
<220>
   <221> misc_feature
   <222> (9)
   <223> The residue at this position can be any nucleotide.
<400> 134
   ggnytnggna aracscat 18
<210> 135
   <211> 18
   <212> DNA
   <213> Artificial Sequence
<220>
   <223> Description of Artificial Sequence: Primer A177Fb
<220>
   <221> misc_feature
   <222> (3)
   <223> The residue at this position can be any nucleotide.
<220>
   <221> misc_feature
   <222> (6)
   <223> The residue at this position can be any nucleotide.
<220>
   <221> misc_feature
   <222> (9)
   <223> The residue at this position can be any nucleotide.
<400> 135
   ggnytnggna aracscac 18
<210> 136
   <211> 18
   <212> DNA
   <213> Artificial Sequence
<220>
   <223> Description of Artificial Sequence: Primer A177Fc
<220>
   <221> misc_feature
   <222> (3)
   <223> The residue at this position can be any nucleotide.
<220>
   <221> misc_feature
   <222> (6)
   <223> The residue at this position can be any nucleotide.
<220>
   <221> misc_feature
   <222> (9)
   <223> The residue at this position can be any nucleotide.
<400> 136
   ggnytnggna aracwcat 18
<210> 137
   <211> 18
   <212> DNA
   <213> Artificial Sequence
<220>
   <223> Description of Artificial Sequence: Primer A117Fc
<220>
   <221> misc_feature
   <222> (3)
   <223> The residue at this position can be any nucleotide.
<220>
   <221> misc_feature
   <222> (6)
   <223> The residue at this position can be any nucleotide.
<220>
   <221> misc_feature
   <222> (9)
   <223> The residue at this position can be any nucleotide.
<400> 137
   ggnytnggna aracwcac 18
<210> 138
   <211> 7
   <212> PRT
   <213> Artificial Sequence
<220>
   <221> SITE
   <222> (2)
   <223> The residue at this position can be either Leu or Phe
<220>
   <223> Description of Artificial Sequence: Consensus
<400> 138
   Glu Leu Phe His Thr Phe Asn
   1 5
<210> 139
   <211> 20
   <212> PRT
   <213> Escherichia coli
<400> 139
   Gln Glu Glu Phe Phe His Thr Phe Asn Ala Leu Leu Glu Gly Asn Gln
   1 5 10 15
   Gln Ile Ile Leu
   20
<210> 140
   <211> 23
   <212> PRT
   <213> Thermotoga maritima
<400> 140
   Thr Gly Val Gln Thr Glu Leu Phe His Thr Phe Asn Glu Leu His Asp
   1 5 10 15
   Ser Gly Lys Gln Ile Val Ile
   20
<210> 141
   <211> 22
   <212> PRT
   <213> Mycobacterium leprae
<400> 141
   Gly Ile Gln Glu Glu Phe Phe His Thr Phe Asn Thr Leu His Asn Ala
   1 5 10 15
   Asn Lys Gln Ile Val Ile
   20

<210> 142
   <211> 20
   <212> PRT
   <213> Bacillus subtilis
<400> 142
   Gln Glu Glu Phe Phe His Thr Phe Asn Thr Leu His Glu Glu Ser Lys
   1 5 10 15
   Gln Ile Val Ile
   20
<210> 143
   <211> 21
   <212> PRT
   <213> Borrelia burgdorferi
<400> 143
   Gly Ile Gln Glu Glu Leu Phe His Thr Phe Asn Ala Leu Tyr Glu Asp
   1 5 10 15
   Asn Lys Gln Leu Val
   20
<210> 144
   <211> 20
   <212> PRT
   <213> Streptomyces coelicolor
<400> 144
   Gln Glu Glu Phe Phe His Thr Phe Asn Thr Leu His Asn Ala Asn Lys
   1 5 10 15
   Gln Ile Val Leu
   20
<210> 145
   <211> 18
   <212> PRT
   <213> Micrococcus luteus
<400> 145
   Glu Phe Phe His Thr Phe Asn Thr Leu Tyr Asn Asn Asn Lys Gln Val
   1 5 10 15
   Val Ile
<210> 146
   <211> 20
   <212> PRT
   <213> Haemophilus influenzae
<400> 146
   Gln Glu Glu Phe Phe His Ile Phe Asn Ser Leu Phe Glu Thr Gly Arg
   1 5 10 15
   Gln Ile Ile Leu
   20
<210> 147
   <211> 20
   <212> PRT
   <213> Pseudomonas putida
<400> 147
   Gln Glu Glu Phe Phe His Thr Phe Asn Ala Leu Leu Glu Gly Gly Gln
   1 5 10 15
   Gln Val Ile Leu
   20
<210> 148
   <211> 20
   <212> PRT
   <213> Buchnera aphidicola
<400> 148
   Gln Glu Glu Phe Phe His Thr Phe Asn Ala Leu Leu Glu Gly Asn Gln
   1 5 10 15
   Gln Ile Ile Leu
   20
<210> 149
   <211> 20
   <212> PRT
   <213> Serratia marcescens
<400> 149
   Gln Glu Glu Phe Phe His Thr Phe Asn Ala Leu Leu Glu Gly Asn Gln
   1 5 10 15
   Gln Ile Ile Leu
   20
<210> 150
   <211> 20
   <212> PRT
   <213> Salmonella typhimurium
<400> 150
   Gln Glu Glu Phe Phe His Thr Phe Asn Ala Leu Leu Glu Gly Asn Gln
   1 5 10 15
   Gln Ile Ile Leu
   20
<210> 151
   <211> 20
   <212> PRT
   <213> Proteus mirabilis
<400> 151
   Gln Glu Glu Phe Phe His Thr Phe Asn Ala Leu Leu Glu Gly Asn Gln
   1 5 10 15
   Gln Ile Ile Leu
   20
<210> 152
   <211> 18
   <212> PRT
   <213> Wolbachia sp.
<400> 152
   Glu Phe Phe Lys Thr Phe Asn Ala Leu Ile Asp Gln Asn Lys Gln Leu
   1 5 10 15
   Val Ile
<210> 153
   <211> 21
   <212> PRT
   <213> Rhizobium Meliloti
<400> 153
   Ile Gln His Glu Phe Cys His Leu Leu Asn Met Leu Leu Asp Ser Ala
   1 5 10 15
   Lys Gln Val Val Val
   20
<210> 154
   <211> 16
   <212> PRT
   <213> Spiroplasma citri
<400> 154
   Phe His Ile Phe Asn Ser Tyr Ile Glu Lys Asn Lys Gln Ile Val Ile
   1 5 10 15
<210> 155
   <211> 7
   <212> PRT
   <213> Artificial Sequence
<220>
   <221> SITE
   <222> (2)
   <223> The residue at this position could be either Leu or Phe
<220>
   <223> Description of Artificial Sequence: Synthetic
<400> 155
   Glu Leu Phe His Thr Phe Asn
   1 5
<210> 156
   <211> 20
   <212> DNA
   <213> Artificial Sequence
<220>
   <223> Description of Artificial Sequence: Synthetic
<220>
   <221> misc_feature
   <222> (6)
   <223> The residue at this position can be any nucleotide.
<220>
   <221> misc_feature
   <222> (15)
   <223> The residue at this position can be any nucleotide.
<400> 156
   ttraangtrt graanaaytc 20
<210> 157
   <211> 20
   <212> DNA
   <213> Artificial Sequence
<220>
   <223> Description of Artificial Sequence: Synthetic
<220>
   <221> misc_feature
   <222> (6)
   <223> The residue at this position can be any nucleotide.
<220>
   <221> misc_feature
   <222> (15)
   <223> The residue at this position can be any nucleotide.
<400> 157
   ttraangtrt graanagytc 20
<210> 158
   <211> 13
   <212> PRT
   <213> Escherichia coli
<400> 158
   Ile Val Leu Asp Thr Glu Thr Thr Gly Met Asn Gln Ile
   1 5 10
<210> 159
   <211> 14
   <212> PRT
   <213> Escherichia coli
<220>
   <221> SIMILAR
   <222> (8)
   <223> The residue at this position can be any amino acid.
<400> 159
<210> 160
   <211> 13
   <212> PRT
   <213> Haemophilus haemolyticus
<400> 160
   Ile Val Leu Asp Thr Glu Thr Thr Gly Met Asn Gln Ile
   1 5 10
<210> 161
   <211> 14
   <212> PRT
   <213> Haemophilus haemolyticus
<220>
   <221> SITE
   <222> (8)
   <223> The residue at this position can be any amino acid.
<400> 161
<210> 162
   <211> 13
   <212> PRT
   <213> Buchnera aphidicola
<400> 162
   Ile Val Leu Asp Thr Glu Thr Thr Gly Met Asn Ser Val
   1 5 10
<210> 163
   <211> 14
   <212> PRT
   <213> Buchnera aphidicola
<400> 163
<210> 164
   <211> 13
   <212> PRT
   <213> Bacillus subtilis
<400> 164
   Val Val Phe Asp Val Glu Thr Thr Gly Leu Ser Ala Val
   1 5 10
<210> 165
   <211> 12
   <212> PRT
   <213> Bacillus subtilis
<220>
   <221> SITE
   <222> (8)
   <223> The residue at this position can be any amino acid.
<400> 165
<210> 166
   <211> 13
   <212> PRT
   <213> Mycoplasma genitalium
<400> 166
   Val Ile Phe Asp Ile Glu Thr Thr Gly Leu His Gly Arg
   1 5 10
<210> 167
   <211> 14
   <212> PRT
   <213> Mycoplasma genitalium
<400> 167
   Met Val Ala His Asn Gly Ile Asn Phe Asp Leu Pro Phe Leu
   1 5 10
<210> 168
   <211> 13
   <212> PRT
   <213> Mycoplasma pulmonis
<400> 168
   Val Val Tyr Asp Ile Glu Thr Thr Gly Leu Ser Pro Met
   1 5 10
<210> 169
   <211> 14
   <212> PRT
   <213> Mycoplasma pulmonis
<220>
   <221> SITE
   <222> (8)
   <223> The residue at this position can be any amino acid.
<400> 169
<210> 170
   <211> 13
   <212> PRT
   <213> Staphylococcus aureus
<400> 170
   Val Val Phe Asp Val Glu Thr Thr Gly Leu Ser Asn Gln
   1 5 10
<210> 171
   <211> 14
   <212> PRT
   <213> Staphylococcus aureus
<220>
   <221> SITE
   <222> (8)
   <223> The residue at this position can be any amino acid.
<400> 171
<210> 172
   <211> 6
   <212> PRT
   <213> Artificial Sequence
<220>
   <221> SITE
   <222> (2)
   <223> The residue at this position could be either Thr, Ile or Val
<220>
   <223> Description of Artificial Sequence: Synthetic
<400> 172
   Asp Thr Glu Thr Thr Gly
    1               5
<210> 173
   <211> 17
   <212> DNA
   <213> Artificial Sequence
<220>
   <223> Description of Artificial Sequence: Synthetic
<220>
   <221> misc_feature
   <222> (6)
   <223> The residue at this position can be any nucleotide.
<220>
   <221> misc_feature
   <222> (12)
   <223> The residue at this position can be any nucleotide.
<220>
   <221> misc_feature
   <222> (15)
   <223> The residue at this position can be any nucleotide.
<400> 173
   gayacngara cnacngg 17
<210> 174
   <211> 17
   <212> DNA
   <213> Artificial Sequence
<220>
   <223> Description of Artificial Sequence: Synthetic
<220>
   <221> misc_feature
   <222> (6)
   <223> The residue at this position can be any nucleotide.
<220>
   <221> misc_feature
   <222> (12)
   <223> The residue at this position can be any nucleotide.
<220>
   <221> misc_feature
   <222> (15)
   <223> The residue at this position can be any nucleotide.
<400> 174
   gayrtngara cnacngg 17
<210> 175
   <211> 6
   <212> PRT
   <213> Artificial Sequence
<220>
   <221> SITE
   <222> (4)
   <223> The residue at this position could be either Ser or Ala
<220>
   <223> Description of Artificial Sequence: Synthetic
<400> 175
   His Asn Ala Ala Phe Asp
    1               5
<210> 176
   <211> 17
   <212> DNA
   <213> Artificial Sequence
<220>
   <223> Description of Artificial Sequence: Synthetic
<220>
   <221> misc_feature
   <222> (6)
   <223> The residue at this position can be any nucleotide.
<220>
   <221> misc_feature
   <222> (9)
   <223> The residue at this position can be any nucleotide.
<400> 176
   tcraangcng crttrtg 17
<210> 177
   <211> 17
   <212> DNA
   <213> Artificial Sequence
<220>
   <223> Description of Artificial Sequence: Synthetic
<220>
   <221> misc_feature
   <222> (6)
   <223> The residue at this position can be any nucleotide.
<220>
   <221> misc_feature
   <222> (9)
   <223> The residue at this position can be any nucleotide.
<400> 177
   tcraanswng crttrtg 17
<210> 178
   <211> 48
   <212> DNA
   <213> Artificial Sequence
<220>
   <223> Description of Artificial Sequence: Synthetic
<220>
   <221> misc_binding
   <222> (5)
   <223> The residue in position 5 is bound to the residue in position 41 in SEQ ID NO:179 such that a duplex is formed between SEQ ID NO:179 and SEQ ID NO:178 wherein the duplex has sticky ends.
<400> 178
   ctaggaggtt ttaatcgatg cggccggatc ctcgagtcta gacactgg 48
<210> 179
   <211> 41
   <212> DNA
   <213> Artificial Sequence
<220>
   <223> Description of Artificial Sequence: Synthetic
<220>
   <221> misc_binding
   <222> (1)
   <223> The residue in position 1 is bound to position 45 in SEQ ID NO:178 such that a duplex is formed between SEQ ID NO:178 and SEQ ID NO:179 wherein the duplex has sticky ends.
<400> 179
   gtgtctagac tcgaggatcc ggccgcatcg attaaaacct c 41
<210> 180
   <211> 444
   <212> DNA
   <213> Thermus thermophilus
<220>
   <221> misc_feature
   <222> (388)
   <223> The residue at this position can be any nucleotide.
<400> 180
   ctgcaccggg atgagcttgg ccaattcctc cgccttcttg tgggggatgc cgtagacccg 60
   ggccacgtcc ttgagggcgg ccttggaggc gaggcttccc agggtgccga tctgggccac 120
   cttgtcctcg ccgtagcgtt cccgcacgta ctggatcacc cggtcccgct cccggtcgga 180
   gaagtccgtg tcaatgtcgg gcatggagac cctctcgggg ttcaggaagc gctcaaagag 240
   gaggccgaag cgcagggggt caatgttggt gatccccacg gcgtaggcca ccaggctccc 300
   ggcggcgctc cccctgccgg gccccacgga gacgccgttt ctccgggccc agttgatgta 360
   ttcctggacg atgaggaagt agccgggnaa accccatgcg ctctatcacg ggaaagctcg 420
   taaagggccc ggtggaaaat ggcc                                        444
<210> 181
   <211> 60
   <212> PRT
   <213> Thermus thermophilus
<220>
   <221> SITE
   <222> (2)
   <223> The residue at this position can be any amino acid.
<400> 181
<210> 182
   <211> 60
   <212> PRT
   <213> Thermus thermophilus
<220>
   <221> SITE
   <222> (12)
   <223> The residue at this position is similar but not identical to tyrosine and/or phenyalanine
<220>
   <221> SITE
   <222> (16)
   <223> The residue at this position is similar but not identical to alanine and/or serine
<220>
   <221> SITE
   <222> (17)
   <223> The residue at this position is similar but not identical to arginine and/or lysine
<220>
   <221> SITE
   <222> (39)
   <223> The residue at this position is similar but not identical to valine and/or leucine
<220>
   <221> SITE
   <222> (43)
   <223> The residue at this position is similar but not identical to asparagine and/or aspartic acid
<220>
   <221> SITE
   <222> (44)
   <223> The residue at this position is similar but not identical to isoleucine and/or leucine
<220>
   <221> SITE
   <222> (2)
   <223> The residue at this position can be any amino acid.
<220>
   <221> SITE
   <222> (10)
   <223> The residue at this position can be any amino acid.
<220>
   <221> SITE
   <222> (14)
   <223> The residue at this position can be any amino acid.
<220>
   <221>. SITE
   <222> (18)
   <223> The residue at this position can be any amino acid.
<220>
   <221> SITE
   <222> (22)
   <223> The residue at this position can be any amino acid.
<220>
   <221> SITE
   <222> (30)
   <223> The residue at this position can be any amino acid.
<220>
   <221> SITE
   <222> (40)
   <223> The residue at this position can be any amino acid.
<220>
   <221> SITE
   <222> (48)
   <223> The residue at this position can be any amino acid.
<220>
   <221> SITE
   <222> (50)
   <223> The residue at this position can be any amino acid.
<400> 182
<210> 183
   <211> 60
   <212> PRT
   <213> Escherichia coli
<220>
   <223> Description of Artificial Sequence: Synthetic
<400> 183
   Gly Phe Pro Gly Tyr Phe Leu Ile Val Met Glu Phe Ile Gln Trp Ser
    1               5                   10                  15
   Lys Asp Asn Gly Val Pro Val Gly Pro Gly Arg Gly Ser Gly Ala Gly
                20                  25                  30
   Ser Leu Val Ala Tyr Ala Leu Lys Ile Thr Asp Leu Asp Pro Leu Glu
           35                  40                  45
   Phe Asp Leu Leu Phe Glu Arg Phe Leu Asn Pro Glu
       50                  55                  60
<210> 184
   <211> 60
   <212> PRT
   <213> Thermus thermophilus
<400> 184
   Arg Val Ser Met Pro Asp Ile Asp Thr Asp Phe Ser Asp Arg Glu Arg
    1               5                  10                  15
   Asp Arg Val Ile Gln Tyr Val Arg Glu Arg Tyr Gly Glu Asp Lys Val
               20                  25                  30
   Ala Gln Ile Gly Thr Leu Gly Ser Leu Ala Ser Lys Ala Ala Leu Lys
           35                  40                  45
   Asp Val Ala Arg Val Tyr Gly Ile Pro His Lys Lys
       50                  55                  60
<210> 185
   <211> 60
   <212> PRT
   <213> Thermus thermophilus
<220>
   <221> SITE
   <222> (15)
   <223> The residue at this position is similar but not identical to glutamic acid and/or lysine
<220>
   <221> SITE
   <222> (18)
   <223> The residue at this position is similar but not identical to arginine and/or glutamine
<220>
   <221> SITE
   <222> (21)
   <223> The residue at this position is similar but not identical to glutamine and/or glutamic acid
<220>
   <221> SITE
   <222> (22)
   <223> The residue at this position is similar but not identical to tyrosine and/or histidine
<220>
   <221> SITE
   <222> (25)
   <223> The residue at this position is similar but not identical to glutamic acid and/or aspartic acid
<220>
   <221> SITE
   <222> (33)
   <223> The residue at this position is similar but not identical to alanine and/or serine
<220>
   <221> SITE
   <222> (40)
   <223> The residue at this position is similar but not identical to serine and/or threonine

<220>
   <221> SITE
   <222> (41)
   <223> The residue at this position is similar but not identical to leucine and/or methionine
<220>
   <221> SITE
   <222> (43)
   <223> The residue at this position is similar but not identical to serine and/or alanine
<220>
   <221> SITE
   <222> (47)
   <223> The residue at this position is similar but not identical to leucine and/or isoleucine
<220>
   <221> SITE
   <222> (48)
   <223> The residue at this position is similar but not identical to lysine and/or arginine
<220>
   <221> SITE
   <222> (58)
   <223> The residue at this position is similar but not identical to histidine and/or tyrosine
<220>
   <221> SITE
   <222> (7)
   <223> The residue at this position can be any amino acid.
<220>
   <221> SITE
   <222> (9)
   <223> The residue at this position can be any amino acid.
<220>
   <221> SITE
   <222> (12)..(19)
   <223> The residue at this position can be any amino acid.
<220>
   <221> SITE
   <222> (24)
   <223> The residue at this position can be any amino acid.
<220>
   <221> SITE
   <222> (26)
   <223> The residue at this position can be any amino acid.
<220>
   <221> SITE
   <222> (29)
   <223> The residue at this position can be any amino acid.
<220>
   <221> SITE
   <222> (31)
   <223> The residue at this position can be any amino acid.
<220>
   <221> SITE
   <222> (33)
   <223> The residue at this position can be any amino acid.
<220>
   <221> SITE
   <222> (36)
   <223> The residue at this position can be any amino acid.
<220>
   <221> SITE
   <222> (38)
   <223> The residue at this position can be any amino acid.
<220>
   <221> SITE
   <222> (46)
   <223> The residue at this position can be any amino acid.
<220>
   <221> SITE
   <222> (51)
   <223> The residue at this position can be any amino acid.
<220>
   <221> SITE
   <222> (54)
   <223> The residue at this position can be any amino acid.
<220>
   <221> SITE
   <222> (56)
   <223> The residue at this position can be any amino acid.
<220>
   <221> SITE
   <222> (59)..(60)
   <223> The residue at this position can be any amino acid.
<400> 185
<210> 186
   <211> 60
   <212> PRT
   <213> Escherichia coli
<400> 186
   Arg Val Ser Met Pro Asp Phe Asp Val Asp Phe Cys Met Glu Lys Arg
   1 5 10 15
   Asp Gln Val Ile Glu His Val Ala Asp Met Tyr Gly Arg Asp Ala Val
   20 25 30
   Ser Gln Ile Ile Thr Phe Gly Thr Met Ala Ala Lys Ala Val Ile Arg
   35 40 45
   Asp Val Gly Arg Val Leu Gly His Pro Tyr Gly Phe
   50 55 60
<210> 187
   <211> 9
   <212> PRT
   <213> Thermus thermophilus
<400> 187
   Ala Glu Glu Leu Ala Lys Leu Ile Pro
   1 5
<210> 188
   <211> 9
   <212> PRT
   <213> Artificial Sequence
<220>
   <221> SITE
   <222> (2)
   <223> The residue at this position is similar but not identical to glutamic and/or aspartic acid
<220>
   <221> SITE
   <222> (4)
   <223> The residue at this position is similar but not identical to leucine and/or isoleucine
<220>
   <221> SITE
   <222> (5)
   <223> The residue at this position is similar but not identical to alanine and/or serine
<220>
   <223> Description of Artificial Sequence: Synthetic
<220>
   <221> SITE
   <222> (1)
   <223> The residue at this position can be any amino acid.
<220>
   <221> SITE
   <222> (3)
   <223> The residue at this position can be any amino acid.
<400> 188
<210> 189
   <211> 9
   <212> PRT
   <213> Escherichia coli
<400> 189
   Val Asp Arg Ile Ser Lys Leu Ile Pro
   1 5
<210> 190
   <211> 17
   <212> DNA
   <213> Thermus thermophilus
<220>
   <221> misc_feature
   <222> (9)
   <223> The residue at this position can be any nucleotide.
<400> 190
   ggttcctcna aggtctt 17
<210> 191
   <211> 7
   <212> PRT
   <213> Thermus thermophilus
<400> 191
   His Leu His Gln His Thr Gln
   1 5
<210> 192
   <211> 6
   <212> PRT
   <213> Thermus thermophilus
<400> 192
   Glu Gly Phe Tyr Glu Lys
   1 5
<210> 193
   <211> 7
   <212> PRT
   <213> Thermus thermophilus
<400> 193
   Tyr Gln Glu Gln Gln Met Gln
   1 5
<210> 194
   <211> 6
   <212> PRT
   <213> Thermus thermophilus
<400> 194
   Asp Gly Gly Tyr Phe His
   1 5
<210> 195
   <211> 8
   <212> PRT
   <213> Artificial: Synthetic
<220>
   <221> SITE
   <222> (5)
   <223> The residue at this position is similar but not identical to arginine and/or glutamine
<400> 195
<210> 196
   <211> 4858
   <212> DNA
   <213> Thermus thermophilus
<400> 196
   ggatcccgag cctctggagg tgccctagaa gggtctcggc ctcggccagg gtcatctccc 60
   cggcgagggc gtagcggtaa agggcgtcgg ccgcttcata aagggccagg gtgggggcgg 120
   cgagggggcg gtcttcctgc ctccagcccc ggtagaggcg tacggcttcg gcccttgggt 180
   ctgccgccaa gagcaggcgg aggaggaagc tagcgtccag caccacccag ctcacggtcc 240
   cgctcctccc tcagggcgga aaccacctcc tcaggggggg taaggggcct gcccaagcgg 300
   gcgtggatgg cccggttaag ggagaggagg gcctccaagg ggtcctcctt ccccgcggtg 360
   aggcgggcgt aggccttcac ggagaggatg accgcctgcg gcctgccccg cccctccacc 420
   acgatcacct ccccttcctc cacccgcctc agcacctcgc cgaagtggac gcgggcctcg 480
   gtggcgctta agacgcgctt gcgatgcatc atttcatgca ttagtttagc cgccccggtc 540
   cctccccgca aggtaaactg gggaccatgg gccgcaaact ccgcttcgcc cacctccacc 600
   agcacaccca gttctccctc ctggacgggg cggcgaagct ttccgacctc ctcaagtggg 660
   tcaaggagac gacccccgag gaccccgcct tggccatgac cgaccacggc aacctcttcg 720
   gggccgtgga gttctacaag aaggccaccg aaatgggcat caagcccatc ctgggctacg 780
   aggcctacgt ggcggcggaa agccgctttg accgcaagcg gggaaagggc ctagacgggg 840
   gctactttca cctcaccctc ctcgccaagg acttcacggg gtaccagaac ctggtgcgcc 900
   tggcgagccg ggcttacctg gaggggtttt acgaaaagcc ccggattgac cgggagatcc 960
   tgcgcgagca cgccgagggc ctcatcgccc tctcggggtg cctcggggcg gagatccccc 1020
   agttcatcct ccaggaccgt ctggacctgg ccgaggcccg gctcaacgag tacctctcca 1080
   tcttcaagga ccgcttcttc attgagatcc agaaccacgg cctccccgag cagaaaaagg 1140
   tcaacgaggt cctcaaggag ttcgcccgaa agtacggcct ggggatggtg gccaccaacg 1200
   acggccatta cgtgaggaag gaggacgccc gggcccacga ggtcctcctc gccatccagt 1260
   ccaagagcac cctggacgac cccgggcgct ggcgcttccc ctgcgacgag ttctacgtga 1320
   agacccccga ggagatgcgg gccatgttcc ccgaggagga gtggggggac gagccctttg 1380
   acaacaccgt ggagatcgcc cgcatgtgca acgtggagct gcccatcggg gacaagatgg 1440
   tctaccgcat cccccgcttc cccctccccg cccgtcggac cgaggcccag tacctcatgg 1500
   agctcacctt taaggggctc ctccgccgct acccggaccg gatcaccgag ggcttctacc 1560
   gggaggtctt ccgccttttg gggaagcttc ccccccacgg ggacggggag gccctggccg 1620
   aggccttggc ccaggtggag cgggaggctt gggagaggct catgaagagc ctccccccct 1680
   tggccggggt caaggagtgg acggcggagg ccattttcca ccgggccctt tacgagcttt 1740
   ccgtgataga gcgcatgggg tttcccggct acttcctcat cgtccaggac tacatcaact 1800
   gggcccggag aaacggcgtc tccgtggggc ccggcagggg gagcgccgcc gggagcctgg 1860
   tggcctacgc cgtggggatc accaacattg accccctgcg cttcggcctc ctctttgagc 1920
   gcttcctgaa ccccgagagg gtctccatgc ccgacattga cacggacttc tccgaccggg 1980
   agcgggaccg ggtgatccag tacgtgcggg aacgctacgg cgaggacaag gtggcccaga 2040
   tcggcaccct gggaagcctc gcctccaagg ccgccctcaa ggacgtggcc cgggtctacg 2100
   gcatccccca caagaaggcg gaggaattgg ccaagctcat cccggtgcag ttcgggaagc 2160
   ccaagcccct gcaggaggcc atccaggtgg tgccggagct tagggcggag atggagaagg 2220
   accccaaggt gcgggaggtc ctcgaggtgg ccatgcgcct ggagggcctg aaccgccacg 2280
   cctccgtcca cgccgccggg gtggtgatcg ccgccgagcc cctcacggac ctcgtccccc 2340
   tcatgcgcga ccaggaaggg cggcccgtca cccagtacga catgggggcg gtggaggcct 2400
   tgggcctttt gaagatggac tttttgggcc tccgcaccct caccttcctg gacgaggtca 2460
   agcgcatcgt caaggcgtcc cagggggtgg agctggacta cgatgccctc cccctggacg 2520
   accccaagac cttcgccctc ctctcccggg gggagaccaa gggggtcttc cagctggagt 2580
   cgggggggat gaccgccacg ctccgcggcc tcaagccgcg gcgctttgag gacctgatcg 2640
   ccatcctctc cctctaccgc cccgggccca tggagcacat ccccacctac atccgccgcc 2700
   accacgggct ggagcccgtg agctacagcg agtttcccca cgccgagaag tacctaaagc 2760
   ccatcctgga cgagacctac ggcatccccg tctaccagga gcagatcatg cagatcgcct 2820
   cggccgtggc ggggtactcc ctgggcgagg cggacctcct caggcgggcc atggggaaga 2880
   agaagctgag gagatgcaga agcaccggga gcgcttcgtc cagggggcca aggaaagggg 2940
   cgtgcccgag gaggaggcca accgcctctt tgacatggct ggaggccttc gccaactacg 3000
   gcttcaacaa atctcatgca gcggcctaca gcctcctctc ctaccagacc gcctacgtga 3060
   aggcccacta ccccgtggag ttcatggccg ccctcctctc cgtggagcgg cacgactccg 3120
   acaaggtggc cgagtacatc cgcgacgccc gggccatggg catagaggtc cttcccccgg 3180
   acgtcaaccg ctccgggttt gactttctgg tccagggccg gcagatcctc ttcggcctct 3240
   ccgcggtgaa aaacgtgggc gaggcggcgg cggaggccat tctccgggag cgggagcggg 3300
   gcggccccta ccggagcctc ggggacttcc tcaagcggct ggacgagaag gtgctcaaca 3360
   agcggaccct ggagtccctc atcaaggcgg gtgccctgga cggcttcggg gaaagggcgc 3420
   ggctcctcgc ctccctggag gggctcctca ggtgggcggc cgagactcgg gagaaggccc 3480
   gctcgggcat gatgggcctc ttcagcgaag tggaggagcc gcctttggcc gaggccgccc 3540
   ccctggacga gatcacccgg ctccgctacg aaaaggaggc cctggggatt tatgtttccg 3600
   gccatcccat cttgcggtat cccgggctcc gggagacggc cacctgcacc ctggaggagc 3660
   ttccccacct ggcccgggac ctgccgcccc ggtctagggt cctcctcgcc ggcatggtgg 3720
   aggaggtggt gcgcaagccc acgaaaagcg gcgggatgat ggcccgcttc gtcctctccg 3780
   acgagacggg ggcgcttgag gcggtggcct tcggccgggc ctacgaccag gtctccccga 3840
   ggctcaagga ggacaccccc gtgctcgtcc tcgccgaggt ggagcgggag gaggggggcg 3900
   tgcgggtgct ggcccaggcc gtttggacct acgaggagct ggagcaggtc ccccgggccc 3960
   tcgaggtgga ggtggaggcc tccctcctgg acgaccgggg ggtggcccac ctgaaaagcc 4020
   tcctggacga gcacgcgggg accctccccc tgtacgtccg ggtccagggc gccttcggcg 4080
   aggccctcct cgccctgagg gaggtgcggg tgggggagga ggccttggcg gccctcgagg 4140
   ccgaggggtt ccgggcctac ctcctgcctg accgggaggt cctcctccag ggcggccagg 4200
   cgggggaggc ccaggaggcg gtgcccttct agggggtggg ccgtgagaca gggtgccatc 4260
   gtcctcgccg ggggcaagga ggcctgggcc gagcgctttg gggtggggag caaggccctc 4320
   gtgccctacc gcggccggcc catggtggag tgggtcctgg aggccctcta cgccgcgggg 4380
   ctttccccgg tgtacgtggg ggagaacccc ggcctcgttc ccgcgcccgc tctcaccctt 4440
   cccgaccgcg gaggcctcct ggaaaacctg gagcaggccc tggagcacgt ggaggggcgc 4500
   gtgctcgtgg ccaccgggga catcccccac ctcacggagg aggcggtccg cttcgttctg 4560
   gataaggctc ctgaggcggc cctggtctac cccattgtgc ccaaggaggc ggtggaggcc 4620
   cgctttcccc agaccaagcg cacctacgcc cgcctccggg aggggacctt caccggcggc 4680
   aaccttctcc ttttggacaa gtccctcttc cgggaggccc ttcccctggc ccggcgggtg 4740
   gtggccctgc gcaagaggcc tttggccctg gcccgcctcg tggggtggga cgtgttgctg 4800
   aagctcctcc tgggccgcct ctccctcgcc gaggtggagg cccgggccca aaggatcc   4858
<210> 197
   <211> 1221
   <212> PRT
   <213> Thermus thermophilus
<400> 197
   Met Gly Arg Lys Leu Arg Phe Ala His Leu His Gln His Thr Gln Phe
    1               5                  10                  15
   Ser Leu Leu Asp Gly Ala Ala Lys Leu Ser Asp Leu Leu Lys Trp Val
               20                  25                  30
   Lys Glu Thr Thr Pro Glu Asp Pro Ala Leu Ala Met Thr Asp His Gly
           35                  40                  45
   Asn Leu Phe Gly Ala Val Glu Phe Tyr Lys Lys Ala Thr Glu Met Gly
       50                  55                  60
   Ile Lys Pro Ile Leu Gly Tyr Glu Ala Tyr Val Ala Ala Glu Ser Arg
   65                  70                  75                  80
   Phe Asp Arg Lys Arg Gly Lys Gly Leu Asp Gly Gly Tyr Phe His Leu
                   85                  90                  95
   Thr Leu Leu Ala Lys Asp Phe Thr Gly Tyr Gln Asn Leu Val Arg Leu
               100                 105                 110
   Ala Ser Arg Ala Tyr Leu Glu Gly Phe Tyr Glu Lys Pro Arg Ile Asp
           115                 120                 125
   Arg Glu Ile Leu Arg Glu His Ala Glu Gly Leu Ile Ala Leu Ser Gly
       130                 135                 140
   Cys Leu Gly Ala Glu Ile Pro Gln Phe Ile Leu Gln Asp Arg Leu Asp
   145                 150                 155                 160
   Leu Ala Glu Ala Arg Leu Asn Glu Tyr Leu Ser Ile Phe Lys Asp Arg
                   165                 170                 175
   Phe Phe Ile Glu Ile Gln Asn His Gly Leu Pro Glu Gln Lys Lys Val
               180                 185                 190
   Asn Glu Val Leu Lys Glu Phe Ala Arg Lys Tyr Gly Leu Gly Met Val
   195 200 205
   Ala Thr Asn Asp Gly His Tyr Val Arg Lys Glu Asp Ala Arg Ala His
   210 215 220
   Glu Val Leu Leu Ala Ile Gln Ser Lys Ser Thr Leu Asp Asp Pro Gly
   225 230 235 240
   Arg Trp Arg Phe Pro Cys Asp Glu Phe Tyr Val Lys Thr Pro Glu Glu
   245 250 255
   Met Arg Ala Met Phe Pro Glu Glu Glu Trp Gly Asp Glu Pro Phe Asp
   260 265 270
   Asn Thr Val Glu Ile Ala Arg Met Cys Asn Val Glu Leu Pro Ile Gly
   275 280 285
   Asp Lys Met Val Tyr Arg Ile Pro Arg Phe Pro Leu Pro Ala Arg Arg
   290 295 300
   Thr Glu Ala Gln Tyr Leu Met Glu Leu Thr Phe Lys Gly Leu Leu Arg
   305 310 315 320
   Arg Tyr Pro Asp Arg Ile Thr Glu Gly Phe Tyr Arg Glu Val Phe Arg
   325 330 335
   Leu Leu Gly Lys Leu Pro Pro His Gly Asp Gly Glu Ala Leu Ala Glu
   340 345 350
   Ala Leu Ala Gln Val Glu Arg Glu Ala Trp Glu Arg Leu Met Lys Ser
   355 360 365
   Leu Pro Pro Leu Ala Gly Val Lys Glu Trp Thr Ala Glu Ala Ile Phe
   370 375 380
   His Arg Ala Leu Tyr Glu Leu Ser Val Ile Glu Arg Met Gly Phe Pro
   385 390 395 400
   Gly Tyr Phe Leu Ile Val Gln Asp Tyr Ile Asn Trp Ala Arg Arg Asn
   405 410 415
   Gly Val Ser Val Gly Pro Gly Arg Gly Ser Ala Ala Gly Ser Leu Val
   420 425 430
   Ala Tyr Ala Val Gly Ile Thr Asn Ile Asp Pro Leu Arg Phe Gly Leu
   435 440 445
   Leu Phe Glu Arg Phe Leu Asn Pro Glu Arg Val Ser Met Pro Asp Ile
   450 455 460
   Asp Thr Asp Phe Ser Asp Arg Glu Arg Asp Arg Val Ile Gln Tyr Val
   465 470 475 480
   Arg Glu Arg Tyr Gly Glu Asp Lys Val Ala Gln Ile Gly Thr Leu Gly
   485 490 495
   Ser Leu Ala Ser Lys Ala Ala Leu Lys Asp Val Ala Arg Val Tyr Gly
   500 505 510
   Ile Pro His Lys Lys Ala Glu Glu Leu Ala Lys Leu Ile Pro Val Gln
   515 520 525
   Phe Gly Lys Pro Lys Pro Leu Gln Glu Ala Ile Gln Val Val Pro Glu
   530 535 540
   Leu Arg Ala Glu Met Glu Lys Asp Pro Lys Val Arg Glu Val Leu Glu
   545 550 555 560
   Val Ala Met Arg Leu Glu Gly Leu Asn Arg His Ala Ser Val His Ala
   565 570 575
   Ala Gly Val Val Ile Ala Ala Glu Pro Leu Thr Asp Leu Val Pro Leu
   580 585 590
   Met Arg Asp Gln Glu Gly Arg Pro Val Thr Gln Tyr Asp Met Gly Ala
   595 600 605
   Val Glu Ala Leu Gly Leu Leu Lys Met Asp Phe Leu Gly Leu Arg Thr
   610 615 620
   Leu Thr Phe Leu Asp Glu Val Lys Arg Ile Val Lys Ala Ser Gln Gly
   625 630 635 640
   Val Glu Leu Asp Tyr Asp Ala Leu Pro Leu Asp Asp Pro Lys Thr Phe
   645 650 655
   Ala Leu Leu Ser Arg Gly Glu Thr Lys Gly Val Phe Gln Leu Glu Ser
   660 665 670
   Gly Gly Met Thr Ala Thr Leu Arg Gly Leu Lys Pro Arg Arg Phe Glu
   675 680 685
   Asp Leu Ile Ala Ile Leu Ser Leu Tyr Arg Pro Gly Pro Met Glu His
   690 695 700
   Ile Pro Thr Tyr Ile Arg Arg His His Gly Leu Glu Pro Val Ser Tyr
   705 710 715 720
   Ser Glu Phe Pro His Ala Glu Lys Tyr Leu Lys Pro Ile Leu Asp Glu
   725 730 735
   Thr Tyr Gly Ile Pro Val Tyr Gln Glu Gln Ile Met Gln Ile Ala Ser
   740 745 750
   Ala Val Ala Gly Tyr Ser Leu Gly Glu Ala Asp Leu Leu Arg Arg Ala
   755 760 765
   Met Gly Lys Lys Lys Leu Arg Arg Cys Arg Ser Thr Gly Ser Ala Ser
   770 775 780
   Ser Arg Gly Pro Arg Lys Gly Ala Cys Pro Arg Arg Arg Pro Thr Ala
   785 790 795 800
   Ser Leu Thr Trp Leu Glu Ala Phe Ala Asn Tyr Gly Phe Asn Lys Ser
   805 810 815
   His Ala Ala Ala Tyr Ser Leu Leu Ser Tyr Gln Thr Ala Tyr Val Lys
   820 825 830
   Ala His Tyr Pro Val Glu Phe Met Ala Ala Leu Leu Ser Val Glu Arg
           835                 840                 845
   His Asp Ser Asp Lys Val Ala Glu Tyr Ile Arg Asp Ala Arg Ala Met
       850                 855                 860
   Gly Ile Glu Val Leu Pro Pro Asp Val Asn Arg Ser Gly Phe Asp Phe
   865                 870                 875                 880
   Leu Val Gln Gly Arg Gln Ile Leu Phe Gly Leu Ser Ala Val Lys Asn
                   885                 890                 895
   Val Gly Glu Ala Ala Ala Glu Ala Ile Leu Arg Glu Arg Glu Arg Gly
               900                 905                 910
   Gly Pro Tyr Arg Ser Leu Gly Asp Phe Leu Lys Arg Leu Asp Glu Lys
           915                 920                 925
   Val Leu Asn Lys Arg Thr Leu Glu Ser Leu Ile Lys Ala Gly Ala Leu
       930                 935                 940
   Asp Gly Phe Gly Glu Arg Ala Arg Leu Leu Ala Ser Leu Glu Gly Leu
   945                 950                 955                 960
   Leu Arg Trp Ala Ala Glu Thr Arg Glu Lys Ala Arg Ser Gly Met Met
                   965                 970                 975
   Gly Leu Phe Ser Glu Val Glu Glu Pro Pro Leu Ala Glu Ala Ala Pro
               980                 985                 990
   Leu Asp Glu Ile Thr Arg Leu Arg Tyr Glu Lys Glu Ala Leu Gly Ile
           995                 1000                1005
   Tyr Val Ser Gly His Pro Ile Leu Arg Tyr Pro Gly Leu Arg Glu Thr
       1010                1015                1020
   Ala Thr Cys Thr Leu Glu Glu Leu Pro His Leu Ala Arg Asp Leu Pro
   1025                1030                1035                1040
   Pro Arg Ser Arg Val Leu Leu Ala Gly Met Val Glu Glu Val Val Arg
                   1045                1050                1055
   Lys Pro Thr Lys Ser Gly Gly Met Met Ala Arg Phe Val Leu Ser Asp
               1060                1065                1070
   Glu Thr Gly Ala Leu Glu Ala Val Ala Phe Gly Arg Ala Tyr Asp Gln
           1075                1080                1085
   Val Ser Pro Arg Leu Lys Glu Asp Thr Pro Val Leu Val Leu Ala Glu
       1090                1095                1100
   Val Glu Arg Glu Glu Gly Gly Val Arg Val Leu Ala Gln Ala Val Trp
   1105                1110                1115                1120
   Thr Tyr Glu Glu Leu Glu Gln Val Pro Arg Ala Leu Glu Val Glu Val
                   1125                1130                1135
   Glu Ala Ser Leu Leu Asp Asp Arg Gly Val Ala His Leu Lys Ser Leu
               1140                1145                1150
   Leu Asp Glu His Ala Gly Thr Leu Pro Leu Tyr Val Arg Val Gln Gly
   1155 1160 1165
   Ala Phe Gly Glu Ala Leu Leu Ala Leu Arg Glu Val Arg Val Gly Glu
   1170 1175 1180
   Glu Ala Leu Ala Ala Leu Glu Ala Glu Gly Phe Arg Ala Tyr Leu Leu
   1185 1190 1195 1200
   Pro Asp Arg Glu Val Leu Leu Gln Gly Gly Gln Ala Gly Glu Ala Gln
   1205 1210 1215
   Glu Ala Val Pro Phe
   1220
<210> 198
   <211> 1297
   <212> PRT
   <213> Thermus thermophilus
<220>
   <221> SITE
   <222> (1)..(14)
   <223> The residue at this position can be any amino acid.
<220>
   <221> SITE
   <222> (136)..(140)
   <223> The residue at this position can be any amino acid.
<220>
   <221> SITE
   <222> (195)
   <223> The residue at this position can be any amino acid.
<220>
   <221> SITE
   <222> (286)..(288)
   <223> The residue at this position can be any amino acid.
<220>
   <221> SITE
   <222> (306)
   <223> The residue at this position can be any amino acid.
<220>
   <221> SITE
   <222> (329)
   <223> The residue at this position can be any amino acid.
<220>
   <221> SITE
   <222> (560)
   <223> The residue at this position can be any amino acid.
<220>
   <221> SITE
   <222> (570)..(572)
   <223> The residue at this position can be any amino acid.
<220>
   <221> SITE
   <222> (626)
   <223> The residue at this position can be any amino acid.
<220>
   <221> SITE
   <222> (629)
   <223> The residue at this position can be any amino acid.
<220>
   <221> SITE
   <222> (672)..(675)
   <223> The residue at this position can be any amino acid.
<220>
   <221> SITE
   <222> (686)..(703)
   <223> The residue at this position can be any amino acid.
<220>
   <221> SITE
   <222> (708)
   <223> The residue at this position can be any amino acid.
<220>
   <221> SITE
   <222> (759)..(760)
   <223> The residue at this position can be any amino acid.
<220>
   <221> SITE
   <222> (778)..(779)
   <223> The residue at this position can be any amino acid.
<220>
   <221> SITE
   <222> (945)
   <223> The residue at this position can be any amino acid.
<220>
   <221> SITE
   <222> (1008)
   <223> The residue at this position can be any amino acid.
<220>
   <221> SITE
   <222> (1027)
   <223> The residue at this position can be any amino acid.
<220>
   <221> SITE
   <222> (1045)
   <223> The residue at this position can be any amino acid.
<220>
   <221> SITE
   <222> (1049)..(1050)
   <223> The residue at this position can be any amino acid.
<220>
   <221> SITE
   <222> (1056)..(1058)
   <223> The residue at this position can be any amino acid.
<220>
   <221> SITE
   <222> (1066)..(1068)
   <223> The residue at this position can be any amino acid.
<220>
   <221> SITE
   <222> (1131)
   <223> The residue at this position can be any amino acid.
<220>
   <221> SITE
   <222> (1180)..(1181)
   <223> The residue at this position can be any amino acid.
<220>
   <221> SITE
   <222> (1295)..(1297)
   <223> The residue at this position can be any amino acid.
<400> 198

<210> 199
   <211> 1297
   <212> PRT
   <213> Borrelia burgdorferi
<220>
   <221> SITE
   <222> (54)..(55)
   <223> The residue at this position can be any amino acid.
<220>
   <221> SITE
   <222> (102)
   <223> The residue at this position can be any amino acid.
<220>
   <221> SITE
   <222> (136)..(140)
   <223> The residue at this position can be any amino acid.
<220>
   <221> SITE
   <222> (195)
   <223> The residue at this position can be any amino acid.
<220>
   <221> SITE
   <222> (284)..(289)
   <223> The residue at this position can be any amino acid.
<220>
   <221> SITE
   <222> (317)..(319)
   <223> The residue at this position can be any amino acid.
<220>
   <221> SITE
   <222> (350)..(361)
   <223> The residue at this position can be any amino acid.
<220>
   <221> SITE
   <222> (368)..(376)
   <223> The residue at this position can be any amino acid.
<220>
   <221> SITE
   <222> (378)..(410)
   <223> The residue at this position can be any amino acid.
<220>
   <221> SITE
   <222> (553)..(554)
   <223> The residue at this position can be any amino acid.
<220>
   <221> SITE
   <222> (556)
   <223> The residue at this position can be any amino acid.
<220>
   <221> SITE
   <222> (569)..(572)
   <223> The residue at this position can be any amino acid.
<220>
   <221> SITE
   <222> (629)
   <223> The residue at this position can be any amino acid.
<220>
   <221> SITE
   <222> (671)..(675)
   <223> The residue at this position can be any amino acid.
<220>
   <221> SITE
   <222> (686)..(703)
   <223> The residue at this position can be any amino acid.
<220>
   <221> SITE
   <222> (708)
   <223> The residue at this position can be any amino acid.
<220>
   <221> SITE
   <222> (759)..(760)
   <223> The residue at this position can be any amino acid.
<220>
   <221> SITE
   <222> (777)..(781)
   <223> The residue at this position can be any amino acid.
<220>
   <221> SITE
   <222> (786)..(787)
   <223> The residue at this position can be any amino acid.
<220>
   <221> SITE
   <222> (945)
   <223> The residue at this position can be any amino acid.
<220>
   <221> SITE
   <222> (1027)
   <223> The residue at this position can be any amino acid.
<220>
   <221> SITE
   <222> (1049)..(1050)
   <223> The residue at this position can be any amino acid.
<220>
   <221> SITE
   <222> (1097)
   <223> The residue at this position can be any amino acid.
<220>
   <221> SITE
   <222> (1111)..(1112)
   <223> The residue at this position can be any amino acid.
<220>
   <221> SITE
   <222> (1180)..(1181)
   <223> The residue at this position can be any amino acid.
<220>
   <221> SITE
   <222> (1205)..(1207)
   <223> The residue at this position can be any amino acid.
<220>
   <221> SITE
   <222> (1218)
   <223> The residue at this position can be any amino acid.
<220>
   <221> SITE
   <222> (1224)
   <223> The residue at this position can be any amino acid.
<220>
   <221> SITE
   <222> (1242)
   <223> The residue at this position can be any amino acid.
<220>
   <221> SITE
   <222> (1254)..(1255)
   <223> The residue at this position can be any amino acid.
<220>
   <221> SITE
   <222> (1259)..(1260)
   <223> The residue at this position can be any amino acid.
<220>
   <221> SITE
   <222> (1271)
   <223> The residue at this position can be any amino acid.
<220>
   <221> SITE
   <222> (1276)
   <223> The residue at this position can be any amino acid.
<220>
   <221> SITE
   <222> (1296)..(1297)
   <223> The residue at this position can be any amino acid.
<400> 199
<210> 200
   <211> 1297
   <212> PRT
   <213> Escherichia coli
<220>
   <223> Description of Artificial Sequence: Synthetic
<220>
   <221> SITE
   <222> (1)..(14)
   <223> The residue at this position can be any amino acid.
<220>
   <221> SITE
   <222> (17)
   <223> The residue at this position can be any amino acid.
<220>
   <221> SITE
   <222> (54)..(55)
   <223> The residue at this position can be any amino acid.
<220>
   <221> SITE
   <222> (93)..(94)
   <223> The residue at this position can be any amino acid.
<220>
   <221> SITE
   <222> (102)..(108)
   <223> The residue at this position can be any amino acid.
<220>
   <221> SITE
   <222> (137)..(140)
   <223> The residue at this position can be any amino acid.
<220>
   <221> SITE
   <222> (195)
   <223> The residue at this position can be any amino acid.
<220>
   <221> SITE
   <222> (284)..(289)
   <223> The residue at this position can be any amino acid.
<220>
   <221> SITE
   <222> (306)
   <223> The residue at this position can be any amino acid.
<220>
   <221> SITE
   <222> (317)..(319)
   <223> The residue at this position can be any amino acid.
<220>
   <221> SITE
   <222> (329) .. (330)
   <223> The residue at this position can be any amino acid.
<220>
   <221> SITE
   <222> (356).. (360)
   <223> The residue at this position can be any amino acid.
<220>
   <221> SITE
   <222> (368)..(374)
   <223> The residue at this position can be any amino acid.
<220>
   <221> SITE
   <222> (379)..(410)
   <223> The residue at this position can be any amino acid.

<220>
   <221> SITE
   <222> (554)..(556)
   <223> The residue at this position can be any amino acid.
<220>
   <221> SITE
   <222> (570)..(572)
   <223> The residue at this position can be any amino acid.
<220>
   <221> SITE
   <222> (626)
   <223> The residue at this position can be any amino acid.
<220>
   <221> SITE
   <222> (686)..(703)
   <223> The residue at this position can be any amino acid.
<220>
   <221> SIMILAR
   <222> (708)
   <223> The residue at this position can be any amino acid.
<220>
   <221> SITE
   <222> (786)..(787)
   <223> The residue at this position can be any amino acid.
<220>
   <221> SITE
   <222> (1027)
   <223> The residue at this position can be any amino acid.
<220>
   <221> SITE
   <222> (1047)
   <223> The residue at this position can be any amino acid.
<220>
   <221> SITE
   <222> (1056)..(1057)
   <223> The residue at this position can be any amino acid.
<220>
   <221> SITE
   <222> (1111)..(1112)
   <223> The residue at this position can be any amino acid.
<220>
   <221> SITE
   <222> (1131)
   <223> The residue at this position can be any amino acid.
<220>
   <221> SITE
   <222> (1205)..(1207)
   <223> The residue at this position can be any amino acid.
<220>
   <221> SITE
   <222> (1218)..(1219)
   <223> The residue at this position can be any amino acid.
<220>
   <221> SITE
   <222> (1224)
   <223> The residue at this position can be any amino acid.
<220>
   <221> SITE
   <222> (1238)..(1239)
   <223> The residue at this position can be any amino acid.
<220>
   <221> SITE
   <222> (1242)
   <223> The residue at this position can be any amino acid.
<220>
   <221> SITE
   <222> (1254)..(1255)
   <223> The residue at this position can be any amino acid.
<220>
   <221> SITE
   <222> (1259)..(1260)
   <223> The residue at this position can be any amino acid.
<220>
   <221> SITE
   <222> (1271)
   <223> The residue at this position can be any amino acid.
<220>
   <221> SITE
   <222> (1276)
   <223> The residue at this position can be any amino acid.
<400> 200
<210> 201
   <211> 1297
   <212> PRT
   <213> Bacillus subtilis
<220>
   <221> SITE
   <222> (1)..(18)
   <223> The residue at this position can be any amino acid.
<220>
   <221> SITE
   <222> (54)..(55)
   <223> The residue at this position can be any amino acid.
<220>
   <221> SITE
   <222> (93)..(96)
   <223> The residue at this position can be any amino acid.
<220>
   <221> SITE
   <222> (102)..(106)
   <223> The residue at this position can be any amino acid.
<220>
   <221> SITE
   <222> (137)..(140)
   <223> The residue at this position can be any amino acid.
<220>
   <221> SITE
   <222> (145)..(148)
   <223> The residue at this position can be any amino acid.
<220>
   <221> SITE.
   <222> (206)..(207)
   <223> The residue at this position can be any amino acid.
<220>
   <221> SITE
   <222> (269)..(270)
   <223> The residue at this position can be any amino acid.
<220>
   <221> SITE
   <222> (285) .. (290)
   <223> The residue at this position can be any amino acid.
<220>
   <221> SITE
   <222> (306)
   <223> The residue at this position can be any amino acid.
<220>
   <221> SITE
   <222> (317)..(319)
   <223> The residue at this position can be any amino acid.
<220>
   <221> SITE
   <222> (329)
   <223> The residue at this position can be any amino acid.
<220>
   <221> SITE
   <222> (350)..(360)
   <223> The residue at this position can be any amino acid.
<220>
   <221> SITE
   <222> (368)..(410)
   <223> The residue at this position can be any amino acid.
<220>
   <221> SITE
   <222> (559)..(556)
   <223> The residue at this position can be any amino acid.
<220>
   <221> SITE
   <222> (570)..(572)
   <223> The residue at this position can be any amino acid.
<220>
   <221> SITE
   <222> (626)
   <223> The residue at this position can be any amino acid.
<220>
   <221> SITE
   <222> (629)
   <223> The residue at this position can be any amino acid.
<220>
   <221> SITE
   <222> (672)..(675)
   <223> The residue at this position can be any amino acid.
<220>
   <221> SITE
   <222> (686)..(703)
   <223> The residue at this position can be any amino acid.
<220>
   <221> SITE
   <222> (708)
   <223> The residue at this position can be any amino acid.
<220>
   <221> SITE
   <222> (759)..(760)
   <223> The residue at this position can be any amino acid.
<220>
   <221> SITE
   <222> (777)..(781)
   <223> The residue at this position can be any amino acid.
<220>
   <221> SITE
   <222> (786)..(787)
   <223> The residue at this position can be any amino acid.
<220>
   <221> SITE
   <222> (945)
   <223> The residue at this position can be any amino acid.
<220>
   <221> SITE
   <222> (970)
   <223> The residue at this position can be any amino acid.
<220>
   <221> SITE
   <222> (1036)..(1038)
   <223> The residue at this position can be any amino acid.
<220>
   <221> SITE
   <222> (1047)..(1050)
   <223> The residue at this position can be any amino acid.
<220>
   <221> SITE
   <222> (1056)..(1057)
   <223> The residue at this position can be any amino acid.
<220> ,
   <221> SITE
   <222> (1097)..(1100)
   <223> The residue at this position can be any amino acid.
<220>
   <221> SITE
   <222> (1131)
   <223> The residue at this position can be any amino acid.
<220>
   <221> SITE
   <222> (1180)..(1181)
   <223> The residue at this position can be any amino acid.
<220>
   <221> SITE
   <222> (1205)..(1207)
   <223> The residue at this position can be any amino acid.
<220>
   <221> SITE
   <222> (1218)..(1220)
   <223> The residue at this position can be any amino acid.
<220>
   <221> SITE
   <222> (1242)..(1245)
   <223> The residue at this position can be any amino acid.
<220>
   <221> SITE
   <222> (1254)..(1256)
   <223> The residue at this position can be any amino acid.
<220>
   <221> SITE
   <222> (1259)..(1261)
   <223> The residue at this position can be any amino acid.
<220>
   <221> SITE
   <222> (1271)
   <223> The residue at this position can be any amino acid.

<220>
   <221> SITE
   <222> (1276)
   <223> The residue at this position can be any amino acid.
<400> 201
<210> 202
   <211> 57
   <212> DNA
   <213> Artificial Sequence
<220>
   <223> Description of Artificial Sequence: Synthetic
<400> 202
   ctagaggagg ttaattaacc atggaaaaaa aaaggtacca aaaaaaaagg ccggcca 57
<210> 203
   <211> 57
   <212> DNA
   <213> Artificial Sequence
<220>
   <223> Description of Artificial Sequence: Synthetic
<400> 203
   tcctccaatt aattggtacc tttttttttc catggttttt ttttccggcc ggtgatc 57
<210> 204
   <211> 44
   <212> DNA
   <213> Artificial Sequence
<220>
   <223> Description of Artificial Sequence: Synthetic
<400> 204
   ctaggaaaaa aaaaggtacc aaaaaaaaag gccggccact agtg 44
<210> 205
   <211> 44
   <212> DNA
   <213> Artificial Sequence
<220>
   <223> Description of Artificial Sequence: Synthetic
<400> 205
   cttttttttt ccatggtttt tttttccggc cggtgatcac agct 44
<210> 206
   <211> 27
   <212> DNA
   <213> Artificial Sequence
<220>
   <223> Description of Artificial Sequence: Synthetic
<400> 206
   gaattcctag gccgcaaact ccgcttc 27
<210> 207
   <211> 28
   <212> DNA
   <213> Artificial Sequence
<220>
   <223> Description of Artificial Sequence: Synthetic
<400> 207
   gtgctcgcgc aggatctccc ggtcaatc 28
<210> 208
   <211> 18
   <212> PRT
   <213> Thermus thermophilus
<400> 208
   Glu Thr Thr Pro Glu Asp Pro Ala Leu Ala Met Thr Asp His Gly Asn
     1               5                  10                  15
   Leu Phe
<210> 209
   <211> 36
   <212> DNA
   <213> Artificial Sequence
<220>
   <223> Description of Artificial Sequence: Synthetic
<400> 209
   cctcgaacac ctcctgccgc aagacccttc gaccca 36
<210> 210
   <211> 22
   <212> DNA
   <213> Artificial Sequence
<220>
   <223> Description of Artificial Sequence: Synthetic
<400> 210
   tatcgatgag cgccctctac cg 22
<210> 211
   <211> 22
   <212> DNA
   <213> Artificial Sequence
<220>
   <223> Description of Artificial Sequence: Synthetic
<400> 211
   cggtggtggc gaagacgaag ag 22
<210> 212
   <211> 20
   <212> DNA
   <213> Artificial Sequence
<220>
   <223> Description of Artificial Sequence: Synthetic
<400> 212
   ccgccatgac cgccctggac 20
<210> 213
   <211> 24
   <212> DNA
   <213> Artificial Sequence
<220>
   <223> Description of Artificial Sequence: Synthetic
<400> 213
   actagttata ccagtacccc ctat 24
<210> 214
   <211> 692
   <212> DNA
   <213> Thermus thermophilus
<220>
   <221> misc_feature
   <222> (27)
   <223> The residue at this position can be any nucleotide.
<400> 214
   agggaagccg gctcgtcctc ctcctcnccg cccgcaaagc gccccccgag gctttaaaga 60
   aagcccccgc ccccagggcc ctggtctacg acctcaccct cctccaggtg cctgcaggtc 120
   tggaggacgt caccctggac cagctccttt acaccgcctt tgacctggag accacgggcc 180
   tggacccgga gcaagatgcc gtggtggccc tcgccggggt ccatatcctg ggtcgaaggg 240
   tcttgcggca ggaggtgttc gaggccctgg tgaacccggg gcgccccatc tcccccgcgg 300
   ccacggcggt ccacggcctc acggcggaga tgctccggga caagcctccc ctagaggcgg 360
   tcctccccgc cttccgcgcc ttcgtccagg acacggtgct ggtggcccac aacggggcct 420
   ttgacctggc ctttctgcgc cgggcgggcc tggaccagcc ccccctcctg gacaccctcc 480
   tcctcgccca gctcctcttc cccgacctca aggactaccg cctcgaggcc ctggcccacc 540
   gcttcggcgt ccccgccacc gggcggcaca ccgccttggg cgatgcgctg atgacggcgg 600
   aggtcttcgt gaggatgcag cccctcctct ttgaacgggg gcttaggcgg ctctgggacg 660
   tggtggaggc ctgccgcgac tccccttggc cg                               692
<210> 215
   <211> 230
   <212> PRT
   <213> Thermus thermophilus
<220>
   <221> SITE
   <222> (9)
   <223> The residue at this position can be any amino acid.
<400> 215
<210> 216
   <211> 195
   <212> PRT
   <213> Thermus thermophilus
<400> 216
   Met Pro Ala Gly Leu Glu Asp Val Thr Leu Asp Gln Leu Leu Tyr Thr
   1               5                   10                  15
   Ala Phe Asp Leu Glu Thr Thr Gly Leu Asp Pro Glu Gln Asp Ala Val
               20                  25                  30
   Val Ala Leu Ala Gly Val His Ile Leu Gly Arg Arg Val Leu Arg Gln
           35                  40                  45
   Glu Val Phe Glu Ala Leu Val Asn Pro Gly Arg Pro Ile Ser Pro Ala
       50                  55                  60
   Ala Thr Ala Val His Gly Leu Thr Ala Glu Met Leu Arg Asp Lys Pro
   65                  70                  75                  80
   Pro Leu Glu Ala Val Leu Pro Ala Phe Arg Ala Phe Val Gln Asp Thr
                   85                  90                  95
   Val Leu Val Ala His Asn Gly Ala Phe Asp Leu Ala Phe Leu Arg Arg
               100                 105                 110
   Ala Gly Leu Asp Gln Pro Pro Leu Leu Asp Thr Leu Leu Leu Ala Gln
           115                 120                 125
   Leu Leu Phe Pro Asp Leu Lys Asp Tyr Arg Leu Glu Ala Leu Ala His
       130                 135                 140
   Arg Phe Gly Val Pro Ala Thr Gly Arg His Thr Ala Leu Gly Asp Ala
   145                 150                 155                 160
   Leu Met Thr Ala Glu Val Phe Val Arg Met Gln Pro Leu Leu Phe Glu
                   165                 170                 175
   Arg Gly Leu Arg Arg Leu Trp Asp Val Val Glu Ala Cys Arg Asp Ser
               180                 185                 190
   Pro Trp Pro
           195
<210> 217
   <211> 253
   <212> PRT
   <213> Thermus thermophilus
<220>
   <221> SITE
   <222> (5) .. (9)
   <223> The residue at this position can be any amino acid.
<220>
   <221> SITE
   <222> (124)..(133)
   <223> The residue at this position can be any amino acid.
<220>
   <221> SITE
   <222> (151)..(152)
   <223> The residue at this position can be any amino acid.
<220>
   <221> SITE
   <222> (187)
   <223> The residue at this position can be any amino acid.
<220>
   <221> SITE
   <222> (197)..(210)
   <223> The residue at this position can be any amino acid.
<220>
   <221> SITE
   <222> (228)..(253)
   <223> The residue at this position can be any amino acid.
<400> 217

<210> 218
   <211> 253
   <212> PRT
   <213> Treponema pallidum
<220>
   <221> SITE
   <222> (5)..(9)
   <223> The residue at this position can be any amino acid.
<220>
   <221> SITE
   <222> (49)..(50)
   <223> The residue at this position can be any amino acid.
<220>
   <221> SITE
   <222> (130)..(133)
   <223> The residue at this position can be any amino acid.
<220>
   <221> SITE
   <222> (227)..(253)
   <223> The residue at this position can be any amino acid.
<400> 218
<210> 219
   <211> 253
   <212> PRT
   <213> Aquiflex aeolicus
<220>
   <221> SITE
   <222> (18)
   <223> The residue at this position can be any amino acid.
<220>
   <221> SITE
   <222> (49)..(50)
   <223> The residue at this position can be any amino acid.
<220>
   <221> SITE
   <222> (130)..(133)
   <223> The residue at this position can be any amino acid.
<220>
   <221> SITE
   <222> (151)..(152)
   <223> The residue at this position can be any amino acid.
<220>
   <221> SITE
   <222> (197)..(210)
   <223> The residue at this position can be any amino acid.
<220>
   <221> SITE
   <222> (223)
   <223> The residue at this position can be any amino acid.
<220>
   <221> SITE
   <222> (227)..(253)
   <223> The residue at this position can be any amino acid.
<400> 219
<210> 220
   <211> 253
   <212> PRT
   <213> Escherichia coli
<220>
   <221> SITE
   <222> (5)..(8)
   <223> The residue at this position can be any amino acid.
<220>
   <221> SITE
   <222> (18)..(20)
   <223> The residue at this position can be any amino acid.
<220>
   <221> SITE
   <222> (50)
   <223> The residue at this position can be any amino acid.
<220>
   <221> SITE
   <222> (151)
   <223> The residue at this position can be any amino acid.
<220>
   <221> SITE
   <222> (223)
   <223> The residue at this position can be any amino acid.
<400> 220
<210> 221
   <211> 1230
   <212> DNA
   <213> Thermus thermophilus
<400> 221
   ctgatgcacg ccgtgggcca ctccgtggcc aagcgcttcc cccacctgaa gattgagtac 60
   gtttccacgg aaactttcac caacgagctc atcaacgcca tccgcgagga ccggatgacg 120
   gagttccggg agcggtaccg ctccgtggac ctcctgctgg tggacgacgt ccagttcatc 180
   gccggaaagg agcgcaccca ggaggagttt ttccacacct tcaacgccct ttacgaggcc 240
   cataagcaga tcatcctctc ctccgaccgg ccgcccaaag gacatcctca ccctggaggc 300
   gcgcctgcgg agccgctttg agtggggctt gatcaccgac atccagcccc ccgacctgga 360
   aacccggatc gccatcctga agatgaacgc cgagcagcgg ggcctgagga tccccgagga 420
   cgccctggag tacatcgccc ggcaggtcac ctccaacatc cgggagctgg aaggggccct 480
   catgcgggca tcgccttcgc ctcgctcaac ggcgttgacg tgacccgcgc cgtggccgcc 540
   aasssstctc cgacatcttc gcccccaggg agctggaggc gaccccttgg agatcatccg 600
   caaagtggcg accacttcgg cctgaaaccg gagagctcac ggggagcggc cgaagaagga 660
   ggtggtcctc ccccggcagc tcgccatgta cctggtgcgg gagctcaccc gggcctccct 720
   gcccgagatc ggccagctct tcggcggccg ggaccacacc acggtcctct acgccatcca 780
   gaaggtccag gagctcgcgg aaagcgaccg ggaggtgcag ggcctcctcc gcaccctccg 840
   ggaaggcgtg cacatgaacc cctgtggata acctgtggat aaccctgtgg ataaccgggr 900
   ctcaaagctg tggataaccc tgtggataac ctgtggaaaa ctccgggggg gccgaacccc 960
   tgtggataac ccgcacttta tccacaggtt atccacaggc cggggccagt tatccacagc 1020
   agttaatcca catgcccctt tcttgtcctg gagggagttt tccaccgcct tatacacaat 1080
   tctctccaca ggacctacta ctacgactac gtatctttta aaagttttaa aaagagagtc 1140
   gtaatagcca ttaaaaaagg cgtcgggcaa tgaacaataa cggttcccaa gaactcctct 1200
   aggaccagct ttccctacct ggtgagatgt                                  1230
<210> 222
   <211> 287
   <212> PRT
   <213> Thermus thermophilus
<220>
   <221> SITE
   <222> (180)..(181)
   <223> The residue at this position can be any amino acid.
<400> 222
<210> 223
   <211> 1255
   <212> PRT
   <213> Artificial Sequence
<220>
   <223> Description of Artificial Sequence: Semi-synthetic N-terminal synthethic sequence attached to Thermus Thermophilus dnaE
<400> 223
   Met Ala Gly Cys Leu Asn Asp Ile Phe Glu Ala Gln Lys Ile Glu Trp
    1               5                  10                  15
   His His His His His His Leu Val Pro Arg Gly Ser Gly Gly Gly Gly
               20                  25                  30
   Leu Gln Asn Leu Gly Arg Lys Leu Arg Phe Ala His Leu His Gln His
           35                  40                  45
   Thr Gln Phe Ser Leu Leu Asp Gly Ala Ala Lys Leu Ser Asp Leu Leu
       50                  55                  60
   Lys Trp Val Lys Glu Thr Thr Pro Glu Asp Pro Ala Leu Ala Met Thr
   65                  70                  75                  80
   Asp His Gly Asn Leu Phe Gly Ala Val Glu Phe Tyr Lys Lys Ala Thr
                   85                  90                  95
   Glu Met Gly Ile Lys Pro Ile Leu Gly Tyr Glu Ala Tyr Val Ala Ala
               100                 105                 110
   Glu Ser Arg Phe Asp Arg Lys Arg Gly Lys Gly Leu Asp Gly Gly Tyr
           115                 120                 125
   Phe His Leu Thr Leu Leu Ala Lys Asp Phe Thr Gly Tyr Gln Asn Leu
       130                 135                 140
   Val Arg Leu Ala Ser Arg Ala Tyr Leu Glu Gly Phe Tyr Glu Lys Pro
   145                 150                 155                 160
   Arg Ile Asp Arg Glu Ile Leu Arg Glu His Ala Glu Gly Leu Ile Ala
                   165                 170                 175
   Leu Ser Gly Cys Leu Gly Ala Glu Ile Pro Gln Phe Ile Leu Gln Asp
               180                 185                 190
   Arg Leu Asp Leu Ala Glu Ala Arg Leu Asn Glu Tyr Leu Ser Ile Phe
           195                 200                 205
   Lys Asp Arg Phe Phe Ile Glu Ile Gln Asn His Gly Leu Pro Glu Gln
       210                 215                 220
   Lys Lys Val Asn Glu Val Leu Lys Glu Phe Ala Arg Lys Tyr Gly Leu
   225                 230                 235                 240
   Gly Met Val Ala Thr Asn Asp Gly His Tyr Val Arg Lys Glu Asp Ala
                   245                 250                 255
   Arg Ala His Glu Val Leu Leu Ala Ile Gln Ser Lys Ser Thr Leu Asp
   260 265 270
   Asp Pro Gly Arg Trp Arg Phe Pro Cys Asp Glu Phe Tyr Val Lys Thr
   275 280 285
   Pro Glu Glu Met Arg Ala Met Phe Pro Glu Glu Glu Trp Gly Asp Glu
   290 295 300
   Phe Asp Asn Thr Val Glu Ile Ala Arg Met Cys Asn Val Glu Leu Pro
   305 310 315 320
   Ile Gly Asp Lys Met Val Tyr Arg Ile Pro Arg Phe Pro Leu Pro Ala
   325 330 335
   Arg Arg Thr Glu Ala Gln Tyr Leu Met Glu Leu Thr Phe Lys Gly Leu
   340 345 350
   Leu Arg Arg Tyr Pro Asp Arg Ile Thr Glu Gly Phe Tyr Arg Glu Val
   355 360 365
   Phe Arg Leu Leu Gly Lys Leu Pro Pro His Gly Asp Gly Glu Ala Leu
   370 375 380
   Ala Glu Ala Leu Ala Gln Val Glu Arg Glu Ala Trp Glu Arg Leu Met
   385 390 395 400
   Lys Ser Leu Pro Pro Leu Ala Gly Val Lys Glu Trp Thr Ala Glu Ala
   405 410 415
   Ile Phe His Arg Ala Leu Tyr Glu Leu Ser Val Ile Glu Arg Met Gly
   420 425 430
   Phe Pro Gly Tyr Phe Leu Ile Val Gln Asp Tyr Ile Asn Trp Ala Arg
   435 440 445
   Arg Asn Gly Val Ser Val Gly Pro Gly Arg Gly Ser Ala Ala Gly Ser
   450 455 460
   Leu Val Ala Tyr Ala Val Gly Ile Thr Asn Ile Asp Pro Leu Arg Phe
   465 470 475 480
   Gly Leu Leu Phe Glu Arg Phe Leu Asn Pro Glu Arg Val Ser Met Pro
   485 490 495
   Asp Ile Asp Thr Asp Phe Ser Asp Arg Glu Arg Asp Arg Val Ile Gln
   500 505 510
   Tyr Val Arg Glu Arg Tyr Gly Glu Asp Lys Val Ala Gln Ile Gly Thr
   515 520 525
   Leu Gly Ser Leu Ala Ser Lys Ala Ala Leu Lys Asp Val Ala Arg Val
   530 535 540
   Tyr Gly Ile Pro His Lys Lys Ala Glu Glu Leu Ala Lys Leu Ile Pro
   545 550 555 560
   Val Gln Phe Gly Lys Pro Lys Pro Leu Gln Glu Ala Ile Gln Val Val
   565 570 575
   Pro Glu Leu Arg Ala Glu Met Glu Lys Asp Pro Lys Val Arg Glu Val
   580 585 590
   Leu Glu Val Ala Met Arg Leu Glu Gly Leu Asn Arg His Ala Ser Val
   595 600 605
   His Ala Ala Gly Val Val Ile Ala Ala Glu Pro Leu Thr Asp Leu Val
   610 615 620
   Pro Leu Met Arg Asp Gln Glu Gly Arg Pro Val Thr Gln Tyr Asp Met
   625 630 635 640
   Gly Ala Val Glu Ala Leu Gly Leu Leu Lys Met Asp Phe Leu Gly Leu
   645 650 655
   Arg Thr Leu Thr Phe Leu Asp Glu Val Lys Arg Ile Val Lys Ala Ser
   660 665 670
   Gln Gly Val Glu Leu Asp Tyr Asp Ala Leu Pro Leu Asp Asp Pro Lys
   675 680 685
   Thr Phe Ala Leu Leu Ser Arg Gly Glu Thr Lys Gly Val Phe Gln Leu
   690 695 700
   Glu Ser Gly Gly Met Thr Ala Thr Leu Arg Gly Leu Lys Pro Arg Arg
   705 710 715 720
   Phe Glu Asp Leu Ile Ala Ile Leu Ser Leu Tyr Arg Pro Gly Pro Met
   725 730 735
   Glu His Ile Pro Thr Tyr Ile Arg Arg His His Gly Leu Glu Pro Val
   740 745 750
   Ser Tyr Ser Glu Phe Pro His Ala Glu Lys Tyr Leu Lys Pro Ile Leu
   755 760 765
   Asp Glu Thr Tyr Gly Ile Pro Val Tyr Gln Glu Gln Ile Met Gln Ile
   770 775 780
   Ala Ser Ala Val Ala Gly Tyr Ser Leu Gly Glu Ala Asp Leu Leu Arg
   785 790 795 800
   Arg Ala Met Gly Lys Lys Lys Leu Arg Arg Cys Arg Ser Thr Gly Ser
   805 810 815
   Ala Ser Ser Arg Gly Pro Arg Lys Gly Ala Cys Pro Arg Arg Arg Pro
   820 825 830
   Thr Ala Ser Leu Thr Trp Leu Glu Ala Phe Ala Asn Tyr Gly Phe Asn
   835 840 845
   Lys Ser His Ala Ala Ala Tyr Ser Leu Leu Ser Tyr Gln Thr Ala Tyr
   850 855 860
   Val Lys Ala His Tyr Pro Val Glu Phe Met Ala Ala Leu Leu Ser Val
   865 870 875 880
   Glu Arg His Asp Ser Asp Lys Val Ala Glu Tyr Ile Arg Asp Ala Arg
   885 890 895
   Ala Met Gly Ile Glu Val Leu Pro Pro Asp Val Asn Arg Ser Gly Phe
   900 905 910
   Asp Phe Leu Val Gln Gly Arg Gln Ile Leu Phe Gly Leu Ser Ala Val
   915 920 925
   Lys Asn Val Gly Glu Ala Ala Ala Glu Ala Ile Leu Arg Glu Arg Glu
   930 935 940
   Arg Gly Gly Pro Tyr Arg Ser Leu Gly Asp Phe Leu Lys Arg Leu Asp
   945 950 955 960
   Glu Lys Val Leu Asn Lys Arg Thr Leu Glu Ser Leu Ile Lys Ala Gly
   965 970 975
   Ala Leu Asp Gly Phe Gly Glu Arg Ala Arg Leu Leu Ala Ser Leu Glu
   980 985 990
   Gly Leu Leu Arg Trp Ala Ala Glu Thr Arg Glu Lys Ala Arg Ser Gly
   995 1000 1005
   Met Met Gly Leu Phe Ser Glu Val Glu Glu Pro Pro Leu Ala Glu Ala
   1010 1015 1020
   Ala Pro Leu Asp Glu Ile Thr Arg Leu Arg Tyr Glu Lys Glu Ala Leu
   1025 1030 1035 1040
   Gly Ile Tyr Val Ser Gly His Pro Ile Leu Arg Tyr Pro Gly Leu Arg
   1045 1050 1055
   Glu Thr Ala Thr Cys Thr Leu Glu Glu Leu Pro His Leu Ala Arg Asp
   1060 1065 1070
   Leu Pro Pro Arg Ser Arg Val Leu Leu Ala Gly Met Val Glu Glu Val
   1075 1080 1085
   Val Arg Lys Pro Thr Lys Ser Gly Gly Met Met Ala Arg Phe Val Leu
   1090 1095 1100
   Ser Asp Glu Thr Gly Ala Leu Glu Ala Val Ala Phe Gly Arg Ala Tyr
   1105 1110 1115 1120
   Asp Gln Val Ser Pro Arg Leu Lys Glu Asp Thr Pro Val Leu Val Leu
   1125 1130 1135
   Ala Glu Val Glu Arg Glu Glu Gly Gly Val Arg Val Leu Ala Gln Ala
   1140 1145 1150
   Val Trp Thr Tyr Glu Glu Leu Glu Gln Val Pro Arg Ala Leu Glu Val
   1155 1160 1165
   Glu Val Glu Ala Ser Leu Leu Asp Asp Arg Gly Val Ala His Leu Lys
   1170 1175 1180
   Ser Leu Leu Asp Glu His Ala Gly Thr Leu Pro Leu Tyr Val Arg Val
   1185 1190 1195 1200
   Gln Gly Ala Phe Gly Glu Ala Leu Leu Ala Leu Arg Glu Val Arg Val
   1205 1210 1215
   Gly Glu Glu Ala Leu Ala Ala Leu Glu Ala Glu Gly Phe Arg Ala Tyr
               1220                1225                1230
   Leu Leu Pro Asp Arg Glu Val Leu Leu Gln Gly Gly Gln Ala Gly Glu
           1235                1240                1245
   Ala Gln Glu Ala Val Pro Phe
       1250                1255

<210> 225
   <211> 26
   <212> PRT
   <213> Thermus thermophilus
<220>
   <221> UNSURE
   <222> (1)
   <223> The residue at this position is uncertain but could be either Gly, Trp or Pro
<220>
   <221> UNSURE
   <222> (16)
   <223> The residue at this position is uncertain but could be Gln
<220>
   <221> UNSURE
   <222> (19)
   <223> The residue at this position is uncertain but could be either Ile or Ala
<220>
   <221> UNSURE
   <222> (21)
   <223> The residue at this position is uncertain but could be either Ala or Ser
<220>
   <221> UNSURE
   <222> (24)
   <223> The residue at this position is uncertain but could be either Gly or Ala
<220>
   <221> UNSURE
   <222> (26)
   <223> The residue at this position is uncertain
<220>
   <221> UNSURE
   <222> (22)
   <223> The residue at this position is uncertain, but could be Ala.
<220>
   <221> UNSURE
   <222> (23)
   <223> The residue at this position is uncertain but could be Val
<220>
   <221> SITE
   <222> (25)
   <223> The residue at this position can be any amino acid.
<400> 225
<210> 226
   <211> 26
   <212> PRT
   <213> Thermus thermophilus
<220>
   <221> UNSURE
   <222> (1)
   <223> The residue at this position is uncertain, but could be either Gly, Trp or Pro
<220>
   <221> UNSURE
   <222> (16)
   <223> The residue at this position is uncertain but could be Gln
<220>
   <221> UNSURE
   <222> (19)
   <223> The residue at this position is uncertain but could be Ile or Ala
<220>
   <221> UNSURE
   <222> (21)
   <223> The residue at this position is uncertain but could be either Ala or Ser
<220>
   <221> UNSURE
   <222> (24)
   <223> The residue at this position is uncertain but could be either Gly or Ala
<220>
   <221> UNSURE
   <222> (26)
   <223> The residue at this position is uncertain
<220>
   <221> UNSURE
   <222> (22)
   <223> The residue at this position is uncertain but could be Ala
<220>
   <221> UNSURE
   <222> (23)
   <223> The residue at this position is uncertain but could be Val
<220>
   <221> SITE
   <222> (25)
   <223> The residue at this position can be any amino acid.
<400> 226
<210> 227
   <211> 13
   <212> PRT
   <213> Thermus thermophilus
<220>
   <221> UNSURE
   <222> (11)
   <223> The residue at this position is uncertain but may be leucine
<220>
   <221> UNSURE
   <222> (12)
   <223> The residue at this position is uncertain but may be phenylalanine
<220>
   <221> UNSURE
   <222> (13)
   <223> The residue at this position is uncertain but may probably Asp or Ala
<400> 227
   Gly Leu Asp Gly Gly Tyr Phe His Leu Thr Leu Phe Ala
    1               5                  10
<210> 228
   <211> 8
   <212> PRT
   <213> Escherichia coli
<400> 228
   Ala Asn Lys Glu Arg Ser Gln Glu
    1               5
<210> 229
   <211> 8
   <212> PRT
   <213> Artificial Sequence
<220>
   <221> VARIANT
   <222> (5)
   <223> The residue at this position could be either "R" or "Q"
<220>
   <223> Description of Artificial Sequence: Synthetic
<400> 229
   Ala Gly Lys Glu Arg Thr Gln Glu
   1               5
<210> 230
   <211> 453
   <212> DNA
   <213> Thermus thermophilus
<220>
   <221> misc_feature
   <222> (89)
   <223> The residue at this position can be any nucleotide.
<220>
   <221> misc_feature
   <222> (392)
   <223> The residue at this position can be any nucleotide.
<400> 230
   aaaaagagag tcgtaatagc cattaaaaaa ggcgtcgggc aatgaaacaa taacggttcc 60
   caagaactcc tctaggacca gctttcctnc tggagcgcat cgtcccctct agaagcgcca 120
   accccctcta cacctacctg gggctttacg ccgaggaagg ggccttgatc ctcttcggga 180
   ccaacgggga ggtggacctc gaggkccggc ttcccggcga ggcccaaagc ctttcccggg 240
   tgctcgtccc cgcccagccc ttcttccaac tggtgcggag ccttyctggg gacctygtgg 300
   cccttgggct tgccttggag ccgggccarg gggggcarct ggagctcttc tccgggcgct 360
   ttcgcatccg gctcaacctg gcccctggcg anggctaccc cgagcttttg gtgccccagg 420
   gggaggacaa gggggccttt cccctkcgga cgc                              453
<210> 231
   <211> 137
   <212> PRT
   <213> Thermus thermophilus
<220>
   <221> SITE
   <222> (11)
   <223> The residue at this position can be any amino acid.
<220>
   <221> SITE
   <222> (55)
   <223> The residue at this position can be any amino acid.
<220>
   <221> SITE
   <222> (82)
   <223> The residue at this position can be any amino acid.
<220>
   <221> SITE
   <222> (96)
   <223> The residue at this position can be any amino acid.
<220>
   <221> SITE
   <222> (99)
   <223> The residue at this position can be any amino acid.
<220>
   <221> SITE
   <222> (117)
   <223> The residue at this position can be any amino acid.
<400> 231
<210> 232
   <211> 135
   <212> PRT
   <213> Thermus thermophilus
<220>
   <221> SITE
   <222> (54)
   <223> The residue at this position can be any amino acid.
<220>
   <221> SITE
   <222> (81)
   <223> The residue at this position can be any amino acid.
<220>
   <221> SITE
   <222> (95)
   <223> The residue at this position can be any amino acid.
<220>
   <221> SITE
   <222> (98)
   <223> The residue at this position can be any amino acid.
<220>
   <221> SITE
   <222> (116)
   <223> The residue at this position can be any amino acid.
<400> 232
<210> 233
   <211> 366
   <212> PRT
   <213> Escherichia coli
<400> 233
   Met Lys Phe Thr Val Glu Arg Glu His Leu Leu Lys Pro Leu Gln Gln
   1               5                   10                  15
   Val Ser Gly Pro Leu Gly Gly Arg Pro Thr Leu Pro Ile Leu Gly Asn
               20                  25                  30
   Leu Leu Leu Gln Val Ala Asp Gly Thr Leu Ser Leu Thr Gly Thr Asp
           35                  40                  45
   Leu Glu Met Glu Met Val Ala Arg Val Ala Leu Val Gln Pro His Glu
       50                  55                  60
   Pro Gly Ala Thr Thr Val Pro Ala Arg Lys Phe Phe Asp Ile Cys Arg
   65                  70                  75                  80
   Gly Leu Pro Glu Gly Ala Glu Ile Ala Val Gln Leu Glu Gly Glu Arg
                   85                  90                  95
   Met Leu Val Pro Ser Gly Arg Ser Arg Phe Ser Leu Ser Thr Leu Pro
               100                 105                 110
   Ala Ala Asp Phe Pro Asn Leu Asp Asp Trp Gln Ser Glu Val Glu Phe
           115                 120                 125
   Thr Leu Pro Gln Ala Thr Met Lys Arg Leu Ile Glu Ala Thr Gln Phe
       130                 135                 140
   Ser Met Ala His Gln Asp Val Arg Tyr Tyr Leu Asn Gly Met Leu Phe
   145                 150                 155                 160
   Glu Thr Glu Gly Glu Glu Leu Arg Thr Val Ala Thr Asp Gly His Arg
                   165                 170                 175
   Leu Ala Val Cys Ser Met Pro Ile Gly Gln Ser Leu Pro Ser His Ser
               180                 185                 190
   Val Ile Val Pro Arg Lys Gly Val Ile Glu Leu Met Arg Met Leu Asp
           195                 200                 205
   Gly Gly Asp Asn Pro Leu Arg Val Gln Ile Gly Ser Asn Asn Ile Arg
       210                 215                 220
   Ala His Val Gly Asp Phe Ile Phe Thr Ser Lys Leu Val Asp Gly Arg
   225                 230                 235                 240
   Phe Pro Asp Tyr Arg Arg Val Leu Pro Lys Asn Pro Asp Lys His Leu
                   245                 250                 255
   Glu Ala Gly Cys Asp Leu Leu Lys Gln Ala Phe Ala Arg Ala Ala Ile
               260                 265                 270
   Leu Ser Asn Glu Lys Phe Arg Gly Val Arg Leu Tyr Val Ser Glu Asn
   275 280 285
   Gln Leu Lys Ile Thr Ala Asn Asn Pro Glu Gln Glu Glu Ala Glu Glu
   290 295 300
   Ile Ile Asp Val Thr Tyr Ser Gly Ala Glu Met Glu Ile Gly Phe Asn
   305 310 315 320
   Val Ser Tyr Val Leu Asp Val Leu Asn Ala Leu Lys Cys Glu Asn Val
   325 330 335
   Arg Met Met Leu Thr Asp Ser Val Ser Ser Val Gln Ile Glu Asp Ala
   340 345 350
   Ala Ser Gln Ser Ala Ala Tyr Val Val Met Pro Met Arg Leu
   355 360 365
<210> 234
   <211> 377
   <212> PRT
   <213> Streptococcus pneumoniae
<400> 234
   Met Ile His Phe Ser Ile Asn Lys Asn Leu Phe Leu Gln Ala Leu Asn
    1               5                  10                  15
   Ile Thr Lys Arg Ala Ile Ser Ser Lys Asn Ala Ile Pro Ile Leu Ser
               20                  25                  30
   Thr Val Lys Ile Asp Val Thr Asn Glu Gly Val Thr Leu Ile Gly Ser
           35                  40                  45
   Asn Gly Gln Ile Ser Ile Glu Asn Phe Ile Ser Gln Lys Asn Glu Asp
       50                  55                  60
   Ala Gly Leu Leu Ile Thr Ser Leu Gly Ser Ile Leu Leu Glu Ala Ser
   65                  70                  75                      80
   Phe Phe Ile Asn Val Val Ser Ser Leu Pro Asp Val Thr Leu Asp Phe
                   85                  90                  95
   Lys Glu Ile Glu Gln Asn Gln Ile Val Leu Thr Ser Gly Lys Ser Glu
               100                 105                 110
   Ile Thr Leu Lys Gly Lys Asp Ser Glu Gln Tyr Pro Arg Ile Gln Glu
           115                 120                 125
   Ile Ser Ala Ser Thr Pro Leu Ile Leu Glu Thr Lys Leu Leu Lys Lys
       130                 135                 140
   Ile Ile Asn Glu Thr Ala Phe Ala Ala Ser Thr Gln Glu Ser Arg Pro
   145                 150                 155                 160
   Ile Leu Thr Gly Val His Phe Val Leu Ser Gln His Lys Glu Leu Lys
                   165                 170                 175
   Thr Val Ala Thr Asp Ser His Arg Leu Ser Gln Lys Lys Leu Thr Leu
               180                 185                 190
   Glu Lys Asn Ser Asp Asp Phe Asp Val Val Ile Ser Arg Ser Leu Arg
           195                 200                 205
   Glu Phe Ser Ala Val Phe Thr Asp Asp Ile Glu Thr Val Glu Ile Phe
       210                 215                 220
   Phe Ala Asn Asn Gln Ile Leu Phe Arg Ser Glu Asn Ile Ser Phe Tyr
   225                 230                 235                 240
   Thr Arg Leu Leu Glu Gly Asn Tyr Pro Asp Thr Asp Arg Leu Ile Pro
                   245                 250                 255
   Thr Asp Glu Asn Thr Thr Ile Thr Phe Asn Val Val Asn Leu Arg Gln
               260                 265                 270
   Ser Met Glu Arg Ala Arg Leu Leu Ser Ser Ala Thr Gln Asn Gly Thr
           275                 280                 285
   Val Lys Leu Glu Ile Lys Asp Gly Val Val Ser Ala His Val His Ser
       290                 295                 300
   Pro Glu Val Gly Lys Val Asn Glu Glu Ile Thr Thr Asp Gln Val Thr
   305                 310                 315                 320
   Gly Glu Asp Leu Thr Ile Ser Phe Asn Pro Thr Tyr Leu Ile Asp Ser
                   325                 330                 335
   Leu Lys Ala Leu Asn Ser Glu Lys Val Thr Ile Ser Phe Ile Ser Ala
               340                 345                 350
   Val Arg Pro Phe Thr Leu Val Pro Ala Asp Thr Asp Glu Asp Phe Met
           355                 360                 365
   Gln Leu Ile Thr Pro Val Arg Thr Asn
       370                 375
<210> 235
   <211> 65
   <212> DNA
   <213> Artificial Sequence
<220>
   <223> Description of Artificial Sequence: Synthetic
<400> 235
   cgatgtctgc tctgtaccgt cgttttcgtc cgctgacttt ccaggaagta gtaggtcagg 60
   aacac                                                             65
<210> 236
   <211> 63
   <212> DNA
   <213> Artificial Sequence
<220>
   <223> Description of Artificial Sequence: Synthetic
<400> 236
   tacagacgag acatggcagc aaaagcaggc gactgaaagg tccttcatca tccagtcctt 60
   gtg                                                               63